(19) Europäisches Patentamt
European Patent Office
Office européen des brevets

(11) EP 4 074 710 A1

(12) EUROPEAN PATENT APPLICATION
published in accordance with Art. 153(4) EPC

(43) Date of publication:
**19.10.2022 Bulletin 2022/42**

(21) Application number: **20898112.6**

(22) Date of filing: **11.12.2020**

(51) International Patent Classification (IPC):
***C07D 471/04*** (2006.01) ***A61K 31/437*** (2006.01)
***A61P 35/00*** (2006.01)

(52) Cooperative Patent Classification (CPC):
**A61K 31/437; A61K 31/496; A61K 31/4995; A61K 31/5386; A61K 31/55; A61P 35/00; C07D 471/04; C07D 519/00**

(86) International application number:
**PCT/CN2020/135934**

(87) International publication number:
**WO 2021/115457 (17.06.2021 Gazette 2021/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2019 CN 201911266900**

(71) Applicant: **Shenzhen Zhongge Biological Technology Co., Ltd**
**Futian District**
**Shenzhen, Guangdong 518017 (CN)**

(72) Inventors:
- **CHENG, Huimin**
  **Shenzhen, Guangdong 518017 (CN)**
- **FANG, Lei**
  **Shenzhen, Guangdong 518017 (CN)**
- **WEN, Xiaoming**
  **Shenzhen, Guangdong 518017 (CN)**
- **LIU, Zhiqiang**
  **Shenzhen, Guangdong 518017 (CN)**
- **CHEN, Yu**
  **Shenzhen, Guangdong 518017 (CN)**
- **MA, Songling**
  **Shenzhen, Guangdong 518017 (CN)**
- **CHEN, Ping**
  **Shenzhen, Guangdong 518017 (CN)**
- **QI, Zhenzhen**
  **Shenzhen, Guangdong 518017 (CN)**
- **NIU, Chunyi**
  **Shenzhen, Guangdong 518017 (CN)**
- **ZHANG, Peiyu**
  **Shenzhen, Guangdong 518017 (CN)**
- **LAI, Lipeng**
  **Shenzhen, Guangdong 518017 (CN)**
- **MA, Jian**
  **Shenzhen, Guangdong 518017 (CN)**
- **WEN, Shuhao**
  **Shenzhen, Guangdong 518017 (CN)**

(74) Representative: **Kluin, Jörg-Eden**
**KLUIN PATENT**
**Benrather Schloßallee 111**
**40597 Düsseldorf (DE)**

# (54) PYRAZOLO[1,5-A]PYRIDINE COMPOUND, PREPARATION METHOD THEREFOR AND USE THEREOF

(57) Disclosed are a pyrazolo[1,5-a]pyridine compound, a preparation method therefor and a use thereof. Also disclosed is a pharmaceutical composition containing the compound as an active ingredient or a pharmaceutically acceptable salt thereof. The present invention further relates to the use of a compound of formula (I) for treating and preventing diseases that can be treated with wild-type, gene fusion-type and mutant (including but not limited to G804 and G810) RET kinase inhibitors, including diseases or conditions mediated by an RET kinase.

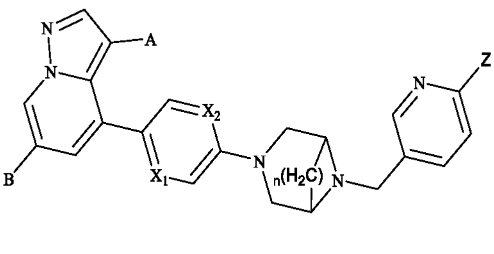

I

EP 4 074 710 A1

## Description

### TECHNICAL FIELD

This disclosure belongs to the field of medicine, and in particular to a pyrazolo[1,5-a]pyridine compound and a preparation method therefor and use thereof.

### BACKGROUND

RET (rearranged during transfection) kinase is a single-transmembrane receptor tyrosine kinase that plays an important role in the development of the kidney and the enteric nervous system and the homeostasis of nerve, endocrine, hematopoiesis, the male reproductive system, and more. The RET structurally includes an extracellular domain, a transmembrane domain and an intracellular kinase domain. Its ligand, GDNF (glial cell line-derived neurotrophic factor) family, does not directly bind to RET, but first forms a complex GFL-GFR$\alpha$ with GDNF family receptor $\alpha$, and then catalyzes the homodimerization of RET, enabling RET to autophosphorylate in the intracellular region, and then recruits adaptor proteins and pathway proteins to activate various signaling pathways including MAPK, PI3K, JAK-STAT, PKA and PKC to participate in cell proliferation, nerve conduction, cell migration and cell differentiation (Alexander Drilon, Nature Reviews Clinical Oncology, 2018, 15:151-167).

The gene encoding the RET protein is located on the long arm of human chromosome 10, and its abnormalities (gene fusions, mutations, etc.) can cause a variety of diseases, including papillary thyroid carcinoma (PTC), medullary thyroid carcinoma (MTC), Hirschsprung's disease, lung adenocarcinoma, irritable bowel syndrome, etc.

The chromosomal rearrangement of the RET gene may lead to the breakage of the RET gene. After the breakage, the 3' end of the RET gene can be fused with different genes such as KIF5B, TRIM33, CCDC6 or NCOA4 to form a fusion gene. The expressed fusion protein manifests as continuous activation and driving tumorigenesis. RET gene fusions have been reported to be present in about 10% to 20% of PTC patients, mainly including CCDC6-RET and NCOA4-RET fusions. About 1% to 2% of lung adenocarcinoma patients have RET fusion genes, mainly including four RET fusion genes: KIF5B-RET, CCDC6-RET, TRIM33-RET, and NCOA4-RET, among which KIF5B-RET is the most common (Rosell R, and Karachaliou N, Lancet Oncol., 2016, 17:1623-1625).

Activating mutations in the RET gene caused by point mutations can cause multiple endocrine neoplasia type 2 (MEN2), which manifests as hyperplasia or tumor of neuroendocrine cells in the thyroid, adrenal medulla, and parathyroid glands (Mulligan LM, Nat Rev Cancer., 2014, 14:173-86). About 60% of MTC patients have RET mutations.

Therefore, compounds that can inhibit gene fusions or mutated RET kinases are very useful for the prevention and treatment of RET-driven tumors.

Several multi-target kinase inhibitors, such as Cabozantinib, Vandetanib, Lenvatini and Ponatinib, have certain inhibitory activity against RET but they are all nonspecific RET inhibitors. In addition, since RET has a large number of homologies with the kinase domain of VEGFR2, these compounds have a certain inhibitory effect on multiple targets including VEGFR2 in addition to inhibiting RET, which leads to a high risk of off-target toxicity, making it difficult to achieve satisfactory efficacy.

Two RET-targeted drugs are currently on the market, namely LOXO-292 (selpercatinib/LY3527723) from Loxo Oncology Company and BLU-667 (pralsetinib/Gavreto) from Blurprint Company. These two targeted drugs show ideal efficacy and safety for RET fusion or mutation-positive patients, especially for RET fusion-positive NSCLC (non small cell lung cancer) and RET mutation-positive MTC (medullary thyroid cancer).

During the treatment of RET fusion-positive NSCLC and RET mutation-positive MTC with selpercatinib, some patients developed drug resistance, and analysis of circulating tumor DNA revealed emergence of RET G810R, G810S, and G810C mutations in the RET solvent front before the emergence of clinical resistance. In Phases 1 and II clinical trials of selpercratinib, acquired mutations in RET G810 were identified in tumor tissue from a patient with CCDC6-RET fusion-positive NSCLC and in plasma from a patient with RET fusion-positive NSCLC.. Preclinical studies reported the presence of RET G810R mutations in a CCDC6-RET patient-derived xenograft model of acquired resistance to selpercatinib. Structural modeling predicted that these mutations sterically hinder the binding of selpercatinib, and in vitro assays confirmed loss of activity to RET G810 mutations for both anti-RET multikinase inhibitors and selective RET inhibitors against G810 mutations (Solomon BJ, Tan L, Lin JJ et al., J Thorac Oncol. 2020 Apr; 15(4):541-549.).

Therefore, there is an urgent need in the field to develop drugs with high specificity and efficient inhibition against wild-type, fusion and mutant (including but not limited to G804 and G810) RET kinases.

### SUMMARY

An objective of this disclosure is to provide a novel compound with RET kinase inhibitory activity and/or good pharmacodynamic/pharmacokinetic properties and use thereof.

According to a first aspect of this disclosure, provided is a compound of formula I or a pharmaceutically acceptable salt, hydrate, solvate, isotopic compound or prodrug thereof;

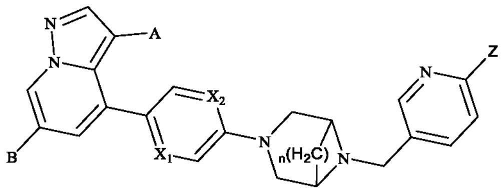

I

wherein

A is selected from: -CN, -$COOR_1$, -$CONR_2R_3$, -$NHCOR_4$ and -$NHCONR_2R_3$;

B is selected from: $R_7$, -O-$(L_1)_{m1}$-$R_8$, -$OCOR_9$, -$NR_{10}R_{11}$, -$COOR_{12}$, -$CONR_{10}R_{11}$,-$(L_2)_{m2}$-$R_{12}$, and -$(L_2)_{m2}$-$NR_{10}R_{11}$, wherein each $L_1$ is independently selected from: -$CR_fR_g$- and-CO-; each $L_2$ is independently selected from: -$CR_fR_g$- and -CO-;

$X_1$ and $X_2$ are each independently CR or N, wherein R is selected from substituted or unsubstituted groups, said group is selected from C1-C6 alkyl, H, halogen or cyano, wherein the term "substituted" means "substituted with one or more groups selected from C1-C6 alkyl, halogen, cyano, hydroxyl, and amino";

Z is selected from substituted or unsubstituted groups, said group is selected from C1-C6 alkyl, C1-C6 alkoxy, C1-C6 haloalkyl, C3-C8 cycloalkyl, C3-C8 cycloalkoxy, C1-C6 haloalkoxy, C1-C6 alkylamino, and C1-C6 alkylthio, wherein the term "substituted" means "substituted with one or more groups selected from: C1-C6 alkyl, halogen, cyano, hydroxyl, and amino";

$R_1$, $R_2$, $R_3$ and $R_4$ are each independently selected from substituted or unsubstituted groups, said group is selected from H, C1-C6 alkyl, C3-C8 cycloalkyl, 3- to 8-membered cycloheteroalkyl, C2-C6 alkenyl, C5-C10 aryl, and 5- to 10-membered heteroaryl; or $R_2$ and $R_3$ together with N atom attached thereto constitute a substituted or unsubstituted 3- to 12-membered heterocyclic group, wherein the term "substituted" means "substituted with one or more groups selected from C1-C6 alkyl, halogen, cyano, hydroxyl, and amino";

$R_7$ is a substituted or unsubstituted 5- to 10-membered heteroaryl, wherein the term "substituted" means "substituted with one or more groups selected from C1-C6 alkyl, halogen, cyano, hydroxyl, and amino";

$R_8$ is selected from substituted or unsubstituted groups, said group is selected from -C1-C6 alkyl-E, C5-C12 fused bicyclic ring, 5- to 12-membered fused heterobicyclic ring, C5-C12 spirobicyclic ring or 5- to 12-membered spiro heterobicyclic ring, wherein E is selected from:-CN, - $COOR_1$, -$OCOR_1$, -$CONR_2R_3$, -$NHCOR_4$, and -$NHCONR_2R_3$, wherein the term "substituted" means "substituted with 1, 2, 3, or 4 groups selected from: H, oxo (=O), halogen, cyano, hydroxy, C1-C6 alkyl, C3-C8 cycloalkyl, C1-C6 haloalkyl, C1-C6 alkoxy, C3-C8 cycloalkoxy, C1-C6 haloalkoxy, C1-C6 alkylamino, and C1-C6 alkylthio";

$R_9$ is selected from substituted or unsubstituted groups, said group is selected from C1-C6 alkoxy, C1-C6 haloalkoxy, -$NR_{10}R_{11}$, -$(L_3)_{m3}$-(3- to 8-membered cycloheteroalkyl), -$(L_3)_{m3}$-(C5-C10 aryl), and -$(L_3)_{m3}$-(5- to 10-membered heteroaryl), wherein each $L_3$ is independently selected from: -$CR_fR_g$- and -$NR_h$-, wherein the term "substituted" means "substituted with one or more groups selected from: C1-C6 alkyl, halogen, cyano, hydroxyl, and amino";

$R_{10}$ and $R_{11}$ are each independently selected from substituted or unsubstituted groups, said group is selected from H, C1-C6 alkyl, -$(L_2)_{m2}$-$NQ_1Q_2$, C3-C8 cycloalkyl, 3- to 8-membered cycloheteroalkyl, C6-C10 aryl, and 5- to 10-membered heteroaryl; alternatively, $R_{10}$ and $R_{11}$ together with N atom attached thereto constitute a 3- to 12-membered substituted or unsubstituted heterocyclyl containing 1 to 3 N atoms and 0, 1 or 2 O or S atoms, wherein $Q_1$ and $Q_2$ are each independently selected from: H and substituted or unsubstituted C1-C6 alkyl, or $Q_1$ and $Q_2$ together with N atom attached thereto constitute a 3- to 10-membered substituted or unsubstituted heterocyclyl containing 1 to 3 N atoms and 0, 1 or 2 O or S atoms, wherein the term "substituted" means "substituted with one or more groups selected from: C1-C6 alkyl, cyano, halogen, hydroxyl, C3-C8 cycloalkyl, 3- to 8-membered cycloheteroalkyl, C6-C10 aryl, and 5- to 10-membered heteroaryl";

each $R_{12}$ is independently selected from substituted or unsubstituted groups, said group is selected from C1-C6 alkyl, C3-C8 cycloalkyl, 3- to 8-membered cycloheteroalkyl, C6-C10 aryl, and 5- to 10-membered heteroaryl, the term "substituted" means "substituted with one or more groups selected from: C1-C6 alkyl, cyano, halogen, hydroxy, C3-C8 cycloalkyl, 3- to 8-membered cycloheteroalkyl, C6-C10 aryl, and 5- to 10-membered heteroaryl";

$R_f$ and $R_g$ are each independently selected from: H, halogen, C1-C4 alkyl, C1-C4 haloalkyl, OH, -$NH_2$, and C3-C6 cycloalkyl;

$R_h$ is independently selected from: H, C1-C4 alkyl, C1-C4 haloalkyl, and C3-C6 cycloalkyl;

n is 0, 1, or 2;
$m_1$ is 1, 2, 3, 4, 5 or 6;
$m_2$ is 1, 2, 3, 4, 5 or 6;
$m_3$ is 0, 1, or 2;
with the limitation that when B is $R_7$, A is selected from: $-COOR_1$, $-CONR_2R_3$, - $NHCOR_4$, and $-NHCONR_2R_3$.

In another preferred embodiment, $R_{12}$ is independently selected from substituted or unsubstituted groups, said group is selected from C1-C3 alkyl, C3-C6 cycloalkyl, 3- to 6-membered cycloheteroalkyl, phenyl, pyrazolyl, pyridinyl, furyl, thiophenyl, oxazolyl, isoxazolyl, and triazolyl, wherein the term "substituted" means "substituted with one or more groups selected from C1-C3 alkyl, cyano, halogen, and hydroxy".

In another preferred example, the "$-(L_2)_{m2}-$" is $-CH_2-$, wherein $L_2$ and $m_2$ are defined as above.

In another preferred embodiment, B is selected from: $-NR_{10}R_{11}$ and $-(L_2)_{m2}-NR_{10}R_{11}$, wherein $R_{10}$, $R_{11}$, $L_2$ and $m_2$ are defined as above.

In another preferred embodiment, B is selected from: -NH-(C1-C8 alkyl) and -NH-(C3-C8 cycloalkyl).

In another preferred embodiment, B is selected from: $-O-(L_1)_{m1}-R_8$ and $-OCOR_9$, wherein $L_1$, m1, $R_8$ and $R_9$ are as defined above.

In another preferred embodiment, m1 is 1, 2, 3 or 4.

In another preferred embodiment, B is selected from: $-O-(CH_2)_{m1}-R_8$, wherein m1 is 1, 2, or 3; $R_8$ is defined as above.

In another preferred embodiment, B is selected from: $-COOR_{12}$, $-CONR_{10}R_{11}$, and-$(L_2)_{m2}-R_{12}$, wherein $R_{10}$, $R_{11}$, $R_{12}$, $L_2$ and $m_2$ are defined as above.

In another preferred embodiment, B is selected from:-$O-(L_1)_{m1}-R_8$, $-OCOR_9$, and-$NR_{10}R_{11}$, wherein $L_1$, $m_1$, $R_8$, $R_9$, $R_{10}$, and $R_{11}$ are defined as above.

In another preferred embodiment, B is $-NR_{10}R_{11}$, wherein $R_{10}$ and $R_{11}$ are each independently selected from substituted or unsubstituted groups, said group is selected from H, C1-C3 alkyl, $-(CH_2)_2-NQ_1Q_2$, C3-C6 cycloalkyl, 3- to 6-membered cycloheteroalkyl, phenyl, pyrazolyl, pyridinyl, furyl, thiophenyl, oxazolyl, isoxazolyl, and triazolyl; alternatively, $R_{10}$ and $R_{11}$ together with N atom attached thereto constitute a 3- to 12-membered substituted or unsubstituted heterocyclyl containing 1 to 3 N atoms and 0, 1 or 2 O or S atoms; $Q_1$ and $Q_2$ are each independently selected from: H and C1-C3 alkyl, wherein the term "substituted" means "substituted with one or more groups selected from: C1-C3 alkyl, cyano, halogen, hydroxyl, cyclopropyl, cyclobutyl, cyclopentyl, cyclohexyl, phenyl, pyrazolyl, pyridinyl, furyl, thiophenyl, oxazolyl, isoxazolyl, and triazolyl".

In another preferred embodiment, when n is 0, the

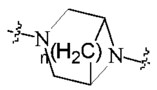

is

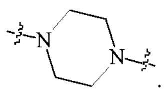

In another preferred embodiment, $R_7$ is

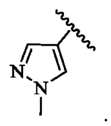

In another preferred embodiment, the compound of formula I or a pharmaceutically acceptable salt, hydrate, solvate, isotopic compound or prodrug thereof has a structure of formula II:

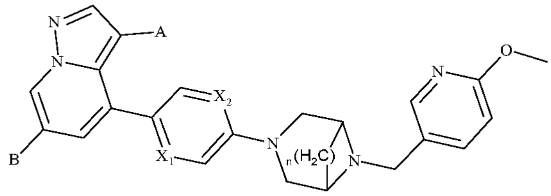

II ,

wherein A, B, $X_1$, $X_2$ and n are defined as above.

In another preferred embodiment, A is selected from:-CN, $-COOR_1$, $-CONR_2R_3$,$-NHCOR_4$ and $-NHCONR_2R_3$, wherein $R_1$, $R_2$ and $R_3$ are independently selected from: H, C1-C6 alkyl, C3-C8 cycloalkyl, 3- to 8-membered cycloheteroalkyl, C2-C6 alkenyl, C5-C10 aryl, and 5- to 10-membered heteroaryl; $R_4$ is selected from substituted or unsubstituted groups, said group is selected from C1-C3 alkyl, C3-C6 cycloalkyl, 3- to 6-membered cycloheteroalkyl, C2-C4 alkenyl, phenyl, pyrazolyl, pyridinyl, furyl, thiophenyl, oxazolyl, isoxazolyl, and triazolyl, wherein the term "substituted" means "substituted with C1-C3 alkyl".

In another preferred embodiment, B is selected from: $R_7$, $-O-(L_1)_{m1}-R_8$, $-NR_{10}R_{11}$,$-CONR_{10}R_{11}$, $-(L_2)_{m2}-R_{12}$, and $-(L_2)_{m2}-NR_{10}R_{11}$;

with the limitation that when B is $R_7$, A is selected from: $-COOR_1$, $-CONR_2R_3$,$-NHCOR_4$, and $-NHCONR_2R_3$, wherein $R_1$, $R_2$, $R_3$, $R_4$, $R_7$, $L_1$, $L_2$, $m_1$, $m_2$, $R_8$, $R_{10}$, $R_{11}$ and $R_{12}$ are defined as above.

In another preferred embodiment, $-(L_1)m_1-$ is selected from :$-(CH_2)_2-$, -CO-, and -CO-NH-.

In another preferred embodiment, $-(L_2)_{m2}-$ is selected from:$-(CH_2)_2-$, -CO-, and -O-CO-

In another preference, $R_8$ is selected from substituted or unsubstituted groups, said group is selected from -C1-C6 alkyl-E, 5- to 12-membered fused heterobicyclic ring, or 5- to 12-membered spiro heterobicyclic ring, wherein E is selected from: -CN, $-COOR_1$, $-OCOR_1$,$-CONR_2R_3$, $-NHCOR_4$, and $-NHCONR_2R_3$, the heterobicyclic ring contains 1 to 3 N atoms and 0, 1 or 2 O or S atoms as ring atoms, and a N atom in the heterobicyclic ring is attached to the $L_1$ moiety, wherein the term "substituted" means "substituted with 1, 2, 3, or 4 groups selected from H, oxo (=O), halogen, cyano, hydroxy, C1-C6 alkyl, C3-C8 cycloalkyl, C1-C6 haloalkyl, C1-C6 alkoxy, C3-C8 cycloalkoxy, C1-C6 haloalkoxy, C1-C6 alkylamino, and C1-C6 alkylthio"; $R_1$, $R_2$, $R_3$ and $R_4$ are defined as above.

In another preferred embodiment, $R_1$, $R_2$, $R_3$ and $R_4$ are each independently selected from: H, C1-C6 alkyl, C3-C8 cycloalkyl, 3- to 8-membered cycloheteroalkyl, C2-C6 alkenyl, C5-C10 aryl, and 5- to 10-membered heteroaryl.

In another preference, $R_8$ is selected from:

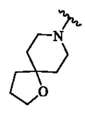 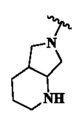 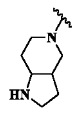 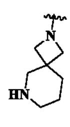 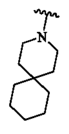 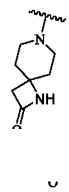 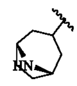

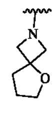 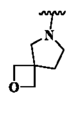 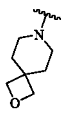 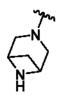 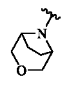 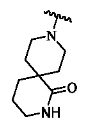 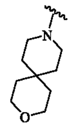

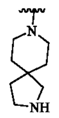 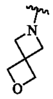 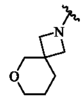 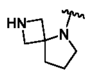 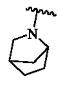 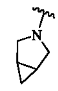 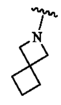

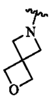 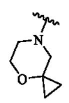 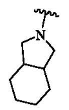 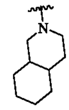 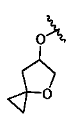 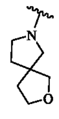 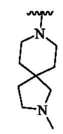 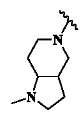 .

In another preferred embodiment, $R_9$ is selected from: C1-C3 alkoxy, C1-C3 haloalkoxy, C1-C3 alkylamino,

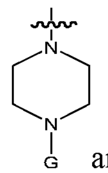 and 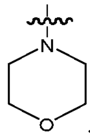,

wherein G is C1-C6 alkyl.

In another preferred embodiment, $R_{10}$ and $R_{11}$ are each independently selected from substituted or unsubstituted groups, said group is selected from H, C1-C3 alkyl, $-(CH_2)_2-NQ_1Q_2$, C3-C6 cycloalkyl, 3- to 6-membered cycloheteroalkyl, phenyl, pyrazolyl, pyridinyl, furyl, thiophenyl, oxazolyl, isoxazolyl, and triazolyl; alternatively, $R_{10}$ and $R_{11}$ together with N atom attached thereto constitute a 3- to 8-membered substituted or unsubstituted heterocyclyl containing 1 to 3 N atoms and 0, 1 or 2 O or S atoms, wherein $Q_1$ and $Q_2$ are each independently selected from: H and C1-C3 alkyl, or $Q_1$ and $Q_2$ together with N atom attached thereto constitute a 3- to 10-membered substituted or unsubstituted heterocyclyl containing 1 to 3 N atoms and 0, 1 or 2 O or S atoms, wherein the term "substituted" means "substituted with one or more groups selected from C1-C3 alkyl, cyano, halogen, and hydroxyl".

In another preferred embodiment, B is selected from: $R_7$, $-O-(CH_2)_m-R_8$, $-OCOR_9$, $-NR_{10}R_{11}$, $-COOR_{12}$, $-CONR_{10}R_{11}$, $-CH_2R_{12}$, and $-CH_2NR_{10}R_{11}$;

wherein $R_7$ is selected from substituted or unsubstituted C5-C10 heteroaryl, wherein the term "substituted" means "substituted with one or more groups selected from C1-C6 alkyl, halogen, cyano, hydroxyl, and amino";

$R_8$ is selected from:

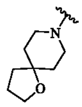 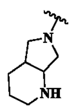 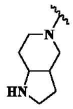 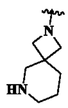 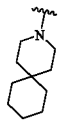 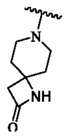 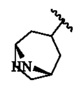

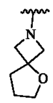 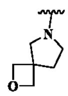 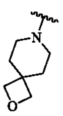 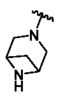 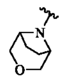 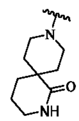 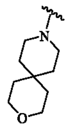

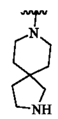 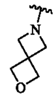 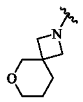 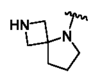 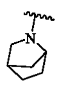 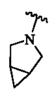 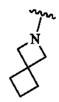

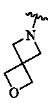 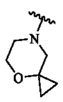 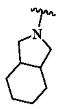 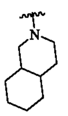 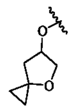 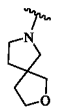 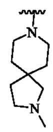 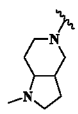;

$R_9$ is selected from: C1-C3 alkoxy, C1-C3 haloalkoxy, C1-C3 alkylamino,

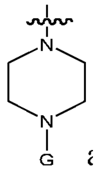

and

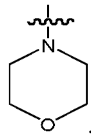,

wherein G is C1-C6 alkyl;

$R_{10}$ and $R_{11}$ are independently selected from substituted or unsubstituted groups, said group is selected from H, C1-C3 alkyl, $-(CH_2)_2-NQ_1Q_2$, C3-C6 cycloalkyl, 3- to 6-membered cycloheteroalkyl, phenyl, pyrazolyl, pyridinyl, furyl, thiophenyl, oxazolyl, isoxazolyl, and triazolyl, wherein $Q_1$ and $Q_2$ are each independently selected from: H and C1-C3 alkyl; the term "substituted" means "substituted with one or more groups selected from C1-C3 alkyl, cyano, halogen, and hydroxy";

$R_{12}$ is independently selected from substituted or unsubstituted groups, said group is selected from C1-C3 alkyl, C3-C6 cycloalkyl, 3- to 6-membered cycloheteroalkyl, phenyl, pyrazolyl, pyridinyl, furyl, thiophenyl, oxazolyl, isoxazolyl, and triazolyl, wherein the term "substituted" means "substituted with one or more groups selected from C1-C3 alkyl, cyano, halogen, and hydroxy";

m is 1, 2 or 3;

with the limitation that

when B is $R_7$, A is selected from: $-COOR_1$, $-CONR_2R_3$, $-NHCOR_4$, and $-NHCONR_2R_3$.

In another preferred embodiment, B is a corresponding group in a specific compound prepared in the embodiments.

In another preferred embodiment, A is a corresponding group in a specific compound prepared in the embodiments.

In another preferred embodiments, the compound or a pharmaceutically acceptable salt, hydrate, solvate, isotopic compound or prodrug thereof is selected from:

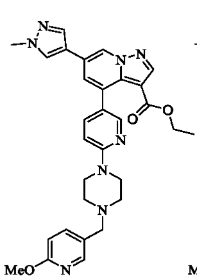
1
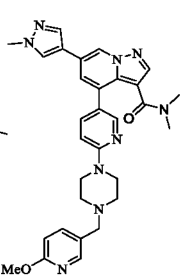
2
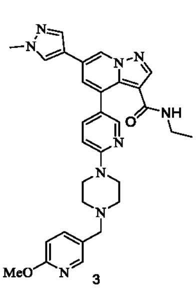
3
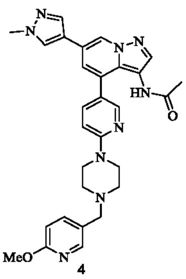
4
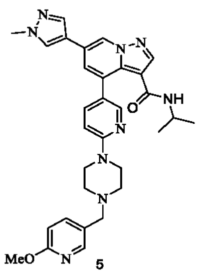
5
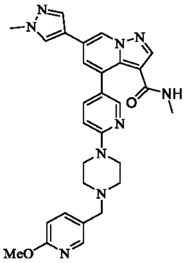
6

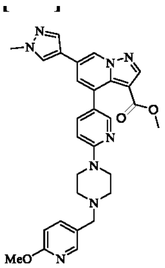
7
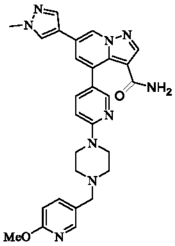
8
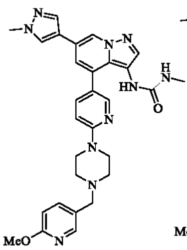
9
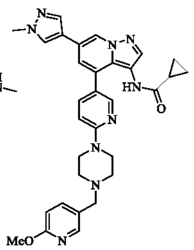
10
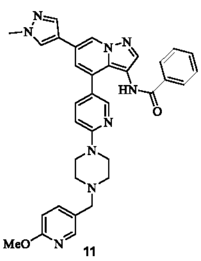
11
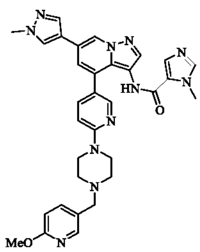
12

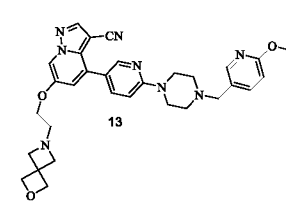
13
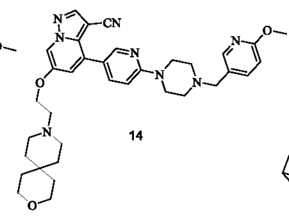
14
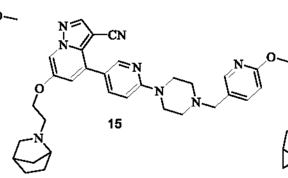
15
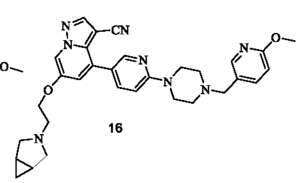
16

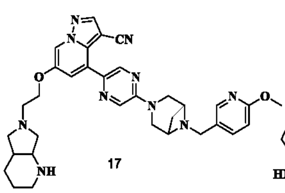
17
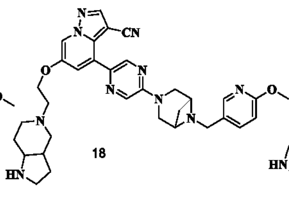
18
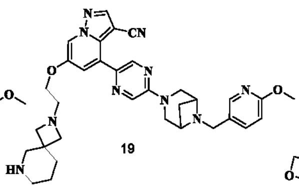
19
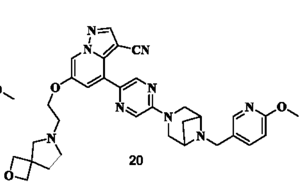
20

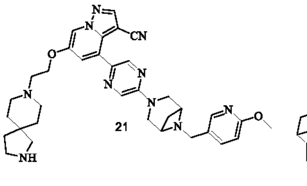
21
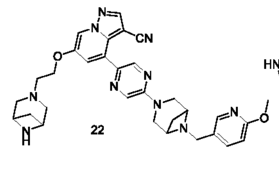
22
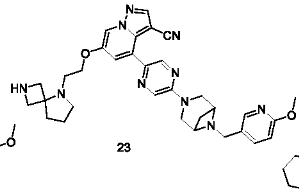
23
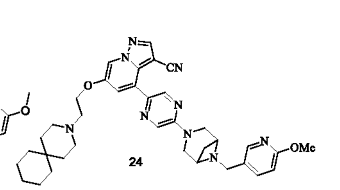
24

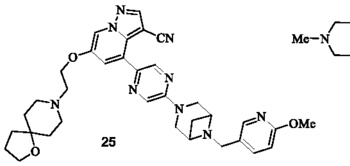
25
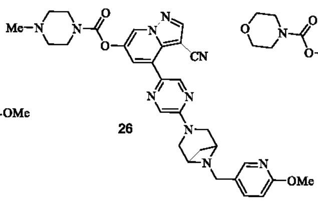
26
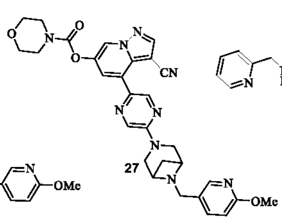
27
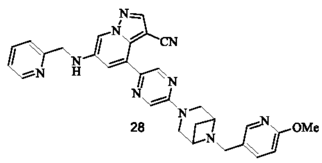
28

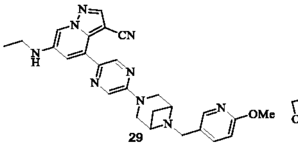
29
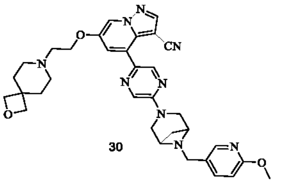
30
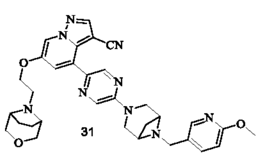
31
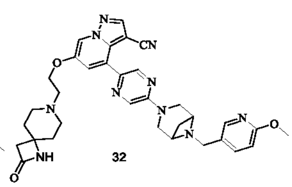
32

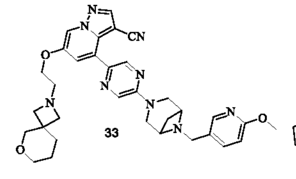
33
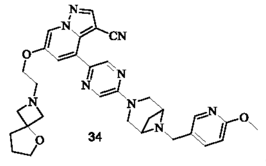
34
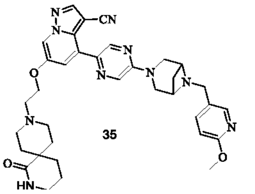
35
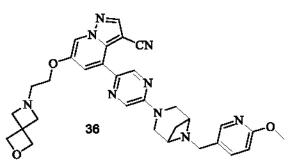
36

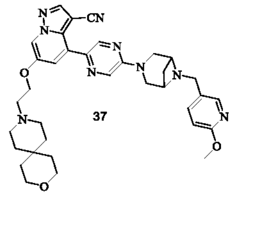
37
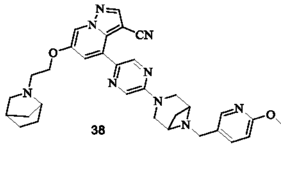
38
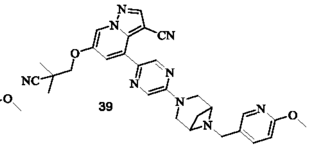
39

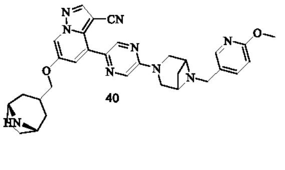
40
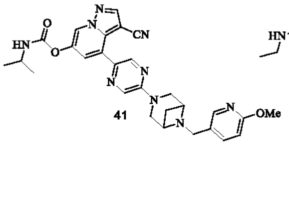
41
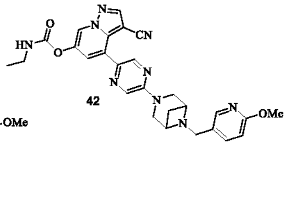
42

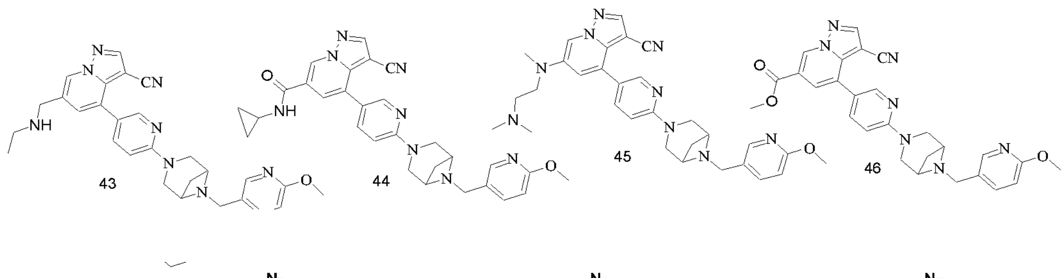

43 44 45 46

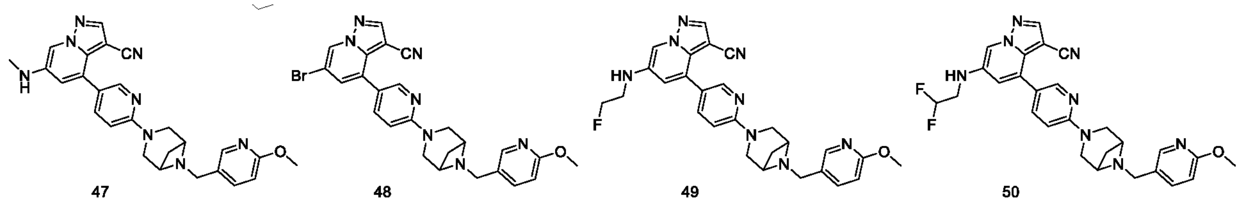

47 48 49 50

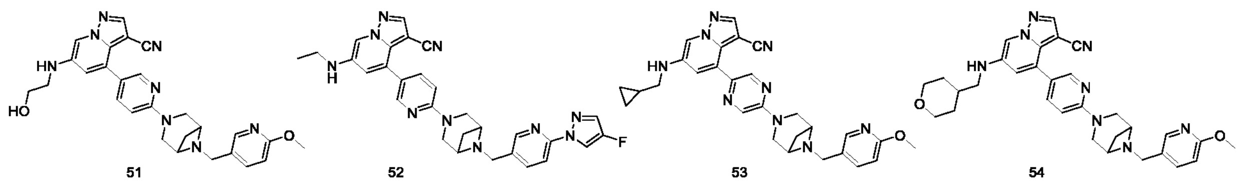

51 52 53 54

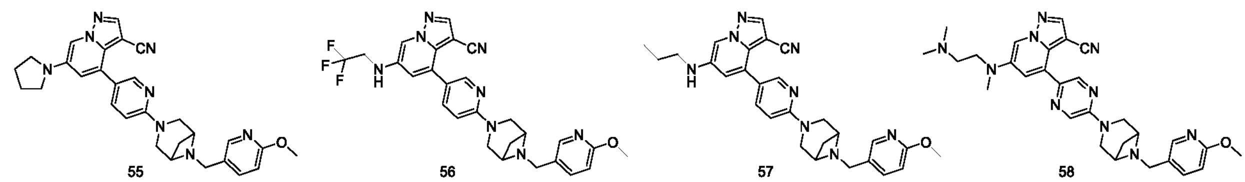

55 56 57 58

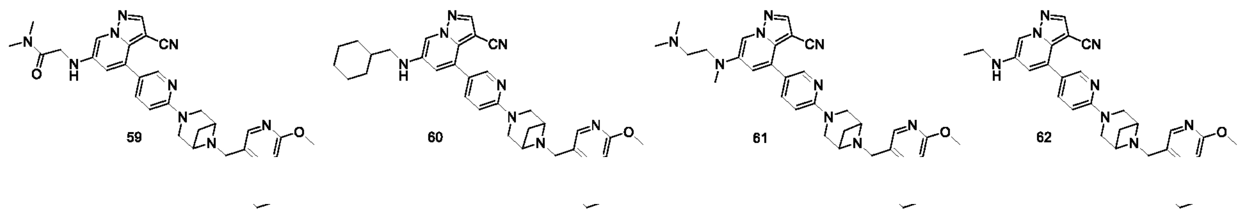

59 60 61 62

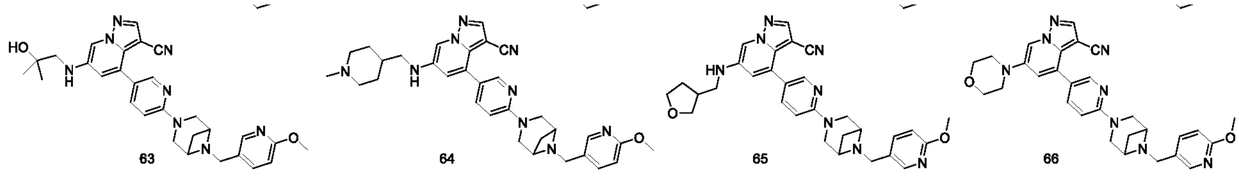

63 64 65 66

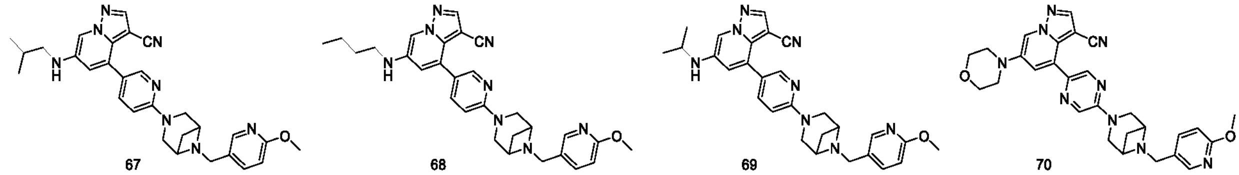

67 68 69 70

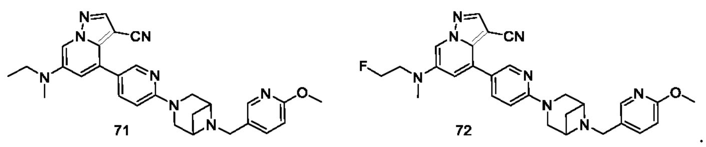

71 72

In another preferred embodiments, the compound or a pharmaceutically acceptable salt, hydrate, solvate, isotopic compound or prodrug thereof is selected from:

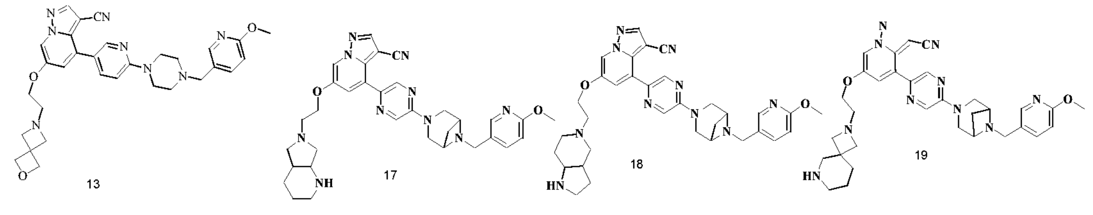

13 17 18 19

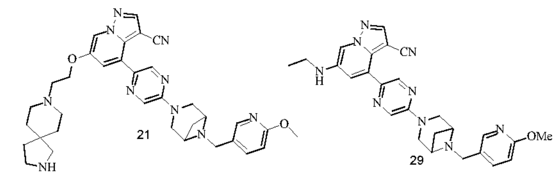

21 29

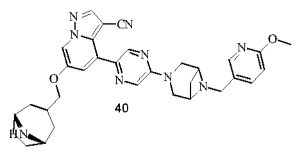

40

In another preferred embodiments, the compound or a pharmaceutically acceptable salt, hydrate, solvate, isotopic compound or prodrug thereof is selected from:

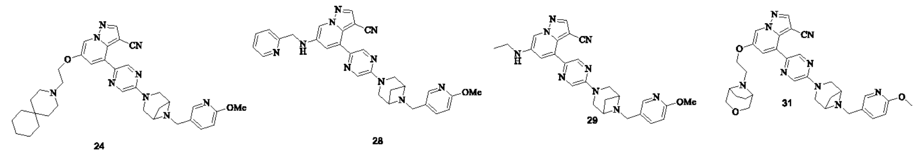

24 28 29 31

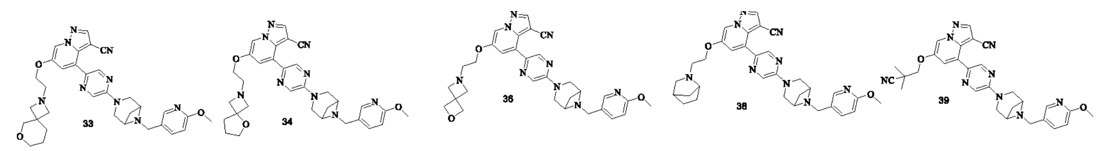

33 34 36 38 39

According to a second aspect of this disclosure, provided is a pharmaceutical composition including the compound described in the first aspect or a pharmaceutically acceptable salt, hydrate, solvate, isotopic compound or prodrug thereof; and a pharmaceutically acceptable carrier or diluent.

In another preferred embodiment, the pharmaceutical composition further includes a second therapeutic agent for a cancer.

In another preferred embodiment, the second therapeutic agent for a cancer includes a radioactive agent, a cytotoxic agent, a kinase inhibitor, an immune targeting inhibitor and an angiogenesis inhibitor.

In another preferred embodiment, the second therapeutic agent for a cancer includes one or two of:
a PD-1 inhibitor (such as Nivolumab, Pembrolizumab, JS-001, SHR-120, BGB-A317, IBI-308, GLS-010, GB-226, STW204, HX008, HLX10, BAT1306, AK105 , LZM 009 or biosimilars thereof, etc.), a PD-L1 inhibitor (such as Durvalumab, Atezolizumab, CS1001, KN035, HLX20, SHR-1316, BGB-A333, JS003, CS1003, KL-A167, F520, GR1405, MSB2311 or biosimilars thereof, etc.), a CD20 antibody (such as Rituximab, Obinutuzumab, Ofatumumab, Tositumumab, Tiimumab, etc.), a CD47 antibody (such as Hu5F9-G4, CC-90002, TTI-621, TTI-622, OSE-172, SRF-231, ALX-148, NI-1701, SHR-1603, IBI188, IMM01), an ALK inhibitor (such as Ceritinib, Alectinib, Brigatinib, Lorlatinib, and Oclatinib), a PI3K inhibitor (such as Ederatinib, Dactolisib, Taselisib, Buparlisib, etc.), a BTK Inhibitor (such as Ibrutinib, Tirabrutinib, Acalabrutinib, etc.), an EGFR inhibitor (such as Afatinib, Gefitinib, Erlotinib, Lapatinib, Dacomitinib, Icotinib, Canetinib, etc.), a VEGFR inhibitor (such as Sorafenib, Pazopanib, Rivatinib, Cabozantinib, Sunitinib, Donafenib, etc.), an HDAC inhibitor (such as Givinostat, Droxinostat, Entinostat, Darcylast, Tycodinaline, etc.), a CDK inhibitor (such as Palbociclib, Ribociclib, Abemaciclib, Lerociclib, etc.), a MEK inhibitor (such as Selumetinib (AZD6244), Trametinib (GSK1120212), PD0325901, U0126, AS-703026, PD184352 (CI-1040), etc.), an Akt inhibitor (such as MK-2206, Ipatasertib, Capivasertib, Afuresertib, Uprosertib, etc.), an mTOR inhibitor (such as Vistusertib, etc.), an SHP2 inhibitor (such as RMC-4630, JAB-3068, TNO155, etc.), an IGF-1R inhibitor (such as Ceritinib, Ocaltinib, linsitinib, BMS-754807, GSK1838705A, etc.), and a combination thereof.

According to a third aspect of this disclosure, provided is use of the compound described in the first aspect or the pharmaceutical composition described in the second aspect in preparation of a drug for inhibiting activity of a RET kinase in a cell or a subject.

In another preferred embodiment, the RET kinase is of a wild type, a gene fusion type or a mutant type.

In another preferred embodiment, the RET kinase is of a mutant type, preferably M918T, G804M, G804L, G810S and G810R.

In another preferred embodiment, the drug is used to treat a RET-related disease or a disorder in the expression or activity or level of RET genes, RET kinases, or any one of the RET genes and the RET kinases.

In another preferred embodiment, the diseases are selected from: eye diseases, rheumatoid arthritis, pulmonary fibrosis, liver fibrosis, and tumors; the tumors include: bladder cancer, ovarian cancer, adenocarcinoma, gastric cancer, pancreatic cancer, prostate cancer, colon cancer, lung cancer, bone cancer, brain cancer, neurocytoma, rectal cancer, colon cancer, familial adenomatous polyposis cancer, hereditary non-polyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, kidney cancer, renal parenchymal cancer, ovarian cancer, cervical cancer, endometrial adenocarcinoma, endometrial cancer, choriocarcinoma, pancreatic cancer, prostate cancer, testicular cancer, urinary cancer, melanoma, acute lymphocytic leukemia, chronic lymphocytic leukemia, acute myeloid leukemia, chronic myeloid leukemia, hepatocellular carcinoma, gallbladder carcinoma, bronchial carcinoma, small cell lung cancer, non-small cell lung cancer, and multiple myeloma.

According to another aspect, provided is a method of treating a RET-related disease, the method including administering to a subject identified or diagnosed as having a RET-related disease a therapeutically effective dose of a compound as described above, or a pharmaceutically acceptable salt or solvate of the compound, or the pharmaceutical composition as described above.

According to another aspect, provided is a method for inhibiting activity of a RET kinase in a cell or a subject, the method including a step of allowing the cell to contact the compound or pharmaceutical composition as described above or administering to the subject the compound or the pharmaceutical composition.

In another preferred embodiment, the cell is from a mammal.

In another preferred embodiment, the subject is a mammal, preferably a human.

It should be appreciated that, within the scope of this disclosure, the above-mentioned technical features of this disclosure and the technical features specifically described in the following (e.g., embodiments) can be combined with each other, thereby constituting new or preferred technical solutions. Due to space limitations, it is not repeated here.

## DETAILED DESCRIPTION

After extensive and in-depth research, the inventors have found a compound with good RET kinase activity (to wild-type, gene fusion and multiple mutant RET kinases) and good selectivity for VEGFR2 kinase through rational design. In addition, the compound has excellent inhibitory activity against RET kinase-sensitive cells and has good pharmacodynamic/pharmacokinetic properties. On this basis, this disclosure is implemented.

### Definition of terms

In this disclosure, unless otherwise specified, the terms used have the ordinary meanings known to those skilled in the art.

Where substituent groups are specified by their conventional chemical formulae, written from left to right, they equally encompass the chemically identical substituents that would result from writing the structure from right to left, e.g., $-CH_2O-$ is equivalent to $-OCH_2-$.

The term "alkyl", by itself or as part of another substituent, means, a straight or branched alkyl having a specified number of carbon atoms (i.e., C1-C6 refers to one to six carbon atoms). Examples of alkyl include, but are not limited to, groups such as methyl, ethyl, n-propyl, isopropyl, n-butyl, tert-butyl, isobutyl, sec-butyl, n-pentyl, n-hexyl and the like. One or more positions in the alkyl can be substituted, and especially 1 to 4 substituents can substitute the alkyl at any position.

The term "C1-C6 alkoxy" refers to a straight chain or branched chain or cycloalkoxy (such as C3-C6 cycloalkoxy) having 1 to 6 carbon atoms, and its typical examples include (but not are limited to): methoxy, ethoxy, propoxy, isopropoxy, butoxy, and the like. Preferably it is C1-C3 alkoxy.

The term "cycloalkyl" refers to a cyclic alkyl including saturated monocyclic, bicyclic or polycyclic alkyl, such as C3-C8 or C3-C12 cycloalkyl. C3-C8 cycloalkyl is meant to include C3, C4, C5, C6, C7, and C8 cycloalkyl. Cycloalkyl can also include cycloalkyls of spiro, bridged, and fused structures. The typical cycloalkyl groups of this disclosure include, but are not limited to: cyclopropyl, cyclobutyl, cyclopentyl, cyclohexyl and norbornyl. It should be appreciated that substituted or unsubstituted cycloalkyl groups, such as branched cycloalkyl groups (such as 1-methylcyclopropyl and 2-methylcyclopropyl), are included in the definition of "cycloalkyl". The C5-C12 fused bicyclic ring refers to include C5, C6, C7, C8, C9, C10, C11, and C12 bicycloalkyl groups, including but not limited to:

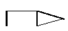, 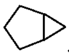, 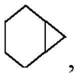, 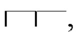, 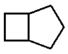, 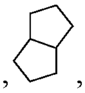, 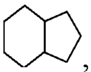, 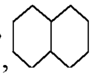,

and the like. C5-C12 spirobicyclic ring include C5, C6, C7, C8, C9, C10, C11, and C12 bicycloalkyl groups, including but not limited to:

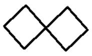, 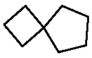, 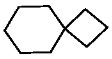, 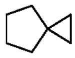, 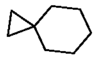, 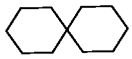,

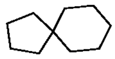, 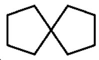

, and the like.

The term "cycloalkoxy" refers to a group in which H on a cycloalkyl is substituted with -O- and O is used as a linker, preferably C3-C8 cycloalkoxy, including but not limited to cyclopropoxy, cyclobutoxy, cyclopentyloxy, cyclohexyloxy, and the like.

The term "cycloheteroalkyl" refers to a cycloalkyl ring having the specified number of ring vertices (or members) and having one to five heteroatoms selected from N, O and S, respectively substituted for carbon atoms in the ring skeleton and wherein N and S atoms are optionally oxidized and the N atom is optionally quaternized. Cycloheteroalkyl is usually a 4- to 12-membered ring. Cycloheteroalkyl can be a monocyclic, bicyclic or polycyclic ring system. Examples of cycloheteroalkyl include, but are not limited to: pyrrolidinyl, imidazolidinyl, pyrazolidinyl, butyrolactam, valerolactam, imidazolidinone, hydantoin, dioxacyclopentyl, phthalimido, piperidinyl, 1,4-dioxanyl, morpholinyl, thiomorpholinyl, thiomorpholine-S-oxide, thiomorpholine-S,S-oxide, piperazinyl, pyranyl, pyridinyl, 3-pyrrolidinyl, thiopyranyl, pyranone, tetrahydrofuranyl, tetrahydrothienyl, quinuclidine and their analogues.

The term "haloalkyl" is meant to include branched and straight saturated aliphatic hydrocarbon groups having a specified number of carbon atoms and substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" having branched and straight saturated aliphatic hydrocarbon groups having a specified number of carbon atoms and substituted with one or more fluorine atoms.

The term "alkylamino" refers to $-NR_1'R_2'$, wherein $R_1'$ and $R_2'$ are each independently H or alkyl, preferably alkyl is C1-C6 alkyl, more preferably C1-C3 alkyl, and $R_1'$ and $R_2'$ are not H at the same time.

The term "alkylthio" refers to -SRi', wherein $R_1'$ is alkyl, preferably C1-C6 alkyl, more preferably C1-C3 alkyl.

The term "alkenyl" refers to a straight or branched hydrocarbon group containing one or more double bonds and usually 2 to 20 carbon atoms (or C2-C8) in length. For example, in this disclosure, "C2-C6 alkenyl" contains two to six carbon atoms. Alkenyl groups include, but are not limited to, for example, vinyl, propenyl, butenyl, 1-methyl-2-buten-1-yl, and the like.

The term "aryl", alone or as part of a larger moiety such as "aralkyl", "aralkoxy" or "aryloxyalkyl", refers to a monocyclic, bicyclic or tricyclic ring system having a total of 5 to 15 ring members (preferably a 6-10 membered aromatic ring), wherein at least one ring in the system is aromatic and wherein each ring in the system contains 3 to 7 ring members. "Aryl" may be substituted or unsubstituted. In some embodiments of this disclosure, "aryl" refers to an aromatic ring system including, but not limited to, phenyl, biphenyl, indanyl, 1-naphthyl, 2-naphthyl, and tetrahydronaphthyl. A fused aryl group can be attached to another group at a suitable position on the cycloalkyl or aromatic ring. The connecting lines drawn from the ring system indicate that the bond may be attached to any suitable ring atom.

The term "heteroaryl" refers to a heteroaromatic system containing 1 to 4 heteroatoms, 5 to 14 ring atoms, wherein the heteroatom is selected from O, N and S. Heteroaryl is preferably a 5- to 10-membered ring, more preferably 5- or 6-membered ring, such as pyrrolyl, pyrazolyl, imidazolyl, oxazolyl, isoxazolyl, thiazolyl, thiadiazolyl, isothiazolyl, furyl, pyridyl, pyrazinyl, pyrimidinyl, pyridazinyl, triazinyl, triazolyl, tetrazolyl, and the like. "Heteroaryl" may be substituted or unsubstituted, and in a case of a substituted heteroaryl, the substituent is preferably one or more of groups independently selected from: alkyl, deuterated alkyl, haloalkyl, alkoxy, haloalkoxy, alkenyl, alkynyl, alkylthio, alkylamino, halogen, amino, nitro, hydroxy, mercapto, cyano, cycloalkyl, heterocyclyl, aryl, heteroaryl, cycloalkylthio, oxo, carboxyl, and carboxylate group.

The term "heterocycle", "heterocyclyl" or "heterocyclic group", which can be interchangeable, refers to a stable 3-, 4-, 5-, or 7-membered monocyclic or bicyclic or 7-, 8-, 9-, 10-membered, 11-membered, 12-membered, 13-membered

or 14-membered polycyclic heterocyclyl, including fused-ring, spiro and/or bridged structures, which are saturated, partially unsaturated or fully unsaturated, and which contain carbon atoms and 1, 2, 3 or 4 heteroatoms independently selected from N, O and S. The term also includes polycyclic groups formed by the fusion of a heterocyclic ring with an aromatic ring such as a benzene ring. The "heterocycle" may be substituted or unsubstituted. N and S heteroatoms as ring atoms can be optionally oxidized. The N atom is substituted or unsubstituted (i.e., N or NR, wherein R is H or, if defined, another substituent). The heterocycle can be attached to its pendant group at any heteroatom or carbon atom that results in a stable structure. The heterocyclyl described herein may be substituted on a C or N atom if the resulting compound is stable. N in the heterocycle may be optionally quaternized. Preferably, when the total number of S and O atoms in the heterocycle exceeds 1, then these heteroatoms are not adjacent to each other. Preferably, the total number of S and O atoms in the heterocycle is not greater than 1. When the term "heterocycle" is used, it is intended to include heteroaryl. Examples of heterocycle include, but are not limited to, acridinyl, azetidinyl, azacinyl, benzimidazolyl, benzofuranyl, benzothiofuranyl, benzothienyl, benzoxazole base, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, dihydrofuro[2, 3-b]tetrahydrofuranyl, furanyl, furazanyl, imidazolidinyl, imidazolinyl, imidazolyl, 1H-indazolyl, imidazopyridyl, indolenyl, dihydroindolyl, indolizinyl, indolyl, 3H-indolyl, isatinoyl, isobenzofuranyl, isochromanyl, isoindazolyl, isodihydroindolyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridyl, oxazolidinyl, rylene diazaphenyl, hydroxyindolyl, pyrimidinyl, phenanthridine, phenanthrolinyl, phenazinyl, phenothiazinyl, phenoxthiyl, phenoxazinyl, phthalazinyl, piperazinyl, piperidinyl, piperidonyl, 4-piperidinyl, piperonyl, pteridyl, purinyl, pyranyl, pyrazinyl, pyrazolidinyl, pyrazolinyl, pyrazolopyridyl, pyrazolyl, pyridazinyl, pyridooxazolyl, pyridoimidazolyl, pyridothiazolyl, pyridyl, pyrimidinyl, pyrrolidinyl, pyrrolidinyl, 2-pyrrolidinyl, 2H-pyrrolidyl, pyrrolyl, quinazolinyl, quinolinyl, 4H-quinazinyl, quinoxalinyl, quinuclidinyl, tetrazolyl, tetrahydrofuranyl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthyl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl, and xanthyl This disclosure further includes fused and spiro compounds containing, for example, the heterocycles described above.

The terms "spiro ring" and "fused ring" indicate that one ring originates from a special cyclic carbon on another ring, for example, a saturated bridged system (ring B and ring B' sharing two carbon atoms) is called a "fused ring", and in a case where ring B and ring B' share a carbon atom in two saturated ring systems, it is called a "spiro ring". "Spiro"and "fused-ring" systems can be attached to the main structure at any ring heteroatom or ring carbon atom to form stable compounds. In some embodiments, the fused ring is a 5- to 12-membered fused ring.

As used herein, the term "fused bicyclic heterocyclic" or "bicyclic heterocyclic" group refers to a stable 5- to 12-membered heterocyclic system containing two fused rings and consisting of carbon atoms and 1, 2, 3 or 4 heteroatoms independently selected from N, O and S. Among the two fused rings, one is a C3-C8 membered alkane ring, which is fused to the second ring. The second ring is a saturated, partially unsaturated or unsaturated C3-C8 monocyclic and bicyclic heterocyclic group can be attached to its pendant group at any heteroatom or carbon atom that results in a stable structure. The bicyclic heterocyclic groups described herein may be substituted on C or N atoms if the resulting compound is stable. When the total number of S and O atoms in the heterocyclyl exceeds 1, then these heteroatoms are not adjacent to each other.

The term "hydroxy" refers to -OH.

In this disclosure, each of the above-mentioned alkyl, haloalkyl, alkoxy, cycloalkyl, aryl, heteroaryl, cycloheteroalkyl, alkenyl, heterocyclyl, heterocyclic groups, and more may be substituted or unsubstituted.

In this disclosure, the term "substituted" means one or more hydrogen atoms on a specific group are substituted with specific substituents. The specific substituents are the substituents correspondingly described above or the substituents that appear in the various examples. Unless otherwise specified, a substituted group may have a substituent, selected from a specific group of groups, at any substitutable position of the group, and the substituent may be the same or different at each position. It will be understood by those skilled in the art that combinations of substituents contemplated by this disclosure are those that are stable or chemically achievable. Typical substituents include, but are not limited to, one or more of the following groups: such as hydrogen, deuterium, halogen (e.g., mono- or poly-halogen substituents, the poly-halogen substituents are such as trifluoromethyl or alkyl containing $Cl_3$), nitrile, nitro, oxo(eg =O), trifluoromethyl, trifluoromethoxy, cycloalkyl, alkenyl, alkynyl, heterocyclyl, aromatic ring, $OR_a$, $SR_a$, $S(=O)R_e$, $S(=O)_2R_e$, $P(=O)_2R_e$, $S(=O)_2OR_e$, $P(=O)_2OR_e$, $NR_bR_c$, $NR_bS(=O)_2R_e$, $NR_bP(=O)_2R_e$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_e$, $NR_dC(=O)NR_bR_c$, $NR_dS(=O)_2NR_bR_c$, $NR_dP(=O)_2NR_bR_c$, $NR_bC(=O)R_a$, and $NR_bP(=O)_2R_e$, wherein $R_a$ may independently represent hydrogen, deuterium, alkyl, cycloalkyl, alkenyl, alkynyl, heterocyclyl or aromatic ring, $R_b$, $R_c$ and $R_d$ may independently represent hydrogen, deuterium, alkyl, cycloalkyl, heterocyclyl or aromatic ring, or $R_b$ and $R_c$ together with the N atom may form a heterocyclyl; $R_e$ may independently represent hydrogen, alkyl, cycloalkyl, alkenyl, alkynyl, heterocyclyl or aromatic ring. The above-mentioned

typical substituents such as alkyl, cycloalkyl, alkenyl, cycloalkenyl, alkynyl, heterocyclyl or aromatic ring may be optionally substituted. The substituents are for example (but not limited to): halogen, hydroxyl, cyano, carboxyl (-COOH), C1-C6 alkyl, C2-C6 alkenyl, C2-C6 alkynyl, C3-C8 cycloalkyl, 3- to 12-membered heterocyclyl, aryl, heteroaryl, C1-C8 aldehyde, C2-C10 acyl, C2-C10 ester, amido, C1-C6 alkoxy, C1-C10 sulfonyl, C1-C6 ureido, and the like.

Unless otherwise stated, it is assumed that any heteroatom that is not in a full valence state has enough hydrogen atoms to complement its valence.

When a substituent is a non-terminal substituent, it is a subunit of the corresponding group, for example, alkyl corresponds to alkylene, cycloalkyl corresponds to cycloalkylene, heterocyclyl corresponds to heterocyclylene, alkoxy corresponds to alkyleneoxy, and the like.

The terms "halo" or "halogen" includes fluorine, chlorine, bromine, or iodine.

The term "cyano" refers to -CN.

**Active ingredient**

As used herein, the terms "compound of this disclosure" and "active ingredient of this disclosure" are used interchangeably to refer to a compound of formula I, or a pharmaceutically acceptable salt, hydrate, solvate, or isotopic compound (e.g., deuterated compound) or prodrug thereof. The terms also include racemates and optical isomers.

The compound of this disclosure has a structure of formula (I),

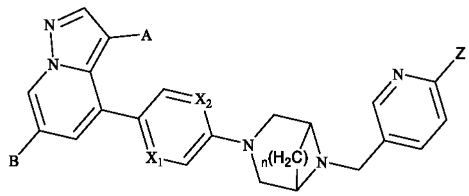

I

wherein A is selected from: -CN, $-COOR_1$, $-CONR_2R_3$, $-NHCOR_4$, and $-NHCONR_2R_3$;

B is selected from: $R_7$, $-O-(L_1)_{m1}-R_8$, $-OCOR_9$, $-NR_{10}R_{11}$, $-COOR_{12}$, $-CO-NR_{10}R_{11}$,$-(L_2)_{m2}-R_{12}$, $-(L_2)_{m2}-NR_{10}R_{11}$; wherein each $L_1$ is independently selected from: $-CR_fR_g-$, -CO-, and -CO-NH-; each $L_2$ is independently selected from: $-CR_fR_g-$, -CO-, and -O-CO-;

$X_1$ and $X_2$ are each independently CR or N, wherein R is selected from substituted or unsubstituted groups, said group is selected from C1-C6 alkyl, H, halogen or cyano, wherein the term "substituted" means "substituted with one or more groups selected from C1-C6 alkyl, halogen, cyano, hydroxyl, and amino";

Z is selected from substituted or unsubstituted groups, said group is selected from C1-C6 alkyl, C1-C6 alkoxy, C1-C6 haloalkyl, C3-C8 cycloalkyl, C3-C8 cycloalkoxy, C1-C6 haloalkoxy, C1-C6 alkylamino, and C1-C6 alkylthio, wherein the term "substituted" means "substituted with one or more groups selected from: C1-C6 alkyl, halogen, cyano, hydroxyl, and amino";

$R_1$, $R_2$, $R_3$ and $R_4$ are each independently selected from substituted or unsubstituted groups, said group is selected from H, C1-C6 alkyl, C3-C8 cycloalkyl, 3- to 8-membered cycloheteroalkyl, C2-C6 alkenyl, C5-C10 aryl, and 5- to 10-membered heteroaryl; or $R_2$ and $R_3$ together with N atom attached thereto constitute a substituted or unsubstituted 3- to 12-membered heterocyclic group, wherein the term "substituted" means "substituted with one or more groups selected from C1-C6 alkyl, halogen, cyano, hydroxyl, and amino";

$R_7$ is a substituted or unsubstituted 5- to 10-membered heteroaryl, wherein the term "substituted" means "substituted with one or more groups selected from C1-C6 alkyl, halogen, cyano, hydroxyl, and amino";

$R_8$ is selected from substituted or unsubstituted groups, said group is selected from -C1-C6 alkyl-E, C5-C12 fused bicyclic ring, 5- to 12-membered fused heterobicyclic ring, C5-C12 spirobicyclic ring or 5- to 12-membered spiro heterobicyclic ring, wherein E is selected from:-CN, - $COOR_1$, $-OCOR_1$, $-CONR_2R_3$, $-NHCOR_4$, and $-NHCONR_2R_3$, wherein the term "substituted" means "substituted with 1, 2, 3, or 4 groups selected from: H, oxo (=O), halogen, cyano, hydroxy, C1-C6 alkyl, C3-C8 cycloalkyl, C1-C6 haloalkyl, C1-C6 alkoxy, C3-C8 cycloalkoxy, C1-C6 haloalkoxy, C1-C6 alkylamino, and C1-C6 alkylthio";

$R_9$ is selected from substituted or unsubstituted groups, said group is selected from C1-C6 alkoxy, C1-C6 haloalkoxy, $-NR_{10}R_{11}$, $-(L_3)_{m3}$-(3- to 8-membered cycloheteroalkyl), $-(L_3)_{m3}$-(C5-C10 aryl), and $-(L_3)_{m3}$-(5- to 10-membered heteroaryl), wherein each $L_3$ is independently selected from: $-CR_fR_g-$ and $-NR_h-$, wherein the term "substituted" means "substituted with one or more groups selected from: C1-C6 alkyl, halogen, cyano, hydroxyl, and amino";

$R_{10}$ and $R_{11}$ are each independently selected from substituted or unsubstituted groups, said group is selected from

H, C1-C6 alkyl, -($L_2$) $_{m2}$-$NQ_1Q_2$, C3-C8 cycloalkyl, 3- to 8-membered cycloheteroalkyl, C6-10 aryl, and 5- to 10-membered heteroaryl, wherein $Q_1$ and $Q_2$ are each independently selected from: H and substituted or unsubstituted C1-C6 alkyl, or $Q_1$ and $Q_2$ together with N atom attached thereto constitute a 3- to 10-membered substituted or unsubstituted heterocyclyl containing 1 to 3 N atoms and 0, 1 or 2 O or S atoms, wherein the term "substituted" means "substituted with one or more groups selected from: C1-C6 alkyl, cyano, halogen, hydroxyl, C3-C8 cycloalkyl, 3- to 8-membered cycloheteroalkyl, C6-C10 aryl, and 5- to 10-membered heteroaryl";

each $R_{12}$ is independently selected from substituted or unsubstituted groups, said group is selected from C1-C6 alkyl, C3-C8 cycloalkyl, 3- to 8-membered cycloheteroalkyl, C6-C10 aryl, and 5- to 10-membered heteroaryl, the term "substituted" means "substituted with one or more groups selected from: C1-C6 alkyl, cyano, halogen, hydroxy, C3-C8 cycloalkyl, 3- to 8-membered cycloheteroalkyl, C6-C10 aryl, and 5- to 10-membered heteroaryl";

$R_f$ and $R_g$ are each independently selected from: H, halogen, C1-C4 alkyl, C1-C4 haloalkyl, OH, $NH_2$, and C3-C6 cycloalkyl;

$R_h$ is independently selected from: H, C1-C4 alkyl, C1-C4 haloalkyl, and C3-C6 cycloalkyl;

n is 0, 1, or 2;

$m_1$ is 1, 2, 3, 4, 5 or 6;

$m_2$ is 1, 2, 3, 4, 5 or 6;

$m_3$ is 0, 1, or 2;

with the limitation that when B is $R_7$, A is selected from: $-COOR_1$, $-CONR_2R_3$,$-NHCOR_4$, and $-NHCONR_2R_3$.

A, B, Z, $X_1$, $X_2$ and n are defined as above.

Preferably, in formula I, B is $-NR_{10}R_{11}$; wherein, $R_{10}$ and $R_{11}$ are each independently selected from substituted or unsubstituted groups, said group is selected from H and C1-C3 alkyl;
wherein the term "substituted" means "substituted with one or more (e.g., 2, 3 or 4) groups selected from: C1-C3 alkyl and halogen (preferably F)".

Preferably, the compound has a structure of formula II:

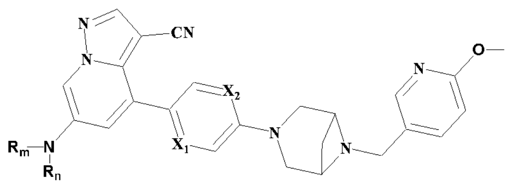

II

wherein

$R_m$ and $R_n$ are each independently selected from substituted or unsubstituted groups, said group is selected from H and C1-C3 alkyl, wherein the term "substituted" means "substituted with 1 to 2 halogen atoms; preferably, $R_m$ and $R_n$ are each independently selected from: H, methyl, ethyl, n-propyl, $-CH_2F$, $-CHF_2$, $-CH_2CH_2F$, $-CH_2CHF_2$, $-CHFCH_3$, $-CHFCH_2F$, $-CH_2CH_2CH_2F$, and $-CH_2CHFCH_2F$";

$X_1$ and $X_2$ are each independently CH or N.

Preferably, in formulae I to II, $X_2$ is N.

Preferably, in formulae I to II, $X_1$ is N.

Preferably, in formulae I to II, $X_1$ and $X_2$ are both N.

Preferably, in formulae I to II, $X_1$ is CH, and $X_2$ is N.

Preferably, $R_n$ is selected from: H and methyl; $R_m$ is selected from: H, methyl, ethyl, n-propyl, $-CH_2F$, $CHF_2$, $-CH_2CH_2F$, $-CH_2CHF_2$, $-CHFCH_3$, $-CHFCH_2F$, $-CH_2CH_2CH_2F$, and-$CH_2CHFCH_2F$.

Preferably, the compound has a structure of formula III

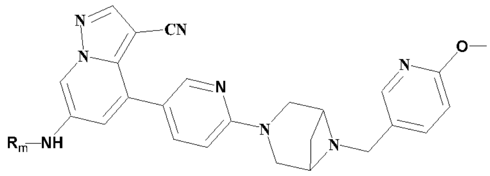

IIi

$R_m$ is selected from: H, methyl, ethyl, n-propyl, $-CH_2F$, $-CHF_2$, $-CH_2CH_2F$, $-CH_2CHF_2$, $-CHFCH_3$, $-CHFCH_2F$, $-CH_2CH_2CH_2F$, and $-CH_2CHFCH_2F$.

Preferably, the compound has a structure of formula IV.

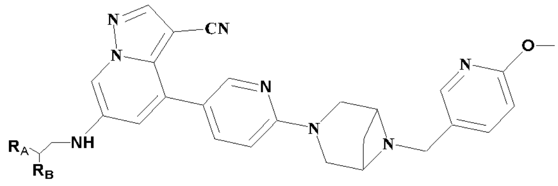

IV

wherein

$R_A$ and $R_B$ are each independently selected from: H, F, and methyl.

The salts that the compounds of this disclosure may form are also within the scope of this disclosure. Unless otherwise specified, the compounds of this disclosure are understood to include their salts. The term "salt", as used herein, refers to salts in an acid or basic form formed using inorganic or organic acids and bases. In addition, when the compound of this disclosure contains a basic moiety, the compound includes, but is not limited to, pyridine or imidazole, and when it contains an acidic moiety, the compound includes, but is not limited to, a carboxylic acid, and the zwitterion ("inner salt") that may be formed is within the scope of the term "salt". Pharmaceutically acceptable (i.e., non-toxic, physiologically acceptable) salts are preferred, although other salts are also used, for example, in a separation or purification step in the preparation process. The compounds of this disclosure may form salts. For example, Compound I reacts with a certain amount, such as an equivalent amount, of an acid or base to produce a salt in a medium, or the compound is lyophilized in an aqueous solution to produce a salt.

The compounds of this disclosure contain basic moieties, including but not limited to amines or pyridine or imidazole rings, which may form salts with organic or inorganic acids. Typical acid-formed salts include acetates (e.g., formed with acetic acid or trihaloacetic acid, such as trifluoroacetic acid), adipates, alginates, ascorbates, aspartates, benzoates, besylates, bisulfates, borates, butyrates, citrates, camphorates, camphor sulfonates, cyclopentane propionates, diglycolates, dodecyl sulfates, ethanesulfonates, fumarates, glucoheptonates, glycerophosphates, hemisulfates, heptanoates, hexanoates, hydrochlorides, hydrobromides, hydroiodides, isethionates (e.g., 2-hydroxyethanesulfonate), lactates, maleates, mesylates, naphthalenesulfonates (e.g., 2-naphthalenesulfonate), nicotinates, nitrates, oxalates, pectates, persulfates, phenylpropionates (e.g., 3-phenylpropionate), phosphates, picrates, pivalates, propionates, salicylates, succinates, sulfates (e.g., formed with sulfuric acid), sulfonates, tartrates, thiocyanates, tosylates such as p-toluenesulfonates, dodecanoates, and the like.

Certain compounds of this disclosure may contain acidic moieties, including but not limited to carboxylic acids, which may form salts with various organic or inorganic bases. Typical base-formed salts include ammonium salts, alkali metal salts (such as sodium, lithium and potassium salts), alkaline earth metal salts such as calcium and magnesium salts and salts formed with organic bases (e.g., organic amines) such as benzathine, dicyclohexylamine, hepamine (salt formed with N,N-bis(dehydroabietyl)ethylenediamine), N-methyl-D-glucosamine, N-methyl-D-glucosamide, tert-butyl amines, and salts with amino acids such as arginine, lysine, and the like. Basic nitrogen-containing groups can form halide quaternary ammonium salts, such as small molecule alkyl halides (e.g., methyl, ethyl, propyl and butyl chlorides, bromides and iodides), dialkyl sulfates (e.g., dimethyl sulfate, diethyl, dibutyl and dipentyl), long-chain halides (e.g., decyl, dodecyl, tetradecyl and tetradecyl chlorides, bromides and iodides), aralkyl halides (such as benzyl and phenyl bromides), and the like.

Prodrugs and solvates of the compounds of this disclosure are also within the scope of this disclosure. The term "prodrug" as used herein refers to a compound that undergoes chemical transformation through metabolic or chemical processes to produce the compound of this disclosure and its salts, or solvates in the treatment of related diseases. The compounds of this disclosure include solvates, such as hydrates.

The compounds, salts or solvates of this disclosure, may exist in tautomeric forms (e.g., amides and imine ethers). All of these tautomers are part of this disclosure.

Stereoisomers of all the compounds (e.g., those due to asymmetric carbon atoms that may exist for various substitutions), including their enantiomeric and diastereomeric forms, are contemplated by this disclosure. Individual stereoisomers of the compounds of this disclosure may not exist concurrently with other isomers (e.g., as a pure or substantially pure optical isomer with specific activity), or may be mixtures, such as racemates, or mixtures with all other stereoisomers or a part thereof. The chiral center of this disclosure has two configurations, S and R, which are defined by the IUPAC Recommendation 1974. The racemic form can be resolved by physical methods, such as fractional crystallization, or by derivatization to diastereomers, separation crystallization, or separation by chiral column chromatography. Individual optical isomers can be obtained from racemates by suitable methods, including but not limited to conventional methods, such as salt formation with an optically active acid followed by crystallization.

For the compounds of this disclosure, the compounds obtained by successive preparation, separation and purification have a weight content equal to or greater than 90%, for example, equal to or greater than 95%, equal to or greater than 99% ("very pure" compounds), listed in the text description. Herein such "very pure" compounds of this disclosure are also intended to be part of this disclosure.

All configurational isomers of the compounds of this disclosure are contemplated, whether in an admixture, pure or very pure form. The definition of compounds of this disclosure includes both cis (Z) and trans (E) olefin isomers, as well as carbocyclic and heterocyclic cis and trans isomers.

Throughout the specification, groups and substituents may be selected to provide stable fragments and compounds.

Definitions of specific functional groups and chemical terms are detailed below. For purposes of this disclosure, chemical elements are as defined in the Periodic Table of the Elements, CAS version, Handbook of Chemistry and Physics, 75th Ed. Definitions of specific functional groups are also described therein. In addition, basic principles of organic chemistry and specific functional groups and reactivity are also stated in "Organic Chemistry", Thomas Sorrell, University Science Books, Sausalito: 1999, the entire contents of which are incorporated by reference.

Some compounds of this disclosure may exist in specific geometric or stereoisomeric forms. This disclosure covers all compounds, including their cis and trans isomers, R and S enantiomers, diastereomers, (D) isomers, (L) isomers, racemic mixtures and other mixtures. In addition, asymmetric carbon atoms may represent substituents such as alkyl. All isomers and their mixtures are incorporated herein.

According to this disclosure, the mixture of isomers may include isomers in various ratios. For example, mixture of only two isomers can have the combinations: 50:50, 60:40, 70:30, 80:20, 90:10, 95:5, 96:4, 97:3, 98:2, 99 :1, and 100:0, and all ratios of isomers are within the scope of this disclosure. Similar ratios readily understood by those of ordinary skill in the art, as well as ratios that are mixtures of more complex isomers, are also within the scope of this disclosure.

This disclosure also includes isotopically labeled compounds that are equivalent to the original compounds disclosed herein. In practice, however, it usually occurs that one or more atoms are substituted with atoms with different atomic weights or mass numbers. Examples of isotopes that may be listed as compounds of this disclosure include hydrogen, carbon, nitrogen, oxygen, phosphorus, sulfur, fluorine and chlorine isotopes such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$. The compounds of this disclosure, or enantiomers, diastereomers, isomers, or pharmaceutically acceptable salts or solvates, which contain isotopes or other isotopic atoms of the above compounds are within the scope of this disclosure. Some isotopically-labeled compounds of this disclosure, such as radioisotopes of $^{3}H$ and $^{14}C$, are also included, and are useful in tissue distribution experiments of drugs and substrates. Tritium, i.e. $^{3}H$, and carbon-14, i.e. $^{14}C$, are relatively easy to prepare and detect. So they are the first choice among isotopes. In addition, a heavier isotope substituent such as deuterium, i.e., $^{2}H$, may be preferred in some cases due to its good metabolic stability, which may have advantages in certain therapies, such as increased half-life or reduced dosage in vivo. Isotopically-labeled compounds can be prepared using general methods by substituting readily available isotopically-labeled reagents for non-isotopically-labeled reagents using the solutions disclosed in the Examples.

If a specific enantiomer of a compound of this disclosure is to be engineered, it can be prepared by asymmetric synthesis, or by derivatization with a chiral auxiliary, separation of the resulting diastereomeric mixture, and removal of the chiral auxiliary to obtain a pure enantiomer. In addition, if the molecule contains a basic functional group, such as an amino acid, or an acidic functional group, such as a carboxyl group, it can, together with a suitable optically active acid or base, form diastereomeric salts, which can be separated by conventional means such as separation crystallization or chromatography, and then the pure enantiomers can be obtained.

As described herein, the compounds of this disclosure may be substituted with any number of substituents or functional groups to extend their scope. In general, whether the term "substituted" appears before or after the term "optional", the formulas that include substituents in the formulations of this disclosure refer to the replacement of hydrogen radicals with the specified structural substituents. When multiple positions in a particular structure are substituted with multiple specified substituents, the substituents may be the same or different at each position. The term "substituted"

as used herein includes all allowable substitutions with organic compounds. Broadly speaking, the permissible substituents include acyclic, cyclic, branched, unbranched, carbocyclic, heterocyclic, aromatic and non-aromatic organic compounds. In this disclosure, for example, the heteroatom nitrogen may have hydrogen substituents or any permissible organic compound described above to complement its valence. Furthermore, this disclosure is not intended to limit in any way the permissible substituted organic compounds. This disclosure contemplates that the combination of substituents and variable groups is well suited for the treatment of diseases in the form of stable compounds. The term "stable" as used herein refers to a compound that is stable enough to be detected for a sufficient period of time to maintain the structural integrity of the compound, preferably for a sufficient period of time, and is used herein for the above-mentioned purposes.

The metabolites of the compounds of this disclosure and pharmaceutically acceptable salts of the compounds, as well as prodrugs that can be converted into the structures of the compounds of this disclosure and their pharmaceutically acceptable salts in vivo, are also included in the claims of this disclosure.

**Preparation method**

The preparation method of the compound of formula (I) of this disclosure is described in more detail below, but these specific methods do not constitute any limitation to this disclosure. The compounds of this disclosure can also be optionally prepared by combining various synthesis methods described in this specification or known in the art, and such combinations can be easily performed by those skilled in the art to which this disclosure belongs.

Usually, in the preparation process, each reaction is usually carried out at room temperature to 90 °C in a suitable solvent under the protection of inert gas, and the reaction time is usually 2 to 24 h.

Method 1:

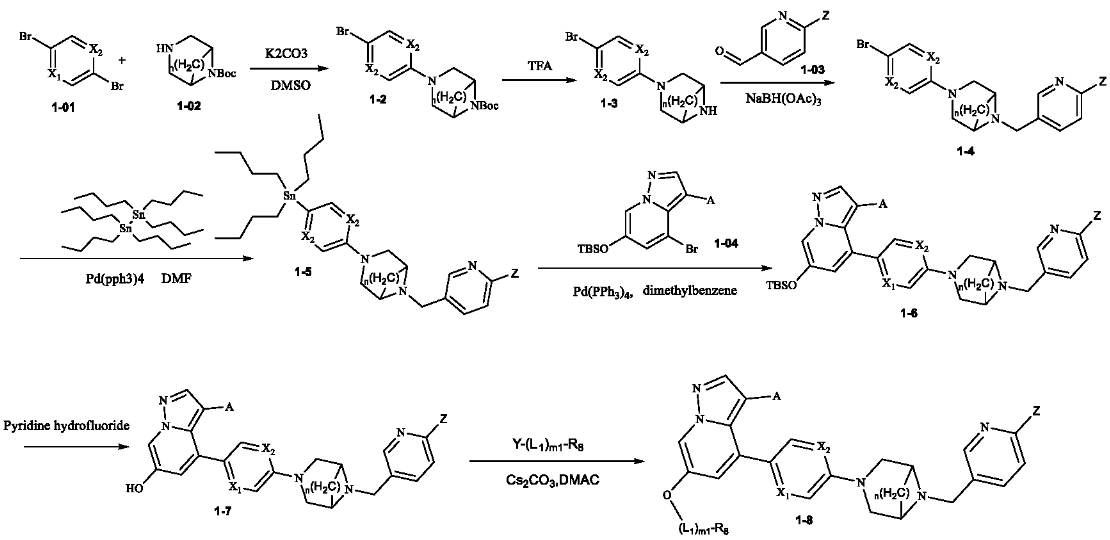


In Method 1, Y can be Cl, Br, or I; A, $X_1$, $X_2$, n, Z, $L_1$, $m_1$, and $R_8$ have the definitions described in this disclosure. The method includes the following steps:

(i) causing compound 1-01 to react with compound 1-02 in an inert solvent (such as DMSO) under a basic condition (e.g. in the presence of $K_2CO_3$, $Na_2CO_3$, $Cs_2CO_3$, and the like) to obtain compound 1-2;
(ii) deprotecting the amino group on the compound 1-2 in an inert solvent under an acidic condition (e.g., in the presence of trifluoroacetic acid) to obtain compound 1-3;
(iii) carrying out reductive amination between the compound 1-3 and compound 1-03 in an inert solvent in the presence of a reducing agent (e.g., $NaBH(OAc)_3$) to obtain compound 1-4;
(iv) causing the compound 1-4 to react with a tin reagent in an inert solvent (e.g., DMF) in the presence of a catalyst (e.g., a palladium catalyst) to obtain compound 1-5;
(v) causing the compound 1-5 to react with compound 1-04 in an inert solvent in the presence of a catalyst (e.g., a palladium catalyst) to obtain compound 1-6;
(vi) deprotecting the compound 1-6 in an inert solvent (e.g., xylene) under an acidic condition (e.g., in the presence of pyridinium hydrofluoride) to obtain compound 1-7; and
(vii) causing the compound 1-7 to react with compound Y-$(L_1)m_1$-$R_8$ in an inert solvent (e.g., DMSO) under a basic

condition (e.g., in the presence of $Cs_2CO_3$) to obtain compound 1-8.

In the above reaction steps, the reaction solvents, reaction temperature, reaction time, catalysts, and the like can be selected according to the specific reactants.

Method 2:

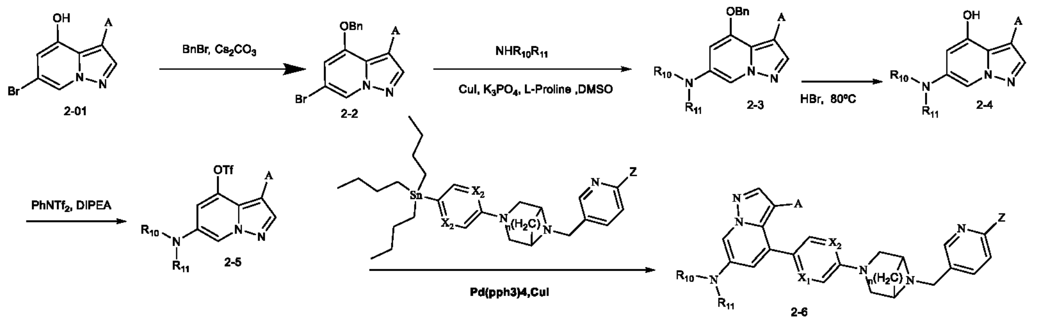


In Method 2, A, $X_1$, $X_2$, n, Z, $R_{10}$, and $R_{11}$ have the definitions described in this disclosure. The method includes the following steps:

(i) causing compound 2-01 to react with a hydroxyl protecting reagent (for example, benzyl bromide) in an inert solvent under a basic condition (e.g., in the presence of $Cs_2CO_3$ and the like) to obtain compound 2-2;
(ii) causing the compound 2-2 to react with an amine compound $NHR_{10}R_{11}$ in an inert solvent under a basic condition (e.g., in the presence of $K_3PO_4$) in the presence of a catalyst (e.g., CuI and L-proline) to obtain compound 2-3;
(iii) deprotecting the compound 2-3 in an inert solvent under an acidic condition (e.g., in the presence of HBr) to obtain compound 2-4;
(iv) causing the compound 2-4 to react with $PhNTf_2$ in an inert solvent to obtain compound 2-5; and
(v) causing the compound 2-5 to react with the compound 1-5 in an inert solvent in the presence of a catalyst (e.g., a palladium catalyst and cuprous halide) to obtain compound 2-6.

In the above reaction steps, the reaction solvents, reaction temperature, reaction time, catalysts, and the like can be selected according to the specific reactants.

**Pharmaceutical composition and its application method**

The pharmaceutical composition of this disclosure is used for preventing and/or treating a disease selected from the following: inflammation, cancers, cardiovascular diseases, infection, immune diseases, and metabolic diseases.

The compound of general formula (I) may be used in combination with other drugs known to treat or ameliorate similar conditions. In a case of co-administration, the administration mode and dosage of the original drug may remain unchanged, while the compound of formula I is administered concurrently or subsequently. When the compound of formula I is administered concomitantly with one or more other drugs, a pharmaceutical composition containing one or more known drugs and the compound of formula I at the same time may be preferred. Combined pharmacotherapy also includes administration of a compound of formula I and one or more other known drugs at overlapping time periods. When a compound of formula I is used in combination with one or more other drugs, the doses of the compound of formula I or known drugs may be lower than their doses when they are used alone.

The drugs or active ingredients that can be used in combination with the compound of general formula (I) include but are not limited to: a PD-1 inhibitor (such as Nivolumab, Pembrolizumab, JS-001, SHR-120, BGB-A317, IBI-308, GLS-010, GB-226, STW204, HX008, HLX10, BAT1306, AK105 , LZM 009 or biosimilars thereof, etc.), a PD-L1 inhibitor (such as Durvalumab, Atezolizumab, CS1001, KN035, HLX20, SHR-1316, BGB-A333, JS003, CS1003, KL-A167, F520, GR1405, MSB2311 or biosimilars thereof, etc.), a CD20 antibody (such as Rituximab, Obinutuzumab, Ofatumumab, Tositumumab, Tiimumab, etc.), a CD47 antibody (such as Hu5F9-G4, CC-90002, TTI-621, TTI-622, OSE-172, SRF-231, ALX-148, NI-1701, SHR-1603, IBI188, IMM01), an ALK inhibitor (such as Ceritinib, Alectinib, Brigatinib, Lorlatinib, and Oclatinib), a PI3K inhibitor (such as Ederatinib, Dactolisib, Taselisib, Buparlisib, etc.), a BTK Inhibitor (such as Ibrutinib, Tirabrutinib, Acalabrutinib, etc.), an EGFR inhibitor (such as Afatinib, Gefitinib, Erlotinib, Lapatinib, Dacomitinib, Icotinib, Canetinib, etc.), a VEGFR inhibitor (such as Sorafenib, Pazopanib, Rivatinib, Cabozantinib, Sunitinib, Donafenib, etc.), an HDAC inhibitor (such as Givinostat, Droxinostat, Entinostat, Darcylast, Tycodinaline, etc.), a CDK inhibitor (such as Palbociclib, Ribociclib, Abemaciclib, Lerociclib, etc.), a MEK inhibitor (such as Selumetinib

(AZD6244), Trametinib (GSK1120212), PD0325901, U0126, AS-703026, PD184352 (CI-1040), etc.), an Akt inhibitor (such as MK-2206, Ipatasertib, Capivasertib, Afuresertib, Uprosertib, etc.), an mTOR inhibitor (such as Vistusertib, etc.), an SHP2 inhibitor (such as RMC-4630, JAB-3068, TNO155, etc.), an IGF-1R inhibitor (such as Ceritinib, Ocaltinib, linsitinib, BMS-754807, GSK1838705A, etc.), and a combination thereof.

The dosage forms of the pharmaceutical composition of this disclosure include (but are not limited to): injections, tablets, capsules, aerosol, suppository, films, drop pills, external liniments, controlled-release or sustained-release or nano preparations.

The pharmaceutical composition of this disclosure includes the compound of this disclosure or a pharmacologically acceptable salt thereof within a safe and effective dose and a pharmacologically acceptable excipient or carrier. The "safe and effective dose" means that the dose of the compound is sufficient to significantly improve the condition without causing serious side effects. Usually, the pharmaceutical composition contains 1 to 2000 mg of the compound of this disclosure/dose, more preferably 10 to 1000 mg of the compound of this disclosure/dose. Preferably, the "one dose" means a capsule or tablet.

The "pharmaceutically acceptable carrier" means: one or more compatible solid or liquid filler or gel substances, which are suitable for human use, and which must be of sufficient purity and sufficiently low toxicity. "Compatible" as used herein means that components in a composition can be blended with the compound of this disclosure and with each other without significantly reducing the efficacy of the compound. Part of examples of the pharmaceutically acceptable carrier include cellulose and its derivatives (such as sodium carboxymethyl cellulose, sodium ethyl cellulose, cellulose acetate, and the like), gelatin, talc, solid lubricants (such as stearic acid and magnesium stearate), calcium sulfate, vegetable oils (such as soybean oil, sesame oil, peanut oil, olive oil, and the like), polyols (such as propylene glycol, glycerin, mannitol, sorbitol, and the like), emulsifiers (such as Tween®), wetting agents (such as sodium lauryl sulfate), colorants, flavors, stabilizers, antioxidants, preservatives, pyrogen-free water, and the like.

The mode of administration of the compounds or pharmaceutical compositions of this disclosure is not particularly limited, and representative modes of administration include (but are not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with: (a) a filler or compatibilizer, such as starch, lactose, sucrose, glucose, mannitol and silicic acid; (b) a binder, such as hydroxymethylcellulose, alginate, gelatin, polyvinylpyrrolidone, sucrose and acacia; (c) a humectant, such as glycerol; (d) a disintegrant, such as agar, calcium carbonate, potato starch or tapioca starch, alginic acid, some complex silicates, and sodium carbonate; (e) a sustained-solubilization agent, such as paraffin; (f) an absorption accelerator, such as a quaternary ammonium compound; (g) a wetting agent, such as cetyl alcohol and glyceryl monostearate; (h) an adsorbent, such as kaolin; and (i) a lubricant, such as talc, calcium stearate, magnesium stearate, solid polyethylene glycol, sodium lauryl sulfate, or mixtures thereof. In capsules, tablets and pills, the dosage form may also include a buffering agent.

Solid dosage forms such as tablets, dragees, capsules, pills and granules can be prepared using coating and shell materials, such as enteric coatings and other materials well known in the art. They may contain an opacifying agent, and the release of the active compound or compounds in such compositions may be in a certain part of the digestive tract in a delayed manner. Examples of an embedding component that can be employed are polymeric substances and waxes. If desired, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, liquid dosage forms may contain inert diluents conventionally employed in the art, such as water or other solvents solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures thereof.

Besides these inert diluents, the compositions can also contain adjuvants such as wetting agents, emulsifiers, suspensions, sweetening agents, flavoring agents and perfuming agents.

In addition to the active compounds, suspensions may contain suspending agents such as ethoxylated isostearyl alcohols, polyoxyethylene sorbitol and sorbitan esters, microcrystalline cellulose, aluminum methoxide and agar or mixtures thereof.

Compositions for parenteral injection may contain physiologically acceptable sterile aqueous or anhydrous solutions, dispersions, suspensions or emulsions and sterile powders for reconstitution into sterile injectable solutions or dispersions. Suitable aqueous and non-aqueous carriers, diluents, solvents or excipients include water, ethanol, polyols and suitable mixtures thereof.

Dosage forms for topical administration of the compounds of this disclosure include ointments, powders, patches, sprays and inhalants. The active ingredient is mixed under a sterile condition with a physiologically acceptable carrier and any preservatives, buffers, or propellants that may be required if necessary.

The treatment methods of this disclosure may be administered alone or in combination with other treatment means or treatment agents.

When the pharmaceutical composition is used, a safe and effective dose of the compound of this disclosure is applied to a mammal (such as a human) in need of treatment, wherein the dose is a pharmaceutically effective dose for administration, and for a person with a body weight of 60kg, the daily dose is usually 1 mg to 2000 mg, preferably 50 mg to 1000 mg. Certainly, the specific dosage should also take into account the route of administration, the patient's health and other factors, which are all within the skill of the skilled physician.

This disclosure further provides a preparation method of a pharmaceutical composition, including the steps of: mixing a pharmaceutically acceptable carrier with the compound of the general formula (I) or its crystal form, pharmaceutically acceptable salt, hydrate or solvate according to this disclosure to form a pharmaceutical composition.

This disclosure further provides a treatment method, including the steps of: applying the compound of general formula (I), or its crystalline form, pharmaceutically acceptable salt, hydrate or solvate according to this disclosure to an object in need of treatment, or applying the pharmaceutical composition of this disclosure, for selectively inhibiting RET.

**This disclosure has the following main advantages:**

1. The compounds of this disclosure have excellent inhibitory ability to RET kinase, and excellent selectivity to RET kinase, and have low inhibitory activity against other kinases such as VEGFR2.
2. The compounds of this disclosure have lower toxic and side effects.
3. The compounds of this disclosure have better pharmacodynamic and pharmacokinetic properties.
4. The compounds of this disclosure have desirable inhibitory activity against wild-type, gene fusion and mutant (including but not limited to G804 and G810) RET kinases.
5. The compounds of this disclosure in which the 6-position ofpyrazolo[1,5-a]pyridine

(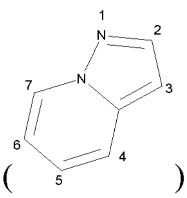)

is an amino group have good inhibitory activity against mutant-type RET G810, especially ethylamino or fluoroethylamino.

This disclosure will be further described below in conjunction with specific embodiments. It should be understood that these examples are only used to illustrate this disclosure and not to limit the scope of this disclosure. The experimental methods in the following examples where their specific conditions are not stated usually are implemented according to conventional conditions such as conditions stated by Sambrook et al., in Molecular Cloning: Laboratory Manual (New York: Cold Spring Harbor Laboratory Press, 1989), or according to conditions as suggested by manufacturers Unless otherwise stated, percentages and parts are by weight.

Unless otherwise defined, all professional and scientific terms used herein have the same meanings as those familiar to those skilled in the art. In addition, any methods and materials similar or equivalent to those described can be used in the methods of this disclosure. Methods and materials for preferred embodiments described herein are provided for illustrative purposes only.

The structures of the compounds of this disclosure were determined by nuclear magnetic resonance (NMR) and liquid chromatography-mass spectrometry (LC-MS).

NMR was performed using Bruker AVANCE-400 and Bruker AVANCE-500 NMR spectrometers; determination solvents include deuterated dimethyl sulfoxide (DMSO-d6), deuterated acetone ($CD_3COCD_3$), deuterated chloroform ($CDCl_3$) and deuterated methanol ($CD_3OD$), and the like. The internal standard uses tetramethylsilane (TMS). The chemical shift is measured in parts per million (ppm).

LC-MS was carried out using an Agilent 1260 mass spectrometer. The HPLC assay was performed using an Agilent 1100 high-pressure chromatograph (Microsorb 5 micron C18 100 x 3.0 mm column).

Qingdao GF254 silica gel plate was used for thin layer chromatography; 0.15-0.20 mm plate was used for TLC and 0.4 mm-0.5 mm plate was used for preparative thin layer chromatography. Column chromatography generally uses Qingdao 200-300 mesh silica gel as a carrier.

The starting materials in the examples of this disclosure are all known and commercially available, or can be synthesized by using or according to reference documents reported in the art.

Unless otherwise specified, all the reactions of this disclosure were carried out under the protection of dry inert gas (such as $N_2$ or Ar) by continuous magnetic stirring, and the reaction temperature was all in degrees Celsius.

**The following abbreviations are used throughout this disclosure:**

THF: tetrahydrofuran
MeOH: methanol
HCl: hydrochloric acid
$Pd(PPh_3)_4$: tetrakistriphenylphosphine palladium
$K_2CO_3$: potassium carbonate
AcOK: potassium acetate
NaOH: sodium hydroxide
$H_2O$: water
TEA: triethylamine
DIEA: N,N-diisopropylethylamine
DMF: N,N-dimethylformamide
DMA: N,N-dimethylacetamide
Py: pyridine
DCE: 1,2-dichloroethane
DMSO: dimethyl sulfoxide
TFA: trifluoroacetic acid
$NaBH(AcO)_3$: sodium triacetylborohydride
$Sn_2(Bu\text{-}n)_6$: hexahexyl ditin
$AlCl_3$: aluminum trichloride
CuI: Cuprous iodide
DPPA: diphenyl phosphate azide
BuOH: tert-butanol
$Cs_2CO_3$: cesium carbonate
$K_3PO_4$: potassium phosphate
BnBr: benzyl bromide
$Pd_2(dba)_3$: tris(dibenzylideneacetone)dipalladium
X-Phos: 2-dicyclohexylphosphorus-2,4,6-triisopropylbiphenyl
EA: ethyl acetate
$NaHCO_3$: sodium bicarbonate
DIPEA: N,N-diisopropylethylamine
HBr: hydrogen bromide

**Examples**

**Synthesis of intermediate 1**

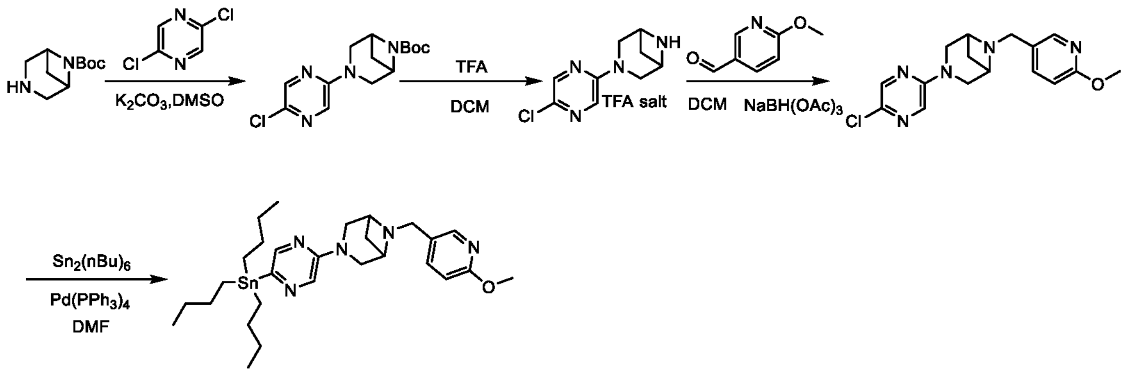


**Step 1: synthesis of tert-butyl 3-(5-chloropyrazin-2-yl)-3,6-diazabicyclo[3.1.1]heptane-6-carboxylate**

Tert-butyl 3,6-diazabicyclo[3.1.1]heptane-6-carboxylate (6.2 g, 31.3 mmol) was dissolved in dimethylsulfoxide (40 mL), 2,5-dichloropyrazine (6.02 g, 40.7 mmol) and potassium carbonate (21.6 g, 156.5 mmol) were then added to the resulting solution, and the reaction solution was stirred at 80 °C overnight. After the reaction was completed, the reaction solution was cooled to room temperature, water (50 mL) and ethyl acetate (100 mL) were added, and the organic phase was separated, washed with water, dried, and subjected to column chromatography (PE:EA=3:1) to obtain 7 g of tert-butyl 3-(5-chloropyrazin-2-yl)-3,6-diazabicyclo[3.1.1]heptane-6-carboxylate with a yield of 72.2%. MS m/z(ESI):311.2 $[M+H]^+$.

**Step 2: synthesis of 3-(5-chloropyrazin-2-yl)-3,6-diazabicyclo[3.1.1]heptane trifluoroacetate**

Tert-butyl 3-(5-chloropyrazin-2-yl)-3,6-diazabicyclo[3.1.1]heptane-6-carboxylate (5 g, 16.12 mmol) was dissolved in dichloromethane (30 mL) at room temperature, trifluoroacetic acid (30 mL) was then added, and the resulting solution reacted at room temperature for 1 h. After the reaction was found to be completed from the monitoring, the reaction solution was concentrated, diethyl ether (150 mL) was added to precipitate a solid, which was filtered, and then 4.9 g of 3-(5-chloropyrazin-2-yl)-3,6-diazabicyclo[3.1.1]heptane trifluoroacetate was obtained with a yield of 98.5%. MS m/z(ESI):211.1 $[M+H]^+$.

**Step 3: synthesis of 3-(5-chloropyrazine-2-yl)-6-((6-methoxypyridin-3-yl)methyl)-3,6-diazabicyclo[3.1.1]heptane**

3-(5-chloropyrazin-2-yl)-3,6-diazabicyclo[3.1.1]heptane trifluoroacetate (4.9 g, 15.96 mmol) was dissolved in dichloromethane (100 mL), sodium triacetylborohydride (17.2 g, 81.2 mmol) and 6-methoxynicotinic aldehyde (6.66 g, 48.6 mmol) were added, and the resulting solution was stirred at room temperature for 2 h to react. After the reaction was found to be completed from the monitoring, dichloromethane was added to dilute the reaction solution, the reaction was quenched with an ammonium chloride solution (50 mL), extraction with dichloromethane (50 mL*2) was then carried out, and the organic phase was washed with water, dried, concentrated, and subjected to column chromatography to obtain 4.3 g of 3-(5-chloropyrazine-2-yl)-6-((6-methoxypyridin-3-yl)methyl)-3,6-diazabicyclo[3.1.1]heptane, with a yield of 81.4%. MS m/z(ESI):332.2$[M+H]^+$.

**Step 4: synthesis of 6((6-methoxypyridin-3-yl)methyl)-3-(5-(tributyltinyl)pyrazin-2-yl)-3,6-diazabicyclo[3.1.1]heptane**

3-(5-chloropyrazine-2-yl)-6-((6-methoxypyridin-3-yl)methyl)-3,6-diazabicyclo[3.1.1]heptane (4.3 g, 12.99 mmol) was dissolved in DMF (40 mL), and then hexabutylditin (9.79 g, 16.89 mmol) and tetrakistriphenylphosphine palladium (1.5 g, 1.299 mmol) were added to the resulting solution, and the mixed solution was stirred at 140 °C for 16 h to react. After the reaction was found to be completed from the monitoring, the reaction was quenched with an ammonium chloride solution (50 mL), extraction with ethyl acetate (30 mL*2) was carried out, and the organic phase was washed, dried, concentrated, and subjected to column chromatography to obtain 2 g of 6((6-methoxypyridin-3-yl)methyl)-3-(5-(tributyltinyl)pyrazin-2-yl)-3,6-diazabicyclo[3.1.1]heptane **(intermediate 1**). MS m/z(ESI):588.3$[M+H]^+$.

**Synthesis of intermediate 2**

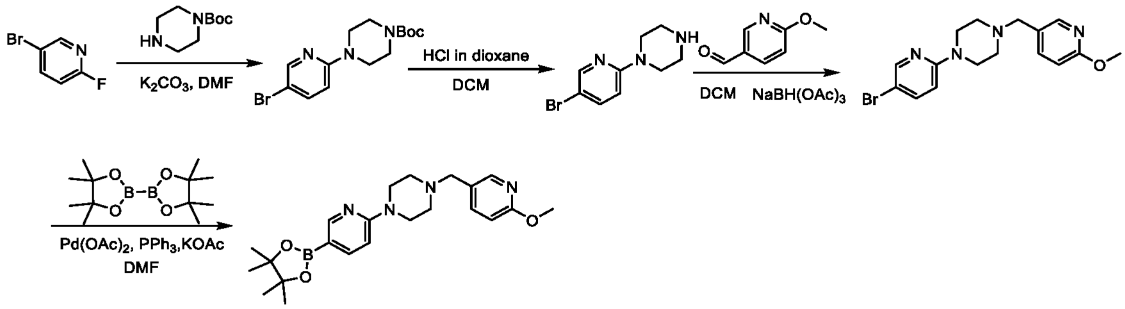


**Step 1: synthesis of tert-butyl 4-(5-bromopyridin-2-yl)piperazine-1-carboxylate**

5-bromo-2-fluoropyridine (10 g, 56.8 mmol), potassium carbonate (31 g, 227.3 mmol) and tert-butylpiperazine-1-carboxylate (10.6 g, 56.8 mmol) were dissolved in DMF (50 mL), and the reaction solution was stirred at 120 °C for 16 h to react. After the reaction was found to be completed from the monitoring, water (50 mL) and EA (30 mL*2) were added for extraction, the organic phase was washed with water, dried, concentrated, and subjected to column chromatography to obtain 15 g of tert-butyl 4-(5-bromopyridin-2-yl)piperazine-1-carboxylate. MS m/z(ESI):342.5[M+H]$^+$.

**Step 2: synthesis of 1-(5-bromopyridin-2-yl)piperazine**

tert-butyl 4-(5-bromopyridin-2-yl)piperazine-1-carboxylate (15 g, 43.99 mmol) was dissolved in dichloromethane (20 mL) at room temperature, and then dioxane hydrochloride (80 mL) was added to the resulting solution, and the mixed solution reacted at room temperature for 1 h. After the reaction was found to be completed from the monitoring, the reaction solution was concentrated, and adjusted to pH = 9 with a potassium carbonate solution; extraction with ethyl acetate was then carried out, the organic phase was washed with water, and then dried, concentrated, and subjected to column chromatography to obtain 8.5 g of 1-(5-bromopyridin-2-yl)piperazine. MS m/z(ESI):242.2[M+H]$^+$.

**Step 3: synthesis of 1-(5-bromopyridin-2-yl)-4-((6-methoxypyridin-3-yl)methyl)piperazine**

1-(5-bromopyridin-2-yl)piperazine (8.5 g, 35.3 mmol) was dissolved in dichloromethane (150 mL), and then sodium triacetylborohydride (22.5 g, 105.9 mmol) and 6-methoxynicotinic aldehyde (9.7 g, 70.6 mmol) were added to the resulting solution, and the mixed solution was stirred at room temperature for 2 h to react. After the reaction was found to be completed from the monitoring, dichloromethane was added to dilute the reaction solution, the reaction was quenched with an ammonium chloride solution (50 mL), and extraction with dichloromethane (50 mL*2) was then carried out, the organic phase was washed with water, dried, and concentrated, and subjected to column chromatography to obtain 8.3 g of 1-(5-bromopyridin-2-yl)-4-((6-methoxypyridin-3-yl)methyl)piperazine. MSm/z(ESI):363.3[M+H]$^+$.

**Step 4: synthesis of 1-((6-methoxypyridin-3-yl)methyl)-4-(5-(4,4,5,5-tetramethyl-1,3,2-dioxaborane-2-yl)pyridin-2-yl)piperazine**

1-(5-bromopyridin-2-yl)-4-((6-methoxypyridin-3-yl)methyl)piperazine (8.3 g, 22.93 mmol) was dissolved in DMF (40 mL), and then pinacol biboronate (11.65 g, 45.86 mmol), palladium acetate (0.26 g, 1.15 mmol), triphenylphosphine (1.2 g, 4.586 mmol) and potassium acetate (6.74 g, 68.78 mmol) were added to the resulting solution. The mixed solution was then stirred at 80 °C for 16 h to react. After the reaction was found to be completed from the monitoring, the reaction solution was diluted with water, extraction with ethyl acetate (30 mL*2) was carried out, the organic phase was washed with water, dried, concentrated, and subjected to column chromatography to obtain 4.98 g of 1-((6-methoxypyridin-3-yl)methyl)-4-(5-(4,4,5,5-tetramethyl-1,3,2-dioxaborane-2-yl)pyridin-2-yl)piperazine (**intermediate 2**). MS m/z(ESI):411.2[M+H]$^+$.

**Synthesis of intermediate 3**

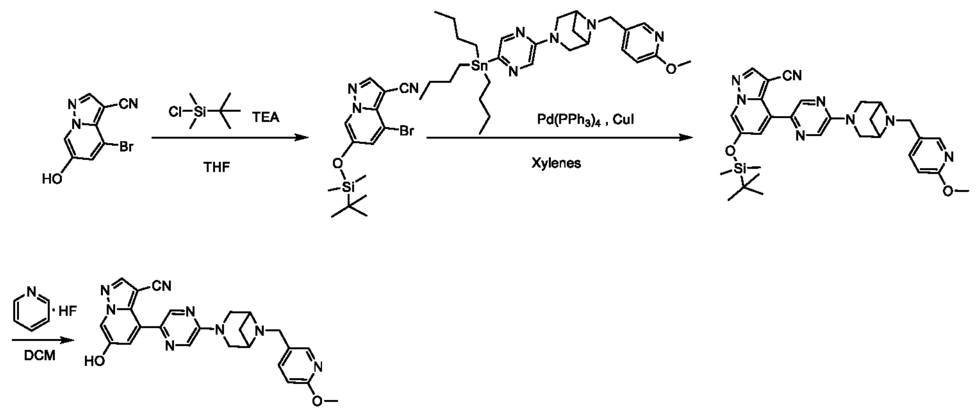


**Step 1: synthesis of 4-bromo-6-((tert-butyldimethylsiloxy)pyrazo[1,5-a]pyridine-3-carbonitrile**

4-bromo-6-hydroxypyrazolo[1,5-a]pyridine-3-carbonitrile (1.6 g, 6.75 mmol) and tert-butyldimethylsilyl chloride (1.12 g, 7.43 mmol) were dissolved in tetrahydrofuran (50 mL), and then triethylamine (1.87 mL) was added dropwise, the reaction solution was then stirred at room temperature for 2 h, an appropriate amount of water was then added, extraction with ethyl acetate was carried out, and the organic phase was then washed with water, dried, concentrated, and subjected to column chromatography to obtain 1.55 g of 4-bromo-6-((tert-butyldimethylsiloxy)pyrazo[1,5-a]pyridine-3-carbonitrile. MS m/z(ESI):352.0[M+H]$^+$.

**Step 2: synthesis of 6-((tert-butyldimethylsilyl)oxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

4-bromo-6-((tert-butyldimethylsilyloxy)pyrazolo[1,5-a]pyridine-3-carbonitrile (1.55 g, 4.43 mmol), 6((6-methoxypyridine-3-yl)methyl)-3-(5-(tributyltinyl)pyrazin-2-yl)-3,6-diazabicyclo[3.1.1]heptane (2.6 g, 4.43 mmol), CuI (84 mg, 0.443 mmol) and tetrakistriphenylphosphine palladium (512 mg, 0.443 mmol) were dissolved in xylene (40 mL), and the reaction solution was stirred at room temperature for 16 h. After the reaction was found to be completed from the monitoring, the reaction was quenched with water (10 mL), extraction with EA (20 mL*3) was carried out, the organic phase was washed with water, dried, concentrated, and subjected to column chromatography to obtain 1.57 g of 6-((tert-butyldimethylsilyl)oxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile. MS m/z(ESI): 569.3 [M+H]$^+$.

**Step 3: synthesis of 6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-((tert-butyldimethylsilyl)oxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (1.57 g, 2.76 mmol) was dissolved in dichloromethane (40 mL), and then hydrogen fluoride-pyridine (0.75 mL) was added dropwise, the reaction solution was stirred at room temperature for 2 h, adjusted to pH of neutrality with a saturated aqueous sodium bicarbonate solution, extraction with ethyl acetate was then carried out, and the organic phase was washed with water and then dried, concentrated, and subjected to column chromatography to obtain 1 g of 6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(intermediate 3**). MS m/z(ESI):455.4[M+H]$^+$.

**Synthesis of intermediate 4**

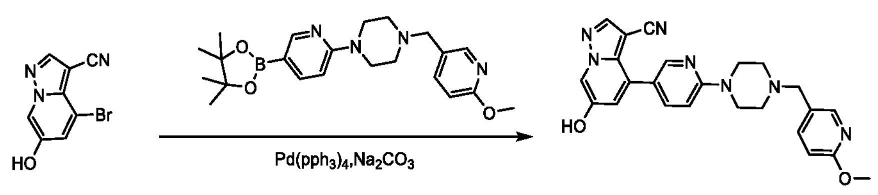

**Step: synthesis of 6-hydroxy-4-(6-(4-((6-methoxypyridin-3-yl)methyl)piperazin-1-yl)pyridin-3-yl)pyrazolo[1,5-a]pyridine-3-carbonitrile**

4-bromo-6-hydroxypyrazolo[1,5-a]pyridine-3-carbonitrile (1.0 mg, 4.2 mmol) and 1-((6-methoxypyridin-3-yl)methyl)-4-(5-(4,4,5,5-tetramethyl-1,3,2-dioxaborolan-2-yl)pyridin-2-yl)piperazine (2.07 g, 5.04 mmol) were dissolved in dioxane:water=(3:1, 20 mL), and then $Pd(PPh_3)_4$ (0.5 g, 0.42 mmol) and sodium carbonate (1.34 g, 12.6 mmol) were added respectively to the resulting solution. The mixed solution was then stirred at 85 °C overnight under the protection of $N_2$. After the reaction was completed, water was added to quench the reaction, the organic phase was separated, washed with water, dried, and subjected to column chromatography (PE:EA=1:1) to obtain 0.7 g of 6-hydroxy-4-(6-(4-((6-methoxypyridin-3-yl)methyl)piperazin-1-yl)pyridin-3-yl)pyrazolo[1,5-a]pyridine-3-carbonitrile (**intermediate 4**). MS m/z(ESI):442.2 $[M+H]^+$.

**Synthesis of intermediate 5**

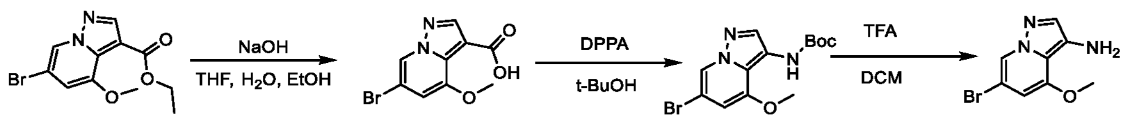


**Step 1: synthesis of 6-bromo-4-methoxypyrazolo[1,5-a]pyridine-3-carboxylic acid**

Ethyl 6-bromo-4-methoxypyrazo[1,5-a]pyridine-3-carboxylate (7.8 g, 26.17 mmol) was dissolved in tetrahydrofuran (10 mL), and then sodium hydroxide solution (5 mol/L, 10 mL) and ethanol (10 mL) were added to the resulting solution, and the reaction solution was stirred at room temperature for 2 h. After the reaction was found to be completed from the monitoring, the reaction solution was concentrated, and adjusted to pH of 3 with dilute hydrochloric acid, extraction with EA (20 mL*3) was carried out, and the organic phase was washed with water, dried, concentrated, and subjected to column chromatography to obtain 6 g of 6-bromo-4-methoxypyrazolo[1,5-a]pyridine-3-carboxylic acid. MS m/z(ESI):271.1$[M+H]^+$.

**Step 2: synthesis of tert-butyl(6-bromo-4-methoxypyrazolo[1,5-a]pyridin-3-yl)carbamate**

6-bromo-4-methoxypyrazolo[1,5-a]pyridine-3-carboxylic acid (6 g, 22.2 mmol) and diphenylphosphoryl azide (31 g, 227.3 mmol) were dissolved in tert-butanol (50 mL), and the reaction solution was then stirred at 80 °C for 4 h. After the reaction was found to be completed from the monitoring, the reaction solution was concentrated to obtain crude tert-butyl(6-bromo-4-methoxypyrazolo[1,5-a]pyridin-3-yl)carbamate for use of next step. MS m/z(ESI):342.5$[M+H]^+$.

**Step 3: synthesis of 6-bromo-4-methoxypyrazolo[1,5-a]pyridin-3-amine**

Tert-butyl(6-bromo-4-methoxypyrazolo[1,5-a]pyridin-3-yl)carbamate (crude product) was dissolved in dichloromethane (50 mL) at room temperature, and then trifluoroacetic acid (50 mL) was added to the resulting solution, and the mixed solution reacted at room temperature for 1 h. After the reaction was found to be completed from the monitoring, the reaction solution was concentrated and adjusted to pH = 9 with potassium carbonate solution, extraction with ethyl acetate was then carried out, and the organic phase was washed with water and then dried, concentrated, and subjected to column chromatography to obtain 3.1 g of 6-bromo-4-methoxypyrazolo[1,5-a]pyridin-3-amine (**intermediate 5**). MS m/z(ESI):242.3$[M+H]^+$.

**Example 1: Synthesis of compound 1**

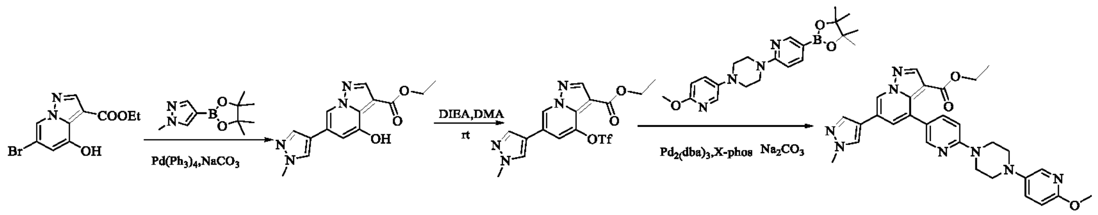


**Step 1: synthesis of ethyl 4-hydroxy-6-(1-methyl-1H-pyrazol-4-yl)H-pyrazolo[1,5-a]pyridine-3-carboxylate**

Ethyl 6-bromo-4-hydroxy-pyrazolo[1,5-a]pyridine-3-carboxylate (400 mg, 1.4 mmol) and 1-methyl-4-(4,4,5,5-tetramethyl-1,3,2-dioxaborolan-2-yl)-1H-pyrazole (351 mg, 1.7 mmol) were dissolved in dioxane:water=(3:1, 20 mL), and then $Pd(PPh_3)_4$ (145 mg, 0.14 mmol) and sodium carbonate (450 mg, 4.2 mmol) were added to the resulting solution. The mixed solution was then stirred at 85 °C overnight under the protection of $N_2$. After the reaction was completed, water was added to quench the reaction, and the organic phase was separated, washed with water, dried, and subjected to column chromatography (PE:EA=1:1) to obtain 250 mg of ethyl 4-hydroxy-6-(1-methyl-1H-pyrazol-4-yl)H-pyrazolo[1,5-a]pyridine-3-carboxylate with a yield of 62%. MS m/z(ESI):287.4 $[M+H]^+$.

**Step 2: synthesis of ethyl 6-(1-methyl-1H-pyrazol-4-yl)-4-(trifluoromethylsulfonyloxy)H-pyrazolo[1,5-a]pyridine-3-carboxylate**

At room temperature, ethyl 4-hydroxy-6-(1-methyl-1H-pyrazol-4-yl)H-pyrazolo[1,5-a]pyridine-3-carboxylate (250 mg, 0.87 mmol) and DIEA (224 mg, 1.74 mmol) were dissolved in DMA (5 mL), and then N-phenylbis(trifluoromethanesulfonyl)imide (343 mg, 1.0 mmol) was added to the resulting solution. The mixed solution was then stirred at room temperature for 6 h under the protection of $N_2$. After the reaction was completed, water was added to quench the reaction, and the organic phase was separated, washed with water, dried, and subjected to column chromatography (DCM:MeOH=20:1) to obtain 230 mg of ethyl 6-(1-methyl-1H-pyrazol-4-yl)-4-(trifluoromethylsulfonyloxy)H-pyrazolo[1,5-a]pyridine-3-carboxylate with a yield of 63%. MS m/z(ESI):419.3 $[M+H]^+$.

**Step 3: synthesis of ethyl 4-(6-(4-(6-methoxypyridin-3-yl)piperazin-1-yl)pyridin-3-yl)-6-(1-methyl-1H-pyrazol-4-yl)H-pyrazolo[1,5-a]pyridine-3-carboxylate**

Ethyl 6-(1-methyl-1H-pyrazol-4-yl)-4-(trifluoromethylsulfonyloxy)H-pyrazolo[1,5-a]pyridine-3-carboxylate (230 mg, 0.55 mmol), 1-(6-methoxypyridin-3-yl)-4-(5-(4,4,5,5-tetramethyl-1,3,2-dioxaborolan-2-yl)pyridin-2-yl)piperazine (293 mg, 0.71 mmol), $Pd_2(dba)_3$ (50 mg, 0.055 mmol), and X-phos (45 mg, 0.11 mmol) were dissolved in dioxane:water=(3:1, 20 mL), and then sodium carbonate (291 mg, 2.75 mmol) was added to the resulting solution. The mixed solution was then stirred at 85 °C overnight under the protection of $N_2$. After the reaction was completed, water was added to quench the reaction, and the organic phase was separated, washed with water, dried, and subjected to column chromatography (DCM:MeOH=20:1) to obtain 121 mg of ethyl 4(6-(4-(6-methoxypyridin-3-yl)piperazin-1-yl)pyridin-3-yl)-6-(1-methyl-1H-pyrazol-4-yl)H-pyrazolo[1,5-a]pyridine-3-carboxylate (**compound 1**) with a yield of 39.9%. MS m/z(ESI):553.0 $[M+H]^+$.

$^1H$ NMR(400 MHz, DMSO) $\delta$ 9.12(s, 1H), 8.41(s, 1H), 8.37(s, 1H), 8.19(d, 1H), 8.09(s, 1H), 8.08(d, 1H), 7.66-7.68(m, 2H), 7.56-7.58(m, 1H), 6.68(d, 1H), 6.80(d, 1H), 3.82-3.87(m, 8H), 3.55(s, 4H), 3.47(s, 2H), 2.49-2.50(s, 3H), 0.89(t, 3H).

**Example 2: Synthesis of compound 2**

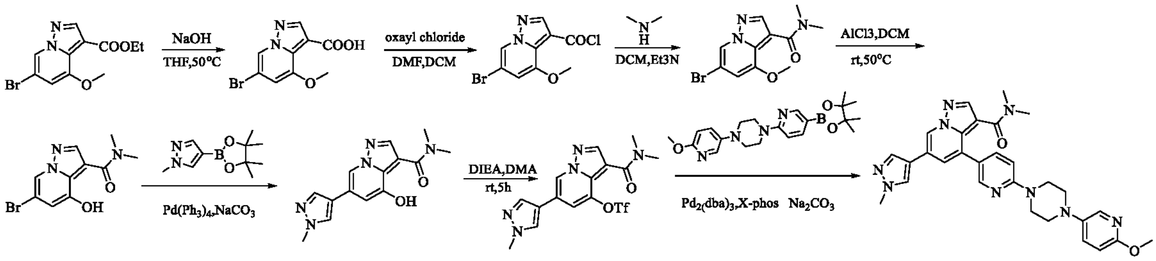

**Step 1: synthesis of 6-bromo-4-methoxyH-pyrazolo[1,5-a]pyridine-3-carboxylic acid**

At room temperature, ethyl 6-bromo-4-methoxyH-pyrazolo[1,5-a]pyridine-3-carboxylate (1.56 g, 5.26 mmol) was dissolved in THF/MeOH=(1:1, 20 mL), and then NaOH (4M, 10 mL) was added to the resulting solution. The mixed solution was then stirred at 50 °C for 1 h. After the reaction was complete, the reaction solution was concentrated at reduced pressure to remove the organic solvent, and then acidified with hydrochloric acid until pH=2-3. The resulting solution was then stirred at 0 °C for 0.5 h, and the solid was filtered, washed with water, and concentrated to obtain 1.3 g of 6-bromo-4-methoxyH-pyrazolo[1,5-a]pyridine-3-carboxylic acid with a yield of 92%. MS m/z(ESI): 268.9 [MH]+.

**Step 2: synthesis of 6-bromo-4-methoxyH-pyrazolo[1,5-a]pyridine-3-carbonyl chloride**

Under the protection of $N_2$, 6-bromo-4-methoxyH-pyrazolo[1,5-a]pyridine-3-carboxylic acid (400 mg, 1.48 mmol) was dissolved in 20 mL of DCM and the resulting solution was then cooled to 0 °C. Oxalyl chloride (282 mg, 2.22 mmol) and 1 drop of DMF were then added to the resulting solution and the mixed solution was stirred at room temperature overnight. After the reaction was complete, the solvent was removed and the product was directly used in the next step.

**Step 3: synthesis of 6-bromo-4-methoxy-N,N-dimethylpyrazolo[1,5-a]pyridine-3-carboxamide**

At room temperature, a dimethylamine tetrahydrofuran solution (0.96 mL, 1.48 mmol) and triethylamine (448 mg, 4.44 mmol) were dissolved in DCM (20 mL) and the resulting solution was then cooled to 0 °C.

6-bromo-4-methoxyH-pyrazolo[1,5-a]pyridine-3-carbonyl chloride (426 mg, 1.48 mmol) was then added to the resulting solution to have thermal reaction for 0.5 h. After the reaction was completed, water was added to quench the reaction, and the organic phase was separated, washed with water, dried, and subjected to column chromatography to obtain 494 mg of 6-bromo-4-methoxy-N,N-dimethylpyrazolo[1,5-a]pyridine-3-carboxamide with a yield of 100%. MS m/z(ESI):297.9 $[M+H]^+$.

**Step 4: synthesis of 6-bromo-4-hydroxy-N,N-dimethylpyrazolo[1,5-a]pyridine-3-carboxamide**

Under the protection of $N_2$, 6-bromo-4-methoxy-N,N-dimethylpyrazolo[1,5-a]pyridine-3-carboxamide (494 mg, 1.6 mmol) was dissolved in DCE ( 20 mL), and then $AlCl_3$ (665 mg, 4.98 mmol) was added the resulting solution. The mixed solution was then stirred at 50 °C for 0.5 h. After the reaction was completed, the reaction solution was adjusted to the pH of 5 to 6 with dilute hydrochloric acid and extracted with DCM. The organic phase was separated, washed with water, dried, and subjected to column chromatography (DCM:MeOH=20:1) to obtain 341 mg of 6-bromo-4-hydroxy-N,N-dimethylpyrazolo[1,5-a]pyridine-3-carboxamide with a yield of 91.4%. MS m/z(ESI):281.9 $[M+H]^+$.

**Step 5: synthesis of 4-hydroxy-N,N-dimethyl-6-(1-methyl-1H-pyrazol-4-yl)pyrazolo[1,5-a]pyridine-3-carboxamide**

6-bromo-4-hydroxy-N,N-dimethylpyrazolo[1,5-a]pyridine-3-carboxamide (341 mg, 1.2 mmol) and 1-methyl-4-(4,4,5,5-tetramethyl-1,3,2-dioxaborolane-2-yl)-1H-pyrazole (123 mg, 1.44 mmol) were dissolved in dioxane:water=(10:1, 22 mL), and then $Pd(Ph_3)_4$ (123 mg, 0.12 mmol) and sodium carbonate (381 mg, 3.6 mmol) were added to the resulting solution. The mixed solution was then stirred at 90 °C overnight under the protection of $N_2$. After the reaction was completed, water was added to quench the reaction, and the organic phase was separated, washed with water, dried, and subjected to column chromatography (DCM:MeOH=20:1) to obtain 409 mg of 4-hydroxy-N,N-dimethyl-6-(1-methyl-1H-pyrazol-4-yl)pyrazolo[1,5-a]pyridine-3-carboxamide with a yield of 100%. MS m/z(ESI):284.2 $[M+H]^+$.

**Step 6: synthesis of 3-(dimethylcarbamoyl)-6-(1-methyl-1H-pyrazol-4-yl)H-pyrazolo[1,5-a]pyridin-4-yl trifluoromethanesulfonate**

At room temperature, 4-hydroxy-N,N-dimethyl-6-(1-methyl-1H-pyrazol-4-yl)pyrazolo[1,5-a]pyridine-3-carboxamide (400 mg, 1.4 mmol) and DIEA (310 mg, 2.8 mmol) were dissolved in DMA (5 mL), and then N-phenylbis(trifluoromethanesulfonyl)imide (600 mg, 1.68 mmol) was added to the resulting solution. The mixed solution was then stirred at room temperature for 3 h under the protection of $N_2$. After the reaction was completed, water was added to quench the reaction, and the organic phase was separated, washed with water, dried, and subjected to column chromatography (DCM:MeOH=20:1) to obtain 300 mg of 3-(dimethylcarbamoyl)-6-(1-methyl-1H-pyrazol-4-yl)H-pyrazolo[1,5-a]pyridin-4-yl trifluoromethanesulfonate with a yield of 85%. MS m/z(ESI):417.9 $[M+H]^+$.

**Step 7: synthesis of 4-(6-(4-(6-methoxypyridin-3-yl)piperazin-1-yl)pyridin-3-yl)-N,N-dimethyl-6-(1-methyl-1H-pyrazol-4-yl)H-pyrazolo [1,5-a]pyridine-3-carboxamide**

3-(dimethylcarbamoyl)-6-(1-methyl-1H-pyrazol-4-yl)H-pyrazolo[1,5-a]pyridin-4-yl trifluoromethanesulfonate (300 mg, 0.72 mmol), 1-(6-methoxypyridin-3-yl)-4-(5-(4,4,5,5-tetramethyl-1,3,2-dioxaborolane-2-yl)pyridin-2-yl)piperazine (383 mg, 0.93 mmol), $Pd_2(dba)_3$ (65 mg, 0.072 mmol), and X-phos (60 mg, 0.144 mmol) were dissolved in dioxane:water=(10:1, 20 mL), and then sodium carbonate (381 mg, 3.6 mmol) was added to the resulting solution. The mixed solution was then stirred at 90 °C overnight under the protection of $N_2$. After the reaction was completed, water was added to quench the reaction, and the organic phase was separated, washed with water, dried, and subjected to column chromatography (DCM:MeOH=10:1) to obtain 188 mg of 4-(6-(4-(6-methoxypyridin-3-yl)piperazin-1-yl)pyridin-3-yl)-N,N-dimethyl-6-(1-methyl-1H-pyrazol-4-yl)H-pyrazolo[1,5-a]pyridine-3-carboxamide (**compound 2**) with a yield of 50%. MS m/z(ESI):552.2[M+H]$^+$.

$^1$H NMR(400 MHz, DMSO) δ 9.03(s, 1H), 8.34(s, 1H), 8.24(d, 1H), 8.07(t, 1H), 7.66-7.69(m, 1H), 7.56-7.59(m, 1H), 6.89(d, 1H), 6.81(d, 1H), 4.14(s, 3H), 3.84(s, 3H), 3.57(s, 4H), 3.48(s, 2H), 2.56(s, 3H),2.45-2.47(m, 3H), 2.41(s, 2H).

**Example 3: Synthesis of compound 3**

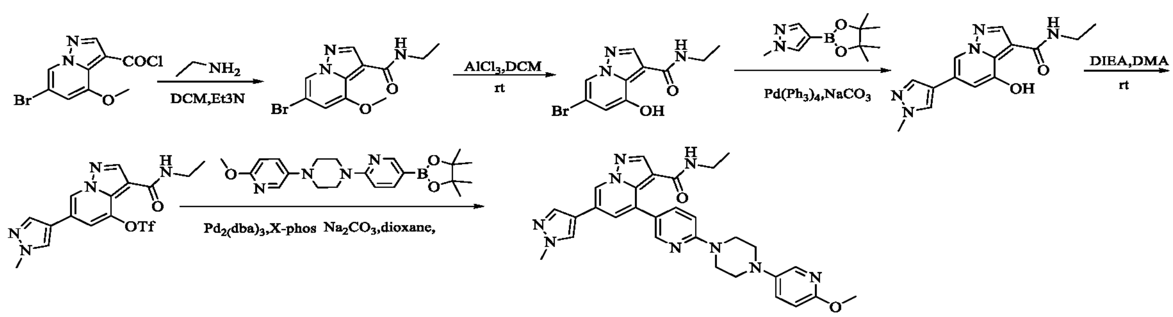


**Step 1: synthesis of 6-bromo-N-ethyl-4-methoxy-pyrazolo[1,5-a]pyridine-3-carboxamide**

At room temperature, an ethylaminetetrahydrofuran solution solution (1 mL, 1.92 mmol) and DIEA (448 mg, 4.44 mmol) were dissolved in DCM (10 mL) and the resulting solution was then cooled to 0 °C. 6-bromo-4-methoxyH-pyrazolo[1,5-a]pyridine-3-carbonyl chloride (426 mg, 1.48 mmol) was then added to the resulting solution to have thermal reaction for 0.5 h. After the reaction was completed, water was added to quench the reaction, and the organic phase was separated, washed with water, dried, and subjected to column chromatography to obtain 420 mg of 6-bromo-N-ethyl-4-methoxy-pyrazolo[1,5-a]pyridine-3-carboxamide with a yield of 90 %.

**Step 2: synthesis of 6-bromo-N-ethyl-4-hydroxy-pyrazolo[1,5-a]pyridine-3-carboxamide**

Under the protection of $N_2$, 6-bromo-N-ethyl-4-methoxy-pyrazolo[1,5-a]pyridine-3-carboxamide (420 mg, 1.4 mmol) was dissolved in DCE (20 mL) at room temperature, and then $AlCl_3$ (567 mg, 4.25 mmol) was added to the resulting solution, and the mixed solution was stirred at 50 °C for 2 h. After the reaction was completed, the reaction solution was adjusted to pH of 5 to 6, extraction with DCM was then carried out, and the organic phase was separated and then washed with water, dried, and subjected to column chromatography (PE:EA=5 : 1) to obtain 336 mg of 6-bromo-N-ethyl-4-hydroxy-pyrazolo[1,5-a]pyridine-3-carboxamide with a yield of 83%. MS m/z(ESI):286.1 [M+H]$^+$.

**Step 3: synthesis of N-ethyl-4-hydroxy-6-(1-methyl-1H-pyrazol-4-yl)pyrazolo[1,5-a]pyridine-3-carboxamide**

6-bromo-N-ethyl-4-hydroxy-pyrazolo[1,5-a]pyridine-3-carboxamide (336 mg, 1.1 mmol) and 1-methyl-4-(4,4,5,5-tetramethyl-1,3,2-dioxaborolane-2-yl)-1H-pyrazole (324 mg, 1.4 mmol) were dissolved in dioxane:water=(10:1, 22 mL), and then $Pd(PPh_3)_4$ (124 mg, 0.11 mmol) and sodium carbonate (385 mg, 3.3 mmol) were added to the resulting solution. The mixed solution was then stirred at 90 °C overnight under the protection of $N_2$. After the reaction was completed, water was added to quench the reaction, and the organic phase was separated, washed with water, dried, and subjected to column chromatography (DCM:MeOH=20:1) to obtain 284 mg of N-ethyl-4-hydroxy-6-(1-methyl-1H-pyrazol-4-yl)pyrazolo[1,5-a]pyridine-3-carboxamide with a yield of 77%.

**Step 4: synthesis of 3-(ethylcarbamoyl)-6-(1-methyl-1H-pyrazol-4-yl)H-pyrazolo[1,5-a]pyridin-4-yl trifluoromethanesulfonate**

At room temperature, N-ethyl-4-hydroxy-6-(1-methyl-1H-pyrazol-4-yl)pyrazolo[1,5-a]pyridine-3-carboxamide (284 mg, 1.40 mmol) and DIEA (256 mg, 2.80 mmol) were dissolved in DMA (5 mL), and then N-phenylbis(trifluoromethanesulfonyl)imide (428 mg, 1.2 mmol) was added to the resulting solution. The mixed solution was then stirred at room temperature for 2 h under the protection of $N_2$. After the reaction was completed, water was added to quench the reaction, and the organic phase was separated, washed with water, dried, and subjected to column chromatography (DCM:MeOH=20:1) to obtain 360 mg of 3-(ethylcarbamoyl)-6-(1-methyl-1H-pyrazol-4-yl)H-pyrazolo[1,5-a]pyridin-4-yl trifluoromethanesulfonate with a yield of 87%. MS m/z(ESI):416.0[M+H]$^+$.

**Step 5: synthesis of N-ethyl-4-(6-(4-(6-methoxypyridin-3-yl)piperazin-1-yl)pyridin-3-yl)-6-(1-methyl-1H-pyridin oxazol-4-yl)H-pyrazolo[1,5-a]pyridine-3-carboxamide**

3-(ethylcarbamoyl)-6-(1-methyl-1H-pyrazol-4-yl)H-pyrazolo[1,5-a]pyridin-4-yl triflate (200 mg, 0.4 mmol), 1-(6-methoxypyridin-3-yl)-4-(5-(4,4,5,5-tetramethyl-1,3,2-dioxaborolane-2-yl)pyridin-2-yl)piperazine (256 mg, 0.62 mmol), $Pd_2(dba)_3$ (37 mg, 0.04 mmol), and X-phos (38 mg, 0.04 mmol) were dissolved in dioxane:water=(10:1, 20 mL), and then sodium carbonate (212 mg, 2.0 mmol) was added to the resulting solution. The mixed solution was then stirred at 90 °C overnight under the protection of $N_2$. After the reaction was completed, water was added to quench the reaction, and the organic phase was separated, washed with water, dried, and subjected to column chromatography (DCM:MeOH=10:1) to obtain 113 mg of N-ethyl-4-(6-(4-(6-methoxypyridin-3-yl)piperazin-1-yl)pyridin-3-yl)-6-(1-methyl-1H-pyridin oxazol-4-yl)H-pyrazolo[1,5-a]pyridine-3-carboxamide (**compound 3**) with a yield of 42 %. MS m/z(ESI):552.1[M+H]$^+$.

$^1$H NMR(400 MHz, DMSO) δ 9.02(s, 1H), 8.35(s, 1H), 8.21(d, 1H), 8.14(s, 1H), 8.08(m, 1H), 7.66-7.69(m, 1H), 6.62-7.64(d, 1H), 7.54-7.59(m, 1H), 7.52(d, 1H), 6.80-6.87(m, 1H), 3.87(s, 3H), 3.85(s, 3H), 3.55(s, 4H), 3.48(s, 2H), 2.86-2.92(m, 2H),2.44-2.47(m, 4H), 0.79(m, 3H).

**Example 4: Synthesis of compound 4**

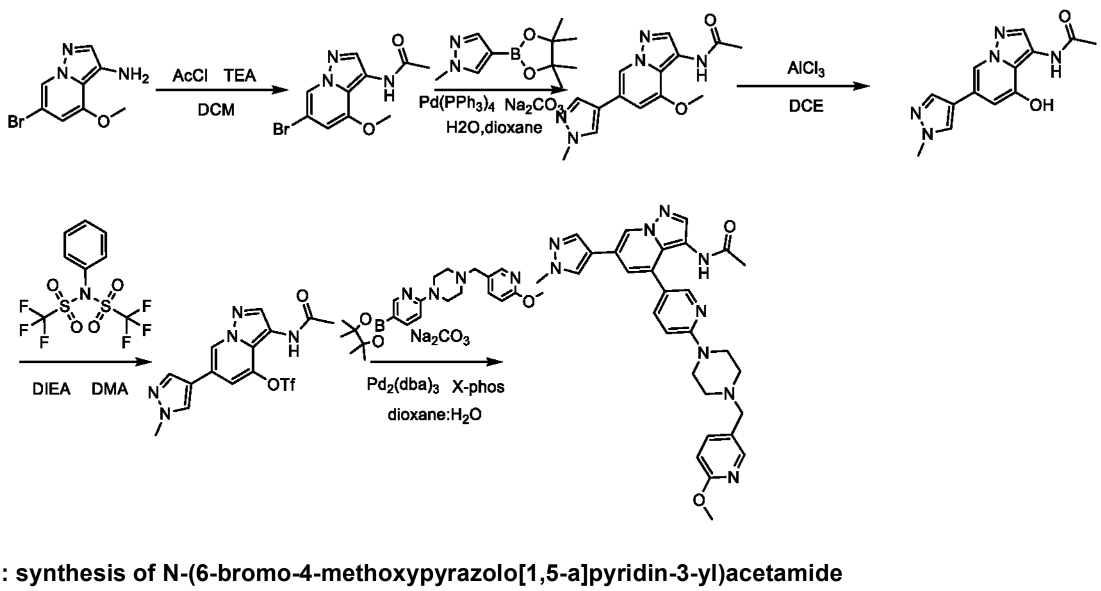


**Step 1: synthesis of N-(6-bromo-4-methoxypyrazolo[1,5-a]pyridin-3-yl)acetamide**

6-bromo-4-methoxypyrazolo[1,5-a]pyridin-3-amine (600 mg, 2.49 mmol) and triethylamine (0.517 mL) were dissolved in dichloromethane (15 mL), and then acetyl chloride (0.27 mL) was added dropwise in the resulting solution in an ice bath, and the reaction solution reacted at room temperature for 1 h. After the reaction was found to be completed from the monitoring, the reaction solution was cooled, water was added to quench the reaction, extraction with EA (10 mL*3) was then carried out, and the organic phase was washed with water and then dried, concentrated and subjected to column chromatography to obtain 580 mg of N-(6-bromo-4-methoxypyrazolo[1,5-a]pyridin-3-yl)acetamide. MS m/z(ESI):284.2[M+H]$^+$.

**Step 2: synthesis of N-(4-methoxy-6-(1-methyl-1H-pyrazol-4-yl)pyrazolo[1,5-a]pyridin-3-yl)acetamide**

N-(6-bromo-4-methoxypyrazolo[1,5-a]pyridin-3-yl)acetamide (580 mg, 2.05 mmol), tetrakistriphenylphosphine palladium (237 mg, 0.205 mmol), sodium carbonate (652 mg, 6.15 mmol), and 1-methyl-4-(4,4,5,5-tetramethyl-1,3,2-dioxolane-2-yl)-pyrazole (512 mg, 2.46 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 520 mg of N-(4-methoxy-6-(1-methyl-1H-pyrazol-4-yl)pyrazolo[1,5-a]pyridin-3-yl)acetamide. MS m/z(ESI):286.0$[M+H]^+$.

**Step 3: synthesis of N-(4-hydroxy-6-(1-methyl-1H-pyrazol-4-yl)pyrazol[1,5-a]pyridin-3-yl)acetamide**

N-(4-methoxy-6-(1-methyl-1H-pyrazol-4-yl)pyrazolo[1,5-a]pyridin-3-yl)acetamide (520 mg, 1.82 mmol) was dissolved in 1,2-dichloroethane (20 mL), and then aluminum trichloride (850 mg, 6.39 mmol) was added to the resulting solution. The reaction solution was stirred at room temperature for 16 h, water (10 mL) was added to quench the reaction, extraction with EA (20 mL*3) was then carried out, and the organic phase was washed with water and then dried, concentrated, and subjected to column chromatography to obtain 250 mg of N-(4-hydroxy-6-(1-methyl-1H-pyrazol-4-yl)pyrazol[1,5-a]pyridin-3-yl)acetamide. MS m/z (ESI): 272.5 $[M+H]^+$.

**Step 4: synthesis of 3-acetylamino-6-(1-methyl-1H-pyrazol-4-yl)pyrazol[1,5-a]pyridin-4-yl trifluoromethanesulfonate**

N-(4-hydroxy-6-(1-methyl-1H-pyrazol-4-yl)pyrazol[1,5-a]pyridin-3-yl)acetamide (250 mg, 0.92 mmol), N-phenylbis(trifluoromethanesulfonyl)imide (396 mg, 1.11 mmol) and N,N-diisopropylethylamine (0.33 mL, 1.85 mmol) were dissolved in DMA (20 mL), and the resulting reaction solution was stirred at room temperature for 16 h to react. After the reaction was found to be completed from the monitoring, water was added to quench the reaction, extraction with EA (30 mL*2) was carried out, and the organic phase was washed with water and then dried, concentrated, and subjected to column chromatography to obtain 300 mg of 3-acetylamino-6-(1-methyl-1H-pyrazol-4-yl)pyrazol[1,5-a]pyridin-4-yl trifluoromethanesulfonate. MS m/z(ESI):404.4$[M+H]^+$.

**Step 5: synthesis of N-(4-(6-(4-((6-methoxypyridin-3-yl)methyl)piperazin-1-yl)pyridin-3-yl)-6-(1-methyl-1H-pyrazol-4-yl)pyrazol[1,5-a]pyridin-3-yl)acetamide**

3-acetylamino-6-(1-methyl-1H-pyrazol-4-yl)pyrazol[1,5-a]pyridin-4-yl trifluoromethanesulfonate (150 mg, 0.372 mmol), 1-((6-methoxypyridin-3-yl)methyl)-4-(5-(4,4,5,5-tetramethyl-1,3,2-dioxolane-2 -yl)pyridin-2-yl)piperazine (183 mg, 0.447 mmol), sodium carbonate (197 mg, 1.86 mmol), tris(dibenzylideneacetone)dipalladium (34 mg, 0.037 mmol), and 2-dicyclohexylphosphorus-2,4,6-triisopropylbiphenyl (35.5 mg, 0.074 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 100 mg of N-(4-(6-(4-((6-methoxypyridin-3-yl)methyl)piperazin-1-yl)pyridin-3-yl)-6-(1-methyl-1H-pyrazol-4-yl)pyrazol[1,5-a]pyridin-3-yl)acetamide (**compound 4**). MS m/z(ESI):537.1$[M+H]^+$.

$^1$H NMR(500 MHz, DMSO-d6) δ 9.02(s, 1H), 8.91(d, J = 1.4 Hz, 1H), 8.31(s, 1H), 8.23 - 8.18(m, 1H), 8.09(d, J = 2.4 Hz, 1H), 8.05(d, J = 0.8 Hz, 1H), 7.86(s, 1H), 7.68(dd, J = 8.5, 2.4 Hz, 1H), 7.58(dd, J = 8.8, 2.5 Hz, 1H), 7.37(d, J = 1.4 Hz, 1H), 6.89(d, J = 8.7 Hz, 1H), 6.82(dd, J = 8.4, 0.7 Hz, 1H), 3.87(s, 3H), 3.85(s, 3H), 3.57(t, J = 5.0 Hz, 4H), 3.49(s, 2H), 2.47(t, J = 5.0 Hz, 4H), 1.66(s, 3H).

**Example 5: Synthesis of compound 5**

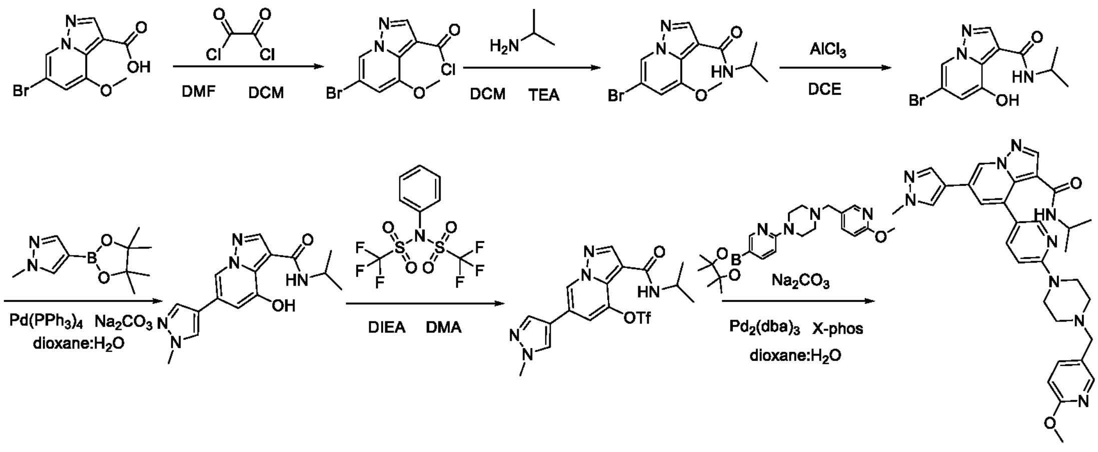


**Step 1: synthesis of 6-bromo-4-methoxypyrazolo[1,5-a]pyridine-3-carbonyl chloride**

6-bromo-4-methoxypyrazolo[1,5-a]pyridine-3-carboxylic acid (400 mg) was dissolved in dichloromethane (10 mL), and then oxalyl chloride (282 mg) was slowly added dropwise, and 2 drops of DMF were then added. The resulting reaction solution was stirred at room temperature for 1 h to react. After the reaction was found to be completed from the monitoring of LCMS, the reaction solution was concentrated to obtain 6-bromo-4-methoxypyrazolo[1,5-a]pyridine-3-carbonyl chloride (426 mg).

**Step 2: synthesis of 6-bromo-N-isopropyl-4-methoxypyrazolo[1,5-a]pyridine-3-carboxamide**

6-bromo-4-methoxypyrazolo[1,5-a]pyridine-3-carbonyl chloride (426 mg) was dissolved in dichloromethane (10 mL), and then isopropylamine (113 mg) was added, and triethylamine (448 mg) was then added dropwise. The resulting reaction solution was stirred at room temperature for 1 h to react. After the reaction was found to be completed from the monitoring of LCMS, water (10 mL) was added to quench the reaction and then the reaction solution was washed with water, dried, concentrated and subjected to column chromatography to obtain 6-bromo-N-isopropyl-4-methoxypyrazolo[1,5-a]pyridine-3-carboxamide (350 mg). MS m/z(ESI):312.1$[M+H]^+$.

**Step 3: synthesis of 6-bromo-4-hydroxy-N-isopropylpyrrolo[1,5-a]pyridine-3-carboxamide**

6-bromo-N-isopropyl-4-methoxypyrazolo[1,5-a]pyridine-3-carboxamide (350 mg) was dissolved in 1,2-dichloroethane (10 mL), and then aluminium trichloride (446 mg) was added, and the resulting reaction solution was stirred at room temperature for 16 h to react. After the reaction was found to be completed from the monitoring of LCMS, water (20 mL) was added to quench the reaction and then the reaction solution was extracted with dichloromethane, washed with water, and then dried and concentrated to obtain 6-bromo-4-hydroxy-N-isopropylpyrrolo[1,5-a]pyridine-3-carboxamide (300 mg). MS m/z(ESI):297.2$[M+H]^+$.

**Step 4: synthesis of 4-hydroxy-N-isopropyl-6-(1-methyl-1H-pyrazol-4-yl)pyrazolo[1,5-a]pyridine-3-carboxamide**

6-bromo-4-hydroxy-N-isopropylpyrrolo[1,5-a]pyridine-3-carboxamide (300 mg, 1.01mmol), tetrakistriphenylphosphine palladium (117 mg, 0.101mmol), sodium carbonate (321 mg, 3.03 mmol), and 1-methyl-4-(4,4,5,5-tetramethyl-1,3,2-dioxolane-2-yl)-pyrazole (252mg, 1.21 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 200 mg of 4-hydroxy-N-isopropyl-6-(1-methyl-1H-pyrazol-4-yl)pyrazolo[1,5-a]pyridine-3-carboxamide. MS m/z(ESI):300.1$[M+H]^+$.

**Step 5: synthesis of 3-(isopropylcarbonyl)-6-(1-methyl-1H-pyrazol-4-yl)pyrazolo[1,5-a]pyridin-4-yl trifluoromethanesulfonate**

4-hydroxy-N-isopropyl-6-(1-methyl-1H-pyrazol-4-yl)pyrazolo[1,5-a]pyridine-3-carboxamide (200 mg, 0.67 mmol), N-phenylbis(trifluoromethanesulfonyl)imide (287 mg, 0.802mmol) and N,N-diisopropylethylamine (0.24 mL, 1.34

mmol) were dissolved in DMA (20 mL), and the resulting reaction solution was stirred at room temperature for 16 h to react. After the reaction was found to be completed from the monitoring, water was added to quench the reaction, extraction with EA (30 mL*2) was carried out, and the organic phase was washed with water and then dried, concentrated, and subjected to column chromatography to obtain 150 mg of 3-(isopropylcarbonyl)-6-(1-methyl-1H-pyrazol-4-yl)pyrazolo[1,5-a]pyridin-4-yl trifluoromethanesulfonate. MS m/z(ESI):432.2[M+H]+.

**Step 6: synthesis of N-isopropyl-4-(6-(4-((6-methoxypyridin-3-yl)methyl)piperazin-1-yl)pyridin-3-yl)-6-(1-methyl)yl-1H-pyrazol-4-yl)pyrazolo[1,5-a]pyridine-3-carboxamide**

3-(isopropylcarbonyl)-6-(1-methyl-1H-pyrazol-4-yl)pyrazolo[1,5-a]pyridin-4-yl trifluoromethanesulfonate (150 mg, 0.348 mmol), 1-((6-methoxypyridin-3-yl)methyl)-4-(5-(4,4,5,5-tetramethyl-1,3,2-dioxolane-2 -yl)pyridin-2-yl)piperazine (171 mg, 0.418mmol), sodium carbonate (185 mg, 1.74mmol), tris(dibenzylideneacetone)dipalladium (32 mg, 0.035mmol), and 2-dicyclohexylphosphorus-2,4,6-triisopropylbiphenyl (33.2 mg, 0.070mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 40 mg of N-isopropyl-4-(6-(4-((6-methoxypyridin-3-yl)methyl)piperazin-1-yl)pyridin-3-yl)-6-(1-methyl)yl-1H-pyrazol-4-yl)pyrazolo[1,5-a]pyridine-3-carboxamide (**compound 5**). MS m/z(ESI):566.1[M+H]+.

$^1H$ NMR(500 MHz, DMSO-d6) δ 9.00(d, J = 1.5 Hz, 1H), 8.34(s, 1H), 8.22(d, J = 2.5 Hz, 1H), 8.12(s, 1H), 8.08(d, J = 2.4 Hz, 1H), 8.07(d, J = 0.8 Hz, 1H), 7.67(dd, J = 8.5, 2.4 Hz, 1H), 7.57(dd, J = 8.8, 2.5 Hz, 1H), 7.53 - 7.47(m, 2H), 6.85(d, J = 8.9 Hz, 1H), 6.81(d, J = 8.5 Hz, 1H), 3.87(s, 3H), 3.84(s, 3H), 3.63(dq, J = 13.5, 6.6 Hz, 1H), 3.54(t, J = 5.0 Hz, 4H), 3.48(s, 2H), 2.46(t, J = 5.0 Hz, 4H), 0.86(s, 3H), 0.85(s, 3H).

**Example 6: Synthesis of compound 6**

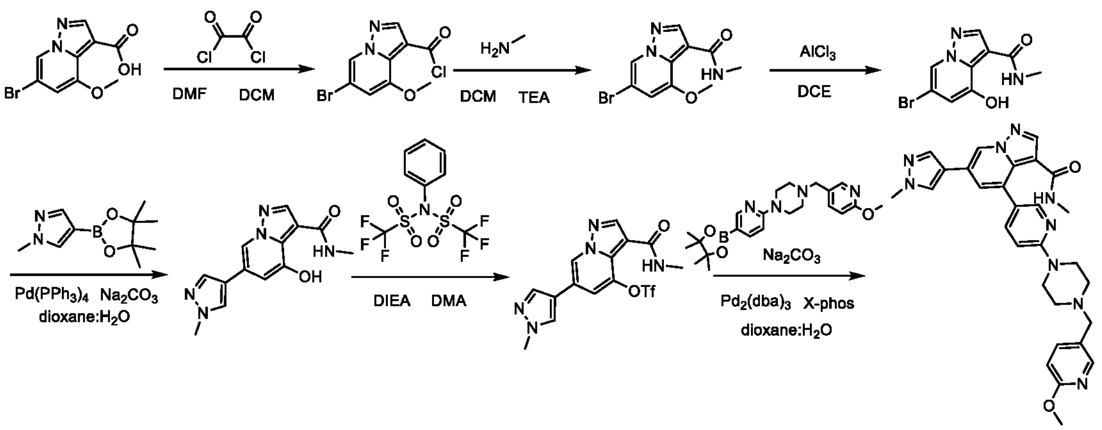


**Step 1: synthesis of 6-bromo-4-methoxypyrazolo[1,5-a]pyridine-3-carbonyl chloride**

6-bromo-4-methoxypyrazolo[1,5-a]pyridine-3-carboxylic acid (400 mg) was dissolved in dichloromethane (10 mL), and then oxalyl chloride (282 mg) was slowly added dropwise, and 2 drops of DMF were then added. The resulting reaction solution was stirred at room temperature for 1 h to react. After the reaction was found to be completed from the monitoring of LCMS, the reaction solution was concentrated to obtain 6-bromo-4-methoxypyrazolo[1,5-a]pyridine-3-carbonyl chloride (430 mg).

**Step 2: synthesis of 6-bromo-4-methoxy-N-methylpyrazolo[1,5-a]pyridine-3-carboxamide**

6-bromo-4-methoxypyrazolo[1,5-a]pyridine-3-carbonyl chloride (430 mg) was dissolved in dichloromethane (10 mL), and then methylamine (59 mg) in tetrahydrofuran was added, and triethylamine (448 mg) was then added dropwise. The resulting reaction solution was stirred at room temperature for 1 h to react. After the reaction was found to be completed from the monitoring of LCMS, water (10 mL) was added to quench the reaction and then the reaction solution was washed with water, dried, concentrated and subjected to column chromatography to obtain 6-bromo-4-methoxy-N-methylpyrazolo[1,5-a]pyridine-3-carboxamide (350 mg). MS m/z(ESI):284.2[M+H]$^+$.

**Step 3: synthesis of 6-bromo-4-hydroxy-N-methylpyrazolopyridine-3-carboxamide**

6-bromo-4-methoxy-N-methylpyrazolo[1,5-a]pyridine-3-carboxamide (350 mg) was dissolved in 1,2-dichloroethane (10 mL), and then aluminium trichloride (490 mg) was added, and the resulting reaction solution was stirred at room temperature for 16 h to react. After the reaction was found to be completed from the monitoring of LCMS, water (20 mL) was added to quench the reaction and then the reaction solution was extracted with dichloromethane, washed with water, and then dried and concentrated to obtain 6-bromo-4-hydroxy-N-methylpyrazolopyridine-3-carboxamide (300 mg). MS m/z(ESI):270.7[M+H]$^+$.

**Step 4: synthesis of 4-hydroxy-N-methyl-6-(1-methyl-1H-pyrazol-4-yl)pyrazolo [1,5-a]pyridine-3-carboxamide**

6-bromo-4-hydroxy-N-methylpyrazolopyridine-3-carboxamide (300 mg, 1.12mmol), tetrakistriphenylphosphine palladium (129 mg, 0.111mmol), sodium carbonate (355 mg, 3.35 mmol), and 1-methyl-4-(4,4,5,5-tetramethyl-1,3,2-dioxolane-2-yl)-pyrazole (279mg, 1.34 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 260 mg of 4-hydroxy-N-methyl-6-(1-methyl-1H-pyrazol-4-yl)pyrazolo[1,5-a]pyridine-3-carboxamide. MS m/z(ESI):272.4[M+H]$^+$.

**Step 5: synthesis of 6-(1-methyl-1H-pyrazol-4-yl)-3-(methylcarbamoyl)pyrazolo[1,5-a]pyridin-4-yl trifluoromethanesulfonate**

4-hydroxy-N-methyl-6-(1-methyl-1H-pyrazol-4-yl)pyrazolo[1,5-a]pyridine-3-carboxamide (260 mg, 0.96 mmol), N-phenylbis(trifluoromethanesulfonyl)imide (411 mg, 1.15mmol) and N,N-diisopropylethylamine (0.34 mL, 1.92 mmol) were dissolved in DMA (20 mL), and the resulting reaction solution was stirred at room temperature for 16 h to react. After the reaction was found to be completed from the monitoring, water was added to quench the reaction, extraction with EA (30 mL*2) was carried out, and the organic phase was washed with water and then dried, concentrated, and subjected to column chromatography to obtain 200 mg of 6-(1-methyl-1H-pyrazol-4-yl)-3-(methylcarbamoyl)pyrazolo[1,5-a]pyridin-4-yl trifluoromethanesulfonate. MS m/z(ESI):404.3 [M+H]$^+$.

**Step 6: synthesis of 4-(6-(4-((6-methoxypyridin-3-yl)methyl)piperazin-1-yl)pyridin-3-yl)-N-methyl-6-(1-methyl-1H-pyrazol-4-yl)pyrazolo[1,5-a]pyridine-3-carboxamide**

6-(1-methyl-1H-pyrazol-4-yl)-3-(methylcarbamoyl)pyrazolo[1,5-a]pyridin-4-yl trifluoromethanesulfonate (200 mg, 0.496 mmol), 1-((6-methoxypyridin-3-yl)methyl)-4-(5-(4,4,5,5-tetramethyl-1,3,2-dioxolane-2 -yl)pyridin-2-yl)piperazine (244 mg, 0.596mmol), sodium carbonate (263 mg, 2.48mmol), tris(dibenzylideneacetone)dipalladium (45mg, 0.0496mmol), and 2-dicyclohexylphosphorus-2,4,6-triisopropylbiphenyl (47 mg, 0.099mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 60 mg of 4-(6-(4-((6-methoxypyridin-3-yl)methyl)piperazin-1-yl)pyridin-3-yl)-N-methyl-6-(1-methyl)-1H-pyrazol-4-yl)pyrazolo[1,5-a]pyridine-3-carboxamide (**compound 6**). MS m/z(ESI):538.2[M+H]$^+$.

$^1$H NMR(400 MHz, DMSO-d6) δ 9.03(d, J = 1.5 Hz, 1H), 8.35(s, 1H), 8.21(d, J = 2.5 Hz, 1H), 8.16(s, 1H), 8.09(s, 1H), 8.08(d, J = 0.8 Hz, 1H), 7.68(d, J = 8.5 Hz, 1H), 7.61(d, J = 4.8 Hz, 1H), 7.56(s, 1H), 7.53(d, J = 1.5 Hz, 1H), 6.86(d, J = 8.9 Hz, 1H), 6.82(d, J = 8.4 Hz, 1H), 3.88(s, 3H), 3.85(s, 3H), 3.56(s, 4H), 3.49(s, 2H), 2.47(s, 4H), 2.39(d, J = 4.6 Hz, 3H).

**Example 7: Synthesis of compound 7**

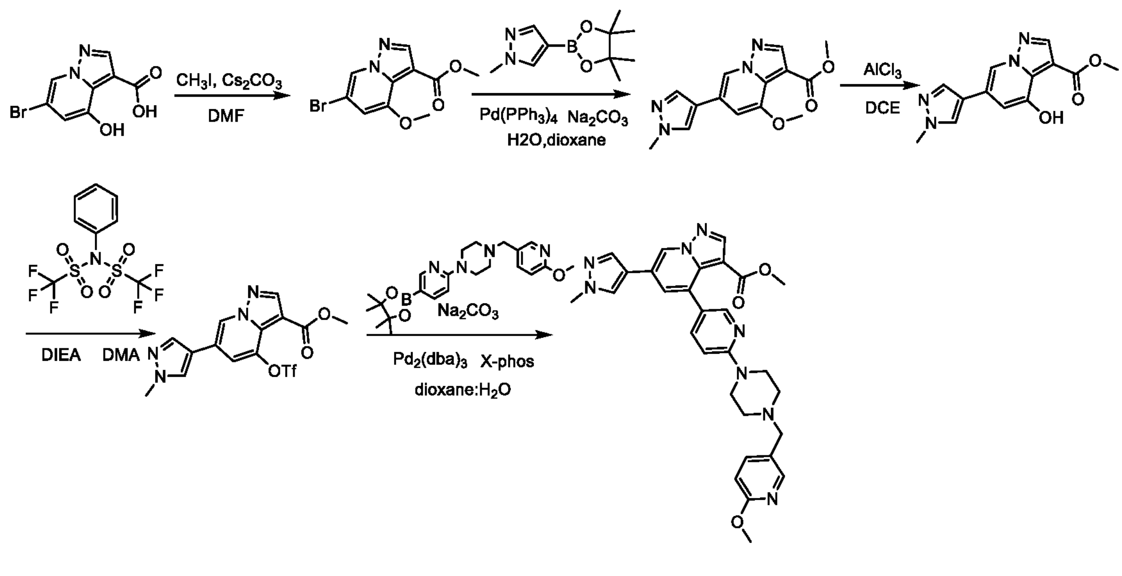


**Step 1: synthesis of methyl 6-bromo-4-methoxypyrazolopyridine-3-carboxylate**

6-bromo-4-hydroxypyrazolopyridine-3-carboxylic acid (1.8 g, 7.04 mmol) was dissolved in DMF (25 mL), and then cesium carbonate (4.59 g, 14.08 mmol) and iodomethane (0.57 mL), 9.15 mmol) were added in sequence, and the resulting reaction solution was stirred at room temperature for 16 h to react. After the reaction was found to be completed from the monitoring of LCMS, water (30 mL) was added and then the reaction solution was extracted with ethyl acetate, washed with water, and then dried, concentrated to obtain methyl 6-bromo-4-methoxypyrazolopyridine-3-carboxylate (1.8 g). MS m/z(ESI):285.2$[M+H]^+$.

**Step 2: synthesis of 4-methoxy-6-(1-methyl-1H-pyrazol-4-yl)pyrazolo[1,5-a]pyridine-3-methyl formate**

Methyl 6-bromo-4-methoxypyrazolopyridine-3-carboxylate (600 mg, 2.11mmol), tetrakistriphenylphosphine palladium (244 mg, 0.211mmol), sodium carbonate (672 mg, 6.34 mmol), and 1-methyl-4-(4,4,5,5-tetramethyl-1,3,2-dioxolane-2-yl)-pyrazole (527 mg, 2.54 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 600 mg of 4-methoxy-6-(1-methyl-1H-pyrazol-4-yl)pyrazolo[1,5-a]pyridine-3-methyl formate. MS m/z(ESI):287.3 $[M+H]^+$.

**Step 3: Synthesis of 4-hydroxy-6-(1-methyl-1H-pyrazol-4-yl)pyrazolo[1,5-a]pyridine-3-methyl formate**

4-methoxy-6-(1-methyl-1H-pyrazol-4-yl)pyrazolo[1,5-a]pyridine-3-methyl formate (600 mg, 2.10 mmol) was dissolved in 1,2-dichloroethane (20 mL), and then aluminum trichloride (977 mg, 7.34 mmol) was added, and the resulting reaction solution was stirred at room temperature for 16 h to react. After the reaction was found to be completed from the monitoring of LCMS, water (20 mL) was added to quench the reaction and then the reaction solution was extracted with dichloromethane, washed with water, and then dried, concentrated to obtain methyl 4-hydroxy-6-(1-methyl-1H-pyrazol-4-yl)pyrazolo[1,5-a]pyridine-3-methyl formate (300 mg). MS m/z(ESI):273.0$[M+H]^+$.

**Step 4: Synthesis of methyl 6-(1-methyl-1H-pyrazol-4-yl)-4-((trifluoromethyl)sulfonyl)oxy)pyrazolo[1,5-a]pyridine-3-carboxylate**

Methyl 4-hydroxy-6-(1-methyl-1H-pyrazol-4-yl)pyrazolo[1,5-a]pyridine-3-formate (300 mg, 1.1 mmol), N-phenylbis(trifluoromethanesulfonyl)imide (473 mg, 1.32mmol) and N,N-diisopropylethylamine (0.36 mL, 2.2 mmol) were dissolved in DMA (20 mL), and the resulting reaction solution was stirred at room temperature for 16 h to react. After the reaction was found to be completed from the monitoring, water was added to quench the reaction, extraction with EA (30 mL*2) was carried out, and the organic phase was washed with water and then dried, concentrated, and subjected to column chromatography to obtain 220 mg of methyl 6-(1-methyl-1H-pyrazol-4-yl)-4-((trifluoromethyl)sulfonyl)oxy)pyrazolo[1,5-a]pyridine-3-carboxylate. MS m/z(ESI):405.5 $[M+H]^+$.

**Step 5: Methyl 4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carboxylate**

Methyl 6-(1-methyl-1H-pyrazol-4-yl)-4-((trifluoromethyl)sulfonyl)oxy)pyrazolo[1,5-a]pyridine-3-carboxylate (220 mg, 0.54 mmol), 1-((6-methoxypyridin-3-yl)methyl)-4-(5-(4,4,5,5-tetramethyl-1,3,2-dioxolane-2-yl)pyridin-2-yl)piperazine (266 mg, 0.65mmol), sodium carbonate (286 mg, 2.7 mmol), tris(dibenzylideneacetone)dipalladium (49.5 mg, 0.054 mmol), and 2-dicyclohexylphosphorus-2,4,6-triisopropylbiphenyl (52 mg, 0.109mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 320 mg of methyl 4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carboxylate (**compound 7**). MS m/z(ESI):539.3[M+H]$^+$.

$^1$H NMR(400 MHz, DMSO-d6) δ 9.13(d, J = 1.5 Hz, 1H), 8.42(s, 1H), 8.36(d, J = 0.7 Hz, 1H), 8.20 - 8.15(m, 1H), 8.09(d, J = 0.8 Hz, 1H), 8.08 - 8.05(m, 1H), 7.69 - 7.63(m, 2H), 7.55(dd, J = 8.8, 2.5 Hz, 1H), 6.86(d, J = 8.9 Hz, 1H), 6.80(dd, J = 8.5, 0.7 Hz, 1H), 3.86(s, 3H), 3.83(s, 3H), 3.54(t, J = 4.9 Hz, 3H), 3.47(s, 2H), 3.37(s, 4H), 2.45(d, J = 5.0 Hz, 4H).

**Example 8: Synthesis of compound 8**

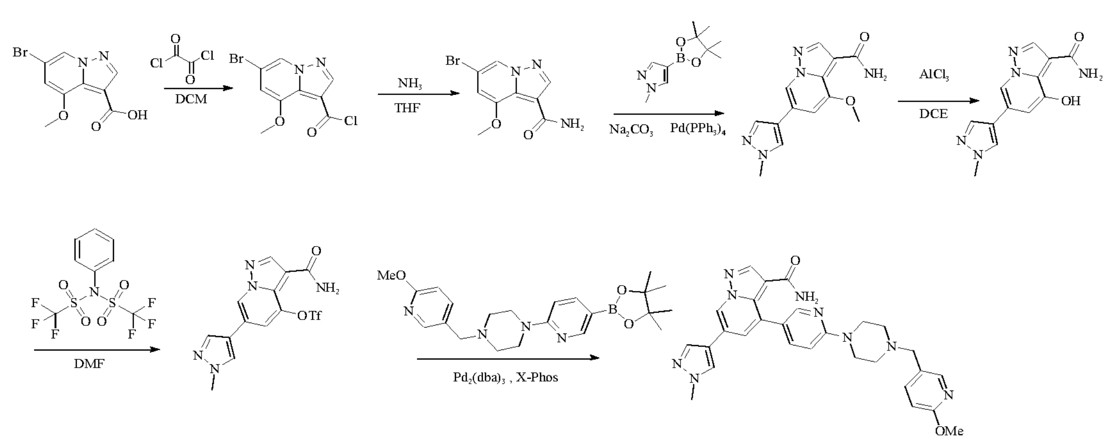


**Step 1: synthesis of 6-bromo-4-methoxypyrazolo[1,5-a]pyridine-3-formyl chloride**

6-bromo-4-methoxypyrazolo[1,5-a]pyridine-3-carboxylic acid (0.6 g, 2.23 mmol) was dissolved in dichloromethane (15 mL) and then oxalyl chloride (0.56 g, 4.46 mmol) was added dropwise to the resulting solution at 0 °C. The reaction solution reacted at room temperature for 2 h. After the reaction was found to be completed from the monitoring, the reaction solution was directly spin-dried and used for next step.

**Step 2: synthesis of 6-bromo-4-methoxypyrazolo[1,5-a]pyridine-3-carboxamide**

6-bromo-4-methoxypyrazolo[1,5-a]pyridine-3-formyl chloride, i.e., the product obtained in the previous step, was dissolved in tetrahydrofuran (10 mL) and then ammonia (1 mL) was added to the resulting solution at 0 °C , and the reaction slution was then stirred at room temperature for 1 h. After the reaction was completed, most of the reaction solution was spin-dried, extraction with dichloromethane (30 mL*2.) was carried out, the organic phase was washed with water and then dried, concentrated, and subjected to column chromatography to obtain 0.4 g of 6-bromo-4-methoxypyrazolo[1,5-a]pyridine-3-carboxamide. MS m/z(ESI):270.1 [M+H]$^+$.

**Step 3: synthesis of 4-methoxy-6-(1-methyl-1H-pyrazol-4-yl)pyrazolo[1,5-a]pyridine-3-carboxamide**

6-bromo-4-methoxypyrazolo[1,5-a]pyridine-3-carboxamide (400 mg, 1.48 mmol), tetrakistriphenylphosphine palladium (170 mg, 0.148mmol), sodium carbonate (470 mg, 4.44 mmol), and 1-methyl-4-(4,4,5,5-tetramethyl-1,3,2-dioxolane-2-yl)-pyrazole (462 mg, 2.22 mmol) were dissolved in dioxane (15 mL) and water (5 mL), and the resulting

reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 0.2 g of 4-methoxy-6-(1-methyl-1H-pyrazol-4-yl)pyrazolo[1,5-a]pyridine-3-carboxamide. MS m/z(ESI):272.3 $[M+H]^+$.

**Step 4: synthesis of 4-hydroxy-6-(1-methyl-1H-pyrazol-4-yl)pyrazolo[1,5-a]pyridine-3-carboxamide**

4-methoxy-6-(1-methyl-1H-pyrazol-4-yl)pyrazolo[1,5-a]pyridine-3-carboxamide (0.4 g, 1.47 mmol) was dissolved in 1,2-dichloroethane (20 mL), and then aluminum trichloride (0.7 g, 2.98 mmol) was added to the resulting solution. The reaction solution was stirred at room temperature for 4 h, water (10 mL) was added to quench the reaction, extraction with DCM (30 mL*3) was then carried out, and the organic phase was washed with water and then dried, concentrated, and subjected to column chromatography to obtain 0.3 g of 4-hydroxy-6-(1-methyl-1H-pyrazol-4-yl)pyrazolo[1,5-a]pyridine-3-carboxamide. MS m/z(ESI):258.3 $[M+H]^+$.

**Step 5: synthesis of 3-carbamoyl-6-(1-methyl-1H-pyrazol-4-yl)pyrazolo[1,5-a]pyridin-4-yltrifluoromethanesulfonate**

4-hydroxy-6-(1-methyl-1H-pyrazol-4-yl)pyrazolo[1,5-a]pyridine-3-carboxamide (0.2 g, 0.78 mmol), N-phenylbis(trifluoromethanesulfonyl)imide (0.33 g, 0.93mmol) and N,N-diisopropylethylamine (0.2 g, 1.56 mmol) were dissolved in DMA (10 mL), and the resulting reaction solution was stirred at room temperature for 8 h to react. After the reaction was found to be completed from the monitoring, water was added to quench the reaction, extraction with EA (30 mL*2) was carried out, and the organic phase was washed with water and then dried, concentrated, and subjected to column chromatography to obtain 0.3 g of 3-carbamoyl-6-(1-methyl-1H-pyrazol-4-yl)pyrazolo[1,5-a]pyridin-4-yltrifluoromethanesulfonate. MS m/z(ESI):390.2$[M+H]^+$.

**Step 6: Synthesis of 4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carboxamide**

3-carbamoyl-6-(1-methyl-1H-pyrazol-4-yl)pyrazolo[1,5-a]pyridin-4-yl trifluoromethanesulfonate (0.2 g, 0.52 mmol), 1-((6-methoxypyridin-3-yl)methyl)-4-(5-(4,4,5,5-tetramethyl-1,3,2-dioxolane-2-yl)pyridin-2-yl)piperazine (0.25 mg, 0.62mmol), sodium carbonate (0.17 mg, 1.56 mmol), tris(dibenzylideneacetone)dipalladium (48 mg, 0.052 mmol), and 2-dicyclohexylphosphorus-2,4,6-triisopropylbiphenyl (50 mg, 0.104 mmol) were dissolved in dioxane (15 mL) and water (5 mL), and the reaction solution was stirred at 80 °C overnight. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 120 mg of 4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carboxamide **(compound 8).** MS m/z(ESI):524.3$[M+H]^+$.

$^1$H NMR(500 MHz, DMSO) δ 8.89(s, 1H), 8.35(s, 1H), 8.21(d, *J*= 2.1 Hz, 1H), 8.12(s, 1H), 8.04(s, 1H), 7.86(s, 1H), 7.65(dd, *J* = 30.6, 7.0 Hz, 2H), 7.40(d, *J* = 1.2 Hz, 1H), 7.10(s, 1H), 6.89 - 6.76(m, 2H), 5.72(d, *J*= 4.4 Hz, 1H), 3.89(d, J= 8.8 Hz, 6H), 3.57(d, *J*= 30.1 Hz, 5H), 2.50(d, *J* = 10.0 Hz, 5H).

**Example 9: Synthesis of compound 9**

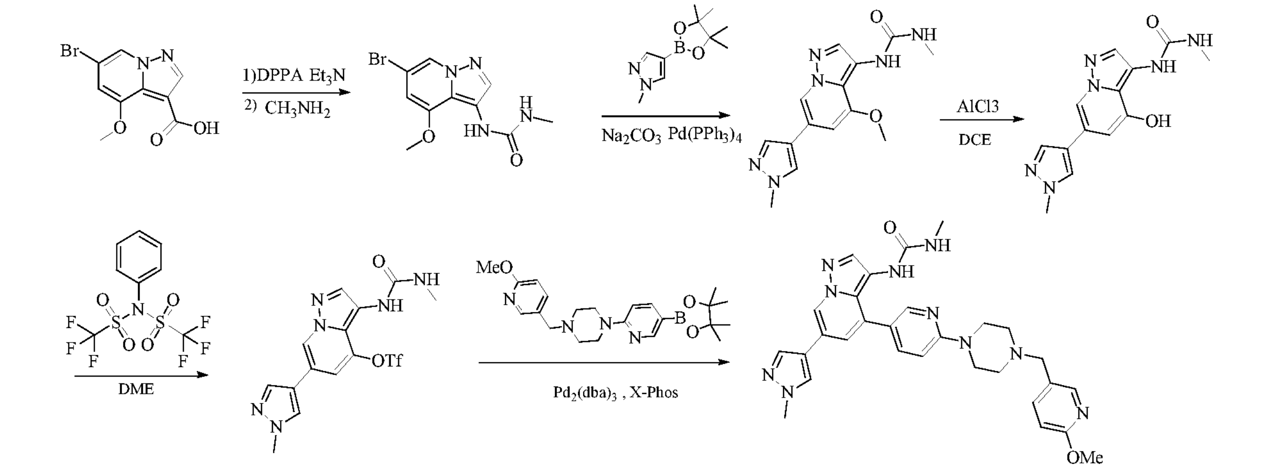


**Step 1: synthesis of 1-(6-bromo-4-methoxypyrazolo[1,5-a]pyridin-3-yl)-3-methylurea**

6-Bromo-4-methoxypyrazolo[1,5-a]pyridine-3-carboxylic acid (0.55 g, 2.04 mmol), diphenylphosphoryl azide (0.84 g, 3.06 mmol), and triethyl The amine (1.23 g, 12.24 mmol) were dissolved in toluene (20 mL), the resulting reaction solution was then stirred at 50 °C for 6 h, methylamine hydrochloride (0.55 g, 8.16 mmol) was then added, and the mixed solution was refluxed overnight. After the reaction was found to be completed from the monitoring, most of the solvent was concentrated, extraction with DCM (30 mL*2) was then carried out, and the organic phase was washed with water and then dried, concentrated, and subjected to column chromatography to obtain 0.4 g of 1-(6-bromo-4-methoxypyrazolo[1,5-a]]pyridin-3-yl)-3-methylurea. MS m/z(ESI):300.0 $[M+H]^+$.

**Step 2: synthesis of 1-(4-methoxy-6-(1-methyl-1H-pyrazol-4-yl)pyrazolo[1,5-a]pyridin-3-yl)-3-methylurea**

1-(6-bromo-4-methoxypyrazolo[1,5-a]]pyridin-3-yl)-3-methylurea (400 g, 1.33 mmol), tetrakistriphenylphosphine palladium (153 mg, 0.133 mmol), sodium carbonate (422 mg, 3.99 mmol), and 1-methyl-4-(4,4,5,5-tetramethyl-1,3,2-dioxolane-2-yl)-pyrazole (332 mg, 1.6 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 0.3 g of 1-(4-methoxy-6-(1-methyl-1H-pyrazol-4-yl)pyrazolo[1,5-a]pyridin-3-yl)-3-methylurea. MS m/z(ESI):300.1 $[M+H]^+$.

**Step 3: synthesis of 1-(4-hydroxy-6-(1-methyl-1H-pyrazol-4-yl)pyrazolo[1,5-a]pyridin-3-yl)-3-methylurea**

1-(4-methoxy-6-(1 -methyl-1H-pyrazol-4-yl)pyrazolo[1,5-a]pyridin-3-yl)-3-methylurea (0.3 g, 1.0 mmol) was dissolved in 1,2-dichloroethane (20 mL), and then aluminum trichloride (0.49 g, 2.0 mmol) was added to the resulting solution. The reaction solution was stirred at room temperature for 4 h, water (10 mL) was added to quench the reaction, extraction with DCM (30 mL*3) was then carried out, and the organic phase was washed with water and then dried, concentrated, and subjected to column chromatography to obtain 0.2 g of 1-(4-hydroxy-6-(1-methyl-1H-pyrazol-4-yl)pyrazolo[1,5-a]pyridin-3-yl)-3-methylurea. MS m/z(ESI):287.3$[M+H]^+$.

**Step 4: synthesis of 6-(1-methyl-1H-pyrazol-4-yl)-3-(3-methylureido)pyrazolo[1,5-a]pyridin-4-yl trifluoromethanesulfonate**

1 -(4-hydroxy-6-(1-methyl-1H-pyrazol-4-yl)pyrazolo[1,5-a]pyridin-3-yl)-3-methylurea (0.2 g, 0.70 mmol), N-phenylbis(trifluoromethanesulfonyl)imide (0.38 g, 1.04 mmol) and N,N-diisopropylethylamine (0.18 mL, 1.4 mmol) were dissolved in DMA (10 mL), and the resulting reaction solution was stirred at room temperature for 8 h to react. After the reaction was found to be completed from the monitoring, water was added to quench the reaction, extraction with EA (30 mL*2) was carried out, and the organic phase was washed with water and then dried, concentrated, and subjected to column chromatography to obtain 0.25 g of 6-(1-methyl-1H-pyrazol-4-yl)-3-(3-methylureido)pyrazolo[1,5-a]pyridin-4-yltrifluoromethanesulfonate. MS m/z(ESI):419.4$[M+H]^+$.

**Step 5: Synthesis of 1-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)-3-methylurea**

6-(1-methyl-1H-pyrazol-4-yl)-3-(3-methylureido)pyrazolo[1,5-a]pyridin-4-yl trifluoromethanesulfonate (0.25 g, 0.6 mmol), 1-((6-methoxypyridin-3-yl)methyl)-4-(5-(4,4,5,5-tetramethyl-1,3,2-dioxolane-2 -yl)pyridin-2-yl)piperazine (0.3 g, 0.72 mmol), sodium carbonate (0.19 g, 1.8 mmol), tris(dibenzylideneacetone)dipalladium (55 mg, 0.06 mmol), and 2-dicyclohexylphosphorus-2,4,6-triisopropylbiphenyl (58 mg, 0.12 mmol) were dissolved in dioxane (15 mL) and water (5 mL), and the resulting reaction solution was stirred at 80 °C overnight to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 100 mg of 1-(4-(6-(4-((6-methoxypyridin-3-yl)methyl)piperazin-1-yl)pyridin-3-yl)-6-(1-methyl-1H -pyrazol-4-yl)pyrazolo[1,5-a]pyridin-3-yl)-3-methylurea **(compound 9).** MS m/z(ESI):553.1[M+H]$^+$.

$^1$H NMR(500 MHz, DMSO) δ 8.87(s, 1H), 8.30(s, 1H), 8.24(d, J= 2.1 Hz, 1H), 8.10(s, 1H), 8.04(s, 1H), 7.86(s, 1H), 7.65(dd, *J* = 30.6, 7.0 Hz, 2H), 7.30(d, *J* = 1.2 Hz, 1H), 7.10(s, 1H), 6.89 - 6.76(m, 2H), 5.72(d, *J*= 4.4 Hz, 1H), 3.86(d, J= 8.8 Hz, 6H), 3.52(d, *J*= 30.1 Hz, 5H), 2.49(d, *J* = 10.0 Hz, 5H), 2.43(d, *J* = 4.5 Hz, 3H).

**Example 10: Synthesis of compound 10**

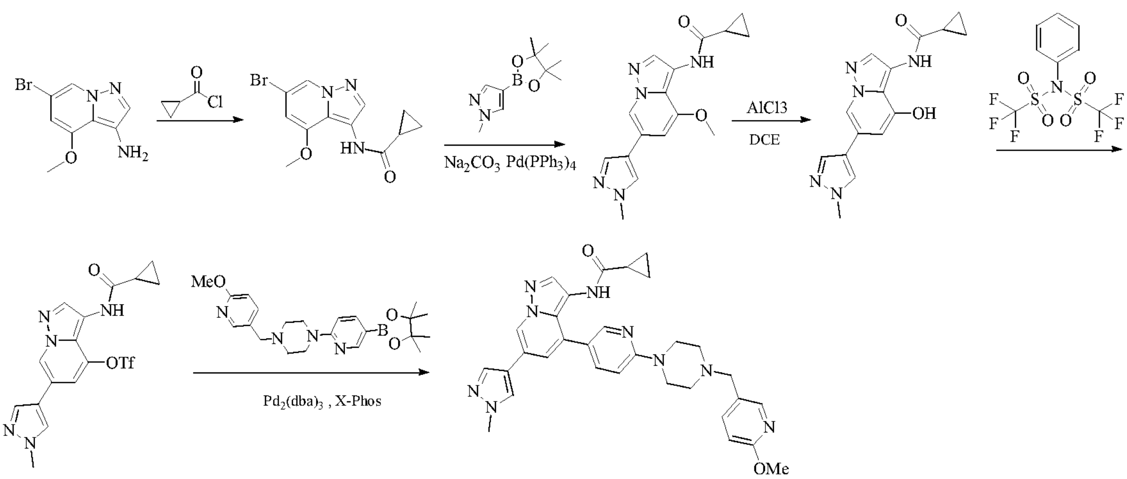


**Step 1: synthesis of N-(6-bromo-4-methoxypyrazolo[1,5-a]pyridin-3-yl)cyclopropanecarboxamide**

6-bromo-4-methoxypyrazolo[1,5-a]pyridin-3-amine (0.5 g, 2.07 mmol) and triethylamine (0.42 g, 4.14 mmol) were dissolved in dichloromethane (15 mL), and then cyclopropanecarbonyl chloride (0.26 g, 2.5 mmol) was added dropwise in the resulting solution in an ice bath, and the reaction solution reacted at room temperature for 1 h. After the reaction was found to be completed from the monitoring, the reaction solution was cooled, water was added to quench the reaction, extraction with DCM (20 mL*2) was then carried out, and the organic phase was washed with water and then dried, concentrated and subjected to column chromatography to obtain 450 mg of N-(6-bromo-4-methoxypyrazolo[1,5-a]pyridin-3-yl)cyclopropanecarboxamide. MS m/z(ESI):310.1 [M+H]$^+$.

**Step 2: synthesis of N-(4-methoxy-6-(1-methyl-1H-pyrazol-4-yl)pyrazolo[1,5-a]pyridin-3-yl)cyclopropanecarboxamide**

N-(6-bromo-4-methoxypyrazolo[1,5-a]pyridin-3-yl)cyclopropanecarboxamide (0.45 g, 1.45 mmol), tetrakistriphenylphosphine palladium (167 mg, 0.145 mmol), sodium carbonate (0.46 g, 4.35 mmol), and 1-methyl-4-(4,4,5,5-tetramethyl-1,3,2-dioxolane-2-yl)-pyrazole (0.36 g, 1.7 mmol) were dissolved in dioxane (15 mL) and water (5 mL), and the resulting reaction solution was stirred at 80 °C for 16 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 0.35 g of N-(4-methoxy-6-(1-methyl-1H-pyrazol-4-yl)pyrazolo[1,5-a]pyridin-3-yl)cyclopropanecarboxamide. MS m/z(ESI):311.2 [M+H]+.

**Step 3: synthesis of N-(4-hydroxy-6-(1-methyl-1H-pyrazol-4-yl)pyrazolo[1,5-a]pyridin-3-yl)cyclopropanecarboxamide**

N-(4-methoxy-6-(1-methyl-1H-pyrazol-4-yl)pyrazolo[1,5-a]pyridin-3-yl)cyclopropanecarboxamide (0.35 g, 1.1 mmol) was dissolved in 1,2-dichloroethane (20 mL), and then aluminum trichloride (0.54 g, 2.2 mmol) was added to the resulting solution. The reaction solution was stirred at room temperature for 4 h, water (10 mL) was added to quench the reaction, extraction with DCM (30 mL*3) was then carried out, and the organic phase was washed with water and then dried, concentrated, and subjected to column chromatography to obtain 0.25 g of N-(4-hydroxy-6-(1-methyl-1H-pyrazol-4-yl)pyrazolo[1,5-a]pyridin-3-yl)cyclopropanecarboxamide. MS m/z(ESI):298.2[M+H]+.

**Step 4: synthesis of 3-(cyclopropanecarboxamido)-6-(1-methyl-1H-pyrazol-4-yl)pyrazolo[1,5-a]pyridin-4-yl trifluoromethanesulfonate**

N-(4-hydroxy-6-(1-methyl-1H-pyrazol-4-yl)pyrazolo[1,5-a]pyridin-3-yl)cyclopropanecarboxamide (0.25 g, 0.84 mmol), N-phenylbis(trifluoromethanesulfonyl)imide (0.36 g, 1.00mmol) and N,N-diisopropylethylamine (0.22 g, 1.68 mmol) were dissolved in DMA (10 mL), and the resulting reaction solution was stirred at room temperature for 8 h to react. After the reaction was found to be completed from the monitoring, water was added to quench the reaction, extraction with EA (30 mL*2) was carried out, and the organic phase was washed with water and then dried, concentrated, and subjected to column chromatography to obtain 0.22 g of 3-(cyclopropanecarboxamido)-6-(1-methyl-1H-pyrazol-4-yl)pyrazolo[1,5-a]pyridin-4-yl trifluoromethanesulfonate. MS m/z(ESI):430.1[M+H]+.

**Step 5: synthesis of N-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)cyclopropanecarboxamide**

3-(cyclopropanecarboxamido)-6-(1-methyl-1H-pyrazol-4-yl)pyrazolo[1,5-a]pyridin-4-yl trifluoromethanesulfonate (0.22 g, 0.51 mmol), 1-((6-methoxypyridin-3-yl)methyl)-4-(5-(4,4,5,5-tetramethyl-1,3,2-dioxolane-2 -yl)pyridin-2-yl)piperazine (0.25 g, 0.62 mmol), sodium carbonate (0.16 g, 1.53 mmol), tris(dibenzylideneacetone)dipalladium (59 mg, 0.051 mmol), and 2-dicyclohexylphosphorus-2,4,6-triisopropylbiphenyl (49 mg, 0.11 mmol) were dissolved in dioxane (15 mL) and water (5 mL), and the resulting reaction solution was stirred at 80 °C overnight to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 80 mg of N-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)cyclopropanecarboxamide **(compound 10).** MS m/z(ESI):564.2[M+H]+.

$^1$H NMR(500 MHz,DMSO) δ 9.27(s, 1H),8.90(d, *J* = 1.3 Hz, 1H), 8.30(s, 1H), 8.19(d, *J* = 2.4 Hz, 1H), 8.10(s, 1H), 8.04(s, 1H), 7.86(s, 1H), 7.68(dd, *J* = 8.5, 1.9 Hz, 1H), 7.56(dd, *J* = 8.8, 2.4 Hz, 1H), 7.34(d, *J*= 1.3 Hz, 1H), 6.90 - 6.75(m, 2H), 3.86(d, *J*= 11.5 Hz, 6H), 3.64 - 3.39(m, 6H), 2.48(s, 4H), 1.46 - 1.37(m, 1H), 0.57 - 0.48(m, 2H), 0.46 - 0.36(m, 2H).

**Example 11: Synthesis of compound 11**

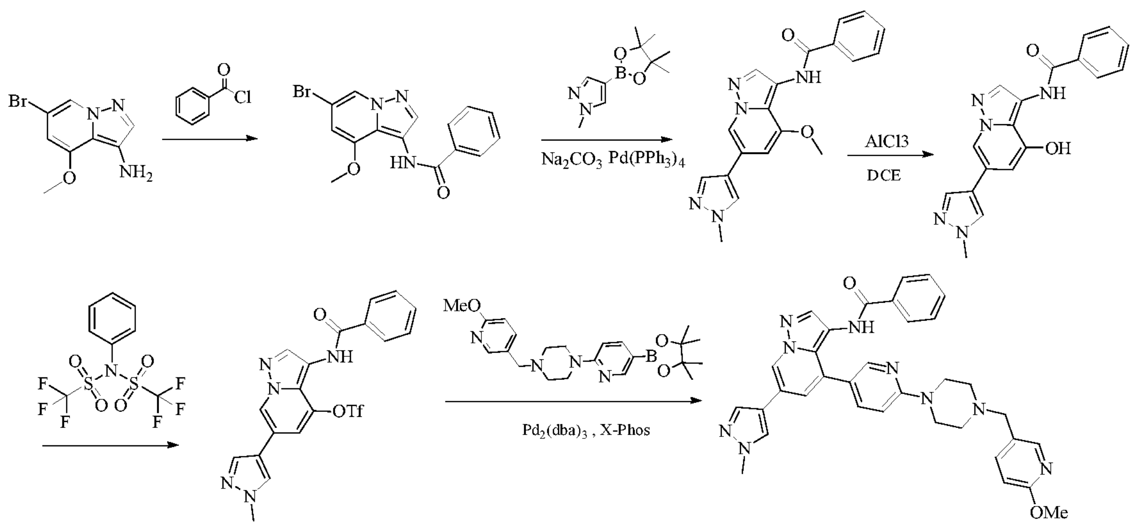


**Step 1: synthesis of N-(6-bromo-4-methoxypyrazolo[1,5-a]pyridin-3-yl)benzamide**

6-bromo-4-methoxypyrazolo[1,5-a]pyridin-3-amine (0.5 g, 2.07 mmol) and triethylamine (0.42 g, 4.14 mmol) were dissolved in dichloromethane (15 mL), and then benzoyl chloride (0.35 g, 2.5 mmol) was added dropwise in the resulting solution in an ice bath, and the reaction solution reacted at room temperature for 1 h. After the reaction was found to be completed from the monitoring, the reaction solution was cooled, water was added to quench the reaction, extraction with DCM (20 mL*2) was then carried out, and the organic phase was washed with water and then dried, concentrated and subjected to column chromatography to obtain 0.5 g of N-(6-bromo-4-methoxypyrazolo[1,5-a]pyridin-3-yl)benzamide. MS m/z(ESI):346.5$[M+H]^+$.

**Step 2: synthesis of N-(4-methoxy-6-(1-methyl-1H-pyrazol-4-yl)pyrazolo[1,5-a]pyridin-3-yl)benzamide**

N-(6-bromo-4-methoxypyrazolo[1,5-a]pyridin-3-yl)benzamide (0.5 g, 1.45 mmol), tetrakistriphenylphosphine palladium (167 mg, 0.145mmol), sodium carbonate (0.46 g, 4.35 mmol), and 1-methyl-4-(4,4,5,5-tetramethyl-1,3,2-dioxolane-2-yl)-pyrazole (0.36 g, 1.7 mmol) were dissolved in dioxane (15 mL) and water (5 mL), and the resulting reaction solution was stirred at 80 °C for 12 h to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 0.41 g of N-(4-methoxy-6-(1-methyl-1H-pyrazol-4-yl)pyrazolo[1,5-a]pyridin-3-yl)benzamide. MS m/z(ESI):348.1 $[M+H]^+$.

**Step 3: synthesis of N-(4-Hydroxy-6-(1-methyl-1H-pyrazol-4-yl)pyrazolo[1,5-a]pyridin-3-yl)benzamide**

N-(4-methoxy-6-(1-methyl-1H-pyrazol-4-yl)pyrazolo[1,5-a]pyridin-3-yl)benzamide (0.42 g, 1.2 mmol) was dissolved in 1,2-dichloroethane (20 mL), and then aluminum trichloride (0.58 g, 2.4 mmol) was added to the resulting solution. The reaction solution was stirred at room temperature for 4 h, water (10 mL) was added to quench the reaction, extraction with DCM (30 mL*3) was then carried out, and the organic phase was washed with water and then dried, concentrated, and subjected to column chromatography to obtain 0.31 g of N-(4-Hydroxy-6-(1-methyl-1H-pyrazol-4-yl)pyrazolo[1,5-a]pyridin-3-yl)benzamide. MS m/z(ESI):334.2$[M+H]^+$.

**Step 4: synthesis of 3-benzamido-6-(1-methyl-1H-pyrazol-4-yl)pyrazolo[1,5-a]pyridin-4-yl trifluoromethanesulfonate**

N-(4-Hydroxy-6-(1-methyl-1H-pyrazol-4-yl)pyrazolo[1,5-a]pyridin-3-yl)benzamide (0.31 g, 0.93 mmol), N-phenylbis(trifluoromethanesulfonyl)imide (0.40 g, 1.12 mmol) and N,N-diisopropylethylamine (0.24 g, 1.86 mmol) were dissolved in DMA (10 mL), and the resulting reaction solution was stirred at room temperature for 8 h to react. After the reaction was found to be completed from the monitoring, water was added to quench the reaction, extraction with EA (30 mL*2) was carried out, and the organic phase was washed with water and then dried, concentrated, and subjected to column chromatography to obtain 0.26 g of 3-benzamido-6-(1-methyl-1H-pyrazol-4-yl)pyrazolo[1,5-a]pyridin-4-yl trifluoromethanesulfonate. MS m/z(ESI):466.0$[M+H]^+$.

**Step 5: Synthesis of N-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)benzamide**

3-benzamido-6-(1-methyl-1H-pyrazol-4-yl)pyrazolo[1,5-a]pyridin-4-yl trifluoromethanesulfonate (0.26 g, 0.56 mmol), 1-((6-methoxypyridin-3-yl)methyl)-4-(5-(4,4,5,5-tetramethyl-1,3,2-dioxolane-2 -yl)pyridin-2-yl)piperazine (0.28 g, 0.67 mmol), sodium carbonate (0.18 g, 1.68 mmol), tris(dibenzylideneacetone)dipalladium (52 mg, 0.056 mmol), and 2-dicyclohexylphosphorus-2,4,6-triisopropylbiphenyl (49 mg, 0.11 mmol) were dissolved in dioxane (15 mL) and water (5 mL), and the resulting reaction solution was stirred at 80 °C overnight to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 90 mg of N-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)benzamide **(compound 11).** MS m/z(ESI):601.5[M+H]$^+$.

$^1$H NMR(500 MHz, DMSO) δ 9.50(s, 1H), 8.96(d, J= 1.3 Hz, 1H), 8.32(s, 1H), 8.23(d, J= 2.4 Hz, 1H), 8.07(dd, *J* = 21.0, 9.0 Hz, 3H), 7.70 - 7.64(m, 1H), 7.64 - 7.57(m, 3H), 7.52(t, *J* = 7.4 Hz, 1H), 7.40(dd, *J* = 10.8, 4.6 Hz, 3H), 6.83(d, *J* = 8.5 Hz, 1H), 6.64(d, *J* = 8.8 Hz, 1H), 3.86(t, J= 9.6 Hz, 6H), 3.54 - 3.35(m, 6H), 2.36(s, 4H).

**Example 12: Synthesis of compound 12**

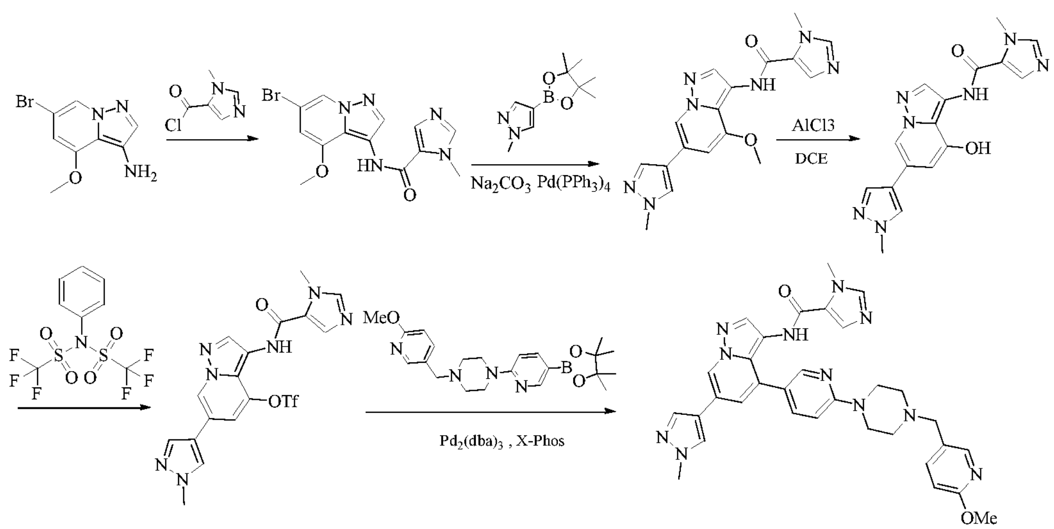


**Step 1: synthesis of N-(6-bromo-4-methoxypyrazolo[1,5-a]pyridin-3-yl)-1-methyl-1H-imidazole-5-carboxamide**

6-bromo-4-methoxypyrazolo[1,5-a]pyridin-3-amine (0.5 g, 2.07 mmol) and triethylamine (0.42 g, 4.14 mmol) were dissolved in dichloromethane (15 mL), and then 1-methyl-1H-imidazole-5-carbonyl chloride (0.36 g, 2.5 mmol) was added dropwise in the resulting solution in an ice bath, and the reaction solution reacted at room temperature for 1 h. After the reaction was found to be completed from the monitoring, the reaction solution was cooled, water was added to quench the reaction, extraction with DCM (20 mL*2) was then carried out, and the organic phase was washed with water and then dried, concentrated and subjected to column chromatography to obtain 0.52 g of N-(6-bromo-4-methoxypyrazolo[1,5-a]pyridin-3-yl)-1-methyl-1H-imidazole-5-carboxamide. MS m/z(ESI):350.7[M+H]$^+$.

**Step 2: synthesis of N-(4-methoxy-6-(1-methyl-1H-pyrazol-4-yl)pyrazolo[1,5-a]pyridin-3-yl)-1-methyl-1H-imidazole-5-carboxamide**

N-(6-bromo-4-methoxypyrazolo[1,5-a]pyridin-3-yl)-1-methyl-1H-imidazole-5-carboxamide (0.52 g, 1.48 mmol), tetrakistriphenylphosphine palladium (171 mg, 0.148mmol), sodium carbonate (0.47 g, 4.44 mmol), and 1-methyl-4-(4,4,5,5-tetramethyl-1,3,2-dioxolane-2-yl)-pyrazole (0.37 g, 1.78 mmol) were dissolved in dioxane (15 mL) and water (5 mL), and the resulting reaction solution was stirred at 80 °C for 12 h to react. After the reaction was found to be completed from the monitoring, the reaction solution was cooled, water was added to quench the reaction, extraction with EA (30 mL*2) was then carried out, and the organic phase was washed with water and then dried, concentrated

and subjected to column chromatography to obtain 0.4 g of N-(4-methoxy-6-(1-methyl-1H-pyrazol-4-yl)pyrazolo[1,5-a]pyridin-3-yl)-1-methyl-1H-imidazole-5-carboxamide. MS m/z(ESI):352.1 [M+H]+.

**Step 3: synthesis of N-(4-hydroxy-6-(1-methyl-1H-pyrazol-4-yl)pyrazolo[1,5-a]pyridin-3-yl)-1-methyl-1H-imidazole-5-carboxamide**

N-(4-methoxy-6-(1-methyl-1H-pyrazol-4-yl)pyrazolo[1,5-a]pyridin-3-yl)-1-methyl-1H-imidazole-5-carboxamide (0.4 g, 1.14 mmol) was dissolved in 1,2-dichloroethane (20 mL), and then aluminum trichloride (0.55 g, 2.28 mmol) was added to the resulting solution. The reaction solution was stirred at room temperature for 4 h, water (10 mL) was added to quench the reaction, extraction with DCM (30 mL*3) was then carried out, and the organic phase was washed with water and then dried, concentrated, and subjected to column chromatography to obtain 0.26 g of N-(4-hydroxy-6-(1-methyl-1H-pyrazol-4-yl)pyrazolo[1,5-a]pyridin-3 -yl)-1-methyl-1H-imidazole-5-carboxamide. MS m/z(ESI):338.2[M+H]+.

**Step 4: synthesis of 3-(1-methyl-1H-imidazole-5-carboxamido)-6-(1-methyl-1H-pyrazol-4-yl)pyrazolo[1,5-a]pyridine-4-trifluoromethanesulfonate**

N-(4-hydroxy-6-(1-methyl-1H-pyrazol-4-yl)pyrazolo[1,5-a]pyridin-3-yl)-1-methyl-1H-imidazole-5-carboxamide (0.26 g, 0.77 mmol), N-phenylbis(trifluoromethanesulfonyl)imide (0.33 g, 0.93 mmol) and N,N-diisopropylethylamine (0.20 g, 1.54 mmol) were dissolved in DMA (10 mL), and the resulting reaction solution was stirred at room temperature for 8 h to react. After the reaction was found to be completed from the monitoring, water was added to quench the reaction, extraction with EA (30 mL*2) was carried out, and the organic phase was washed with water and then dried, concentrated, and subjected to column chromatography to obtain 0.33 g of 3-(1-methyl-1H-imidazole-5-carboxamido)-6-(1-methyl-1H-pyrazol-4-yl)pyrazolo[1,5-a]pyridine-4-trifluoromethanesulfonate. MS m/z(ESI):470.1[M+H]+.

**Step 5: Synthesis of N-(4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-3-yl)-1-methyl-1H-imidazole-5-carboxamide**

3-(1-methyl-1H-imidazole-5-carboxamido)-6-(1-methyl-1H-pyrazol-4-yl)pyrazolo[1,5-a]pyridine-4-trifluoromethanesulfonate (0.33 g, 0.71 mmol), 1-((6-methoxypyridin-3-yl)methyl)-4-(5-(4,4,5,5-tetramethyl-1,3,2-dioxolane-2 -yl)pyridin-2-yl)piperazine (0.35 g, 0.84 mmol), sodium carbonate (0.23 g, 2.13 mmol), tris(dibenzylideneacetone)dipalladium (65 mg, 0.071 mmol), and 2-dicyclohexylphosphorus-2,4,6-triisopropylbiphenyl (68 mg, 0.14 mmol) were dissolved in dioxane (15 mL) and water (5 mL), and the resulting reaction solution was stirred at 80 °C overnight to react. After the reaction was found to be completed from the monitoring, the reaction solution was cooled, water was added to quench the reaction, extraction with EA (30 mL*2) was then carried out, and the organic phase was washed with water and then dried, concentrated and subjected to column chromatography to obtain 100 mg of N-(4-(6-(4-((6-methoxypyridin-3-yl)methyl)piperazin-1-yl)pyridin-3-yl)-6-(1-methyl-1H -pyrazol-4-yl)pyrazolo[1,5-a]pyridin-3-yl)-1-methyl-1H-imidazole-5-carboxamide **(compound 12).** MS m/z(ESI):605.3[M+H]+.

$^1$H NMR(500 MHz, DMSO) δ 9.36(s, 1H), 8.94(d, *J*= 1.3 Hz, 1H), 8.32(s, 1H), 8.21(d, J= 2.4 Hz, 1H), 8.10(d, *J* = 2.1 Hz, 1H), 8.06(s, 1H), 7.99(s, 1H), 7.75 - 7.66(m, 2H), 7.60(dd, *J* = 8.8, 2.5 Hz, 1H), 7.43(s, 1H), 7.39(d, *J* = 1.2 Hz, 1H), 6.83(d, *J* = 8.4 Hz, 1H), 6.62(d, *J* = 8.8 Hz, 1H), 3.87(d, J= 12.9 Hz, 6H), 3.59(s, 3H), 3.43(d, *J*= 46.0 Hz, 6H), 2.40(s, 4H).

**Example 13: Synthesis of compound 13**

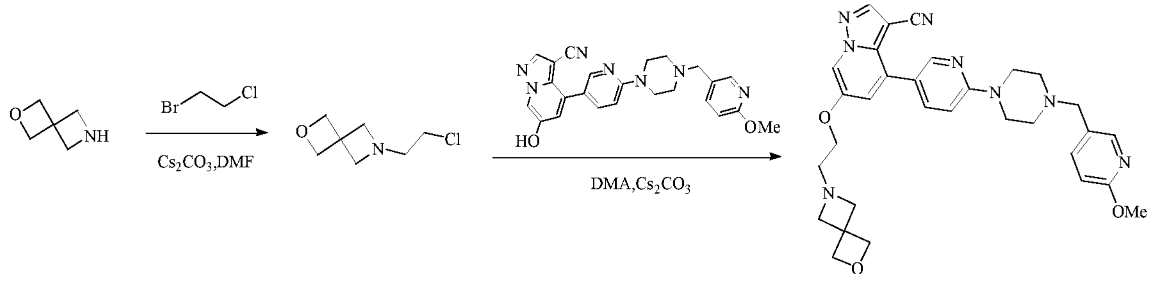

**Step 1: synthesis of 6-(2-chloroethyl)-2-oxa-6-azaspiro[3.3]heptane**

2-oxa-6-azaspiro[3.3]heptane (0.3 g, 3.03 mmol) and cesium carbonate (2.96 g, 9.09 mmol) were added to DMF (5 mL), and then 1-bromo-2-chloroethane (1.3 g, 9.09 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 150 mg of 6-(2-chloroethyl)-2-oxa-6-azaspiro[3.3]heptane. MS m/z(ESI):162.3 $[M+H]^+$.

**Step 2: synthesis of 6-(2-(2-oxa-6-azaspiro[3.3]hept-6-yl)ethoxy)-4-(6-(4-((6-methoxypyridin-3-yl)methyl)piperazin-1-yl)pyridin-3-yl)pyrazolo[1,5-a]pyridine-3-carbonitrile**

6-hydroxy-4-(6-(4-((6-methoxypyridin-3-yl)methyl)piperazin-1-yl)pyridin-3-yl)pyrazolo[1,5-a]pyridine-3-carbonitrile (0.1 g, 0.23 mmol), 6-(2-chloroethyl)-2-oxa-6-azaspiro[3.3]heptane (74 mg, 0.45 mmol), and cesium carbonate (0.15 g, 0.45 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 50 mg of 6-(2-(2-oxa-6-azaspiro[3.3]hept-6-yl)ethoxy)-4-(6-(4-((6-methoxypyridin-3-yl)methyl)piperazin-1-yl)pyridin-3-yl)pyrazolo[1,5-a]pyiidine-3-carbonitrile **(compound 13).** MS m/z(ESI):567.1 $[M+H]^+$.

$^1H$ NMR(500 MHz, DMSO) $\delta$ 8.64(d, $J$= 2.1 Hz, 1H), 8.64(d, $J$= 2.1 Hz, 1H), 8.56(s, 1H), 8.56(s, 1H), 8.32(d, $J$ = 2.5 Hz, 1H), 8.08(d, $J$ = 2.1 Hz, 1H), 7.76(dd, $J$ = 8.8, 2.5 Hz, 1H), 7.67(dd, $J$ = 8.5, 2.4 Hz, 1H), 7.26(d, $J$ = 2.1 Hz, 1H), 6.93(d, $J$ = 8.9 Hz, 1H), 6.81(d, $J$ = 8.5 Hz, 1H), 4.59(s, 4H), 4.04(t, $J$ = 5.3 Hz, 2H), 3.84(s, 3H), 3.55(dd, $J$ = 29.6, 24.6 Hz, 8H), 2.72(t, $J$= 5.2 Hz, 2H), 2.49 - 2.39(m, 5H), 2.08 - 1.88(m, 2H).

**Example 14: Synthesis of compound 14**

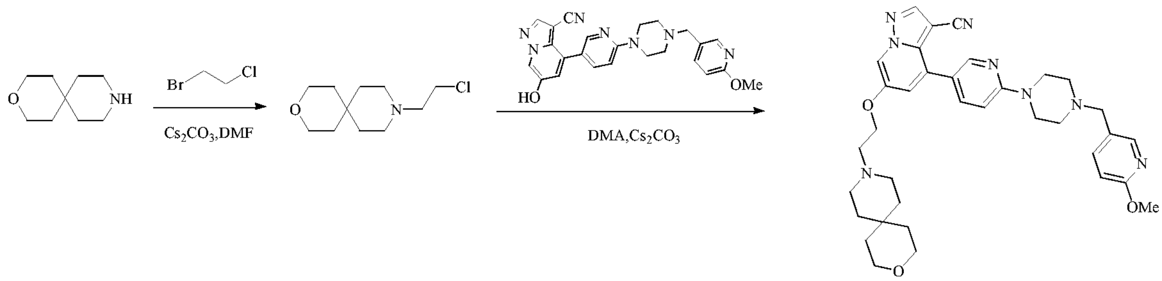


**Step 1: synthesis of 9-(2-chloroethyl)-3-oxa-9-azaspiro[5.5]undecane**

3-oxa-9-azaspiro[5.5]undecane (0.3 g, 1.93 mmol) and cesium carbonate (1.88 g, 5.80 mmol) were added to DMF (5 mL), and then 1-bromo-2-chloroethane (0.83 g, 5.80 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 180 mg of 9-(2-chloroethyl)-3-oxa-9-azaspiro[5.5]undecane. MS m/z(ESI):218.5 $[M+H]^+$.

**Step 2: synthesis of 6-(2-(3-oxa-9-azaspiro[5.5]undec-9-yl)ethoxy)-4-(6-(4-((6-methoxypyridine-3-yl)methyl)piperazin-1-yl)pyridin-3-yl)pyrazolo[1,5-a]pyridine-3-carbonitrile**

6-hydroxy-4-(6-(4-((6-methoxypyridin-3-yl)methyl)piperazin-1-yl)pyridin-3-yl)pyrazolo[1,5-a]pyridine-3-carbonitrile (0.1 g, 0.23 mmol), 9-(2-chloroethyl)-3-oxa-9-azaspiro[5.5]undecane (98 mg, 0.45 mmol), and cesium carbonate (0.15 g, 0.45 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 48 mg of 6-(2-(3-oxa-9-azaspiro[5.5]undec-9-yl)ethoxy)-4-(6-(4-((6-methoxypyridine-3-yl)methyl)piperazin-1-yl)pyridin-3-yl)pyrazolo[1,5-a]pyridine-3-carbonitrile **(compound** 14). MS

m/z(ESI):623.3 [M+H]+. $^1$H NMR(500 MHz, DMSO) δ 8.73(s, 1H), 8.58(s, 1H), 8.32(d, *J* = 2.4 Hz, 1H), 8.09(s, 1H), 7.76(dd, J= 8.8, 2.5 Hz, 1H), 7.67(dd, J= 8.4, 1.8 Hz, 1H), 7.31(s, 1H), 6.93(d, J= 8.9 Hz, 1H), 6.80(d, J= 8.4 Hz, 1H), 4.23(d, J= 62.7 Hz, 2H), 3.84(s, 3H), 3.66 - 3.42(m, 13H), 2.47(s, 4H), 1.62 - 1.33(m, 11H).

**Example 15: Synthesis of compound 15**

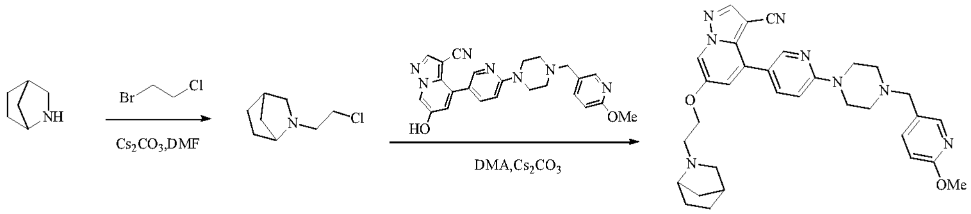


**Step 1: synthesis of 2-(2-chloroethyl)-2-azabicyclo[2.2.1]heptane**

2-azabicyclo[2.2.1]heptane (0.2 g, 2.06 mmol) and cesium carbonate (2.01 g, 6.18 mmol) were added to DMF (5 mL), and then 1-bromo-2-chloroethane (0.88 g, 6.18 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 100 mg of 2-(2-chloroethyl)-2-azabicyclo[2.2.1]heptane. MS m/z(ESI):160.7 [M+H]+ .

**Step 2: synthesis of 6-(2-(2-azabicyclo[2.2.1]hept-2-yl)ethoxy)-4-(6-(4-((6-methoxypyridin-3-yl)methyl)piperazin-1-yl)pyridin-3-yl)pyrazolo[1,5-a]pyridine-3-carbonitrile**

6-hydroxy-4-(6-(4-((6-methoxypyridin-3-yl)methyl)piperazin-1-yl)pyridin-3-yl)pyrazolo[1,5-a]pyridine-3-carbonitrile (0.12 g, 0.27mmol), 2-(2-chloroethyl)-2-azabicyclo[2.2.1]heptane (87 mg, 0.54 mmol), and cesium carbonate (0.18 g, 0.54 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 90 mg of 6-(2-(2-azabicyclo[2.2.1]hept-2-yl)ethoxy)-4-(6-(4-((6-methoxypyridin-3-yl)methyl)piperazin-1-yl)pyridin-3-yl)pyrazolo[1,5-a]pyridine-3-carbonitrile **(compound 15).** MS m/z(ESI):565.3 [M+H]+. $^1$H NMR(500 MHz, DMSO) δ 8.74(s, 1H), 8.59(s, 1H), 8.32(d, J= 2.5 Hz, 1H), 8.08(d, *J* = 2.1 Hz, 1H), 7.77(dd, *J* = 8.8, 2.5 Hz, 1H), 7.67(dd, *J* = 8.5, 2.4 Hz, 1H), 7.33(s, 1H), 6.93(d, *J*= 8.9 Hz, 1H), 6.81(d, J= 8.5 Hz, 1H), 4.30(s, 2H), 3.84(s, 3H), 3.55(dd, J= 29.6, 24.7 Hz, 8H), 2.49 - 2.40(m, 5H), 1.99(dt, J= 12.5, 7.1 Hz, 1H), 1.84(s, 2H), 1.63 - 1.28(m, 6H).

**Example 16: Synthesis of compound 16**

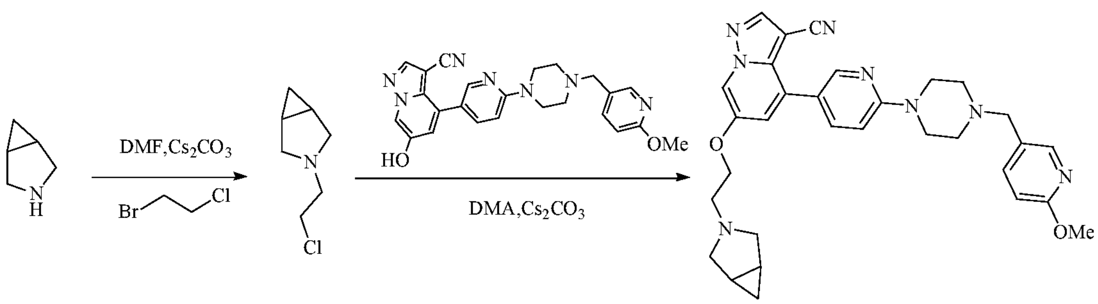


**Step 1: Synthesis of 3-(2-chloroethyl)-3-azabicyclo[3.1.0]hexane**

3-azabicyclo[3.1.0]hexane (0.2 g, 2.41 mmol) and cesium carbonate (1.56 g, 4.82 mmol) were added to DMF

(5 mL), and then 1-bromo-2-chloroethane (0.75 g, 4.82 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 110 mg of 3-(2-chloroethyl)-3-azabicyclo[3.1.0]hexane. MS m/z(ESI): 146.6 $[M+H]^+$.

**Step 2: synthesis of 6-(2-(3-azabicyclo[3.1.0]hex-3-yl)ethoxy)-4-(6-(4-((6-methoxypyridin-3-yl)methyl)piperazin-1-yl)pyridin-3-yl)pyrazolo[1,5-a]pyridine-3-carbonitrile**

6-hydroxy-4-(6-(4-((6-methoxypyridin-3-yl)methyl)piperazin-1-yl)pyridin-3-yl)pyrazolo[1,5-a]pyridine-3-carbonitrile (0.1 g, 0.23 mmol), 3-(2-chloroethyl)-3-azabicyclo[3.1.0]hexane (66 mg, 0.45 mmol), and cesium carbonate (0.15 g, 0.45 mmol) were dissolved in DMA (5 mL) and the resulting solution was stirred at 50 °C overnight to react until the reaction was found to be completed from the monitoring. Water was added to quench the reaction, extraction with dichloromethane was then carried out, and the organic phase was washed with water and then dried, concentrated, and subjected to column chromatography to obtain 60 mg of 6-(2-(3-azabicyclo[3.1.0]hex-3-yl)ethoxy)-4-(6-(4-((6-methoxypyridin-3-yl)methyl)piperazin-1-yl)pyridin-3-yl)pyrazolo[1,5-a]pyridine-3-carbonitrile **(compound** 16). MS m/z(ESI):551.2 $[M+H]^+$.

$^1$H NMR(500 MHz, DMSO) δ 8.68(s, 1H), 8.57(s, 1H), 8.32(d, J= 2.5 Hz, 1H), 8.08(d, *J* = 2.0 Hz, 1H), 7.76(dd, J= 8.8, 2.5 Hz, 1H), 7.67(dd, J= 8.5, 2.3 Hz, 1H), 7.28(s, 1H), 6.92(d, *J* = 8.9 Hz, 1H), 6.80(d, *J* = 8.4 Hz, 1H), 4.15(s, 2H), 3.84(s, 3H), 3.53(d, J= 48.6 Hz, 7H), 2.91(d, *J*= 97.1 Hz, 4H), 2.48 - 2.30(m, 5H), 1.34(d, *J*= 10.2 Hz, 2H), 0.56(s, 1H), 0.29(s, 1H).

**Example 17: Synthesis of compound 17**

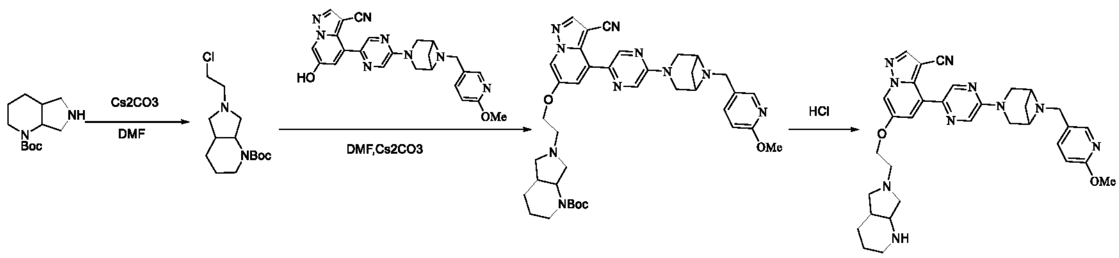


**Step 1: synthesis of tert-butyl-3-(5-bromopyrazin-2-yl)-3,6-diazabicyclo[3.1.1]heptane-6-formate**

1-BOC-octahydro-pyrrole[3,4-B]pyridine (350 mg, 1.5 mmol) and cesium carbonate (1.5 g, 4.6 mmol) were dissolved in DMF (5 mL), and then 1-bromo-2-chloroethane (665 mg, 4.6 mmol) was added to the resulting solution at room temperature, and the reaction solution was stirred at room temperature overnight to react until the reaction was completed. The reaction solution was diluted with ethyl acetate and then filtered, water was then added to quench the reaction, the reaction solution was extracted and then concentrated under reduced pressure to remove the solvent, and column chromatography (PE:EA=3:1) was then carried out to obtain 270 mg of tert-butyl 6-(2-chloroethyl)octahydro-1H-pyrrolo [3,4-b]pyridine-1-carboxylate with a yield of 60%. MS m/z(ESI):289 $[M+H]^+$.

**Step 2: synthesis of tert-butyl 2-(2-((3-cyano-4-(5-(6-((6-methoxypyridin-3-yl)methyl)-3,6-diazabicyclo[3.1.1]hept-3-yl)pyrazin-2-yl)pyrazolo[1,5-a]pyridin-6-yl)oxy)ethyl)octahydro-1h-isoindole-4-carboxylate**

Tert-butyl 6-(2-chloroethyl)octahydro-1H-pyrrolo[3,4-b]pyridine-1-carboxylate (126 mg, 0.44 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (4 mL), and then cesium carbonate (214 mg, 0.66 mmol) was added to the resulting solution, and the reaction solution was stirred at 50 °C for 3 h to react until the reaction was completed. The reaction solution was then diluted with DCM and concentrated under reduced pressure to remove solvent, and column chromatography (DCM:MeOH=20:1) was carried out to obtain 129 mg of tert-butyl 2-(2-((3-cyano-4-(5-(6-((6-methoxypyridin-3-yl)methyl)-3,6-diazabicyclo[3.1.1]hept-3-yl)pyrazin-2-yl)pyrazolo[1,5-a]pyridin-6-yl)oxy)ethyl)octahydro-1h-isoindole-4-carboxylate with a yield of 83%. MS m/z(ESI):707 $[M+H]^+$.

**Step 3: synthesis of 4-(5-(6-((6-methoxypyridin-3-yl)methyl)-3,6-diazabicyclo[3.1.1]hept-3-yl)pyrazin-2-yl)-6-(2-(octahydro-6H-pyrrole[3,4-b]pyridin-6-yl)ethoxy)pyrazolo[1,5-a]pyridine-3-carbonitrile**

Tert-butyl 2-(2-((3-cyano-4-(5-(6-((6-methoxypyridin-3-yl)methyl)-3,6-diazabicyclo[3.1.1]hept-3-yl)pyrazin-2-yl)pyrazolo[1,5-a]pyridin-6-yl)oxy)ethyl)octahydro-1h-isoindole-4-carboxylate (126 mg, 0.178 mmol) was dissolved in DCM (2 mL), and then 2 mL of dioxane hydrochloride was then added to the resulting solution, and the reaction solution was stirred for 1 h to react until the reaction was completed. The reaction was quenched with saturated aqueous $NaHCO_3$ solution, and the reaction solution was extracted with DCM and then washed with water, dried, and subjected to column chromatography (DCM:MeOH=10:1) to obtain 62 mg of 4-(5-(6-((6-methoxypyridin-3-yl)methyl)-3,6-diazabicyclo[3.1.1]hept-3-yl)pyrazin-2-yl)-6-(2-(octahydro-6H-pyrrole[3,4-b]pyridin-6-yl)ethoxy)pyrazolo[1,5-a]pyridine-3-carbonitrile **(compound 17)** with a yield of 57%. MS m/z(ESI):607 $[M+H]^+$.

$^1H$ NMR(400 MHz, DMSO) δ 8.73(s, 1H), 8.67(s, 1H), 8.60(s, 1H), 8.29(s, 1H), 8.09(d, 1H), 7.68-7.70(m, 1H), 7.62(d, 2H), 6.76(d, 1H), 4.18(m, 1H), 3.82(m, 3H), 3.79(s, 1H), 3.68(d, 2H), 3.61(d, 2H), 3.54(s, 2H),3.10-3.13(m,1H),2.91-2.95(m,3H),2.69-2.79(m,3H),2.41-2.47(m,2H),2.00-2.04(m, 2H),1.57-1.63(m, 3H),1.23-1.34(m, 2H).

**Example 18: Synthesis of compound 18**

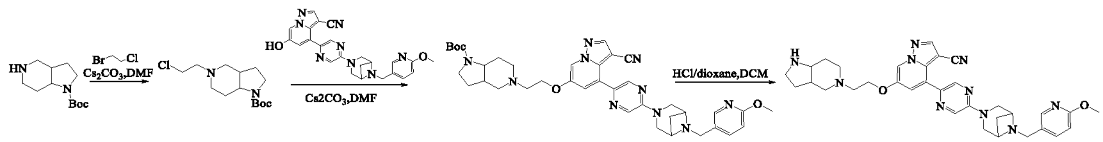


**Step 1: synthesis of tert-butyl 5-(2-chloroethyl)octahydro-1H-pyrro[3,2-c]pyridine-1-carboxylate**

Tert-butyl octahydro-1H-pyrrolo[3,2-C]pyridine-1-carboxylate (350 mg, 1.5 mmol) and cesium carbonate (1.5 g, 4.6 mmol) were dissolved in DMF (3 mL), and then 1-bromo-2-chloroethane (665 mg, 4.6 mmol) was added to the resulting solution at room temperature, and the reaction solution was stirred at room temperature overnight to react until the reaction was completed. The reaction solution was diluted with ethyl acetate and then filtered, water was then added to quench the reaction, the reaction solution was extracted and then concentrated under reduced pressure to remove the solvent, and column chromatography (PE:EA=3:1) was then carried out to obtain 250 mg of tert-butyl 5-(2-chloroethyl)octahydro-1H-pyrro[3,2-c]pyridine-1-carboxylate with a yield of 56%. MS m/z(ESI):289 $[M+H]^+$.

**Step 2: synthesis of tert-butyl 5-(2-((3-cyano-4-(5-(6-((6-methoxypyridin-3-yl)methyl)-3,6-diazabicyclo[ 3.1.1]heptan-3-yl)pyrazin-2-yl)pyrazolo[1,5-a]pyridin-6-yl)oxy)ethyl)octahydro-1H-pyrrolo[3,2-c]pyridine-1-carboxylate**

Tert-butyl 5-(2-chloroethyl)octahydro-1H-pyrro[3,2-c]pyridine-1-carboxylate (250 mg, 0.86 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (4 mL), and then cesium carbonate (214 mg, 0.66 mmol) was added to the resulting solution, and the reaction solution was stirred at 50 °C for 18 h to react until the reaction was completed. The reaction solution was then diluted with DCM, filtered and concentrated under reduced pressure to remove solvent, and column chromatography (DCM:MeOH=20:1) was carried out to obtain 148 mg of tert-butyl 5-(2-((3-cyano-4-(5-(6-((6-methoxypyridin-3-yl)methyl)-3,6-diazabicyclo[ 3.1.1]heptan-3-yl)pyrazin-2-yl)pyrazolo[1,5-a]pyridin-6-yl)oxy)ethyl)octahydro-1H-pyrrolo[3, 2-c]pyridine-1-carboxylate with a yield of 95%. MS m/z(ESI):707 $[M+H]^+$.

**Step 3: synthesis of 4-(5-(6-((6-methoxypyridin-3-yl)methyl)-3,6-diazabicyclo[3.1.1]hept-3-yl)pyrazin-2-yl)-6-(2-(octahydro-5H-pyrrolo[3,2-c]pyridin-5-yl)ethoxy)pyrazolo[1,5-a]pyridine-3-carbonitrile**

Tert-butyl 5-(2-((3-cyano-4-(5-(6-((6-methoxypyridin-3-yl)methyl)-3,6-diazabicyclo[ 3.1.1]heptan-3-yl)pyrazin-2-yl)pyrazolo[1,5-a]pyridin-6-yl)oxy)ethyl)octahydro-1H-pyrrolo[3, 2-c]pyridine-1-carboxylate (148 mg, 0.2 mmol) was dissolved in 3 mL of DCM, and then 6 mL of dioxane hydrochloride was then added to the resulting solution, and the reaction solution was stirred for 1 h to react until the reaction was completed. The solvent was removed and the reaction solution was diluted with the aqueous $NaHCO_3$ solution, and then extracted with DCM, washed with water, and then dried, concentrated and subjected to column chromatography (DCM:MeOH=10:1) to obtain 35 mg of 4-(5-(6-((6-methoxypyridin-3-yl)methyl)-3,6-diazabicyclo[3.1.1]hept-3-yl)pyrazin-2-yl)-6-(2-(octahydro-5H-pyrrolo[3,2-c]pyridin-5-yl)ethoxy)pyrazolo[1,5-a]pyridine-3-carbonitrile **(compound 18)** with a yield of 27%. MS m/z(ESI):607 [M+H]+.

$^1$H NMR(400 MHz, DMSO) δ 8.74(s, 1H), 8.67(s, 1H), 8.60(s, 1H), 8.29(s, 1H), 8.09(d, 1H), 7.68-7.70(m, 1H), 7.62(d, 2H), 6.76(d, 1H), 4.23(m, 2H), 3.82(m, 3H),3.79(s, 1H), 3.68(d, 2H), 3.61(d, 2H), 3.54(s, 2H),3.92-3.00(m, 2H),2.78-2.82(m,1H), 2.70-2.72(m, 2H),2.53-2.55(m, 2H),2.40-2.43(m, 2H),2.16-2.22(m, 1H),2.03-2.09(m, 1H),1.67-1.76(m,2H),1.57-1.63(m,2H),1.44-1.50(m,1H),1.23(s,2H).

**Example 19: Synthesis of compound 19**

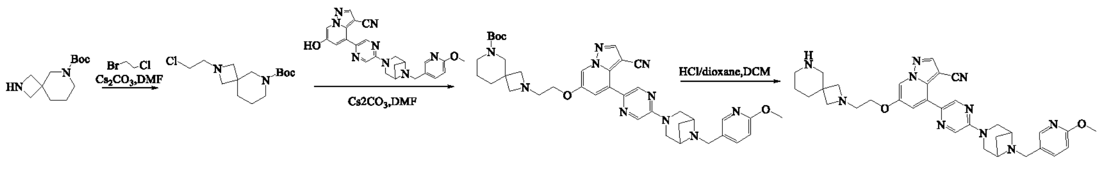


**Step 1: synthesis of tert-butyl 2-(2-chloroethyl)-2,6-diazaspiro[3.5]nonane-6-2-formate**

Tert-butyl 2,6-diazaspiro[3.5]nonane-6-carboxylate (300 mg, 0.55 mmol) and cesium carbonate (899 mg, 2.76 mmol) were dissolved in DMF (5 mL), and then 1-bromo-2-chloroethane (238 mg, 1.66 mmol) was added to the resulting solution at room temperature, and the reaction solution was stirred at room temperature overnight to react until the reaction was completed. The reaction solution was diluted with ethyl acetate and then filtered, water was then added to quench the reaction, the reaction solution was extracted and then concentrated under reduced pressure to remove the solvent, and column chromatography (PE:EA=3:1) was then carried out to obtain 250 mg of tert-butyl 2-(2-chloroethyl)-2,6-diazaspiro[3.5]nonane-6-carboxylate with a yield of 100%. MS m/z(ESI):289 [M+H]+.

**Step 2: synthesis of tert-butyl 2-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,6-diazaspiro[3.5]nonane-6-carboxylate**

Tert-butyl 2-(2-chloroethyl)-2,6-diazaspiro[3.5]nonane-6-carboxylate (126 mg, 0.44 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (2 mL), and then cesium carbonate (214 mg, 0.66 mmol) was added to the resulting solution, and the reaction solution was stirred at 50 °C for 18 h to react until the reaction was completed. The reaction solution was then diluted with DCM, filtered and concentrated under reduced pressure to remove solvent, and column chromatography (DCM:MeOH=20:1) was carried out to obtain 61 mg of tert-butyl 2-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,6-diazaspiro[3.5]nonane-6-carboxylate with a yield of 39%. MS m/z(ESI):706 [M+H]+.

**Step 3: synthesis of 6-(2-(2,6-diazaspiro[3.5]non-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

Tert-butyl 2-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,6-diazaspiro[3.5]nonane-6-carboxylate (61 mg, 0.086 mmol) was dissolved in DCM (3 mL), TFA (1 mL) was then added to the resulting solution, and the reaction solution was stirred for 1 h to react until the reaction was completed. The solvent was removed and the reaction solution was neutralized with the aqueous $NaHCO_3$ solution, and then extracted with DCM, washed with water, and then dried, concentrated and subjected to column chromatography (DCM:MeOH=10:1) to obtain 20 mg of 6-(2-(2,6-diazaspiro[3.5]non-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 19)** with a yield of 38%. MS m/z(ESI):607 [M+H]+.

$^1$H NMR(400 MHz, DMSO) δ 8.71(d, 1H), 8.66(d, 1H), 8.61(s, 1H), 8.29(d, 1H), 8.09(d, 1H), 7.68-7.70(m, 1H), 7.57(d, 1H), 6.76(d, 1H), 4.09(m, 2H), 3.82(m, 3H),3.79(s, 1H), 3.68(d, 2H), 3.61(d, 2H), 3.54(s, 2H),3.15(d, 2H),3.00(s,1H), 2.95(d, 2H),2.79-2.85(m, 4H), 2.55-2.58(m, 1H),1.67-1.70(t, 2H),1.61(d, 1H),1.51-1.52(m, 2H),1.25-1.27(m, 2H).

**Example 20: Synthesis of compound 20**

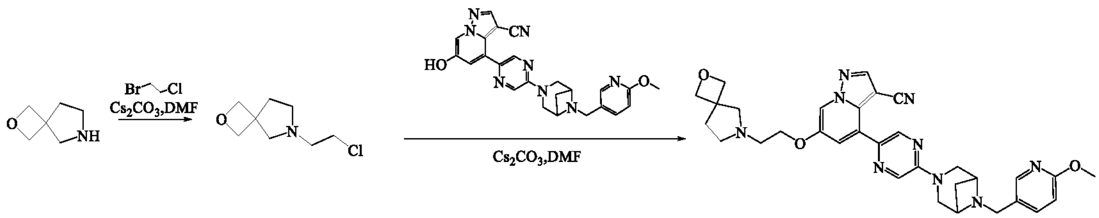


**Step 1: synthesis of 6-(2-chloroethyl)-2-oxa-6-azaspiro[3.4]octane**

2-oxa-6-azaspiro[3.4]octane (300 mg, 0.94 mmol) and cesium carbonate (925 mg, 2.84 mmol) were dissolved to DMF (5 mL), and then 1-bromo-2-chloroethane (407 mg, 2.84 mmol) was added to the resulting solution at room temperature, and the mixed solution was stirred at room temperature overnight to react until the reaction was found to be completed from the monitoring. The reaction solution was diluted with ethyl acetate and then filtered, water was then added to quench the reaction, the reaction solution was concentrated under reduced pressure to remove the solvent, and column chromatography (PE:EA=3:1) was then carried out to obtain 100mg of 6-(2-chloroethyl)-2-oxa-6-azaspiro[3.4]octane with a yield of 18%. MS m/z(ESI):176 $[M+H]^+$.

**Step 2: synthesis of 6-(2-(2-oxa-6-azaspiro[3.4]oct-6-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-(2-chloroethyl)-2-oxa-6-azaspiro[3.4]octane (77 mg, 0.44 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (2 mL), and then cesium carbonate (214 mg, 0.66 mmol) was added to the resulting solution, and the reaction solution was stirred at 50 °C for 18 h to react until the reaction was completed. The reaction solution was then diluted with DCM, filtered and concentrated under reduced pressure to remove solvent, and column chromatography (DCM:MeOH=20:1) was carried out to obtain 10 mg of 6-(2-(2-oxa-6-azaspiro[3.4]oct-6-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 20)** with a yield of 7.6%. MS m/z(ESI):594 $[M+H]^+$.

$^1H$ NMR(400 MHz, DMSO) δ 8.71(d, 1H), 8.64(d, 1H), 8.58(s, 1H), 8.27(d, 1H), 8.07(d, 1H), 7.66-7.68(m, 1H), 7.60(d, 1H), 6.74(d, 1H), 4.21(t, 2H), 3.80(m, 3H),3.77(s, 1H), 3.66(d, 2H), 3.58-3.61(d, 3H), 3.55(s, 3H), 2.80(m, 5H),2.47-2.57(m, 5H), 2.01(t, 2H),1.59-1.62(m, 1H).

**Example 21: Synthesis of compound 21**

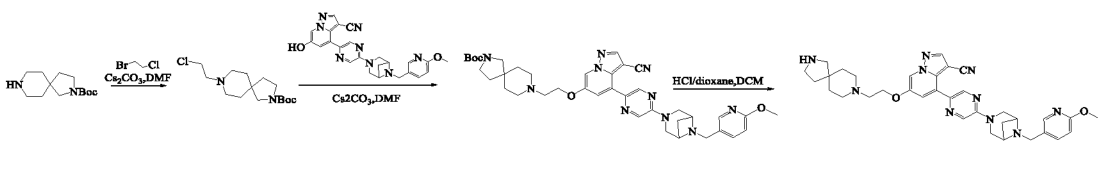


**Step 1: synthesis of tert-butyl 8-(2-chloroethyl)-2,8-diazaspiro[4.5]decane-2-carboxylate**

Tert-butyl 2,8-diazaspiro[4.5]decane-2-2-carboxylate (350 mg, 1.45 mmol) and cesium carbonate (1.5 g, 4.36 mmol) were dissolved in DMF (5 mL), and then 1-bromo-2-chloroethane (624 mg, 4.36 mmol) was added to the resulting solution at room temperature, and the reaction solution was stirred at room temperature overnight to react until the reaction was completed. The reaction solution was diluted with ethyl acetate and then filtered, water was then added to quench the reaction, the reaction solution was extracted and then concentrated under reduced pressure to remove the solvent, and column chromatography (PE:EA=3:1) was then carried out to obtain 192 mg of tert-butyl 8-(2-chloroethyl)-2,8-diazaspiro[4.5]decane-2-carboxylate with a yield of 43%.

**Step 2: synthesis of tert-butyl 8-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,8-diazaspiro[4.5]decane-2-carboxylate**

Tert-butyl 8-(2-chloroethyl)-2,8-diazaspiro[4.5]decane-2-carboxylate (192 mg, 0.66 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (2 mL), and then cesium carbonate (214 mg, 0.66 mmol) was added to the resulting solution, and the reaction solution was stirred at 50 °C for 3 h to react until the reaction was completed. The reaction solution was then diluted with DCM, filtered and concentrated under reduced pressure to remove solvent, and column chromatography (DCM:MeOH=20:1) was carried out to obtain 158 mg of tert-butyl 8-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,8-diazaspiro[4.5]decane-2-carboxylate with a yield of 100%. MS m/z(ESI):721 $[M+H]^+$.

**Step 3: synthesis of 6-(2,8-diazaspiro[4.5]dec-8-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

Tert-butyl 8-(2-((3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-yl)oxy)ethyl)-2,8-diazaspiro[4.5]decane-2-carboxylate (158 mg, 0.21 mmol) was dissolved in DCM (6 mL), TFA (2 mL) was then added to the resulting solution, and then the reaction solution was stirred for 1 h to react until the reaction was completed. The solvent was removed and the reaction solution was neutralized with the aqueous $NaHCO_3$ solution, and then extracted with DCM, washed with water, and then dried, concentrated and subjected to column chromatography (DCM:MeOH=10:1) to obtain 54 mg of 6-(2,8-diazaspiro[4.5]dec-8-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 21)** with a yield of 39%. MS m/z(ESI):621$[M+H]^+$.

1H NMR(400 MHz, DMSO) δ 8.71(d, 1H), 8.64(d, 1H), 8.58(s, 1H), 8.26(d, 1H), 8.07(d, 1H), 7.65-7.68(m, 1H), 7.59(d, 1H), 6.73(d, 1H), 4.21(t, 2H), 3.80(m, 3H),3.77(s, 1H), 3.66(d, 2H), 3.61(d, 2H), 3.58(s, 2H),3.51(s, 2H),2.98(t,1H), 271-2.73(m, 3H),2.54-2.55(m, 1H), 2.39-2.43(m, 4H),1.54-1.60(m, 3H),1..48(m, 4H).

**Example 22: Synthesis of compound 22**

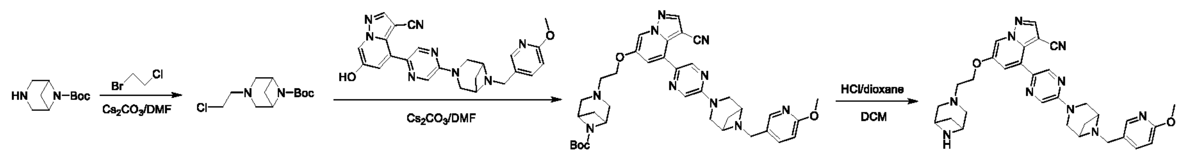


**Step 1: synthesis of tert-butyl 3-(2-chloroethyl)-3,6-diazabicyclo[3.1.1]heptane-6-carboxylate**

Tert-butyl 3,6-Diazabicyclo[3.1.1]heptane-6-carboxylate (300.0 mg, 1.51 mmol) and cesium carbonate (1.47 g, 4.51 mmol) were dissolved in DMF (3 mL), and then 1-bromo-2-chloroethane (650.0 mg, 4.51 mmol) was added to the resulting solution, and the mixed solution was stirred at room temperature overnight until the reaction was found to be completed from the monitoring. Water was then added, extraction with dichloromethane was then carried out, and the organic phase was washed with water and then dried, concentrated, and subjected to column chromatography to obtain 170 mg of tert-butyl 3-(2-chloroethyl)-3,6-diazabicyclo[3.1.1]heptane-6-carboxylate with a yield of 43%. MS m/z(ESI):261.0 $[M+H]^+$.

**Step 2: synthesis of ethyl 3-(2-((3-cyano-4-(5-(6-((6-methoxypyridin-3-yl)methyl)-3,6-diazabicyclo[3.1.1]hept-3-yl tert-butyl)pyrazin-2-yl)pyrazolo[1,5-a]pyridin-6-yl)oxy)ethyl)-3,6-diazabicyclo[3.1.1]heptane-6-carboxylate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (100.0 mg, 0.22 mmol), tert-butyl 3-(2-chloroethyl)-3,6-diazabicyclo[3.1.1]heptane-6-carboxylate (170.0 mg, 0.65 mmol), and cesium carbonate (210.0 mg, 0.65 mmol) were dissolved in DMF (3 mL) and the resulting solution was stirred at 50 °C overnight until the reaction was found to be completed from the monitoring. Water was then added, extraction with dichloromethane was carried out, and the organic phase was then washed with water, and then dried, concentrated, and subjected to column chromatography to obtain 130 mg of ethyl 3-(2-((3-cyano-4-(5-(6-((6-methoxypyridin-3-yl)methyl)-3,6-diazabicyclo[3.1.1]hept-3-yl tert-butyl)pyrazin-2-yl)pyrazolo[1,5-a]pyridin-6-yl)oxy)ethyl)-3,6-diazabicyclo[3.1. 1]heptane-6-carboxylate with a yield of 87%. MS m/z(ESI):679.2 $[M+H]^+$.

**Step 3: synthesis of 6-(2-(3,6-diazabicyclo[3.1.1]heptan-3-yl)ethoxy)-4-(5-(6-((6-methoxypyridine-3-yl)methyl)-3,6-diazabicyclo[3.1.1]heptan-3-yl)pyrazin-2-yl)pyrazolo[1,5-a]pyridine-3-carbonitrile**

Ethyl 3-(2-((3-cyano-4-(5-(6-((6-methoxypyridin-3-yl)methyl)-3,6-diazabicyclo[3.1.1]hept-3 -yl tert-butyl)pyrazin-2-yl)pyrazolo[1,5-a]pyridin-6-yl)oxy)ethyl)-3,6-diazabicyclo[3.1.1]heptane-6-carboxylate (130.0 mg, 0.20 mmol) was dissolved in dichloromethane (2 mL), and then 4M HCl/dioxane (5 mL) was added to the resulting solution, and the mixed solution was then stirred at room temperature for 2 h until the reaction was found to be completed from the monitoring. The reaction solution was then concentrated, water was then added, saturated sodium bicarbonate solution was then added to adjust pH to neutral or alkaline, extraction with dichloromethane was then carried out, and the organic phase was washed with water and then dried, concentrated, and subjected to column chromatography to obtain 15 mg of 6-(2-(3,6-diazabicyclo[3.1.1]heptan-3-yl)ethoxy)-4-(5-(6-((6-methoxypyridine-3-yl)methyl)-3,6-diazabicyclo[3.1.1]heptan-3-yl)pyrazin-2-yl)pyrazolo[1,5-a]pyridine-3-carbonitrile **(compound** 22) with a yield of 14%. MS m/z(ESI):579.1[M+H]$^+$.

$^1$H NMR(500 MHz, DMSO) $\delta$ 8.77(d, $J$ = 2.1 Hz, 1H), 8.67(d, $J$= 1.4 Hz, 1H), 8.62(s, 1H), 8.30(d, $J$= 1.4 Hz, 1H), 8.10(d, $J$= 2.1 Hz, 1H), 7.70(dd, J= 8.5, 2.4 Hz, 1H), 7.63(d, $J$ = 2.1 Hz, 1H), 6.78(d, J= 8.5 Hz, 1H), 4.32(t, $J$= 5.5 Hz, 2H), 4.00(s, 2H), 3.83(s, 3H), 3.69(d, $J$ = 5.9 Hz, 2H), 3.62(d, $J$ = 12.3 Hz, 2H), 3.54(s, 2H), 3.26(d, $J$ = 11.3 Hz, 4H), 3.06(dd, $J$ = 12.5, 8.4 Hz, 4H), 2.00(dd, $J$ = 19.4, 8.0 Hz, 2H), 1.62(d, $J$ = 8.7 Hz, 1H), 1.35(d, $J$ = 8.9 Hz, 1H).

**Example 23: Synthesis of compound 23**

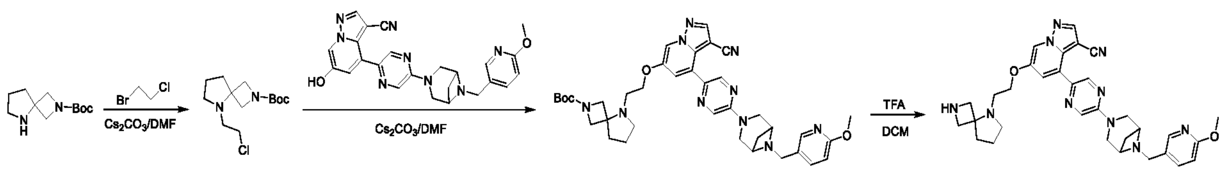


**Step 1: synthesis of tert-butyl 5-(2-chloroethyl)-2,5-diazaspiro[3.4]octane-2-carboxylate**

Tert-butyl 2,5-diazaspiro[3.4]octane-2-carboxylate (200.0 mg, 0.94 mmol) and cesium carbonate (0.92 g, 2.83 mmol) were dissolved in DMF (3 mL), and then 1-bromo-2-chloroethane (400.0 mg, 2.83 mmol) was added to the resulting solution, and the mixed solution was stirred at room temperature overnight until the reaction was found to be completed from the monitoring. Water was then added, extraction with dichloromethane was then carried out, and the organic phase was washed with water and then dried, concentrated, and subjected to column chromatography to obtain 155 mg of tert-butyl 5-(2-chloroethyl)-2,5-diazaspiro[3.4]octane-2-carboxylate with a yield of 60%. MS m/z(ESI):275.0 [M+H]$^+$.

**Step 2: synthesis of methyl 5-(2-((3-cyano-4-(5-(6-((6-methoxypyridin-3-yl)methyl)-3,6-diazabicyclo[3.1.1] Hept-3-yltert-butyl)pyrazin-2-yl)pyrazolo[1,5-a]pyridin-6-yl)oxy)ethyl)-2,5-diazaspiro[3.4]octane-2-carboxylate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (100.0 mg, 0.22mmol), tert-butyl 5-(2-chloroethyl)-2,5-diazaspiro[3.4]octane-2-carboxylate (155.0 mg, 0.57 mmol), and cesium carbonate (210.0 mg, 0.65 mmol) were dissolved in DMF (3 mL) and the resulting solution was stirred at 50 °C overnight until the reaction was found to be completed from the monitoring. Water was then added, extraction with dichloromethane was carried out, and the organic phase was then washed with water, and then dried, concentrated, and subjected to column chromatography to obtain 123 mg of methyl 5-(2-((3-cyano-4-(5-(6-((6-methoxypyridin-3-yl)methyl)-3,6-diazabicyclo[3.1.1] Hept-3-yl tert-butyl)pyrazin-2-yl)pyrazolo[1,5-a]pyridin-6-yl)oxy)ethyl)-2,5-diazaspiro[3.4]octane-2-carboxylate with a yield of 81%. MS m/z(ESI):693.2 [M+H]$^+$.

**Step 3: synthesis of 6-(2-(2,5-diazaspiro[3.4]octan-5-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

Methyl 5-(2-((3-cyano-4-(5-(6-((6-methoxypyridin-3-yl)methyl)-3,6-diazabicyclo[3.1.1] Hept-3-yl tert-butyl)pyrazin-2-yl)pyrazolo[1,5-a]pyridin-6-yl)oxy)ethyl)-2,5-diazaspiro[3.4]octane-2-carboxylate (123.0 mg, 0.18 mmol) was dissolved in dichloromethane (3 mL), and then TFA (0.1 mL) was added to the resulting solution, and the mixed solution was then stirred at room temperature for 3 h until the reaction was found to be completed from the monitoring.

The reaction solution was then concentrated, water was then added, saturated sodium bicarbonate solution was then added to adjust pH to neutral or alkaline, extraction with dichloromethane was then carried out, and the organic phase was washed with water and then dried, concentrated, and subjected to column chromatography to obtain 80 mg of 6-(2-(2,5-diazaspiro[3.4]octan-5-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 23)** with a yield of 76%. MS m/z(ESI):593.2[M+H]$^+$.

$^1$H NMR(500 MHz, DMSO) δ 8.80(d, *J* = 32.2 Hz, 1H), 8.69(s, 1H), 8.59(d, *J* = 5.1 Hz, 1H), 8.28(s, 1H), 8.14(d, J= 23.3 Hz, 1H), 7.69(s, 2H), 6.78(d, J= 7.0 Hz, 1H), 4.26(s, 2H), 3.88(s, 3H), 3.82(d, *J*= 13.5 Hz, 3H), 3.75 - 3.62(m, 2H), 3.61(d, *J*= 12.4 Hz, 2H), 3.53(s, 2H), 2.16(d, *J* = 13.5 Hz, 2H), 1.33(d, *J*= 8.7 Hz, 1H), 1.25(dd, *J*= 18.1, 13.5 Hz, 8H), 0.84(d, *J* = 7.0 Hz, 2H).

**Example 24: Synthesis of compound 24**

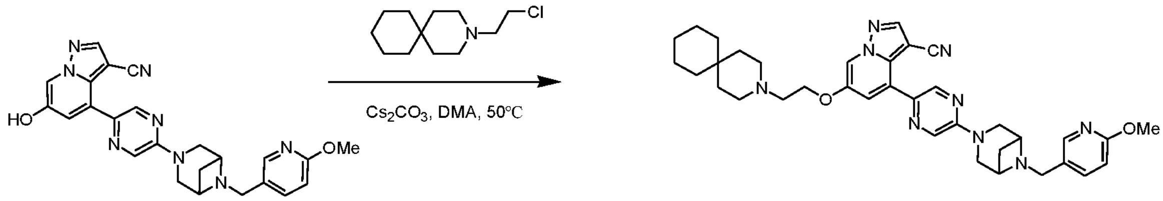


**Step 1: Synthesis of 3-(2-chloroethyl)-3-azaspiro[5.5]undecane**

3-azaspiro[5.5]undecane (300 mg, 1.96 mmol) and cesium carbonate (1.91 g, 5.87 mmol) were dissolved to DMF (5 mL), and then 1-bromo-2-chloroethane (1.12 g, 7.83 mmol) was added to the resulting solution at room temperature, and the mixed solution was stirred at room temperature overnight to react until the reaction was found to be completed from the monitoring. The reaction solution was diluted with ethyl acetate and then filtered, water was then added to quench the reaction, the reaction solution was concentrated under reduced pressure to remove the solvent, and column chromatography (PE:EA=3:1) was then carried out to obtain 192 mg of 3-(2-chloroethyl)-3-azaspiro[5.5]undecane. MS m/z(ESI):216.1 [M+H]$^+$.

**Step 2: synthesis of 6-(2-(3-azaspiro[5.5]undecane-3-yl)ethoxy)-4-(5-(6-((6-methoxypyridin-3-yl)methyl)-3,6diazabicyclo[3.1.1]heptan-3-yl)pyrazin-2-yl)pyrazolo[1,5-a]pyridine-3-carbonitrile**

3-(2-chloroethyl)-3-azaspiro[5.5]undecane (192 mg, 8.9 mmol) and 6-hydroxy-4-(5-(6-((6-methoxypyridin-3-yl)methyl)-3,6-diazabicyclo[3.1.1]heptan-3-yl)pyrazin-2-yl)pyrazole [1,5-a]pyridine-3-carbonitrile (100 mg, 0.22 mmol) were dissolved in DMF (6 mL), and then cesium carbonate (215.04 mg, 0.66 mmol) was added to the resulting solution, and the reaction solution was stirred at 50 °C for 3 h to react until the reaction was completed. The reaction solution was diluted with DCM, and then filtered, concentrated under reduced pressure to remove solvent, and column chromatography (DCM:MeOH=20:1) was then carried out to obtain 35 mg of 6-(2-(3-azaspiro[5.5]undecane-3-yl)ethoxy)-4-(5-(6-((6-methoxypyridin-3-yl)methyl)-3,6diazabicyclo[3.1.1]heptan-3-yl)pyrazin-2-yl)pyrazolo[1,5-a]pyridine-3-carbonitrile. MS m/z(ESI):634.2 [M+H]$^+$.

$^1$H NMR(500 MHz, DMSO-$d_6$) δ 8.74(d, *J* = 1.9 Hz, 1H), 8.66(d, *J* = 1.4 Hz, 1H), 8.60(s, 1H), 8.28(d, *J* = 1.3 Hz, 1H), 8.09(d, *J* = 2.1 Hz, 1H), 7.69(dd, *J* = 8.5, 2.4 Hz, 1H), 7.62(d, *J* = 2.1 Hz, 1H), 6.77(d, *J* = 8.5 Hz, 1H), 4.23(t, *J* = 5.4 Hz, 2H), 3.82(s, 4H), 3.82 - 3.77(m, 2H), 3.69(d, *J*= 5.7 Hz, 2H), 3.62(d, J= 12.2 Hz, 2H), 3.54(s, 2H), 2.78 - 2.68(m, 2H), 2.48 - 2.38(m, 4H), 1.42 - 1.34(m, 10H), 1.32 - 1.26(m, 4H), 1.24(d, J= 7.2 Hz, 2H), 1.05(t, J= 7.0 Hz, 1H).

**Example 25: Synthesis of compound 25**

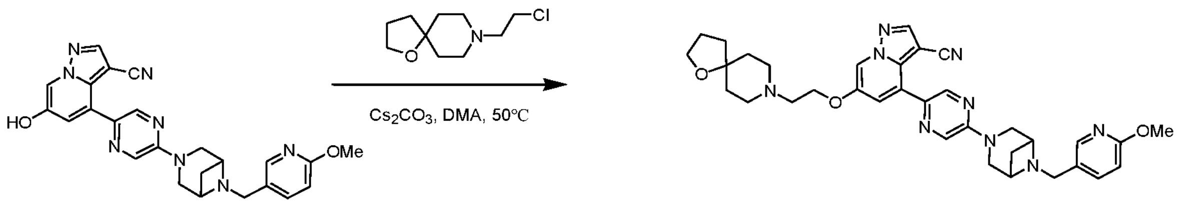


**Step 1: Synthesis of 8-(2-chloroethyl)-1-oxa-8-azaspiro[4.5]decane**

1-oxa-8-azaspiro[4.5]decane (300 mg, 1.69 mmol) and cesium carbonate (1.65 g, 5.07 mmol) were dissolved to DMF (5 mL), and then 1-bromo-2-chloroethane (968.01 mg, 6.75 mmol) was added to the resulting solution at room temperature, and the mixed solution was stirred at room temperature overnight to react until the reaction was found to be completed from the monitoring. The reaction solution was diluted with ethyl acetate and then filtered, water was then added to quench the reaction, the reaction solution was concentrated under reduced pressure to remove the solvent, and column chromatography (PE:EA=3:1) was then carried out to obtain 150 mg of 8-(2-chloroethyl)-1-oxa-8-azaspiro[4.5]decane. MS m/z(ESI):204.1 $[M+H]^+$.

Step 2: synthesis of **6-(2-(1-oxa-8-azaspiro[4.5]dec-8-yl)ethoxy)-4-(5-(6-((6-**methoxypyridin-3-yl)methyl)-3,6-diazabicyclo[3.1.1]heptan-3-yl)pyrazin-2-**yl)pyrazolo[1,5-a]pyridine-3-carbonitrile**

8-(2-chloroethyl)-1-oxa-8-azaspiro[4.5]decane (150 mg, 0.73 mmol) and 6-hydroxy-4-(5-(6-((6-methoxypyridin-3-yl)methyl)-3,6-diazabicyclo[3.1.1]heptan-3-yl)pyrazin-2-yl)pyrazole **[1,5-a]pyridine-3-carbonitrile**(100 mg, 0.22 mmol) were dissolved in DMF (6 mL), and then cesium carbonate (215.04 mg, 0.66 mmol) was added to the resulting solution, and the reaction solution was stirred at 50 °C for 3 h to react until the reaction was completed. The reaction solution was diluted with DCM, and then filtered, concentrated under reduced pressure to remove solvent, and column chromatography (DCM:MeOH=20:1) was then carried out to obtain 45 mg of 6-(2-(1-oxa-8-azaspiro[4.5]dec-8-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (compound 25). $^1H$ NMR(500 MHz, DMSO-$d_6$) δ 8.74(d, *J* = 1.9 Hz, 1H), 8.66(d, *J* = 1.4 Hz, 1H), 8.60(s, 1H), 8.28(d, *J* = 1.3 Hz, 1H), 8.09(d, *J* = 2.1 Hz, 1H), 7.69(dd, *J* = 8.5, 2.4 Hz, 1H), 7.62(d, *J* = 2.1 Hz, 1H), 6.77(d, *J* = 8.5 Hz, 1H), 4.23(t, *J* = 5.4 Hz, 2H), 3.82(s, 4H), 3.82 - 3.77(m, 2H), 3.69(d, *J*= 5.7 Hz, 2H), 3.62(d, *J* = 12.2 Hz, 2H), 3.54(s, 2H), 2.78 - 2.68(m, 2H), 2.48 - 2.38(m, 4H), 1.42 - 1.34(m, 10H), 1.32 - 1.26(m, 4H), 1.24(d, *J* = 7.2 Hz, 2H), 1.05(t, *J* = 7.0 Hz, 1H).MS m/z(ESI):622.1 $[M+H]^+$.

**Example 26: Synthesis of compound 26**

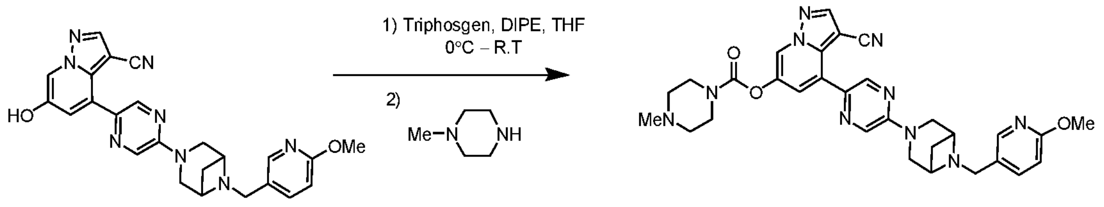


**Step 1: synthesis of 3-cyano-4-(5-(6-((6-methoxypyridin-3-yl)methyl)-3,6-diazabicyclo[3.1.1]heptan-3-yl)pyrazin)pyrazolo[1,5-a]pyridin-6-yl 4-methylpiperazine-1-carboxylate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (100 mg, 0.22 mmol) was dissolved in THF (10 mL) at 0 °C under the protection of $N_2$, DIPEA (113.8 mg, 0.88 mmol) was then added to the resulting solution, triphosgene (65.3 mg, 0.22 mmol) was then slowly added, the reaction solution was stirred for 2 h, and N-methylpiperazine (66.1 mg, 0.66 mmol) was then added dropwise to the reaction system, and the mixed solution was further stirred overnight until the reaction was completed. The reaction was then quenched with saturated sodium bicarbonate, extraction with EA(2 * 10 mL) was carried out, and the organic phases were combined, and then washed with a saturated NaCl solution (20 mL), then concentrated under reduced pressure to remove the solvent, and subjected to column chromatography (DCM:MeOH=10:1) to obtain 65 mg of 3-cyano-4-(5-(6-((6-methoxypyridin-3-yl)methyl)-3,6-diazabicyclo[3.1.1]heptan-3-yl)pyrazin)pyrazolo[1,5-a]pyridin-6-yl 4-methylpiperazine-1-carboxylate **(compound 26).** MS m/z(ESI):581.1 [M+H]+.

$^1$H NMR(500 MHz, DMSO-$d_6$) δ 9.10(d, *J* = 1.9 Hz, 1H), 8.74(s, 1H), 8.66(d, *J* = 1.4 Hz, 1H), 8.32(d, *J* = 1.3 Hz, 1H), 8.10(s, 1H), 7.83(d, *J* = 1.9Hz, 1H), 7.70(dd, *J* = 8.5, 2.3 Hz, 1H), 6.78(d, *J* = 8.5 Hz, 1H), 3.83(s, 4H), 3.80(s, 2H), 3.74 - 3.43(m, 11H), 2.41(d, *J* = 17.1 Hz, 4H), 2.25(s, 3H).

**Example 27: Synthesis of compound 27**

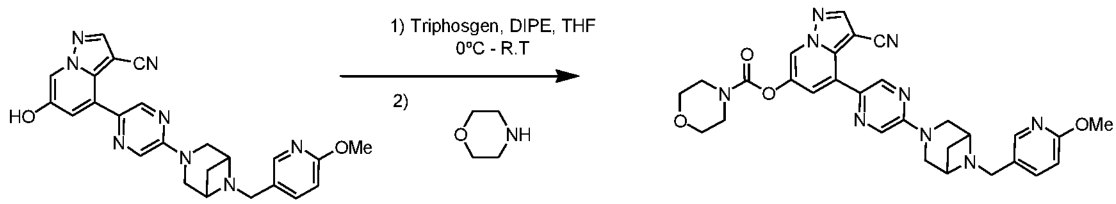


**Step 1: synthesis of 3-cyano-4-(5-(6-((6-methoxypyridin-3-yl)methyl)-3,6-diazabicyclo[3.1.1]heptan-3-yl)pyrazin-2-yl]pyrazolo[1,5-a]pyridin-6-ylmorpholine-4-carboxylate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (100 mg, 0.22 mmol) was dissolved in THF (10 mL) at 0 °C under the protection of $N_2$, DIPEA (113.8 mg, 0.88 mmol) was then added to the resulting solution, triphosgene (65.3 mg, 0.22 mmol) was then slowly added, the reaction solution was stirred for 2 h, andmorpholine (57.6 mg, 0.66 mmol) was then added dropwise to the reaction system, and the mixed solution was further stirred overnight until the reaction was completed. The reaction was then quenched with saturated sodium bicarbonate, extraction with EA(2 * 10 mL) was carried out, and the organic phases were combined, and then washed with a saturated NaCl solution (20 mL), then concentrated under reduced pressure to remove the solvent, and subjected to column chromatography (DCM:MeOH=10:1) to obtain 36 mg of 3-cyano-4-(5-(6-((6-methoxypyridin-3-yl)methyl)-3,6-diazabicyclo[3.1.1]heptan-3-yl)pyrazin-2-yl]pyrazolo[1,5-a]pyridin-6-ylmorpholine-4-carboxylate **(compound 27).** MS m/z(ESI):721 [M+H]+.

$^1$H NMR(500 MHz, DMSO-$d_6$) δ 9.10(d, *J* = 1.9 Hz, 1H), 8.73(s, 1H), 8.65(d, *J* = 1.3 Hz, 1H), 8.31(d, *J* = 1.3 Hz, 1H), 8.09(d, *J* = 1.9 Hz, 1H), 7.84(d, *J* = 1.9 Hz, 1H), 7.69(dd, *J* = 8.5, 2.4 Hz, 1H), 6.77(d, J= 8.5 Hz, 1H), 3.81(d, *J*= 12.0 Hz, 5H), 3.74 - 3.59(m, 11H), 3.54(s, 2H), 3.46(s, 2H), 2.55(d, *J* = 6.7 Hz, 1H), 1.62(d, *J* = 8.5 Hz, 1H). MS m/z(ESI):568.3 [M+H]+.

**Example 28: Synthesis of compound 28**

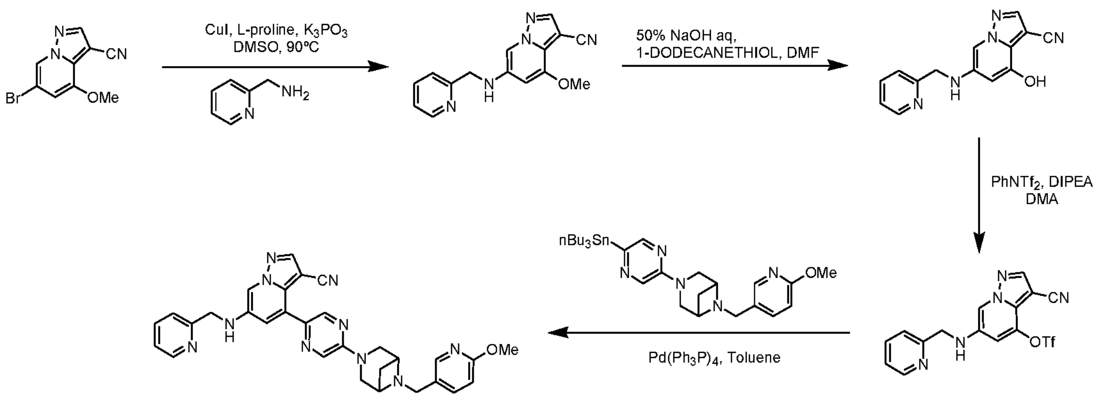


**Step 1: synthesis of 4-methoxy-6-((pyridin-2-ylmethyl)amino)pyrazolo[1,5-a]pyridine-3-carbonitrile**

Compound 6-bromo-4-methoxy-pyrazolo[1,5-a]pyridine-3-carbonitrile (1.0 g, 3.96 mmol), pyridin-2-ylmethanamine (643.4 mg, 5.95 mmol), CuI (75.4 mg, 0.396 mmol), L-proline (91.3 mg, 0.793 mmol) and $K_3PO_4$ (1.68 mg, 7.93 mmol) were dissolved in DMSO (8 mL), and then under the protection of $N_2$, the reaction solution was stirred at 90 °C

overnight. After the reaction was completed, the reaction solution was washed with a saturated NaCl solution (10 mL), and the organic phase was then concentrated and purified by column chromatography (PE:EA=3:1) to obtain 300 mg of 4-methoxy-6-((pyridin-2-ylmethyl)amino)pyrazolo[1,5-a]pyridine-3-carbonitrile. MS m/z(ESI):280.1 $[M+H]^+$.

**Step 2: synthesis of 4-hydroxy-6-((pyridin-2-ylmethyl)amino)pyrazolo[1,5-a]pyridine-3-carbonitrile**

4-methoxy-6-((pyridin-2-ylmethyl)amino)pyrazolo[1,5-a]pyridine-3-carbonitrile (0.2 g, 0.71 mmol) was dissolved in DMF (4 mL), and then n-dodecanethiol (0.34 mL) was added, and the resulting solution was heated to react to 45 °C; then, 12N aqueous NaOH solution (0.13 mL) was added slowly, the resulting solution was then heated up to 50 °C, and the reaction solution was stirred overnight under the protection of $N_2$. After the reaction was completed, the reaction solution was diluted with water (10 mL) and then adjusted to pH of 5 to 6 with 50% aqueous citric acid solution; extraction with EA (2*10 mL) was then carried out, and the organic phases were combined and washed with a saturated NaCl solution (20 mL) and then concentrated and purified by ISCO column chromatography (DCM/MeOH=10:1) to obtain 100 mg of 4-hydroxy-6-((pyridin-2-ylmethyl)amino)pyrazolo[1,5-a]pyridine-3-carbonitrile. MS m/z(ESI):266.2 $[M+H]^+$.

**Step 3: synthesis of 3-cyano-6-((pyridin-2-ylmethyl)amino)pyrazolo[1,5-a]pyridin-4-yl trifluoromethanesulfonate**

4-hydroxy-6-((pyridin-2-ylmethyl)amino)pyrazolo[1,5-a]pyridine-3-carbonitrile (100 mg, 0.38 mmol) was dissolved in DMA (10 mL), and then DIPEA (0.13 mL, 0.76 mmol) was added, and N-phenylbis(trifluoromethanesulfonyl)imide (161.7 mg, 0.45 mmol) was then slowly added to the reaction solution and the resulting solution was stirred at room temperature for 2 h to react. After the reaction was found to be completed from the monitoring of LCMS, the reaction was quenched with water (10 mL), extraction with EA (10 mL*3) was carried out, and the organic phase was washed with water and then dried, concentrated, and subjected to column chromatography to obtain 88 mg of 3-cyano-6-((pyridin-2-ylmethyl)amino)pyrazolo[1,5-a]pyridin-4-yl trifluoromethanesulfonate. MS m/z(ESI):398.2 $[M+H]^+$.

**Step 4: synthesis of 4-(5-(6-((6-methoxypyridin-3-yl)methyl)-3,6-diazabicyclo[3.1.1]heptan-3-yl)pyrazin-2-yl)-6-((pyridin-2-ylmethyl)amino)pyrazolo[1,5-a]pyridine-3-carbonitrile**

Compound 3-cyano-6-((pyridin-2-ylmethyl)amino)pyrazolo[1,5-a]pyridin-4-yl trifluoromethanesulfonate (88 mg, 0.22 mmol), 6-((6-methoxypyridin-3-yl)methyl)-3-(5-(tributylstannyl)pyrazin-2-yl)-3,6-diazabicyclo[3.1.1 ]heptane (195.2 mg, 0.33 mmol), $Pd(Ph_3P)_4$ (25.4 mg, 0.022 mmol) and CuI (4.2 mg, 0.022 mmol) were dissolved in 1,4-xylene (10 mL) at 0 °C under the protection of $N_2$, and the reaction system was stirred thoroughly. The reaction system was then slowly heated up to 140 °C and further stirred overnight to react. After the reaction was found to be completed from the monitoring of LCMS, the reaction was quenched with saturated aqueous $NaHCO_3$ solution (10 mL), extraction with EA (10 mL*3) was then carried out, and the organic phase was then washed with saturated NaCl solution, and then dried, concentrated, and purified by column chromatography with DCM/MeOH (10:1) to obtain 32 mg of 4-(5-(6-((6-methoxypyridin-3-yl)methyl)-3,6-diazabicyclo[3.1.1]heptan-3-yl)pyrazin-2-yl)-6-((pyridin-2-ylmethyl)amino)pyrazolo[1,5-a]pyridine-3-carbonitrile **(compound 28).**

$^1H$ NMR(400 MHz, DMSO-d6) δ 8.57(d, J = 4.1 Hz, 1H), 8.53(d, J = 1.3 Hz, 1H), 8.39(s, 1H), 8.29(d, J = 1.2 Hz, 1H), 8.10(s, 1H), 7.95(d, J = 1.8 Hz, 1H), 7.79(td, J = 7.7, 1.8 Hz, 1H), 7.70(dd, J = 8.4, 2.1 Hz, 1H), 7.48(t, J = 4.6 Hz, 2H), 7.30(dd, J = 6.7, 5.0 Hz, 1H), 6.75(dd, J = 16.2, 7.3 Hz, 2H), 5.43 - 5.40(m, 1H), 4.46(d, J = 6.0 Hz, 2H), 3.82(s, 4H), 3.79(s, 2H), 3.74 - 3.48(m, 7H), 2.55(s, 1H), 1.62(d, J = 8.4 Hz, 1H). MS m/z(ESI):545.3 [M+H]+.

**Example 29: Synthesis of compound 29**

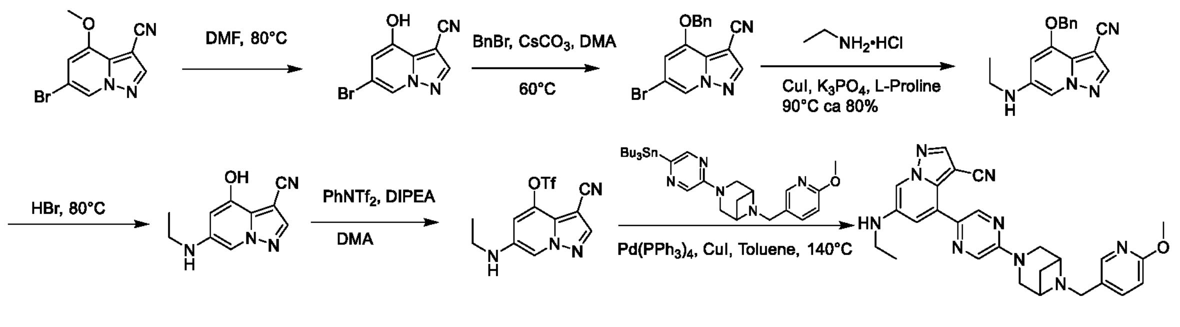

**Step 1: synthesis of 6-bromo-4-(benzyloxy)-pyrazolo[1,5-a]pyridine-3-carbonitrile**

6-bromo-4-methoxy-pyrazolo[1,5-a]pyridine-3-carbonitrile (1.0 g, 0.71 mmol) was dissolved in DMF (4 mL), and then n-dodecanethiol (1.5 mL) was added, and the resulting solution was heated to react to 45 °C; then, aqueous NaOH solution 12 N (0.6 mL) was then added slowly, the resulting solution was then heated up to 50 °C, and the reaction solution was stirred overnight under the protection of $N_2$. After the reaction was completed, the reaction solution was diluted with water (10 mL) and then adjusted to pH of 5 to 6 with 50% aqueous citric acid solution; extraction with EA (2*10 mL) was then carried out, and the organic phases were combined and washed with a saturated NaCl solution (20 mL) and then concentrated to obtain 0.8 g of the product 6-bromo-4-(benzyloxy)-pyrazolo[1,5-a]pyridine-3-carbonitrile, and the obtained product was directly used in the next step of reaction. The product was dissolved in the solvent DMA (10 mL), and then benzyl bromide (1.2 g, 0.67 mmol) and $Cs_2CO_3$ (3.28 g, 10.08 mmol) were added, and the resulting solution was heated and stirred overnight. After the reaction was completed, the reaction solution was washed with saturated NaCl (30 mL), and the organic phase was concentrated and purified by ISCO column chromatography (PE:EA=1:2) to obtain 1.0 g of 6-bromo-4-(benzyloxy)-pyrazolo[1,5-a]pyridine-3-carbonitrile. MS m/z(ESI):328.1 $[M+H]^+$.

**Step 2: synthesis of 4-(benzyloxy)-6-(ethylamino)pyrazolo[1,5-a]pyridine-3-carbonitrile**

Ethylamine hydrochloride (0.92 g, 9.14 mmol) was dissolved in the solvent DMSO (30 mL) in advance, and then $K_2CO_3$ (0.92 g, 9.14 mmol) was added to neutralize the hydrochloric acid in the system, the reaction solution was then stirred for 2 h and then set still, and centrifugation was then carried out to take the supernatant.

In a 100mL high-pressure reaction flask, Compound 6-bromo-4-(benzyloxy)-pyrazolo[1,5-a]pyridine-3-carbonitrile (1.0 g, 3.07 mmol), CuI (16.2 mg, 0.61 mmol), L-proline (141.6 mg, 1.23 mmol) and $K_3PO_4$ (1.3 g, 6.14 mmol) were dissolved in ethylamine (30 mL), and then under the protection of $N_2$, the reaction solution was stirred at 90 °C overnight in a sealed state. After the reaction was completed, the reaction solution was washed with a saturated NaCl solution (30 mL), and the organic phase was then concentrated and purified by column chromatography (PE:EA=3:1) to obtain 0.68 g of 4-(benzyloxy)-6-((pyridin-2-ylmethyl)amino)pyrazolo[1,5-a]pyridine-3-carbonitrile. MS m/z(ESI):293.2 $[M+H]^+$.

**Step 3: synthesis of 6-(ethylamino)-4-hydroxypyrazolo[1,5-a]pyridine-3-carbonitrile**

4-(benzyloxy)-6-((pyridin-2-ylmethyl)amino)pyrazolo[1,5-a]pyridine-3-carbonitrile (0.6 g, 2.05mmol) was dissolved in HBr (20 mL), and the resulting solution was then slowly heated to 80 °C and refluxed for 2 h. After the reaction was completed, the reaction solution was diluted with water (10 mL) and then adjusted to pH of 4 to 5 with 4 N aqueous NaOH solution; extraction with EA (2*10 mL) was then carried out, and the organic phases were combined and washed with a saturated NaCl solution (20 mL) and then concentrated and purified by ISCO column chromatography (DCM/MeOH=10:1) to obtain 0.26 g of 6-(ethylamino)-4-hydroxypyrazolo[1,5-a]pyridine-3-carbonitrile. MS m/z(ESI):203.5 $[M+H]^+$.

**Step 4: synthesis of 3-cyano-6-(ethylamino)pyrazolo[1,5-a]pyridin-4-yl trifluoromethanesulfonate**

6-(ethylamino)-4-hydroxypyrazolo[1,5-a]pyridine-3-carbonitrile (160 mg, 0.79 mmol) was dissolved in DMA (10 mL), and then DIPEA (0.27 mL, 1.58 mmol) was added, and $PhNTf_2$ (340 mg, 0.95 mmol) was then slowly added to the reaction solution and the resulting solution was stirred at room temperature for 2 h to react. After the reaction was found to be completed from the monitoring of LCMS, the reaction was quenched with water (10 mL), extraction with EA (10 mL*3) was carried out, and the organic phase was washed with water and then dried, concentrated, and subjected to column chromatography to obtain 270 mg of 3-cyano-6-(ethylamino)pyrazolo[1,5-a]pyridin-4-yl trifluoromethanesulfonate. MS m/z(ESI):335.2 $[M+H]^+$.

**Step 5: synthesis of 6-(ethylamino)-4-(5-(-6-((6-methoxypyridin-3-yl)methyl)-3,6-diazabicyclo[3.1.1[heptane-3-yl)pyrazin-2-yl)pyrazolo[1,5-a[pyridine-3-carbonitrile**

Compound 3-cyano-6-(ethylamino)pyrazolo[1,5-a]pyridin-4-yl trifluoromethanesulfonate (100 mg, 0.30 mmol), 6-((6-methoxypyridin-3-yl)methyl)-3-(5-(tributylstannyl)pyrazin-2-yl)-3,6-diazabicyclo[3.1.1 ]heptane (263.6 mg, 0.45 mmol), $Pd(Ph_3P)_4$ (34.65 mg, 0.03 mmol) and CuI (5.7 mg, 0.03 mmol) were dissolved in 1,4-xylene (10 mL) at 0 °C under the protection of $N_2$, and the reaction system was stirred thoroughly. The reaction system was then slowly heated up to 140 °C and further stirred overnight to react. After the reaction was found to be completed from the monitoring of LCMS, the reaction was quenched with saturated aqueous $NaHCO_3$ solution (10 mL), extraction with EA (10 mL*3) was then carried out, and the organic phase was then washed with saturated NaCl solution, and then dried, concentrated, and purified by column chromatography with DCM/MeOH (10:1) to obtain 34 mg of 6-(ethylamino)-4-(5-(-6-((6-methoxypyridin-3-yl)methyl)-3,6-diazabicyclo[3.1.1]heptane-3-yl)pyrazin-2-yl)pyrazolo[1,5-a]pyridine-3-carbonitrile **(com-**

**pound 29).** MS m/z(ESI):482.2 [M+H]+.

$^1$H NMR(400 MHz, DMSO-$d_6$) δ 8.52(d, *J* = 1.3 Hz, 1H), 8.40(s, 1H), 8.28(d, *J* = 1.2 Hz, 1H), 8.10(s, 1H), 7.97(d, J= 1.8 Hz, 1H), 7.69(d, J= 8.5 Hz, 1H), 7.32(d, *J*= 1.9 Hz, 1H), 6.76(d, *J*= 8.4 Hz, 1H), 5.95(t, *J*= 5.3 Hz, 1H), 3.81(s, 1H), 3.80(d, *J*= 8.1 Hz, 1H), 3.74 - 3.47(m, 2H), 3.13 - 2.99(m, 1H), 1.21(t, *J*= 7.1 Hz, 1H).

**Example 30: Synthesis of compound 30**

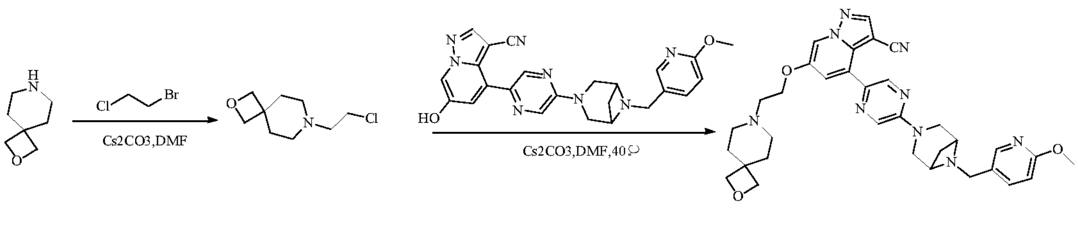


**Step 1: synthesis of 7-(2-chloroethyl)-2-oxa-7-azaspiro[3.5]nonane**

2-oxa-7-azaspiro[3.5]nonane (0.2 g, 1.57 mmol) and cesium carbonate (1.54 g, 4.72 mmol) were added to DMF (5 mL), and then 1-bromo-2-chloroethane (0.68 g, 4.72 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 150 mg of 7-(2-chloroethyl)-2-oxa-7-azaspiro[3.5]nonane. MS m/z(ESI): 190.1 [M+H]+.

**Step 2: synthesis of 6-(2-(2-oxa-7-azaspiro[3.5]non-7-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 7-(2-chloroethyl)-2-oxa-7-azaspiro[3.5]nonane (84 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 50 mg of 6-(2-(2-oxa-7-azaspiro[3.5]non-7-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 30).** MS m/z(ESI):608.3 [M+H]+.

$^1$H NMR(500 MHz, DMSO) δ 8.83(s, 1H), 8.58(s, 1H), 8.32(d, J= 2.4 Hz, 1H), 8.19(s, 1H), 7.67(dd, J= 8.4, 1.8 Hz, 1H), 7.31(s, 1H), 6.93(d, J= 8.9 Hz, 1H), 6.80(d, J= 8.4 Hz, 1H), 4.23(d, *J* = 62.7 Hz, 2H), 3.84(s, 3H), 3.66 - 3.42(m, 13H), 2.27(s, 4H), 1.62-1.33(m, 7H).

**Example 31: Synthesis of compound 31**

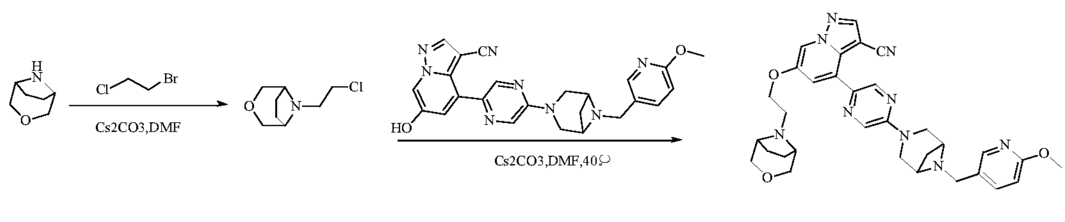


**Step 1: synthesis of 8-(2-chloroethyl)-3-oxa-8-azabicyclo[3.2.1]octane**

3-oxa-8-azabicyclo[3.2.1]octane (0.3 g, 2.65 mmol) and cesium carbonate (1.72 g, 5.30 mmol) were added to DMF (5 mL), and then 1-bromo-2-chloroethane (0.76 g, 5.30 mmol) was added to the resulting solution, and the mixed solution was stirred at room temperature overnight until the reaction was found to be completed from the monitoring.

Water was added to quench the reaction, extraction with dichloromethane was then carried out, and the organic phase was washed with water and then dried, concentrated, and subjected to column chromatography to obtain 120 mg of 8-(2-chloroethyl)-3-oxa-8-azabicyclo[3.2.1]octane. MS m/z(ESI): 176.1 [M+H]+.

**Step 2: synthesis of 6-(2-(3-oxa-8-azabicyclo[3.2.1]oct-8-yl)ethoxy)-4-(5-(6-((6-methoxypyridine-3-yl)methyl)-3,6diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 8-(2-chloroethyl)-3-oxa-8-azabicyclo[3.2.1]octane (78 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 60 mg of 6-(2-(3-oxa-8-azabicyclo[3.2.1]oct-8-yl)ethoxy)-4-(5-(6-((6-methoxypyridine-3-yl)methyl)-3,6diazabicyclo[3.1.1]heptan-3-yl)pyrazin-2-yl)pyrazolo[1,5-a]pyridine-3-carbonitrile **(compound 31).** MS m/z(ESI): 594.3 [M+H]+.

$^1$H NMR(500 MHz, DMSO) δ 8.75(d, J= 1.8 Hz, 1H), 8.67(d, J= 1.0 Hz, 1H), 8.61(s, 1H), 8.30(s, 1H), 8.10(s, 1H), 7.70(d, *J* = 6.4 Hz, 1H), 7.63(d, *J* = 2.0 Hz, 1H), 6.78(d, *J* = 8.5 Hz, 1H), 4.23(s, 2H), 3.81(d, *J*= 14.3 Hz, 5H), 3.74 - 3.49(m, 8H), 3.42(d, *J*= 10.0 Hz, 2H), 3.19(s, 2H), 2.68(s, 2H), 2.55(s, 1H), 1.88(s, 2H), 1.75(d, *J*= 7.1 Hz, 2H), 1.63(d, *J*= 8.5 Hz, 1H).

**Example 32: Synthesis of compound 32**

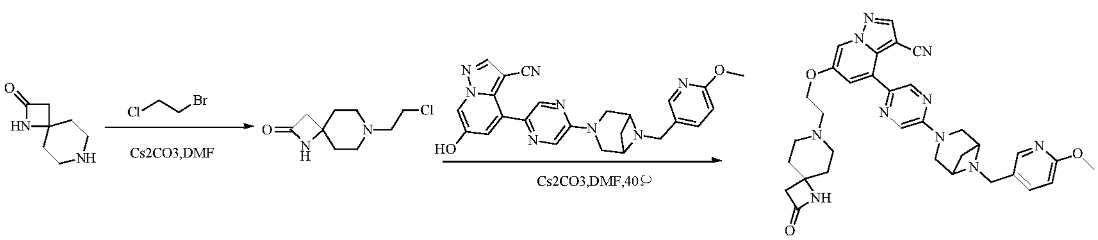


**Step 1: synthesis of 7-(2-chloroethyl)-1,7-diazaspiro[3.5]nonan-2-one**

1,7-diazaspiro[3.5]nonan-2-one (0.3 g, 2.12 mmol) and cesium carbonate (2.09 g, 6.43 mmol) were added to DMF (5 mL), and then 1-bromo-2-chloroethane (0.91 g, 6.43 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 150 mg of 7-(2-chloroethyl)-1,7-diazaspiro[3.5]nonan-2-one. MS m/z(ESI):203.12 [M+H]+.

**Step 2: synthesis of 4-(5-(6-((6-methoxypyridin-3-yl)methyl)-3,6-diazabicyclo[3.1.1]heptan-3-yl)pyrazin-2-yl)-6-(2-(2-oxo-1,7-diazaspiro[3.5]non-7-yl)ethoxy)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.15 g, 0.33 mmol), 7-(2-chloroethyl)-1,7-diazaspiro[3.5]nonan-2-one (134 mg, 0.66 mmol), and cesium carbonate (0.22 g, 0.66 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 40 mg of 4-(5-(6-((6-methoxypyridin-3-yl)methyl)-3,6-diazabicyclo[3.1.1]heptan-3-yl)pyrazin-2-yl)-6-(2-(2-oxo-1,7-diazaspiro[3.5]non-7-yl)ethoxy)pyrazolo[1,5-a]pyridine-3-carbonitrile. MS m/z(ESI):621.3 [M+H]+.

$^1$H NMR(500 MHz, DMSO) δ 8.74(d, J= 2.1 Hz, 1H), 8.67(d, *J*= 1.4 Hz, 1H), 8.61(s, 1H), 8.29(d, *J* = 1.3 Hz, 1H), 8.21(s, 1H), 8.10(d, *J* = 2.1 Hz, 1H), 7.70(dd, J= 8.5, 2.4 Hz, 1H), 7.62(d, *J* = 2.1 Hz, 1H), 6.77(d, *J* = 8.5 Hz, 1H), 4.25(t, *J* = 5.6 Hz, 2H), 3.84 - 3.75(m, 5H), 3.62(dt, *J* = 49.8, 10.5 Hz, 7H), 2.77(t, J= 5.4 Hz, 2H), 2.60(dd, J= 33.9, 6.2 Hz, 4H), 2.42(s, 2H), 1.67(dd, *J*= 19.3, 11.5 Hz, 4H), 1.47(s, 1H).

**Example 33: Synthesis of compound 33**

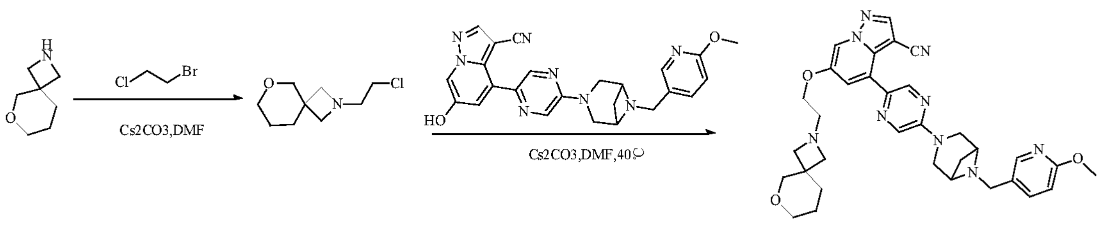


**Step 1: synthesis of 2-(2-chloroethyl)-6-oxa-2-azaspiro[3.5]nonane**

6-oxa-2-azaspiro[3.5]nonane (0.3 g, 2.36 mmol) and cesium carbonate (1.53 g, 4.72 mmol) were added to DMF (5 mL), and then 1-bromo-2-chloroethane (0.68 g, 4.72 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 120 mg of 2-(2-chloroethyl)-6-oxa-2-azaspiro[3.5]nonane. MS m/z(ESI): 190.2 $[M+H]^+$.

**Step 2: synthesis of 6-(2-(6-oxa-2-azaspiro[3.5]non-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 2-(2-chloroethyl)-6-oxa-2-azaspiro[3.5]nonane (84 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 50 mg of 6-(2-(6-oxa-2-azaspiro[3.5]non-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 33).** MS m/z(ESI):608.3 $[M+H]^+$. $^1H$ NMR(500 MHz, DMSO) δ 8.83(s, 1H), 8.58(s, 1H), 8.32(d, *J* = 2.4 Hz, 1H), 8.19(s, 1H), 7.67(dd, *J* = 8.4, 1.8 Hz, 1H), 7.31(s, 1H), 6.93(d, *J* = 8.9 Hz, 1H), 6.80(d, J= 8.4 Hz, 1H), 4.23(d, J= 62.7 Hz, 2H), 3.84(s, 3H), 3.66 - 3.42(m, 11H), 2.27(s, 6H), 1.62-1.33(m, 7H).

**Example 34: Synthesis of compound 34**

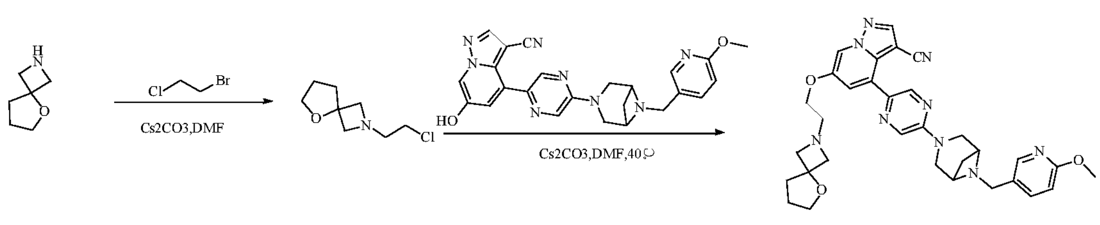


**Step 1: synthesis of 2-(2-chloroethyl)-5-oxa-2-azaspiro[3.4]octane**

5-oxa-2-azaspiro[3.4]octane (0.3 g, 2.65 mmol) and cesium carbonate (1.72 g, 5.30 mmol) were added to DMF (5 mL), and then 1-bromo-2-chloroethane (0.76 g, 5.30 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 130 mg of 2-(2-chloroethyl)-5-oxa-2-azaspiro[3.4]octane. MS m/z(ESI): 176.5 $[M+H]^+$.

**Step 2: synthesis of 6-(2-(5-oxa-2-azaspiro[3.4]oct-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 2-(2-chloroethyl)-5-oxa-2-azaspiro[3.4]octane (78 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 50 mg of 6-(2-(5-oxa-2-azaspiro[3.4]oct-2-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 34).** MS m/z(ESI):594.3 $[M+H]^+$.

$^1H$ NMR(500 MHz, DMSO) δ 8.71(d, *J*= 1.9 Hz, 1H), 8.67(d, J= 1.3 Hz, 1H), 8.61(s, 1H), 8.30(d, *J* = 1.2 Hz, 1H), 8.10(s, 1H), 7.70(d, *J* = 7.1 Hz, 1H), 7.61(d, *J*= 2.0 Hz, 1H), 6.78(d, *J* = 8.5 Hz, 1H), 4.15(s, 2H), 3.82(d, *J* = 12.8 Hz, 5H), 3.74 - 3.42(m, 11H), 3.23(s, 2H), 2.94(s, 2H), 2.56(s, 1H), 2.02(t, J= 7.2 Hz, 2H), 1.86 - 1.77(m, 2H).

**Example 35: Synthesis of compound 35**

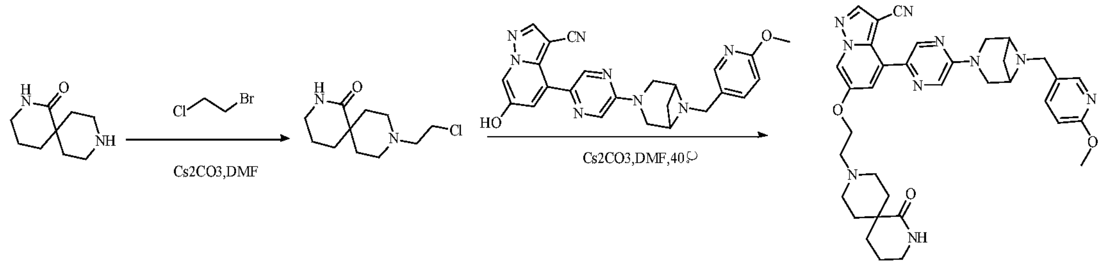


**Step 1: Synthesis of 9-(2-chloroethyl)-2,9-azaspiro[5.5]undecan-1-one**

2,9-diazaspiro[5.5]undecan-1-one (0.4 g, 2.17 mmol) and cesium carbonate (2.12 g, 6.51 mmol) were added to DMF (10 mL), and then 1-bromo-2-chloroethane (0.94 g, 6.51 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 160 mg of 9-(2-chloroethyl)-2,9-diazaspiro[5.5]undecan-1-one. MS m/z(ESI):247.3 $[M+H]^+$.

**Step 2: synthesis of 4-(5-(6-((6-methoxypyridin-3-yl)methyl)-3,6-diazabicyclo[3.1.1]heptan-3-yl)pyrazin-2-yl)-6-(2-(-1-oxo-2,9-diazaspiro[5.5]undecan-9-yl)ethoxy)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.15 g, 0.33 mmol), 9-(2-chloroethyl)-2,9-diazaspiro[5.5]undecan-1-one (163 g, 0.66 mmol), and cesium carbonate (0.22 g, 0.66 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 40 mg of 4-(5-(6-((6-methoxypyridin-3-yl)methyl)-3,6-diazabicyclo[3.1.1]heptan-3-yl)pyrazin-2-yl)-6-(2-(-1-oxo-2,9-diazaspiro[5.5]undecan-9-yl)ethoxy)pyrazolo[1,5-a]pyridine-3-carbonitrile. MS m/z(ESI):649.6 $[M+H]^+$.

$^1H$ NMR(500 MHz, DMSO) δ 8.77(d, J= 2.1 Hz, 1H), 8.67(d, *J*= 1.4 Hz, 1H), 8.62(s, 1H), 8.29(d, J= 1.3 Hz, 1H), 8.21(s, 1H), 8.00(d, J= 2.1 Hz, 1H), 7.70(dd, J= 8.5, 2.4 Hz, 1H), 7.62(d, *J* = 2.1 Hz, 1H), 6.77(d, *J* = 8.5 Hz, 1H), 4.25(t, *J* = 5.6 Hz, 2H), 3.84 - 3.75(m, 5H), 3.62(dt, *J* = 49.8, 10.5 Hz, 7H), 2.77(t, J= 5.4 Hz, 4H), 2.60(dd, J= 33.9, 6.2 Hz, 6H), 2.42(s, 2H), 1.67(dd, *J*= 19.3, 11.5 Hz, 4H), 1.57(s, 1H).

**Example 36: Synthesis of compound 36**

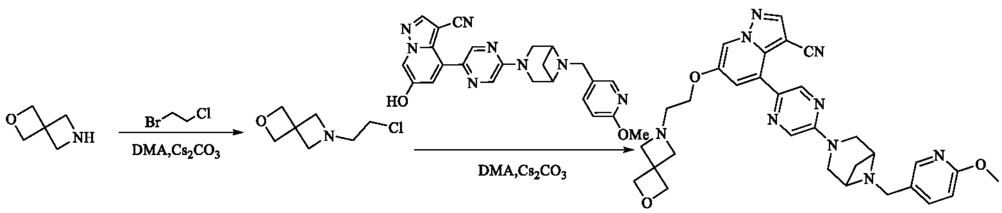


**Step 1: synthesis of 6-(2-chloroethyl)-2-oxa-6-azaspiro[3.3]heptane**

2-oxa-6-azaspiro[3.3]heptane (0.3 g, 3.03 mmol) and cesium carbonate (2.96 g, 9.09 mmol) were added to DMF (5 mL), and then 1-bromo-2-chloroethane (1.3 g, 9.09 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 180mg of 6-(2-chloroethyl)-2-oxa-6-azaspiro[3.3]heptane. MS m/z(ESI):162.3 $[M+H]^+$.

**Step 2: synthesis of 6-(2-(2-oxa-6-azaspiro[3.3]hept-6-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22mmol), 6-(2-chloroethyl)-2-oxa-6-azaspiro[3.3]heptane (74 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 60 mg of 6-(2-(2-oxa-6-azaspiro[3.3]hept-6-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 36).** MS m/z(ESI):580.5 $[M+H]^+$.

$^1$H NMR(400 MHz, DMSO) $\delta$ 8.66(dd, *J* = 7.0, 1.7 Hz, 2H), 8.59(s, 1H), 8.28(d, *J* = 1.3 Hz, 1H), 8.08(d, *J* = 2.0 Hz, 1H), 7.68(dd, *J* = 8.5, 2.4 Hz, 1H), 7.58(d, *J* = 2.1 Hz, 1H), 6.76(d, *J* = 8.5 Hz, 1H), 4.59(s, 4H), 4.05(t, *J*= 5.2 Hz, 2H), 3.79(d, *J* = 10.8 Hz, 5H), 3.71 - 3.48(m, 7H), 3.37(s, 5H), 2.73(t, *J* = 4.9 Hz, 2H).

**Example 37: Synthesis of compound 37**

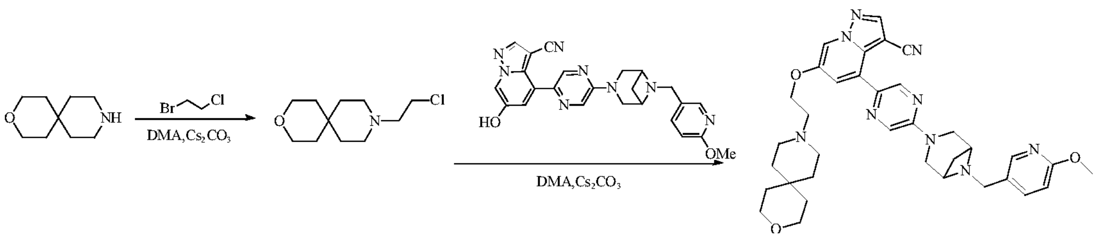


**Step 1: synthesis of 9-(2-chloroethyl)-3-oxa-9-azaspiro[5.5]undecane**

3-oxa-9-azaspiro[5.5]undecane (0.3 g, 1.93 mmol) and cesium carbonate (1.88 g, 5.80 mmol) were added to DMF (5 mL), and then 1-bromo-2-chloroethane (0.83 g, 5.80 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 180 mg of 9-(2-chloroethyl)-3-oxa-9-azaspiro[5.5]undecane. MS m/z(ESI):218.5 $[M+H]^+$.

**Step 2: synthesis of 6-(2-(3-oxa-9-azaspiro[5.5]undec-9-yl)ethoxy)-4-(5-(6-((6-methoxypyridin-3-yl)methyl)-3,6-diazabicyclo[3.1.1]heptan-3-yl)pyrazin-2-yl)pyrazolo [1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 9-(2-chloroethyl)-3-oxa-9-azaspiro[5.5]undecane (97 mg, 0.44 mmol), and cesium carbonate (0.15 g, 0.44 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 50 mg of 6-(2-(3-oxa-9-azaspiro[5.5]undec-9-yl)ethoxy)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 37).** MS m/z(ESI):636.4 [M+H]+. $^1$H NMR(500 MHz, DMSO) δ 8.73(s, 1H), 8.58(s, 1H), 8.32(d, *J* = 2.4 Hz, 1H), 7.76(dd, *J* = 8.8, 2.5 Hz, 1H), 7.67(dd, *J* = 8.4, 1.8 Hz, 1H), 7.31(s, 1H), 6.93(d, *J*= 8.9 Hz, 1H), 6.80(d, *J* = 8.4 Hz, 1H), 4.23(d, *J* = 62.7 Hz, 2H), 3.84(s, 3H), 3.66 - 3.42(m, 13H), 2.47(s, 4H), 1.62 - 1.33(m, 11H).

**Example 38: Synthesis of compound 38**

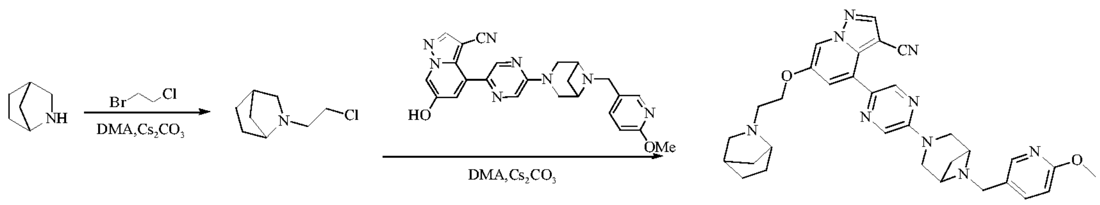


**Step 1: synthesis of 2-(2-chloroethyl)-2-azabicyclo[2.2.1]heptane**

2-azabicyclo[2.2.1]heptane (0.2 g, 2.06 mmol) and cesium carbonate (2.01 g, 6.18 mmol) were added to DMF (5 mL), and then 1-bromo-2-chloroethane (0.88 g, 6.18 mmol) was added to the resulting solution, and the mixed solution was stirred at room temperature overnight until the reaction was found to be completed from the monitoring. Water was added to quench the reaction, extraction with dichloromethane was then carried out, and the organic phase was washed with water and then dried, concentrated, and subjected to column chromatography to obtain 100 mg of 2-(2-chloroethyl)-2-azabicyclo[2.2.1]heptane. MS m/z(ESI):160.7 [M+H]+.

**Step 2: synthesis of 6-(2-(2-azabicyclo[2.2.1]hept-2-yl)ethoxy)-4-(5-(6-((6-methoxypyridine-3-yl)methyl)-3,6-diazabicyclo[3.1.1]heptan-3-yl)pyrazin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol), 2-(2-chloroethyl)-2-azabicyclo[2.2.1]heptane (71 mg, 0.44 mmol), and cesium carbonate (0.15 g, 0.44 mmol) were dissolved in DMA (5 mL) and the resulting solution was stirred at 80 °C overnight until the reaction was found to be completed from the monitoring. Water was added to quench the reaction, extraction with dichloromethane was then carried out, and the organic phase was washed with water and then dried, concentrated, and subjected to column chromatography to obtain 45 mg of 6-(2-(2-azabicyclo[2.2.1]hept-2-yl)ethoxy)-4-(5-(6-((6-methoxypyridine-3-yl)methyl)-3,6-diazabicyclo[3.1.1]heptan-3-yl)pyrazin-2-yl)pyrazolo[1,5-a]pyridine-3-carbonitrile **(compound 38).** MS m/z(ESI):578.1 [M+H]+. $^1$H NMR(400 MHz, DMSO) δ 8.81(s, 1H), 8.69 - 8.60(m, 2H), 8.29(d, *J* = 1.2 Hz, 1H), 8.08(s, 1H), 7.67(t, *J* = 5.1 Hz, 2H), 6.76(d, *J* = 8.5 Hz, 1H), 5.75(s, 1H), 4.44(s, 2H), 3.79(d, *J* = 8.8 Hz, 5H), 3.61(dd, *J* = 28.5, 15.9 Hz, 7H), 2.53(s, 2H), 2.02 - 1.79(m, 2H), 1.68 - 1.21(m, 7H), 0.91 - 0.74(m, 2H).

**Example 39: Synthesis of compound 39**

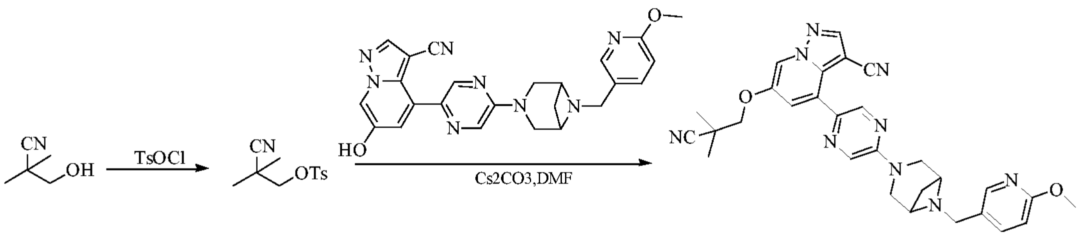

**Step 1: Synthesis of 2-cyano-2-methylpropyl 4-methylbenzenesulfonate**

3-hydroxy-2,2-dimethylpropionitrile (1.0 g, 10.1 mmol) and triethylamine (1.53 g, 15.2 mmol) were added to DCM (10 mL), the reaction solution was cooled to 0 °C, and then p-toluenesulfonyl chloride (1.92 g, 10.1 mmol) was slowly added. The reaction solution was then stirred at room temperature for 2 h to react, water was then added to quench the reaction, extraction with dichloromethane was then carried out, and the organic phase was washed with water and then dried, concentrated, and subjected to column chromatography to obtain 1.5 g of 2-cyano-2-methylpropyl 4-methylbenzenesulfonate.

**Step 2: synthesis of 6-(2-cyano-2-methylpropoxy)-4-(5-(6-((6-methoxypyridin-3-yl)methyl)-3,6-diazabicyclo[3.1.1]heptan-3-yl)pyrazinpyridin-2-yl)pyrazolo[1,5-a]pyridine-3-carbonitrile**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.15 g, 0.33 mmol), 2-cyano-2-methylpropyl 4-methylbenzenesulfonate (170 mg, 0.66 mmol), and cesium carbonate (0.22 g, 0.66 mmol) were dissolved in DMA (5 mL) and the resulting solution was stirred at 50 °C overnight until the reaction was found to be completed from the monitoring. Water was added to quench the reaction, extraction with dichloromethane was then carried out, and the organic phase was washed with water and then dried, concentrated, and subjected to column chromatography to obtain 60 mg of 6-(2-cyano-2-methylpropoxy)-4-(5-(6-((6-methoxypyridin-3-yl)methyl)-3,6-diazabicyclo[3.1.1]heptan-3-yl)pyrazinpyridin-2-yl)pyrazolo[1,5-a]pyridine-3-carbonitrile **(compound 39).** MS m/z(ESI):536.1 $[M+H]^+$.

$^1$H NMR(500 MHz, DMSO) δ 8.79(d, *J* = 2.1 Hz, 1H), 8.68(d, *J* = 1.4 Hz, 1H), 8.62(s, 1H), 8.28(d, *J* = 1.4 Hz, 1H), 8.08(s, 1H), 7.68(dd, *J* = 7.4, 2.2 Hz, 2H), 6.75(d, *J* = 8.5 Hz, 1H), 4.20(s, 2H), 3.79(d, *J* = 11.5 Hz, 5H), 3.71 - 3.49(m, 7H), 2.53(s, 1H), 1.44(s, 6H).

**Example 40: Synthesis of compound 40**

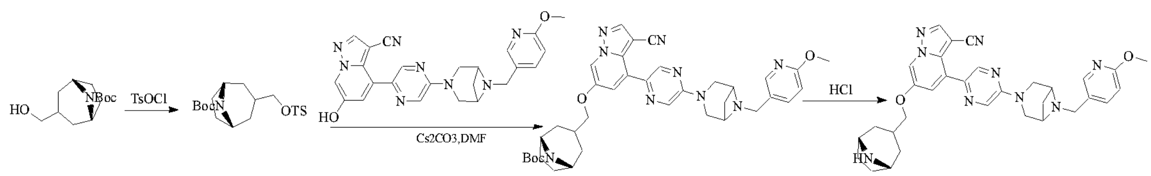


**Step 1: Synthesis of tert-butyl (1R, 5S)-3-((toluenesulfonyloxy)methyl)-8-azabicyclo[3.2.1]octane-8-carboxylate**

Tert-butyl (1R, 5S)-3-(hydroxymethyl)-8-azabicyclo[3.2.1]octane-8-carboxylate (0.5 g, 2.07 mmol) and triethylamine (0.32 g, 3.11 mmol) were added to DCM (10 mL), the reaction solution was cooled to 0 °C, and then p-toluenesulfonyl chloride (0.4 g, 2.07 mmol) was slowly added. The reaction solution was then stirred at room temperature for 2 h to react, water was then added to quench the reaction, extraction with dichloromethane was then carried out, and the organic phase was washed with water and then dried, concentrated, and subjected to column chromatography to obtain 0.6 g of tert-butyl (1R, 5S)-3-((toluenesulfonyloxy)methyl)-8-azabicyclo[3.2.1]octane-8-carboxylate.

**Step 2: synthesis of tert-butyl(1R, 3s, 5S)-3-(((3-cyano-4-(5-(6-((6-methoxypyridin-3-yl)methyl)-3,6-diazabicyclo[3.1.1]]heptan-3-yl)pyrazin-2-yl)pyrazolo[1,5-a]pyridin-6-yl)oxy)methyl)-8-azabicyclo[3.2.1]octane-8-tert-butyl formate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.15 g, 0.33 mmol), tert-butyl (1R, 5S)-3-((toluenesulfonyloxy)methyl)-8-azabicyclo[3.2.1]octane-8-carboxylate (182 mg, 0.66 mmol), and cesium carbonate (0.22 g, 0.66 mmol) were dissolved in DMF (8 mL) and the resulting solution was stirred at 80 °C overnight until the reaction was found to be completed from the monitoring. Water was then added to quench the reaction, extraction with dichloromethane was carried out, and the organic phase was then washed with water, and then dried, concentrated, and subjected to column chromatography to obtain 110 mg of tert-butyl(1R, 3s, 5S)-3-(((3-cyano-4-(5-(6-((6-methoxypyridin-3-yl)methyl)-3,6-diazabicyclo[3.1.1]]heptan-3-yl)pyrazin-2-yl)pyrazolo[1,5-a]pyridin-6-yl)oxy)methyl)-8-azabicyclo[3.2.1]octane-8-tert-butyl formate. MS m/z(ESI):678.4 $[M+H]^+$.

**Step 3: synthesis of 6-(((1R, 5S)-8-azabicyclo[3.2.1]oct-3-yl)methoxy)-4-(5-(6-((6-methoxypyridine-3-yl)methyl)-3,6diazabicyclo[3.1.1]heptan-3-yl)pyrazin-2-yl)pyrazolo[1,5-a]pyridine-3-carbonitrile**

Tert-butyl(1R, 3s, 5S)-3-(((3-cyano-4-(5-(6-((6-methoxypyridin-3-yl)methyl)-3,6-diazabicyclo[3.1.1]]heptan-3-yl)pyrazin-2-yl)pyrazolo[1,5-a]pyridin-6-yl)oxy)methyl)-8-azabicyclo[3.2.1]octane-8-tert-butyl formate (0.11 g, 0.16 mmol) was dissolved in DCM (5 mL), the resulting solution was then cooled to 0°C, a dioxane hydrochloride solution (2 mL, 4 mol/L) was then added, and the reaction solution was stirred for 2 h to react until the reaction was completed from the monitoring. The reaction solution was spin-dried, and then a small amount of water was added. Extraction with dichloromethane was then carried out, and the organic phase was washed with water and then dried, concentrated, and subjected to column chromatography to obtain 40 mg of 6-(((1R, 5S)-8-azabicyclo[3.2.1]oct-3-yl)methoxy)-4-(5-(6-((6-methoxypyridine-3-yl)methyl)-3,6diazabicyclo[3.1.1]heptan-3-yl)pyrazin-2-yl)pyrazolo[1,5-a]pyridine-3-carbonitrile. MS m/z(ESI):578.3 [M+H]+.

$^1$H NMR(400 MHz, DMSO) δ 8.77(d, *J* = 2.0 Hz, 1H), 8.65(d, *J* = 1.2 Hz, 1H), 8.59(s, 1H), 8.27(d, *J* = 1.1 Hz, 1H), 8.08(d, *J* = 2.1 Hz, 1H), 7.68(dd, *J* = 8.5, 2.4 Hz, 1H), 7.59(d, *J* = 2.0 Hz, 1H), 6.75(d, *J* = 8.5 Hz, 1H), 4.06(d, *J*= 7.9 Hz, 2H), 3.79(d, *J*= 11.4 Hz, 5H), 3.71 -3.48(m, 7H), 3.44(s, 4H), 2.22(dd, *J* = 15.8, 7.9 Hz, 1H), 2.03 - 1.89(m, 2H), 1.69(s, 3H), 1.56(dd, *J* = 24.8, 11.3 Hz, 3H).

**Example 41: Synthesis of compound 41**

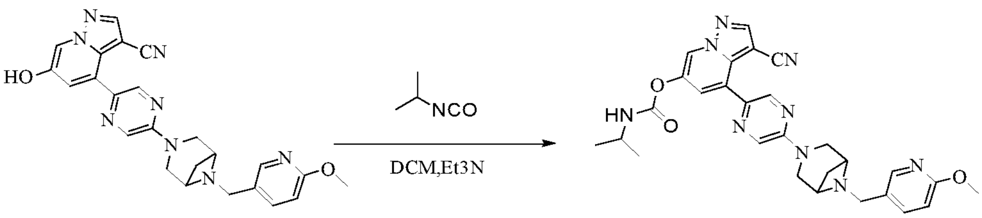


**Step 1: synthesis of 3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-ylisopropylcarbamate**

6-hydroxy-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile (0.1 g, 0.22 mmol) and triethylamine (0.067 g, 0.66 mmol) were dissolved in DCM (5 mL), the mixed solution was cooled to 0 °C, isopropyl isocyanate (0.057 g, 0.66 mmol) was then added to the mixed solution, and the reaction was carried out for 1 h. Water was then added to quench the reaction, extraction with dichloromethane was then carried out, and the organic phase was washed with water and then dried, concentrated, and subjected to column chromatography to obtain 45 mg of 3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-ylisopropylcarbamate **(compound 41).** MS m/z(ESI):540.7 $[M+H]^+$.

$^1$H NMR(500 MHz, DMSO) δ 9.04(d, *J* = 1.8 Hz, 1H), 8.71(s, 1H), 8.66(d, *J* = 1.1 Hz, 1H), 8.31(d, *J* = 1.2 Hz, 1H), 8.10(d, *J* = 1.9 Hz, 1H), 7.99(d, *J* = 7.7 Hz, 1H), 7.75 - 7.65(m, 2H), 6.76(d, *J* = 8.5 Hz, 1H), 3.81(d, *J* = 12.0 Hz, 5H), 3.74 - 3.50(m, 7H), 2.55(d, *J* = 7.0 Hz, 1H), 1.62(d, *J* = 8.5 Hz, 1H), 1. 16(d, *J* = 6.6 Hz, 6H).

**Example 42: Synthesis of compound 42**

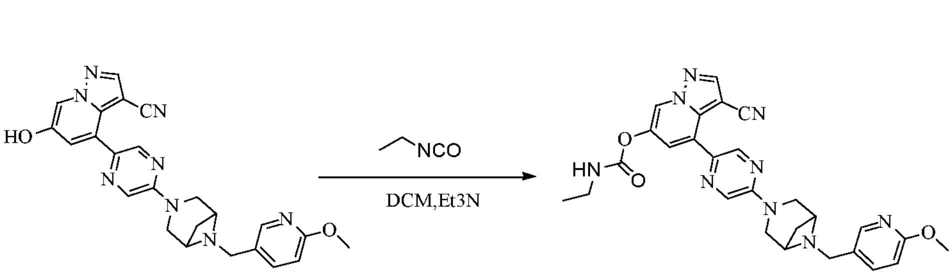

**Step 1: synthesis of 3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-ylethylcarbamate**

6-hydroxy-4-(5-(6-((6-methoxypyridin-3-yl)methyl)-3,6-diazabicyclo[3 .1.1]heptan-3-yl)pyrazin-2-yl)pyrazolo[1,5-a]pyridine-3-carbonitrile (0.1 g, 0.22 mmol) and triethylamine (0.067 g, 0.66 mmol) were dissolved in DCM (5 mL), the mixed solution was cooled to 0 °C, ethyl isocyanate (0.047 g, 0.66 mmol) was then added to the mixed solution, and the reaction was carried out for 1 h. Water was then added to quench the reaction, extraction with dichloromethane was then carried out, and the organic phase was washed with water and then dried, concentrated, and subjected to column chromatography to obtain 35 mg of 3-cyano-4-(5-(6-((6-methoxypyridin-3-yl)methyl)-3,6-diazabicyclo[3.1.1]heptan-3-yl)pyrazin-2-yl)pyrazolo[1,5-a]pyridin-6-ylethylcarbamate (compound 42). MS m/z(ESI):526.3 $[M+H]^+$.

$^1H$ NMR(500 MHz, DMSO) δ 9.05(d, *J* = 1.9 Hz, 1H), 8.72(s, 1H), 8.66(d, *J* = 1.2 Hz, 1H), 8.31(d, *J* = 1.2 Hz, 1H), 8.13 - 8.01(m, 2H), 7.76 - 7.65(m, 2H), 6.77(d, *J* = 8.5 Hz, 1H), 3.81(d, *J* = 12.3 Hz, 5H), 3.72 - 3.49(m, 7H), 3.14(dt, *J* = 12.9, 6.5 Hz, 2H), 2.98(dd, *J* = 7.1, 5.7 Hz, 1H), 2.55(d, *J* = 7.4 Hz, 1H), 1.12(t, *J* = 7.2 Hz, 3H).

**Example 47: Synthesis of compound 47**

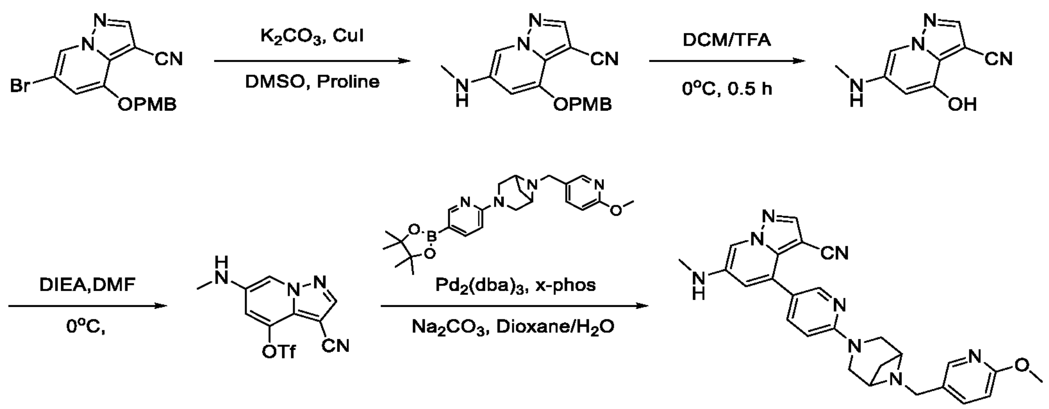


**Step 1: synthesis of 4-((4-methoxybenzyl)oxy)-6-(methylamino)pyrazolo[1,5-a]pyridine-3-carbonitrile**

At room temperature, 6-bromo-4-((4-methoxybenzyl)oxy)pyrazolo[1,5-a]pyridine-3-carbonitrile (500 mg, 1.4 mmol), CuI (40 mg, 0.21 mmol), L-proline (32 mg, 0.28 mmol), and potassium carbonate (1.9 g, 14 mmol) were dissolved in anhydrous DMSO (15 mL), and methylamine hydrochloride (940 mg, 14 mmol) was then added to the resulting solution under the protection of $N_2$. The reaction was carried out at 100 °C for 10 h. After the reaction was completed, the reaction solution was cooled to room temperature, extracted with EA (50 mL*2), diluted with water (50 mL), stirred, filtered, and separated; and the organic phase was extracted, dried, filtered, concentrated, and subjected to column chromatography (PE: EA=3:1) to obtain 4-((4-methoxybenzyl)oxy)-6-(methylamino)pyrazolo[1,5-a]pyridine-3-carbonitrile (150 mg). MS m/z (ESI): 309.1 $[M+H]^+$.

**Step 2: synthesis of 4-hydroxy-6-(methylamino)pyrazolo[1,5-a]pyridine-3-carbonitrile**

At room temperature, 4-((4-methoxybenzyl)oxy)-6-(methylamino)pyrazolo[1,5-a]pyridine-3-carbonitrile (150 mg, 0.48 mmol) was dissolved in 3 mL of DCM, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 189.1 $[M+H]^+$.

**Step 3: synthesis of 3-cyano-6-(methylamino)pyrazolo[1,5-a]pyridin-4-yl trifluoromethanesulfonate**

4-hydroxy-6-(methylamino)pyrazolo[1,5-a]pyridine-3-carbonitrile (150 mg, 0.8 mmol) and DIEA (307 mg, 2.4 mmol) were dissolved in 5 mL of DMF, N-phenylbis(trifluoromethanesulfonyl)imide (286 mg, 0.8 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-(methylamino)pyrazolo[1,5-a]pyridin-4-yl trifluoromethanesulfonate (180

mg). MS m/z (ESI): 321.3 [M+H]+.

**Step 4: synthesis of 4-(6-(6-((6-methoxypyridin-3-yl)methyl)-3,6-diazabicyclo[3.1.1]heptyl-3-yl)pyridin-3-yl)-6-(methylamino)pyrazolo[1,5-a]pyridine-3-carbonitrile**

At room temperature, 3-cyano-6-(methylamino)pyrazolo[1,5-a]pyridin-4-yl trifluoromethanesulfonate (0.15 g, 0.46 mmol), 6-((6-methoxypyridin-3-yl)methyl)-3-(5-(4,4,5,5-tetramethyl-1,3-dioxaboran-2-yl)pyridine-2-yl)-3,6-diazabicyclo[3.1.1]heptane (0.20 g, 0.46 mmol), $Pd_2(dba)_3$ (46 mg, 0.05 mmol), and x-phos (24 mg, 0.05 mmol) were dissolved in Dioxane/$H_2O$=20 mL/4 mL, nitrogen replacement was carried out three times, and the resulting solution reacted at 100 °C for 6 h. After the reaction was completed, the reaction solution was cooled, concentrated, and extracted with DCM; the organic phase was dried, filtered, concentrated, and subjected to column chromatography (DCM:MeOH=10:1) to obtain 60 mg of 4-(6-(6-((6-methoxypyridin-3-yl)methyl)-3,6-diazabicyclo[3.1.1]heptyl-3-yl)pyridin-3-yl)-6-(methylamino)pyrazolo[1,5-a]pyridine-3-carbonitrile **(compound 47).** MS m/z (ESI): 467.2 [M+H]+.

$^1$H NMR (500 MHz, DMSO) δ 8.28 (s, 1H), 8.13 (d, *J* = 1.4 Hz, 1H), 8.07 (s, 1H), 7.92 (d, *J* = 2.7 Hz, 1H), 7.79 (dd, *J* = 8.8, 2.5 Hz, 1H), 7.67 (d, *J* = 9.8 Hz, 1H), 7.05 (d, *J* = 1.8 Hz, 1H), 6.76 (t, *J* = 8.1 Hz, 2H), 5.92 (t, *J* = 5.4 Hz, 1H), 3.85 (d, *J* = 3.4 Hz, 4H), 3.76 - 3.62 (m, 5H), 3.50 (s, 4H), 1.03 (t, *J* = 7.4 Hz, 3H).

**Example 48: Synthesis of compound 48**

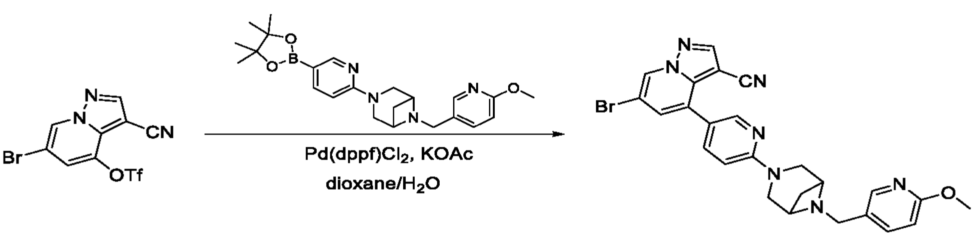


**Step 1: synthesis of 6-bromo-4-(6-(6-(((6-methoxypyridin-3-yl)methyl)-3,6-diazabicyclo[3.1.1]heptyl-3-yl)pyridin-3-yl)pyrazolo[1,5-a]pyridine-3-carbonitrile**

6-bromo-3-cyanopyrazolo[1,5-a]pyridin-4-yl trifluoromethanesulfonate (1.0 g, 2.7 mmol) was dissolved in 1,4-dioxane (15 mL) and water (2 mL), and then 6-((6-methoxypyridin-3-yl)methyl)-3-(5-(4,4,5,5-tetramethyl-1,3-dioxaboran-2-yl)pyridin-2-yl)-3,6-diazabicyclo[3.1.1]heptane (1.1 g, 2.7 mmol), $Pd(dppf)_2Cl_2$ (0.22 g , 0.27 mmol), KOAc (0.8 g, 8.1 mmol) were added, and nitrogen replacement was carried out. The reaction solution was stirred at room temperature for 16 h. After the reaction was found to be completed from the monitoring, the reaction solution was diluted with water and then extracted with ethyl acetate (30 mL*2), and the organic phase was washed with water and then dried, concentrated, and subjected to column chromatography to obtain 0.7 g of 6-bromo-4-(6-(6-(((6-methoxypyridin-3-yl)methyl)-3,6-diazabicyclo[3.1.1]heptyl-3-yl)pyridin-3-yl)pyrazolo[1,5-a]pyridine-3-carbonitrile **(compound 48)** with a yield of 54%. MS m/z (ESI): 517.2 [M+H]+.

$^1$H NMR (500 MHz, DMSO) δ 9.31 (d, *J* = 1.6 Hz, 1H), 8.69 (s, 1H), 8.45 - 8.34 (m, 1H), 8.04 (d, *J* = 2.0 Hz, 1H), 7.82 (dd, *J* = 8.8, 2.5 Hz, 1H), 7.64 (t, *J* = 1.9 Hz, 2H), 6.76 (dd, *J* = 18.0, 8.6 Hz, 2H), 3.80 (s, 4H), 3.72 (d, *J* = 11.8 Hz, 2H), 3.66 (d, *J* = 5.4 Hz, 2H), 3.51 (d, *J* = 20.6 Hz, 5H).

**Example 49: Synthesis of compound 49**

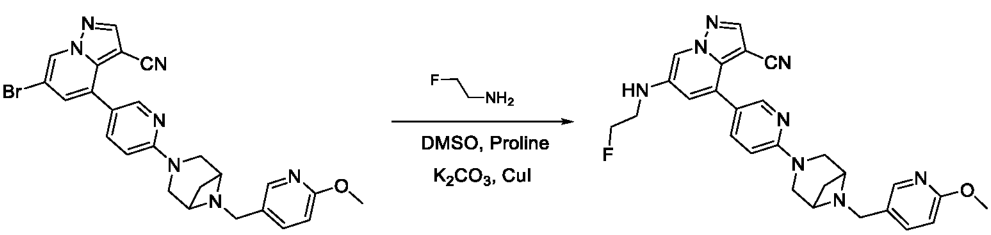

**Step 1: synthesis of 6-((2-fluoroethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile**

At room temperature and under the protection of nitorgen gas, 6-bromo-4-(6-(6-(((6-methoxypyridin-3-yl)methyl)-3,6-diazabicyclo[3.1.1]heptyl-3-yl)pyridin-3-yl)pyrazolo[1,5-a]pyridine-3-carbonitrile (600 mg, 1.2 mmol), CuI (22 mg, 0.12 mmol), L-proline (14 mg, 0.12 mmol), and potassium carbonate (1.6 g, 12 mmol) were dissolved in anhydrous DMSO (15 mL), and 2-fluoroethylamine hydrochloride (1.2 g, 12 mmol) was then added to the resulting solution. Reaction was carried out at 100 °C for 10 h. After the reaction was completed, the reaction solution was cooled to room temperature and then diluted with water (30 mL), extracted with EA (50 mL*2), stirred and filtered, and separated. The organic phase was dried, filtered, concentrated, and subjected to column chromatography (DCM:MeOH=10 : 1) to obtain 50 mg of 6-((2-fluoroethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile **(compound 49).** MS m/z (ESI): 499.3 $[M+H]^+$.

$^1$H NMR (400 MHz, DMSO) δ 8.40 (s, 1H), 8.34 (d, *J* = 2.4 Hz, 1H), 8.09 (t, *J* = 3.2 Hz, 2H), 7.77 (dd, *J* = 8.8, 2.5 Hz, 1H), 7.68 (d, *J* = 7.6 Hz, 1H), 7.11 (d, *J* = 1.8 Hz, 1H), 6.77 (t, *J* = 7.7 Hz, 2H), 6.25 (t, *J* = 5.8 Hz, 1H), 5.74 (s, 1H), 4.68 (t, *J* = 4.7 Hz, 1H), 4.56 (t, *J* = 4.7 Hz, 1H), 3.81 (s, 3H), 3.78 - 3.62 (m, 4H), 3.50 (dd, *J* = 25.0, 11.4 Hz, 6H).

**Example 50: Synthesis of compound 50**

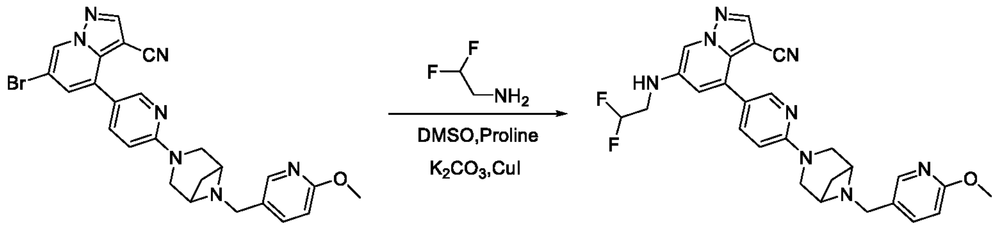


**Step 1: synthesis of 6-(((2,2-Difluoroethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile**

At room temperature and under the protection of nitorgen gas, 6-bromo-4-(6-(6-(((6-methoxypyridin-3-yl)methyl)-3,6-diazabicyclo[3.1.1]heptyl-3-yl)pyridin-3-yl)pyrazolo[1,5-a]pyridine-3-carbonitrile (600 mg, 1.2 mmol), CuI (22 mg, 0.12 mmol), L-proline (14 mg, 0.12 mmol), and potassium carbonate (1.6 g, 12 mmol) were dissolved in anhydrous DMSO (15 mL), and 2-fluoroethylamine hydrochloride (1.2 g, 12 mmol) was then added to the resulting solution. Reaction was carried out at 100 °C for 10 h. After the reaction was completed, the reaction solution was cooled to room temperature and then diluted with water (30 mL), extracted with EA (50 mL*2), stirred and filtered, and separated. The organic phase was dried, filtered, concentrated, and subjected to column chromatography (DCM:MeOH=10 : 1) to obtain 80 mg of 6-(((2,2-Difluoroethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile **(compound** 50). MS m/z (ESI): 517.3 $[M+H]^+$.

$^1$H NMR (500 MHz, DMSO) δ 8.43 (s, 1H), 8.37 - 8.32 (m, 1H), 8.24 (d, *J* = 1.9 Hz, 1H), 8.07 (s, 1H), 7.78 (dd, *J* = 8.8, 2.5 Hz, 1H), 7.68 (dd, *J* = 8.5, 2.3 Hz, 1H), 7.14 (d, *J* = 2.0 Hz, 1H), 6.77 (dd, *J* = 10.8, 8.8 Hz, 2H), 6.32 (t, *J* = 6.4 Hz, 1H), 3.81 (s, 3H), 3.76 - 3.47 (m, 10H), 2.53 (s, 1H), 1.58 (d, *J* = 8.4 Hz, 1H), 1.22 (s, 1H).

**Example 51: Synthesis of compound 51**

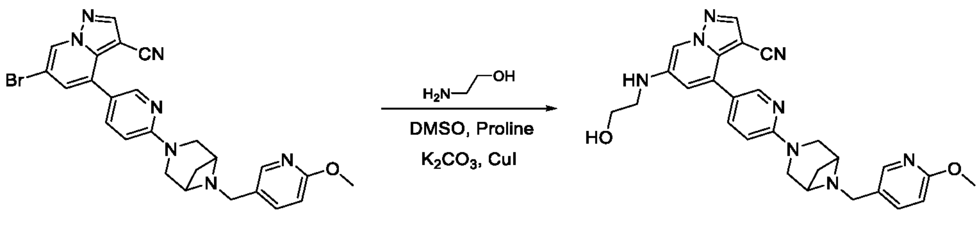

**Step 1: synthesis of 6-((2-hydroxyethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile**

At room temperature and under the protection of nitorgen gas, 6-bromo-4-(6-(6-(((6-methoxypyridin-3-yl)methyl)-3,6-diazabicyclo[3.1.1]heptyl-3-yl)pyridin-3-yl)pyrazolo[5-a]pyridine-3-carbonitrile (600 mg, 1.2 mmol), CuI (22 mg, 0.12 mmol), L-proline (14 mg, 0.12 mmol), and potassium carbonate (500 mg, 3.6 mmol) were dissolved in anhydrous DMSO (15 mL), and 2-hydroxyethylamine (220 mg, 3.6 mmol) was then added to the resulting solution. Reaction was carried out at 100 °C for 10 h. After the reaction was completed, the reaction solution was cooled to room temperature and then diluted with water (30 mL), extracted with EA (50 mL*2), stirred and filtered, and separated. The organic phase was dried, filtered, concentrated, and subjected to column chromatography (DCM:MeOH=10 : 1) to obtain 40 mg of 6-((2-hydroxyethyl)amino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile **(compound** 51). MS m/z (ESI): 497.3 $[M+H]^+$. $^1H$ NMR (400 MHz, DMSO) $\delta$ 8.39 (s, 1H), 8.33 (d, $J$ = 2.4 Hz, 1H), 8.07 (s, 1H), 8.00 (d, $J$ = 1.8 Hz, 1H), 7.77 (dd, $J$ = 8.8, 2.3 Hz, 1H), 7.68 (d, $J$ = 7.9 Hz, 1H), 7.12 (d, $J$ = 1.9 Hz, 1H), 6.77 (t, $J$ = 8.0 Hz, 2H), 5.96 (t, $J$ = 5.6 Hz, 1H), 4.78 (t, $J$ = 5.3 Hz, 1H), 3.81 (s, 3H), 3.61 (ddd, $J$= 41.1, 32.8, 17.3 Hz, 9H), 3.14 (q, $J$= 5.6 Hz, 2H), 1.58 (s, 1H).

**Example 52: Synthesis of compound 52**

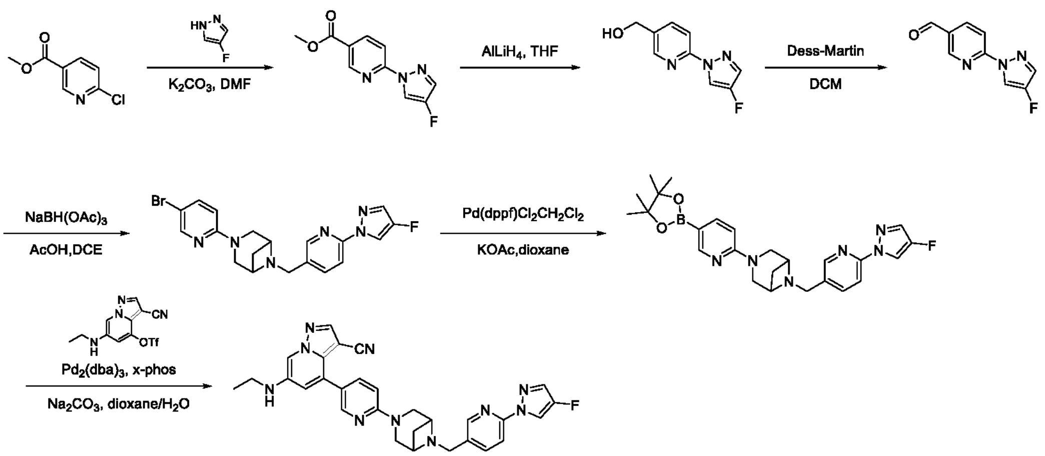


**Step 1: Synthesis of methyl 6-(4-fluoro-1H-pyrazol-1-yl)nicotinate**

Methyl 6-chloronicotinate (2.0 g, 11.65 mmol), 4-fluoro-1H-pyrazole (770 mg, 8.96 mmol), and potassium carbonate (3.7 g, 34.95 mmol) were dissolved in 30 mL of anhydrous DMF at room temperature, under nitrogen protection, and the resulting solution reacted at 80 °C overnight under the protection of $N_2$. After the reaction was completed, the reaction was cooled to room temperature, 250 mL of water was then added, and the reaction solution was then stirred for 0.5 h and then filtered; the filter cake was washed with water for many times, and then dried to obtain 1.9 g of methyl 6-(4-fluoro-1H-pyrazol-1-yl)nicotinate with a yield of 95%. MS m/z (ESI): 222.1 $[M+H]^+$.

**Step 2: Synthesis of (6-(4-fluoro-1H-pyrazol-1-yl)pyridin-3-yl)methanol**

At room temperature, methyl 6-(4-fluoro-1H-pyrazol-1-yl)nicotinate (1.8 g, 8.0 mmol) was dissolved in 50 mL of anhydrous THF, and the resulting solution was then cooled to 0 °C under the protection of $N_2$; lithium aluminum hydrogen (300 mg, 8.0 mmol) was then added, and the mixed solution reacted at 0 °C for 15 min. After the reaction was completed, the reaction was quenched with 1 mL of methanol, and the reaction solution was then diluted with EA (50 mL) and water (50 mL), extracted, dried, filtered, and concentrated to obtain 1.44 g of (6-(4-fluoro-1H-pyrazol-1-yl)pyridin-3-yl)methanol with a yield of 91%. MS m/z (ESI): 194.1 $[M+H]^+$.

**Step 3: Synthesis of 6-(4-fluoro-1H-pyrazol-1-yl)nicotinaldehyde**

At room temperature, (6-(4-fluoro-1H-pyrazol-1-yl)pyridin-3-yl)methanol (1.1 g, 5.6 mmol) was dissolved in 40 mL of anhydrous DCM, and the resulting solution was then cooled to 0 °C; Dess-Martin (2.8 g, 6.74 mmol) was then

added, and the mixed solution reacted at room temperature for 0.5 h. After the reaction was completed, the reaction solution was then extracted, dried, filtered, concentrated, and subjected to column chromatography to obtain 0.8 g of 6-(4-fluoro-1H-pyrazol-1-yl)nicotinaldehyde with a yield of 74 %. MS m/z (ESI): 191.1 [M+H]$^+$.

**Step 4: synthesis of 3-(5-bromopyridin-2-yl)-6-((6-(4-fluoro-1H-pyrazol-1-yl)pyridin-3-yl)methyl)-3,6-diazabicyclo[3.1.1]heptane**

At room temperature, 6-(4-fluoro-1H-pyrazol-1-yl)nicotinaldehyde (0.8 g, 4.1 mmol), 3-(5-bromopyridin-2-yl)-3,6-diazabicyclo[3.1.1]heptane (815 mg, 3.2 mmol), and 1 drop of acetic acid were dissolved in 40 mL of DCE, and the resulting solution was then cooled to 0 °C; after the reaction was carried out for 0.5 h, sodium borohydride acetate (2.0 g, 9.6 mmol) was then added, and the mixed solution reacted at room temperature for 2 h. After the reaction was completed, the reaction solution was then extracted, dried, filtered, concentrated, and subjected to column chromatography to obtain 310 mg of 3-(5-bromopyridin-2-yl)-6-((6-(4-fluoro-1H-pyrazol-1-yl)pyridin-3-yl)methyl)-3,6-diazabicyclo[3.1.1]heptane with a yield of 21%. MS m/z (ESI): 429.1 [M+H]$^+$.

**Step 5: synthesis of 6-((6-(4-fluoro-1H-pyrazol-1-yl)pyridin-3-yl)methyl)-3-(5-(4,4,5,5-tetramethyl-1,3,2-dioxaboran-2-yl)pyridin-2-yl)-3,6-diazabicyclo[3.1.1]heptane**

At room temperature, 3-(5-bromopyridin-2-yl)-6-((6-(4-fluoro-1H-pyrazol-1-yl)pyridin-3-yl)methyl)-3,6-diazabicyclo[3.1.1]heptane (306 mg, 0.7 mmol), pinacol diboronate (533 mg, 2.1 mmol), Pd(dppf)$Cl_2CH_2Cl_2$ (57 mg, 0.07 mol), and KOAc (206 mg, 2.1 mol) were dissolved in 10 mL of Dioxane, and the resulting solution reacted at 90 °C overnight. After the reaction was completed, the reaction solution was cooled to room temperature and then extracted, dried, filtered, concentrated, and subjected to column chromatography to obtain 160 mg of 6-((6-(4-fluoro-1H-pyrazol-1-yl)pyridin-3-yl)methyl)-3-(5-(4,4,5,5-tetramethyl-1,3,2-dioxaboran-2-yl)pyridin-2-yl)-3,6-diazabicyclo[3.1.1]heptane with a yield of 46%. MS m/z (ESI): 477.1 [M+H]$^+$.

**Step 6: synthesis of 6-(ethylamino)-4-(6-(6-((6-(4-fluoro-1H-pyrazol-1-yl)pyridin-3-yl)methyl)-3,6-diazabicyclo[3.1.1]heptan-3-yl)pyridin-3-yl)pyrazolo[1,5-a]pyridine-3-carbonitrile**

At room temperature, 3-cyano-6-(ethylamino)pyrazo[1,5-a]pyridin-4-yl trifluoromethanesulfonate (102 mg, 0.3 mmol), 6-((6-(4-fluoro-1H-pyrazol-1-yl)pyridin-3-yl)methyl)-3-(5-(4,4,5,5-tetramethyl-1,3,2-dioxaboran-2-yl)pyridin-2-yl)-3,6-diazabicyclo[3.1.1]heptane (160 mg, 0.33 mmol), $Pd_2(dba)_3$ (27 mg, 0.03 mmol), and x-phos (28 mg, 0.06 mmol) were dissolved in Dioxane/$H_2O$=10 mL/1 mL, nitrogen replacement was carried out three times, and the resulting solution reacted at 90 °C overnight. After the reaction was completed, the reaction solution was cooled, concentrated, diluted and extracted; the organic phase was dried, filtered, concentrated, and subjected to column chromatography (DCM:MeOH=10:1) to obtain 51 mg of 6-(ethylamino)-4-(6-(6-((6-(4-fluoro-1H-pyrazol-1-yl)pyridin-3-yl)methyl)-3,6-diazabicyclo[3.1.1]heptan-3-yl)pyridin-3-yl)pyrazolo[1,5-a]pyridine-3-carbonitrile **(compound 52)** with a yield of 33%. MS m/z (ESI): 535.1 [M+H]$^+$.

$^1$H NMR (400 MHz, DMSO) δ 8.67 (s, 1H), 8.41 (d, 2H), 8.34 (s, 1H), 7.80-8.00 (m, 4H), 7.77-7.79 (d 1H), 7.04 (s, 1H), 6.78 (d, 2H), 5.93 (m, 1H), 3.73-3.77 (m, 4H), 3.64 (s, 2H), 3.56-3.59 (m, 2H), 3.06-3.09(m, 2H),2.56-2.58(m, 1H),1.61-1.63(d, 1H), 1.21-1.24 (m, 4H).

**Example 53: Synthesis of compound 53**

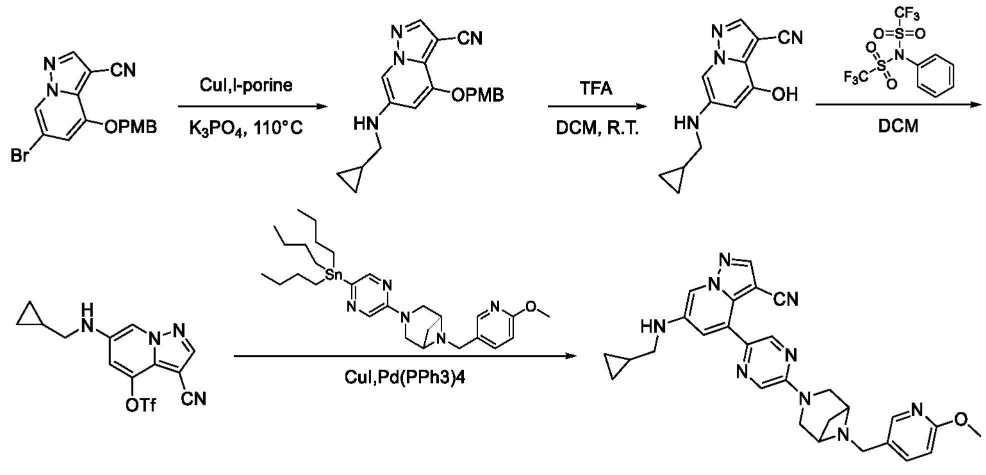


**Step 1: synthesis of 6-((cyclopropylmethyl)amino)-4-((4-methoxybenzyl)oxy)pyrazolo[1,5-a] pyridine-3-carbonitrile**

At room temperature, 6-bromo-4-((4-methoxybenzyl)oxy)pyrazolo[1,5-a]pyridine-3-carbonitrile (0.6 g, 1.7 mmol), CuI (32 mg, 0.17 mmol), L-proline (39 mg, 0.34 mmol), and potassium phosphate (1.1 g, 5.1 mmol) were dissolved in anhydrous DMSO (10 mL), and cyclopropylmethylamine (0.36 g, 5.1 mmol) was then added to the resulting solution under the protection of $N_2$. Reaction was carried out at 120 °C for 5 h. After the reaction was completed, the reaction solution was cooled to room temperature, water (10 mL) was then added to quench the reaction, extraction with EA (30 mL*2) was then carried out, and the organic phase was dried, filtered, concentrated, and subjected to column chromatography (PE:EA=3:1) to obtain 0.3 g of 6-((cyclopropylmethyl)amino)-4-((4-methoxybenzyl)oxy)pyrazolo[1,5-a]pyridine-3-carbonitrile. MS m/z (ESI): 349.1 $[M+H]^+$.

**Step 2: synthesis of 6-((cyclopropylmethyl)amino)-4-hydroxypyrazolo[1,5-a]pyridine-3-carbonitrile**

6-((cyclopropylmethyl)amino)-4-((4-methoxybenzyl)oxy)pyrazolo[1,5-a]pyridine-3-carbonitrile (0.2 g, 0.57 mmol) was dissolved in DCM (6 mL) at room temperature, the resulting solution was then cooled to 0 °C, TFA (2 mL) was then added, and the reaction was continued for half an hour. After the reaction was completed, the reaction solution was directly spin-dried for the next step without further purification. MS m/z (ESI) :228.9 $[M+H]^+$.

**Step 3: synthesis of 3-cyano-6-((cyclopropylmethyl)amino)pyrazolo[1,5-a]pyridin-4-yl trifluoromethanesulfonate**

6-((cyclopropylmethyl)amino)-4-hydroxypyrazolo[1,5-a]pyridine-3-carbonitrile (0.15 g, 0.66 mmol) and DIEA (0.26 g, 1.96 mmol) were dissolved in DMF (5 mL), N-phenylbis(trifluoromethanesulfonyl)imide (0.35 g, 1.0 mmol) was then added, and the mixed solution was stirred at room temperature for 0.5 h to react. After the reaction was completed, water (10 mL) was added to quench the reaction, extraction with EA (20 mL*2) was then carried out, and the organic phase was dried, filtered, concentrated, and subjected to column chromatography (PE:EA =7:1) to obtain 0.12 g of 3-cyano-6-(cyclopropylmethyl)amino)pyrazolo[1,5-a]pyridin-4-yl trifluoromethanesulfonate. MS m/z (ESI): 361.1 $[M+H]^+$.

**Step 4: synthesis of 6-((cyclopropylmethyl)amino)-4-(5-(6-(((6-methoxypyridin-3-yl)methyl)-3,6-diazabicyclo[3.1.1]hept-3-yl)pyrazin-2-yl)pyrazolo[1,5-a]pyridine-3-carbonitrile**

3-cyano-6-((cyclopropylmethyl)amino)pyrazolo[1,5-a]pyridin-4-yl trifluoromethanesulfonate (0.12 g, 0.33 mmol), 6-((6-methoxypyridin-3-yl)methyl)-3-(5-(tributyltinyl)pyrazin-2-yl)-3,6-diazabicyclo[3.1.1]heptane (0.19 g, 0.33 mmol), $Pd(PPh_3)_4$ (38 mg, 0.033 mmol), and CuI (6.2 mg, 0.033 mmol) were dissolved in 10 mL of xylene , nitrogen replacement was then carried out, and the reaction was carried out at 130 °C for 3 h. After the reaction was completed, the reaction solution was cooled, water (10 ml) was added to quench the reaction, extraction with EA (20 mL*2) was then carried out, and the organic phase was dried, filtered, concentrated, and subjected to column chromatography (DCM:MeOH=10:1) to obtain 50 mg of 6-((cyclopropylmethyl)amino)-4-(5-(6-(((6-methoxypyridin-3-yl)methyl)-3,6-diazabicyclo[3.1.1]hept-3-yl)pyrazin-2-yl)pyrazolo[1,5-a]pyridine-3-carbonitrile **(compound 53).** MS m/z (ESI): 508.2 $[M+H]^+$.

$^1$H NMR (500 MHz, DMSO) δ 8.52 (d, *J* = 1.4 Hz, 1H), 8.40 (s, 1H), 8.28 (d, *J* = 1.4 Hz, 1H), 8.09 (s, 1H), 7.97

(d, $J$ = 1.9 Hz, 1H), 7.69 (dd, $J$ = 8.5, 2.3 Hz, 1H), 7.39 (d, $J$ = 1.9 Hz, 1H), 6.76 (d, $J$ = 8.5 Hz, 1H), 6.09 (t, $J$ = 5.4 Hz, 1H), 3.84 - 3.75 (m, 5H), 3.61 (dd, $J$ = 41.9, 29.6 Hz, 6H), 3.43 (qd, $J$ = 7.0, 5.1 Hz, 1H), 2.96 - 2.89 (m, 2H), 2.58-2.51 (m, 1H), 1.04 (t, $J$ = 7.0 Hz, 1H), 0.52 (dd, $J$ = 8.0, 1.6 Hz, 2H), 0.26 (dd, $J$ = 4.8, 1.3 Hz, 2H).

**Example 54: Synthesis of compound 54**

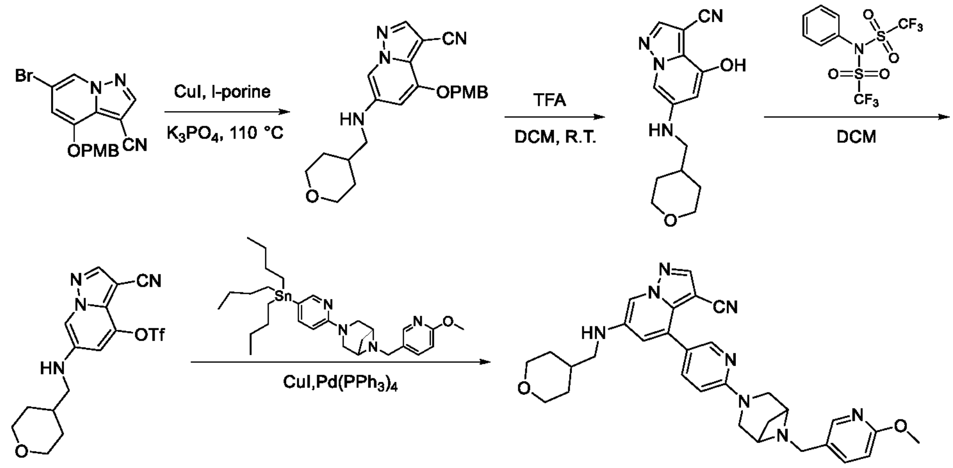


**Step 1: synthesis of 4-((4-methoxybenzyl)oxy)-6-((((tetrahydro-2H-pyran-4-yl)methyl)amino)pyrazolo[1,5-a]pyridine-3-carbonitrile**

At room temperature, 6-bromo-4-((4-methoxybenzyl)oxy)pyrazolo[1,5-a]pyridine-3-carbonitrile (0.4 g, 1.1 mmol), CuI (22 mg, 0.11 mmol), L-proline (26 mg, 0.22 mmol), and potassium phosphate (0.49 g, 2.2 mmol) were dissolved in anhydrous DMSO (10 mL), and 4-aminomethyltetrahydropyran (0.26 g, 2.2 mmol) was then added to the resulting solution under the protection of $N_2$. Reaction was carried out at 120 °C for 5 h. After the reaction was completed, the reaction solution was cooled to room temperature, water (10 mL) was then added to quench the reaction, extraction with EA (30 mL*2) was then carried out, and the organic phase was dried, filtered, concentrated, and subjected to column chromatography (PE:EA=3:1) to obtain 0.2 g of 4-((4-methoxybenzyl)oxy)-6-((((tetrahydro-2H-pyran-4-yl)methyl)amino)pyrazolo[1,5-a]pyridine-3-carbonitrile. MS m/z (ESI): 392.3 $[M+H]^+$.

**Step 2: synthesis of 4-hydroxy-6-((((tetrahydro-2H-pyran-4-yl)methyl)amino)amino]pyrazolo[1,5-a] pyridine-3-carbonitrile**

4-((4-methoxybenzyl)oxy)-6-((((tetrahydro-2H-pyran-4-yl)methyl)amino)pyrazolo[1,5-a]pyridine-3-carbonitrile (0.2 g, 0.51 mmol) was dissolved in DCM (6 mL) at room temperature, the resulting solution was then cooled to 0 °C, TFA (2 mL) was then added, and the reaction was continued for half an hour. After the reaction was completed, the reaction solution was directly spin-dried for the next step without further purification. MS m/z (ESI): 272.9 $[M+H]^+$.

**Step 3: synthesis of 3-cyano-6-(((tetrahydro-2H-pyran-4-yl)methyl)amino)pyrazolo[1,5-a]pyridin-4-yl trifluoromethanesulfonate**

4-hydroxy-6-((((tetrahydro-2H-pyran-4-yl)methyl)amino)amino)pyrazolo[1,5-a]pyridine-3-carbonitrile (0.1 g, 0.37 mmol) and DIEA (0.15 g, 1.1 mmol) were dissolved in DMF (5 mL), N-phenylbis(trifluoromethanesulfonyl)imide (0.20 g, 0.56 mmol) was then added at room temperature, and the mixed solution was stirred at room temperature for 0.5 h to react. After the reaction was completed, water (10 mL) was added to quench the reaction, extraction with EA (20 mL*2) was then carried out, and the organic phase was dried, filtered, concentrated, and subjected to column chromatography (PE:EA =7:1) to obtain 0.1 g of 3-cyano-6-(((tetrahydro-2H-pyran-4-yl)methyl)amino)pyrazolo[1,5-a]pyridin-4-yl trifluoromethanesulfonate. MS m/z (ESI): 405.3 $[M+H]^+$.

**Step 4: synthesis of 4-(6-(6-((6-methoxypyridin-3-yl)methyl)-3,6-diazabicyclo[3.1.1]heptyl-3-yl)pyridin-3-yl)-6-(((-2H-pyran-4-yl)methyl)amino)pyrazolo[1,5-a]pyridine-3-carbonitrile**

3-cyano-6-(((tetrahydro-2H-pyran-4-yl)methyl)amino)pyrazolo[1,5-a]pyridin-4-yl trifluoromethanesulfonate (0.1 g, 0.25 mmol), 6-(((6-methoxypyridin-3-yl)methyl)-3-(5-(tributylstannyl)pyridin-2-yl)-3,6-diazabicyclo[3.1.1]heptane (0.15 g, 0.25 mmol), $Pd(PPh_3)_4$ (29 mg, 0.025 mmol), and CuI (4.8 mg, 0.025 mmol) were dissolved in 10 mL of xylene, nitrogen replacement was then carried out, and the reaction was carried out at 130 °C for 3 h. After the reaction was completed, the reaction solution was cooled, water (10 mL) was added to quench the reaction, extraction with EA (20 mL*2) was then carried out, and the organic phase was dried, filtered, concentrated, and subjected to column chromatography (DCM:MeOH=10:1) to obtain 30 mg of 4-(6-(6-((6-methoxypyridin-3-yl)methyl)-3,6-diazabicyclo[3.1.1]heptyl-3-yl)pyridin-3-yl)-6-(((-2H-pyran-4-yl)methyl)amino)pyrazolo[1,5-a]pyridine-3-carbonitrile **(compound 54).** MS m/z (ESI): 551.2 $[M+H]^+$.

$^1H$ NMR (500 MHz, DMSO) $\delta$ 8.39 (s, 1H), 8.33 (d, $J$ = 2.3 Hz, 1H), 8.06 (s, 1H), 7.97 (d, $J$ = 1.7 Hz, 1H), 7.79 (dd, $J$ = 7.6, 3.8 Hz, 1H), 7.67 (d, $J$ = 8.1 Hz, 1H), 7.08 (d, $J$ = 1.8 Hz, 1H), 6.77 (t, $J$ = 8.0 Hz, 2H), 6.05 (t, $J$ = 5.7 Hz, 1H), 3.86 (dd, $J$ = 11.3, 2.8 Hz, 2H), 3.81 (d, $J$ = 3.9 Hz, 4H), 3.74 - 3.63 (m, 4H), 3.50 (s, 4H), 2.96 (t, $J$ = 6.2 Hz, 2H), 1.90 - 1.79 (m, 1H), 1.70 (d, $J$ = 12.4 Hz, 2H), 1.58 (d, $J$ = 7.9 Hz, 1H), 1.04 (t, $J$ = 7.0 Hz, 2H), 0.83 (ddd, $J$ = 10.9, 7.5, 2.8 Hz, 2H).

**Example 55: Synthesis of compound 55**

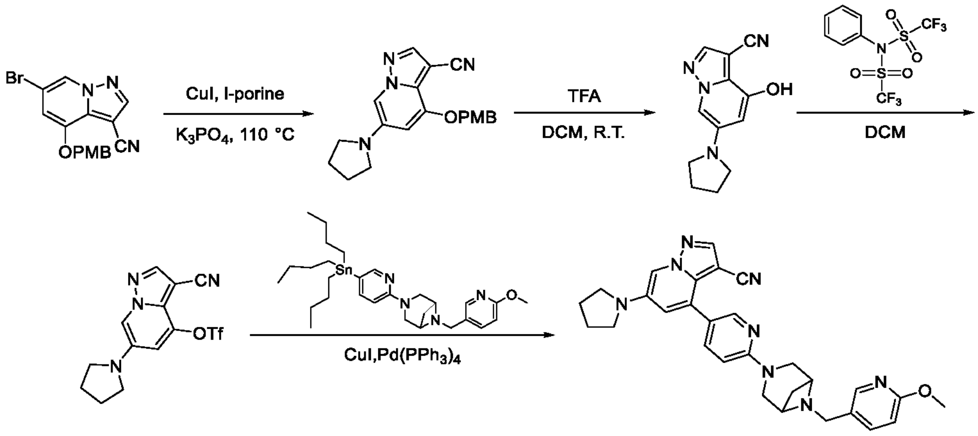


**Step 1: synthesis of 4-((4-methoxybenzyl)oxy)-6-(pyrrolidin-1-yl)pyrazolo[1,5-a]pyridine-3-carbonitrile**

At room temperature, 6-bromo-4-((4-methoxybenzyl)oxy)pyrazolo[1,5-a]pyridine-3-carbonitrile (0.6 g, 1.7 mmol), CuI (32 mg, 0.17 mmol), L-proline (39 mg, 0.34 mmol), and potassium phosphate (1.1 g, 5.1 mmol) were dissolved in anhydrous DMSO (10 mL), and tetrahydropyrrole (0.36 g, 5.1 mmol) was then added to the resulting solution under the protection of $N_2$. Reaction was carried out at 120 °C for 5 h. After the reaction was completed, the reaction solution was cooled to room temperature, water (10 mL) was then added to quench the reaction, extraction with EA (30 mL*2) was then carried out, and the organic phase was dried, filtered, concentrated, and subjected to column chromatography (PE:EA=3:1) to obtain 0.35 g of 4-((4-methoxybenzyl)oxy)-6-(pyrrolidin-1-yl)pyrazolo[1,5-a]pyridine-3-carbonitrile. MS m/z (ESI): 349.5 $[M+H]^+$.

**Step 2: synthesis of 4-hydroxy-6-(pyrrolidin-1-yl)pyrazolo[1,5-a]pyridine-3-carbonitrile**

4-((4-methoxybenzyl)oxy)-6-(pyrrolidin-1-yl)pyrazolo[1,5-a]pyridine-3-carbonitrile (0.25 g, 0.72 mmol) was dissolved in DCM (6 mL) at room temperature, the resulting solution was then cooled to 0 °C, TFA (2 mL) was then added, and the reaction was continued for half an hour. After the reaction was completed, the reaction solution was directly spin-dried for the next step without further purification. MS m/z (ESI): 229.2 $[M+H]^+$.

**Step** 3: **synthesis of 3-cyano 6-(pyrrolidin-1-yl)pyrazolo[1,5-a]pyridin-4-yl trifluoromethanesulfonate**

4-hydroxy-6-(pyrrolidin-1-yl)pyrazolo[1,5-a]pyridine-3-carbonitrile (0.2 g, 0.87 mmol) and DIEA (0.34 g, 2.62 mmol) were dissolved in DMF (5 mL), N-phenylbis(trifluoromethanesulfonyl)imide (0.46 g, 1.3 mmol) was then added, and the mixed solution was stirred at room temperature for 0.5 h to react. After the reaction was completed, water (10 mL) was added to quench the reaction, extraction with EA (20 mL*2) was then carried out, and the organic phase was dried, filtered, concentrated, and subjected to column chromatography (PE:EA =7:1) to obtain 0.25 g of 3-cyano 6-(pyrrolidin-1-yl)pyrazolo[1,5-a]pyridin-4-yl trifluoromethanesulfonate. MS m/z (ESI): 361.5 $[M+H]^+$.

**Step 4: synthesis of 4-(6-(6-((6-methoxypyridin-3-yl)methyl)-3,6-diazabicyclo[3.1.1]heptyl-3-yl)pyridin-3-yl)-6-(pyrrolidin-1-yl)pyrazolo[1,5-a]pyridine-3-carbonitrile**

3-cyano 6-(pyrrolidin-1-yl)pyrazolo[1,5-a]pyridin-4-yl trifluoromethanesulfonate (0.2 g, 0.55 mmol), 6-(((6-methoxypyridin-3-yl)methyl)-3-(5-(tributylstannyl)pyridin-2-yl)-3,6-diazabicyclo[3.1.1]heptane (0.32 g, 0.55 mmol), $Pd(PPh_3)_4$ (63 mg, 0.055 mmol), and CuI (10 mg, 0.055 mmol) were dissolved in 10 mL of xylene, nitrogen replacement was then carried out, and the reaction was carried out at 130 °C for 3 h. After the reaction was completed, the reaction solution was cooled, water (10 mL) was added to quench the reaction, extraction with EA (20 mL*2) was then carried out, and the organic phase was dried, filtered, concentrated, and subjected to column chromatography (DCM:MeOH=10:1) to obtain 60 mg of 4-(6-(6-((6-methoxypyridin-3-yl)methyl)-3,6-diazabicyclo[3.1.1]heptyl-3-yl)pyridin-3-yl)-6-(pyrrolidin-1-yl)pyrazolo[1,5-a]pyridine-3-carbonitrile **(compound** 55). MS m/z (ESI): 507.5 $[M+H]^+$.

$^1$H NMR (400 MHz, DMSO) δ 8.44 - 8.33 (m, 2H), 8.01 (dd, *J* = 18.8, 1.9 Hz, 2H), 7.80 (dd, *J* = 8.8, 2.5 Hz, 1H), 7.64 (dd, *J* = 8.5, 2.2 Hz, 1H), 7.08 (d, *J* = 2.0 Hz, 1H), 6.74 (t, *J* = 8.4 Hz, 2H), 3.78 (d, *J* = 2.8 Hz, 3H), 3.74 - 3.37 (m, 9H), 3.28 (s, 4H), 1.95 (t, *J* = 6.5 Hz, 4H), 1.55 (d, *J* = 8.3 Hz, 1H).

**Example 56: Synthesis of compound 56**

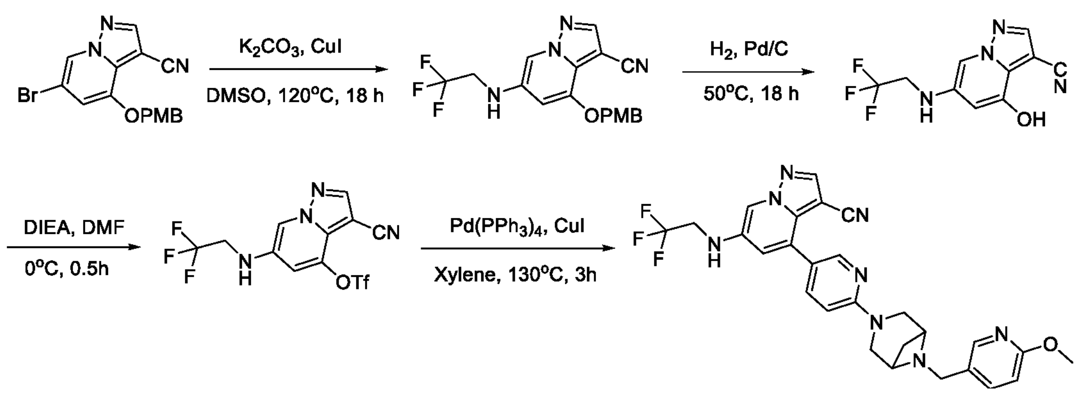


**Step 1: synthesis of 4-((4-methoxybenzyl)oxy)-6-((2,2,2-trifluoroethyl)amino)pyrazolo[1,5-a] pyridine-3-carbonitrile**

At room temperature, 6-bromo-4-((4-methoxybenzyl)oxy)pyrazolo[1,5-a]pyridine-3-carbonitrile (1.0 g, 2.8 mmol), CuI (78 mg, 0.4 mmol), L-proline (64 mg, 0.55 mmol), and potassium carbonate (1.1 g, 7.9 mmol) were dissolved in anhydrous DMSO (10 mL), and trifluoroethylamine (2.7 g, 28 mmol) was then added to the resulting solution under the protection of $N_2$. The reaction was carried out at 120 °C overnight. After the reaction was completed, the reaction solution was cooled to room temperature, diluted with EA (50 mL) and water (50 mL), stirred, and filtered; and the organic phase was extracted, dried, filtered, concentrated, and subjected to column chromatography (PE: EA=3:1) to obtain 4-((4-methoxybenzyl)oxy)-6-((2,2,2-trifluoroethyl)amino)pyrazolo[1,5-a]pyridine-3-carbonitrile (180 mg) with a yield of 18%. MS m/z (ESI): 377.1 $[M+H]^+$.

**Step 2: synthesis of 4-hydroxy-6-((2,2,2-trifluoroethyl)amino)pyrazolo[1,5-a]pyridine-3-carbonitrile**

At room temperature, 4-((4-methoxybenzyl)oxy)-6-((2,2,2-trifluoroethyl)amino)pyrazolo[1,5-a]pyridine-3-carbonitrile (150 mg, 0.39 mmol) was dissolved in 10 mL of methanol, 10% palladium on carbon (70 mg) was then added,

hydrogen replacement was carried out three times, and the reaction was carried out at 50 °C overnight. After the reaction was completed, the orgnatic phase was filtered and concentrated, and the product was directly used in the next step without further purification. MS m/z (ESI): 257.1 [M+H]$^+$.

**Step 3: synthesis of 3-cyano-6-((2,2,2-trifluoroethyl)amino)pyrazolo[1,5-a]pyridine-4-trifluoromethanesulfonate**

4-hydroxy-6-((2,2,2-trifluoroethyl)amino)pyrazolo[1,5-a]pyridine-3-carbonitrile (102 mg, 0.39 mmol) and DIEA (0.2 mL, 1.17 mmol) were dissolved in 5 mL of DMF at room temperature, N-phenylbis(trifluoromethanesulfonyl)imide (142 mg, 0.39 mmol) was then added at 0 °C, and the mixed solution was stirred at room temperature for 0.5 h to react until the reaction was completed. Extraction was then carried out, and the organic phase was dried, filtered, concentrated, and subjected to column chromatography (PE:EA =5:1) to obtain 3-cyano-6-((2,2,2-trifluoroethyl)amino)pyrazolo[1,5 -a]pyridine-4-trifluoromethanesulfonate (130 mg) with a yield of 85%. MS m/z (ESI): 389.1 [M+H]$^+$.

**Step 4: synthesis of 4-(6-(6-((6-methoxypyridin-3-yl)methyl)-3,6-diazabicyclo[3.1.1]heptan-3-yl)pyridin-3-yl)-6-((2,2,2-trifluoroethyl)amino)pyrazolo[1,5-a]pyridine-3-carbonitrile**

At room temperature, 3-cyano-6-((2,2,2-trifluoroethyl)amino)pyrazolo[1,5-a]pyridine-4-trifluoromethanesulfonate (130 mg, 0.33 mmol), 6-((6-methoxypyridin-3-yl)methyl)-3-(5-(tributyltinyl)pyridin-2-yl)-3,6-diazabicyclo[3.1.1]heptane (206 mg, 0.35 mmol), $Pd(PPh_3)_4$ (38 mg, 0.033 mmol), and CuI (6 mg, 0.033 mmol) were dissolved in 10 mL of xylene, nitrogen replacement was carried out three time, and the reaction was carried out at 130 °C for 3 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 22 mg of 4-(6-(6-((6-methoxypyridin-3-yl)methyl)-3,6-diazabicyclo[3.1.1]heptan-3-yl)pyridin-3-yl)-6-((2,2,2-trifluoroethyl)amino)pyrazolo[1,5-a]pyridine-3-carbonitrile **(compound 56)** with a yield of 11%. MS m/z (ESI): 535.1 [M+H]$^+$.

$^1$H NMR (400 MHz, DMSO) δ 8.44 (s, 1H), 8.34 (d, 2H), 8.06 (s, 1H), 7.77 (d, 1H), 7.66(d, 1H), 7.14 (s, 1H), 6.74-6.78 (m, 2H), 6.56-6.59 (m, 1H), 4.05-4.14(m, 2H), 4.57 (d, 2H), 3.49-3.80 (m, 9H), 1.56(d, 2H).

**Example 57: Synthesis of compound 57**

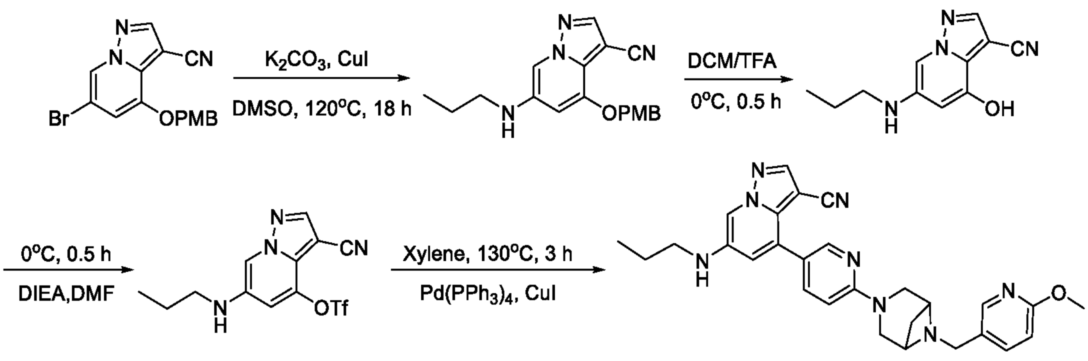


**Step** 1: **synthesis of 4-((4-methoxybenzyl)oxy)-6-(propylamino)pyrazolo[1,5-a]pyridine-3-carbonitrile**

At room temperature, 6-bromo-4-((4-methoxybenzyl)oxy)pyrazolo[1,5-a]pyridine-3-carbonitrile (500 mg, 1.4 mmol), CuI (40 mg, 0.21 mmol), L-proline (32 mg, 0.28 mmol), and potassium carbonate (966 mg, 7.0 mmol) were dissolved in anhydrous DMSO (10 mL), and n-propylamine (827 mg, 14 mmol) was then added to the resulting solution under the protection of $N_2$. The reaction was carried out at 120 °C overnight. After the reaction was completed, the reaction solution was cooled to room temperature, diluted with EA (50 mL) and water (50 mL), stirred, and filtered; and the organic phase was extracted, dried, filtered, concentrated, and subjected to column chromatography (PE: EA=3:1) to obtain 4-((4-methoxybenzyl)oxy)-6-(propylamino)pyrazolo[1,5-a]pyridine-3-carbonitrile (170 mg) with a yield of 36%. MS m/z (ESI): 337.1 [M+H]$^+$.

**Step 2: 4-hydroxy-6-(propylamino)pyrazo[1,5-a]pyridine-3-carbonitrile**

At room temperature, 4-((4-methoxybenzyl)oxy)-6-(propylamino)pyrazolo[1,5-a]pyridine-3-carbonitrile (170 mg, 0.5 mmol) was dissolved in 1 mL of DCM, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 217.1 $[M+H]^+$.

**Step 3: synthesis of 3-cyano-6-(propylamino)pyrazo[1,5-a]pyridine-4-trifluoromethanesulfonate**

4-hydroxy-6-(propylamino)pyrazo[1,5-a]pyridine-3-carbonitrile (109 mg, 0.5 mmol) and DIEA (193 mg, 1.5 mmol) were dissolved in 5 mL of DMF, N-phenylbis(trifluoromethanesulfonyl)imide (178 mg, 0.55 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-(propylamino)pyrazo[1,5-a]pyridine-4-trifluoromethanesulfonate (170 mg) with a yield of 96%. MS m/z (ESI): 349.1 $[M+H]^+$.

**Step 4: synthesis of 4-(6-(6-((6-methoxypyridin-3-yl)methyl)-3,6-diazabicyclo [3.1.1]heptan-3-yl)pyridin-3-yl)-6-(propylamino)pyrazolo [1,5-a] pyridine-3-carbonitrile**

At room temperature, 3-cyano-6-(propylamino)pyrazo[1,5-a]pyridine-4-trifluoromethanesulfonate (170 mg, 0.48 mmol), 6-((6-methoxypyridin-3-yl)methyl)-3-(5-(tributyltinyl)pyridin-2-yl)-3,6-diazabicyclo[3.1.1]heptane (281 mg, 0.48 mmol), $Pd(PPh_3)_4$ (55 mg, 0.048 mmol), and CuI (9 mg, 0.048 mmol) were dissolved in xylene (10 mL), nitrogen replacement was carried out three time, and the reaction was carried out at 130 °C for 3 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 35 mg of 4-(6-(6-((6-methoxypyridin-3-yl)methyl)-3,6-diazabicyclo[3.1.1]heptan-3-yl)pyridin-3-yl)-6-(propylamino)pyrazolo[1,5-a]pyridine-3-carbonitrile **(compound 57)** with a yield of 14%. MS m/z (ESI): 494.1 $[M+H]^+$.

$^1H$ NMR (400 MHz, DMSO) δ 8.38 (s, 1H), 8.32 (d, 1H), 8.06 (s, 1H), 7.92 (s, 1H), 7.77 (d, 1H), 7.66 (d, 1H), 7.04(s,1H), 6.74-6.78 (m, 2H), 5.95 (m, 1H), 3.80 (s,3H), 3.50-3.74 (m, 9H),2.97-3.02 (m,2H),1.55-1.63(m, 3H),0.93-0.98(m,3H).

**Example 58: Synthesis of compound 58**

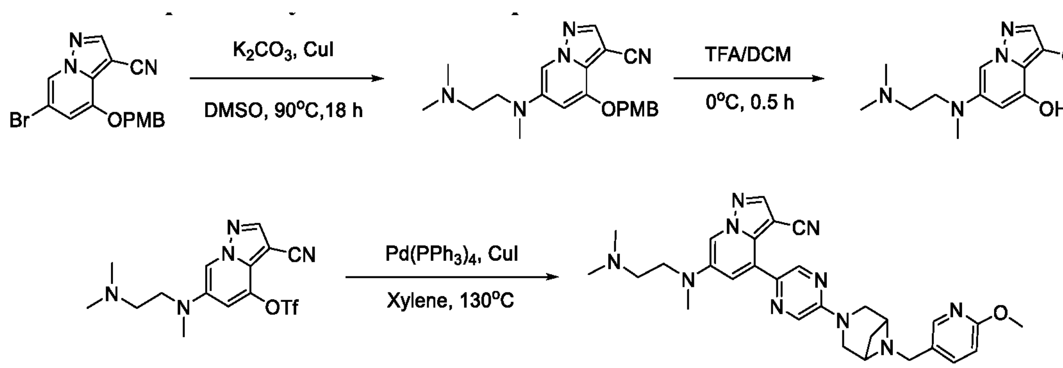


**Step 1: synthesis of 6-((2-(dimethylamino)ethyl)(methyl)amino)-4-((4-methoxybenzyl)oxy)pyrazolo[1,5-a] pyridine-3-carbonitrile**

At room temperature, 6-bromo-4-((4-methoxybenzyl)oxy)pyrazolo[1,5-a]pyridine-3-carbonitrile (500 mg, 1.4 mmol), CuI (26 mg, 0.14 mmol), L-proline (32 mg, 0.28 mmol), and potassium phosphate (600 mg, 2.8 mmol) were dissolved in 10 mL of anhydrous DMSO, and then N1, N1, N2-trimethylethane-1,2-diamine (430 mg, 4.2 mmol) was added under the protection of $N_2$, and the mixed solution reacted at 90 °C overnight. After the reaction was completed, the reaction solution was cooled to room temperature, and then diluted with ethyl acetate (50 mL) and water (50 mL), stirred and filtered. The organic phase was then extracted, dried, filtered, concentrated, and subjected to column chromatography (DCM:MeOH=20:1) to obtain 6-((2-(dimethylamino)ethyl)(methyl)amino)-4-((4-methoxybenzyl)oxy)pyrazolo[1,5-a]pyridine-3-carbonitrile (240 mg) with a yield of 45%. MS m/z (ESI): 380.2 [M+H]$^+$.

**Step 2: synthesis of 6-((2-(dimethylamino)ethyl)(methyl)amino)-4-hydroxypyrazolo[1,5-a] pyridine-3-carbonitrile**

6-((2-(dimethylamino)ethyl)(methyl)amino)-4-((4-methyl)oxybenzyl)oxy)pyrazolo[1,5-a]pyridine-3-carbonitrile (240 mg, 0.63 mmol) was dissolved in 2 mL of DCM, 3 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 260.1 [M+H]$^+$.

**Step 3: synthesis of 3-cyano-6-((2-(dimethylamino)ethyl)(methyl)amino)pyrazolo[1,5-a]pyridine-4-trifluoromethanesulfonate**

6-((2-(dimethylamino)ethyl)(methyl)amino)-4-hydroxypyrazolo[1,5-a]pyridine-3-carbonitrile (164 mg, 0.63 mmol) and DIEA (244 mg, 1.89 mmol) were dissolved in 5 mL of DMF. N-phenylbis(trifluoromethanesulfonyl)imide (338 mg, 0.94 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-((2-(dimethylamino)ethyl)(methyl)amino)pyrazolo[1,5-a]pyridine-4-trifluoromethanesulfonate (120 mg) with a yield of 33%. MS m/z (ESI): 391.1 [M+H]$^+$.

**Step 4: synthesis of 6-((2-(dimethylamino)ethyl)(methyl)amino)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile**

3-cyano-6-((2-(dimethylamino)ethyl)(methyl)amino)pyrazolo[1,5-a]pyridine-4-trifluoromethanesulfonate (70 mg, 0.18 mmol), 6-((6-methoxypyridin-3-yl)methyl)-3-(5-(tributyltinyl)pyrazin-2-yl)-3,6-diazabicyclo[3.1.1]heptane (115 mg, 0.19 mmol), $Pd(PPh_3)_4$ (20 mg, 0.018 mmol), and CuI (3.4 mg, 0.018 mmol) were dissolved in 10 mL of xylene, nitrogen replacement was carried out three time, and the reaction was carried out at 130 °C for 3 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 19 mg of 6-((2-(dimethylamino)ethyl)(methyl)amino)-4-(5-(6-((6-methoxypyridin-3-yl)methyl)-3,6-diazabicyclo[3.1.1]heptan-3-yl)pyrazin-2-yl)pyrazolo[1,5-a]pyridine-3-carbonitrile **(compound 58)** with a yield of 19%. MS m/z (ESI): 539.1 [M+H]+.

$^1$H NMR (400 MHz, DMSO) δ 8.64 (s, 1H), 8.47 (s, 1H), 8.28 (s, 1H), 8.18 (s, 1H), 8.05 (s, 1H), 7.68 (d, 1H), 7.58 (d, 1H), 7.04(s,1H), 6.73 (d, 2H), 3.35-3.81 (m, 15 H),2.96 (s, 3H), 2.56 (s, 2H),2.29(m,3H).

**Example 59: Synthesis of compound 59**

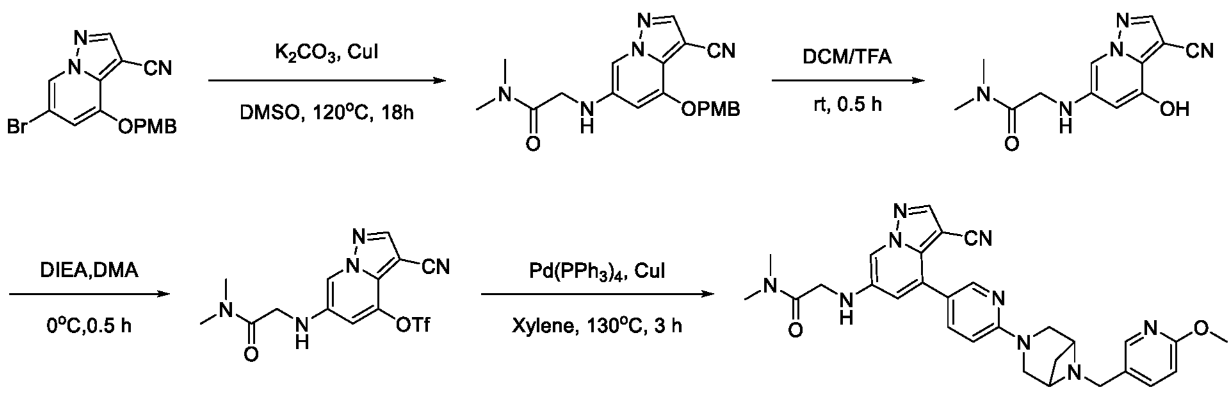


**Step 1: synthesis of 2-((3-cyano-4-((4-methoxybenzyl)oxy)pyrazolo[1,5-a]pyridin-6-yl)amino)-N,N-dimethyl acetamide**

At room temperature and under the protection of $N_2$, 6-bromo-4-((4-methoxybenzyl)oxy)pyrazolo[1,5-a]pyridine-3-carbonitrile (400 mg, 1.12 mmol), CuI (20 mg, 0.1 mmol), L-proline (25 mg, 0.21 mmol), and potassium phosphate (480 mg, 2.2 mmol) were dissolved in 10 mL of anhydrous DMSO, and then 2-amino-N,N-dimethylacetamide (344 mg, 3.3 mmol) was added, and the mixed solution reacted at 120 °C overnight. After the reaction was completed, the reaction solution was cooled to room temperature, and then diluted with EA (50 mL) and water (50 mL), stirred and filtered. The organic phase was then extracted, dried, filtered, concentrated, and subjected to column chromatography (DCM:MeOH=20:1) to obtain2-((3-cyano-4-((4-methoxybenzyl)oxy)pyrazolo[1,5 -a]pyridin-6-yl)amino)-N,N-dimethyl acetamide (280 mg) with a yield of 66%. MS m/z (ESI): 378 $[M+H]^+$.

**Step 2: synthesis of 2-((3-cyano-4-hydroxypyrazolo[1,5-a]pyridin-6-yl)amino)-N,N-dimethylacetamide**

At room temperature, 2-((3-cyano-4-((4-methoxybenzyl)oxy)**pyrazolo[1,5-a]pyridin**-6-yl)amino)-N,N-dimethyl acetamide (280 mg, 0.73 mmol) was dissolved in 10 mL of DCM, 3 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 259.1 $[M+H]^+$.

**Step 3: synthesis of 3-cyano-6-((2-(dimethylamino)-2-oxoethyl)amino)pyrazolo[1,5-a]pyridin-4-yl trifluoromethanesulfonate**

2-((3-cyano-4-hydroxypyrazolo[1,5-a]pyridin-6-yl)amino)-N,N-dimethylacetamide (190 mg, 0.73 mmol) and DIEA (285 mg, 2.2 mmol) were dissolved in 5 mL of DMF at room temperature, N-phenylbis(trifluoromethanesulfonyl)imide (395 mg, 1.1 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =1:1) to obtain 3-cyano-6-((2-(dimethylamino)-2-oxoethyl)amino)pyrazolo[1,5 -a]pyridin-4-yl trifluoromethanesulfonate (74 mg) with a yield of 25%. MS m/z (ESI): 391.1 $[M+H]^+$.

**Step 4: synthesis of 2-((3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)amino)-N,N-dimethylacetamide**

3-cyano-6-((2-(dimethylamino)-2-oxoethyl)amino)pyrazolo[1,5-a]pyridin-4-yl trifluoromethanesulfonate (74 mg, 0.18 mmol), 6-((6-methoxypyridin-3-yl)methyl)-3-(5-(tributyltinyl)pyridin-2-yl)-3,6-diazabicyclo[3.1.1]heptane (121 mg, 0.2 mmol), $Pd(PPh_3)_4$ (22 mg, 0.018 mmol), and CuI (3.6 mg, 0.018 mmol) were dissolved in 10 mL of xylene at room temperature, nitrogen replacement was carried out three time, and the reaction was carried out at 130 °C for 5 h. After the reaction was completed, the reaction solution was cooled and concentrated to remove xylene, and then diluted and extracted; the organic phase was dried, filtered, concentrated, and subj ected to column chromatography (DCM:MeOH=10:1) to obtain 20 mg of 2-((3-cyano-4-(6-(6-((6-methoxypyridin-3-yl)methyl)-3,6-diazabicyclo[3.1.1]heptan-3-yl)pyridin-3-yl)pyrazolo[1,5-a]pyridin-6-yl)amino)-N,N-dimethylacetamide **(compound 59)** with a yield of 19%. MS m/z (ESI): 538.1 $[M+H]^+$.

$^1$H NMR (400 MHz, DMSO) δ 8.41 (s, 1H), 8.34 (s, 1H), 8.12 (s, 1H), 8.08 (s, 1H), 7.79 (d, 1H), 7.67 (d, 1H), 7.34(s,1H), 6.75-6.82 (m, 3H), 3.80 (s,3H), 3.63-3.97 (m, 8H),3.06 (s,3H),2.87(s, 3H),1.89(s, 2H),1.23(s,3H).

**Example 60: Synthesis of compound 60**

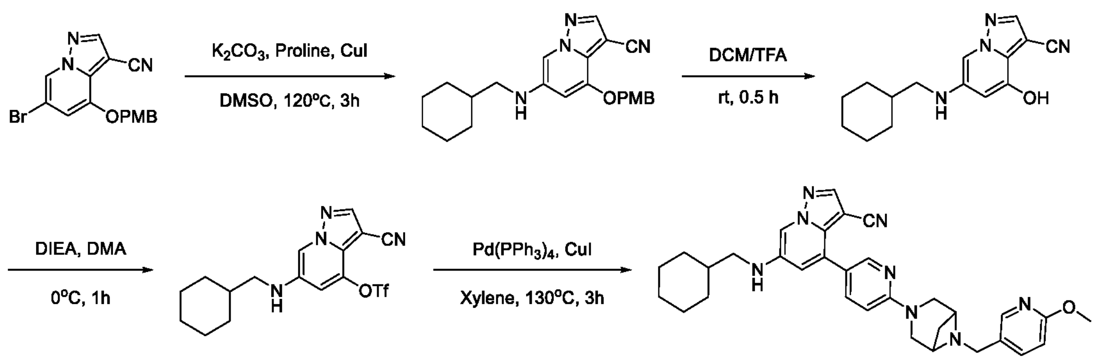

**Step 1: synthesis of 6-((cyclohexylmethyl)amino)-4-((4-methoxybenzyl)oxy)pyrazolo[1,5-a] pyridine-3-carbonitrile**

At room temperature, 6-bromo-4-((4-methoxybenzyl)oxy)pyrazolo[1,5-a]pyridine-3-carbonitrile (400 mg, 1.12 mmol), CuI (20 mg, 0.1 mmol), L-proline (25 mg, 0.21 mmol), and potassium phosphate (480 mg, 2.2 mmol) were dissolved in 5 mL of anhydrous DMSO, and then cyclohexylmethylamine (379 mg, 3.3 mmol) was added under the protection of $N_2$, and the mixed solution reacted at 120 °C for 3 h. After the reaction was completed, the reaction solution was cooled to room temperature, and then diluted with ethyl acetate (50 mL) and water (50 mL), stirred and filtered. The organic phase was then extracted, dried, filtered, concentrated, and subjected to column chromatography (PE:EA=5:1) to obtain 6-((cyclohexylmethyl)amino)-4-((4-methoxybenzyl)oxy)pyrazolo[1,5-a]pyridine-3-carbonitrile (150 mg) with a yield of 36%. MS m/z (ESI): 391.2 $[M+H]^+$.

**Step 2: synthesis of 6-((cyclohexylmethyl)amino)-4-hydroxypyrazolo[1,5-a]pyridine-3-carbonitrile**

6-((cyclohexylmethyl)amino)-4-((4-methoxybenzyl)oxy)pyrazolo[1,5-a]pyridine-3-carbonitrile(215 mg, 0.54 mmol) was dissolved in 5 mL of DCM at room temperature, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 259.1 $[M+H]^+$.

**Step 3: synthesis of 3-cyano-6-((cyclohexylmethyl)amino)pyrazolo[1,5-a]pyridin-4-yl trifluoromethanesulfonate**

6-((cyclohexylmethyl)amino)-4-hydroxypyrazolo[1,5-a]pyridine-3-carbonitrile (150 mg, 0.54 mmol) and DIEA (212 mg, 1.6 mmol) were dissolved in 5 mL of DMF at room temperature. N-phenylbis(trifluoromethanesulfonyl)imide (250 mg, 0.7 mmol) was then added at 0 °C, and the mixed solution was stirred at room temperature for 1 h to react until the reaction was completed. Extraction was carried out, and the organic phase was dried, filtered, concentrated, and subjected to column chromatography (PE:EA =1:1) to obtain 3-cyano-6-((cyclohexylmethyl)amino)pyrazolo[1,5-a]pyridin-4-yl trifluoromethanesulfonate (132 mg) with a yield of 59%. MS m/z (ESI): 402.1 $[M+H]^+$.

**Step 4: synthesis of 6-((cyclohexylmethyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile**

At room temperature, 3-cyano-6-((cyclohexylmethyl)amino)pyrazolo[1,5-a]pyridin-4-yl trifluoromethanesulfonate (132 mg, 0.32 mmol), 6-((6-methoxypyridin-3-yl)methyl)-3-(5-(tributyltinyl)pyridin-2-yl)-3,6-diazabicyclo[3.1.1]heptane (187 mg, 0.32 mmol), $Pd(PPh_3)_4$ (37 mg, 0.032 mmol), and CuI (9 mg, 0.048 mmol) were dissolved in 10 mL of xylene, nitrogen replacement was carried out three time, and the reaction was carried out at 130 °C for 3 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 10 mg of 6-((cyclohexylmethyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile **(compound 60)** with a yield of 5.5%. MS m/z (ESI): 549.1 $[M+H]^+$.

$^1$H NMR (400 MHz, DMSO) δ 8.38 (s, 1H), 8.32 (d, 1H), 8.06 (s, 1H), 7.90 (s, 1H), 7.77 (d, 1H), 7.66 (d, 1H), 7.07 (s,1H), 6.75-6.78 (t, 2H), 3.81 (s,3H), 3.63-3.72 (m, 4H),3.49-3.52(m, 3H), 2.88-2.91(t, 2H),1.58-1.84(m,7H),0.96-1.24(m, 6H).

**Example 61: Synthesis of compound 61**

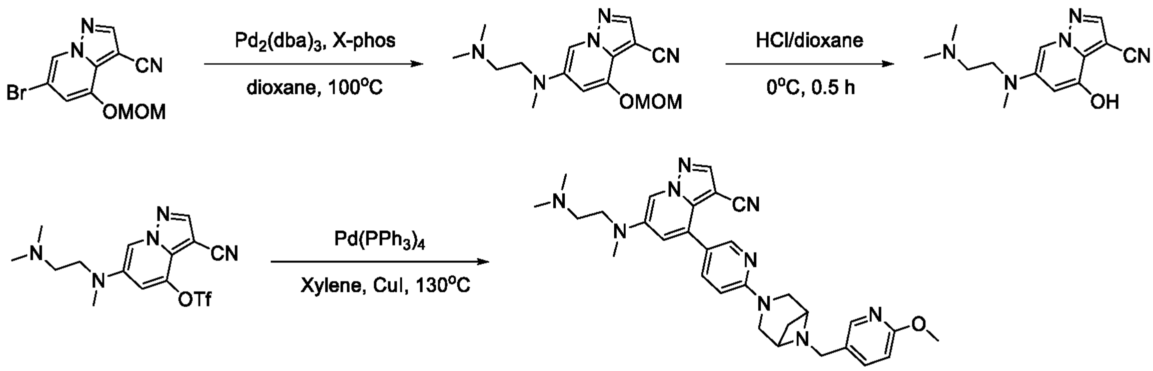


**Step 1: synthesis of 6-((2-(dimethylamino)ethyl)(methyl)amino)-4-(methoxymethoxy)pyrazolo[1,5-a]pyridine-3-carbonitrile**

At room temperature, 6-bromo-4-(methoxymethoxy)pyrazolo[1,5-a]pyridine-3-carbonitrile (1.0 g, 3.5 mmol), N1, N1, N2-trimethylethane-1,2-diamine (2.16 g, 21.1 mmol), $K_3PO_4$ (3.74 g, 17.6 mmol), $Pd_2(dba)_3$ (915 mg, 1.04 mmol), and Xantphos (500 mg, 1.0 mmol) were dissolved in 20 mL of Dioxane, nitrogen replacement was carried out three times, and the reaction was carried out at 100 °C for 3 h. After the reaction was completed, the reaction solution was cooled, concentrated, diluted, and extracted; and the organic phase was dried, filtered, concentrated, and then subjected to column chromatography (PE:EA=3:1) to obtain 6-((2-(dimethylamino)ethyl)(methyl)amino)-4-(methoxymethoxy)pyrazolo[1,5-a]pyridine-3-carbonitrile (170 mg) with a yield of 17%. MS m/z (ESI): 303.1 $[M+H]^+$.

**Step 2: synthesis of 6-((2-(dimethylamino)ethyl)(methyl)amino)-4-hydroxypyrazolo[1,5-a] pyridine-3-carbonitrile**

6-((2-(dimethylamino)ethyl)(methyl)amino)-4-hydroxypyrazolo[1,5-a]pyridine-3-carbonitrile (170 mg, 0.56 mmol) was dissolved in 5 mL of DCM at room temperature, 3 mL of 4M HCl/Dioxane was then added at 0 °C, and the reaction was carried out for 0.5 h. After the reaction was completed, the reaction solution was concentrated and dried in vacuum, and the product was directly used in the next step without further purification. MS m/z (ESI): 259.1 $[M+H]^+$.

**Step 3: synthesis of 3-cyano-6-((2-(dimethylamino)ethyl)(methyl)amino)pyrazolo[1,5-a]pyridine-4-trifluoromethanesulfonate**

6-((2-(dimethylamino)ethyl)(methyl)amino)-4-hydroxypyrazolo[1,5-a]pyridine-3-carbonitrile (94 mg, 0.36 mmol) and DIEA (212 mg, 0.72 mmol) were dissolved in 5 mL of DMF at room temperature. N-phenylbis(trifluoromethanesulfonyl)imide (250 mg, 0.46 mmol) was then added at 0 °C, and the mixed solution was stirred at room temperature for 1 h to react until the reaction was completed. Extraction was carried out, and the organic phase was dried, filtered, concentrated, and subjected to column chromatography (PE:EA =1:1) to obtain 3-cyano-6-((2-(dimethylamino)ethyl)(methyl)amino)pyrazolo[1,5-a]pyridine-4-trifluoromethanesulfonate (107 mg) with a yield of 75%. MS m/z (ESI): 392.1 $[M+H]^+$.

**Step 4: synthesis of 6-((2-(dimethylamino)ethyl)(methyl)amino)-4-(6-((6-methoxypyridin-3-yl)methyl)-3,6-diazabicyclo[3.1.1]heptan-3-yl)pyridin-3-yl)pyrazolo[1,5-a]pyridine-3-carbonitrile**

3-cyano-6-((2-(dimethylamino)ethyl)(methyl)amino)pyrazolo[1,5-a]pyridine-4-trifluoromethanesulfonate (107 mg, 0.27 mmol), 6-((6-methoxypyridin-3-yl)methyl)-3-(5-(tributyltinyl)pyridin-2-yl)-3,6-diazabicyclo[3.1.1]heptane (187 mg, 0.32 mmol), $Pd(PPh_3)_4$ (31 mg, 0.027 mmol), and CuI (8 mg, 0.042 mmol) were dissolved in 10 mL of xylene, nitrogen replacement was carried out three time, and the reaction was carried out at 130 °C for 3 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 11 mg of 6-((2-(dimethylamino)ethyl)(methyl)amino)-4-(6-((6-methoxypyridin-3-yl)methyl)-3,6-diazabicyclo[3.1.1]heptan-3 -yl)pyridin-3-yl)pyrazolo[1,5-a]pyridine-3 - carbonitrile **(compound 61)** with a yield of 7.5%. MS m/z (ESI): 537.1 $[M+H]^+$.

$^1H$ NMR (400 MHz, DMSO) δ 8.64 (s, 1H), 8.47 (s, 1H), 8.28 (s, 1H), 8.18 (s, 1H), 8.05 (s, 1H), 7.58 (s, 1H), 7.04(s,1H), 6.73 (d, 2H), 3.35-3.81 (m, 15 H),2.96 (s,3H), 2.56 (s, 2H),2.29(m,3H).

**Example 62: Synthesis of compound 62**

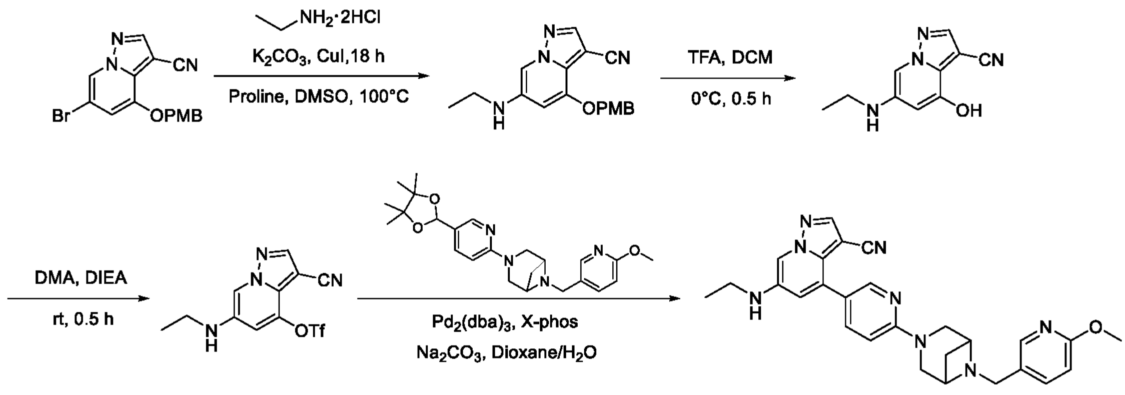


**Step 1: synthesis of 6-(ethylamino)-4-((4-methoxybenzyl)oxy)pyrazolo[1,5-a]pyridine-3-carbonitrile**

At room temperature, 6-bromo-4-((4-methoxybenzyl)oxy)pyrazolo[1,5-a]pyridine-3-carbonitrile (1.0 g, 2.8 mmol), CuI (53 mg, 0.28 mmol), L-proline (48 mg, 0.42 mmol), and potassium carbonate (3.8 g, 28 mmol) were dissolved in anhydrous DMSO (15 mL), and ethylamine hydrochloride (2.28 g, 28 mmol) was then added to the resulting solution under the protection of $N_2$. The reaction was carried out at 100 °C overnight. After the reaction was completed, the reaction solution was cooled to room temperature, diluted with EA (100 mL) and water (50 mL), stirred, and filtered; and the organic phase was extracted, dried, filtered, concentrated, and subjected to column chromatography (PE: EA=3:1) to obtain 6-(ethylamino)-4-((4-methoxybenzyl)oxy)pyrazolo[1,5-a]pyridine-3-carbonitrile (470 mg) with a yield of 52%. MS m/z (ESI): 323.1 $[M+H]^+$.

**Step 2: synthesis of 6-(ethylamino)-4-hydroxypyrazolo[1,5-a]pyridine-3-carbonitrile**

6-(ethylamino)-4-((4-methoxybenzyl)oxy)pyrazolo[1,5-a]pyridine-3-carbonitrile (2.28 g, 7 mmol) was dissolved in 10 mL of DCM at room temperature, 10 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 203.1 $[M+H]^+$.

**Step 3: synthesis of 3-cyano-6-(ethylamino)pyrazolo[1,5-a]pyridin-4-yl trifluoromethanesulfonate**

6-(ethylamino)-4-hydroxypyrazolo[1,5-a]pyridine-3-carbonitrile (1.43 g, 7 mmol) and DIEA (2.7 g, 21 mmol) were dissolved in 10 mL of DMF at room temperature. N-phenylbis(trifluoromethanesulfonyl)imide (2.5 g, 7 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-(ethylamino)pyrazolo[1,5-a]pyridin-4-yl trifluoromethanesulfonate (1.74 g) with a yield of 76%. MS m/z (ESI): 335.1 $[M+H]^+$.

**Step 4: synthesis of 6-(ethylamino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile**

At room temperature, 6-(ethylamino)pyrazo[1,5-a]pyridin-4-yl trifluoromethanesulfonate (0.9 g, 2.69 mmol), 6-((6-methoxypyridin-3-yl)methyl)-3-(5-(4,4,5,5-tetramethyl-1,3-dioxan-2-yl)pyridin-2-yl)-3,6-diazabicyclo[3.1.1]heptane (1.47 g, 3.5 mmol), $Pd_2(dba)_3$ (246 mg, 0.269 mmol), and x-phos (256 mg, 0.053 mmol) were dissolved in Dioxane/$H_2O$=20 mL/4 mL, nitrogen replacement was carried out three times, and the resulting solution reacted at 100 °C for 16 h. After the reaction was completed, the reaction solution was cooled, concentrated, and diluted; the organic phase was extracted, dried, filtered, concentrated, and subjected to column chromatography (DCM:MeOH=10:1) to obtain 1.0 g of 6-(ethylamino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile **(compound 62).** The product was purified by reslurrying with ether, thus obtaining 400 mg of purified product with a yield of 31%. MS m/z (ESI): 481.1 $[M+H]^+$.

$^1$H NMR (400 MHz, DMSO) δ 8.38 (s, 1H), 8.32 (d, 1H), 8.06 (s, 1H), 7.92 (s, 1H), 7.77 (d, 1H), 7.66 (d, 1H),

7.04(s,1H), 6.74-6.78 (m, 2H), 5.95 (m, 1H), 3.80 (s,3H), 3.50-3.74 (m, 9H), 1.55-1.63(m, 3H),0.93-0.98(m,3H).

**Example 63: Synthesis of compound 63**

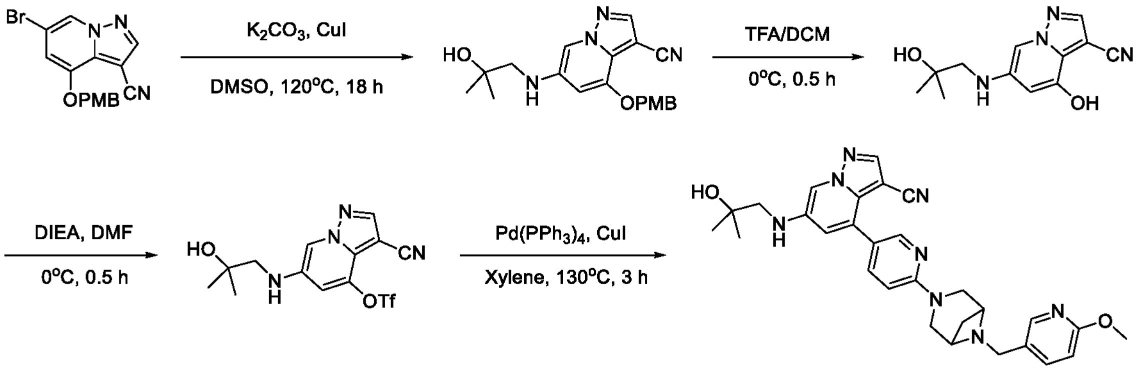


**Step 1: synthesis of 6-((2-hydroxy-2-methylpropyl)amino)-4-((4-methoxybenzyl)oxy)pyrazolo[1,5-a] pyridine-3-carbonitrile**

6-bromo-4-((4-methoxybenzyl)oxy)pyrazolo[1,5-a]pyridine-3-carbonitrile (500 mg, 1.4 mmol), CuI (40 mg, 0.21 mmol), L-proline (32 mg, 0.28 mmol), and potassium carbonate (966 mg, 7.0 mmol) were dissolved in anhydrous DMSO (10 mL) at room temperature, and 1-amino-2-methylpropan-2-ol (623 mg, 7 mmol) was then added to the resulting solution under the protection of $N_2$. The reaction was carried out at 100 °C overnight. After the reaction was completed, the reaction solution was cooled to room temperature, diluted with EA (50 mL) and water (50 mL), stirred, and filtered; and the organic phase was extracted, dried, filtered, concentrated, and subjected to column chromatography (PE: EA=3:1) to obtain 6-((2-hydroxy-2-methylpropyl)amino)-4-((4-methoxybenzyl)oxy)pyrazolo[1,5-a]pyridine-3-carbonitrile (240 mg) with a yield of 47%. MS m/z (ESI): 367.1 $[M+H]^+$.

**Step 2: synthesis of 4-hydroxy-6-((2-hydroxy-2-methylpropyl)amino)pyrazolo[1,5-a]pyridine-3-carbonitrile**

6-((2-hydroxy-2-methylpropyl)amino)-4-((4-methoxybenzyl)oxy)pyrazolo[1,5-a]pyridine-3-carbonitrile (240 mg, 0.65 mmol) was dissolved in 2 mL of DCM at room temperature, 2 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 247.1 $[M+H]^+$.

**Step 3: synthesis of 3-cyano-6-((2-hydroxy-2-methylpropyl)amino)pyrazolo[1,5-a]pyridine-4-trifluoromethanesulfonate**

4-hydroxy-6-((2-hydroxy-2-methylpropyl)amino)pyrazolo[1,5-a]pyridine-3-carbonitrile (161 mg, 0.6 mmol) and DIEA (234 mg, 1.8 mmol) were dissolved in 5 mL of DMF, N-phenylbis(trifluoromethanesulfonyl)imide (321 mg, 0.9 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-((2-hydroxy-2-methylpropyl)amino)pyrazolo[1,5-a]pyridine-4-trifluoromethanesulfonate (80 mg) with a yield of 32%. MS m/z (ESI): 379.1 $[M+H]^+$.

**Step 4: synthesis of 6-((2-hydroxy-2-methylpropyl)amino)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile**

3-cyano-6-((2-hydroxy-2-methylpropyl)amino)pyrazolo[1,5-a]pyridine-4-trifluoromethanesulfonate (80 mg, 0.2 mmol), 6-((6-methoxypyridin-3-yl)methyl)-3-(5-(tributyltinyl)pyridin-2-yl)-3,6-diazabicyclo[3.1.1]heptane (136 mg, 0.23 mmol), $Pd(PPh_3)_4$ (24 mg, 0.02 mmol), and CuI (4 mg, 0.02 mmol) were dissolved in 10 mL of xylene, nitrogen replacement was carried out three time, and the reaction was carried out at 130 °C for 4 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 13 mg of 6-((2-hydroxy-2-methylpropyl)amino)-4-(6-(6-((6-methoxypyridin-3-yl)methyl)-3,6-diazabicyclo[3.1.1]heptan-3 -yl)pyridin-3-yl)pyrazolo[1,5-a]pyridine-3 - carbonitrile **(compound 63)** with a yield of 11 %. MS m/z (ESI): 524.1 $[M+H]^+$.

$^1$H NMR (400 MHz, DMSO) δ 8.37 (s, 1H), 8.33 (s, 1H), 8.06 (s, 1H), 8.02 (s, 1H), 7.75-7.77(m,1H), 7.67 (d, 1H), 7.24 (s, 1H),6.75 (t, 2H),5.81 (t, 1H), 4.54 (s, 1H),3.81 (s, 1H),3.65-3.72 (m, 4H), 3.48-3.52 (m, 4H),2.97 (d, 2H),1.58(s, 1H),1.18 (s,6H).

**Example 64: Synthesis of compound 64**

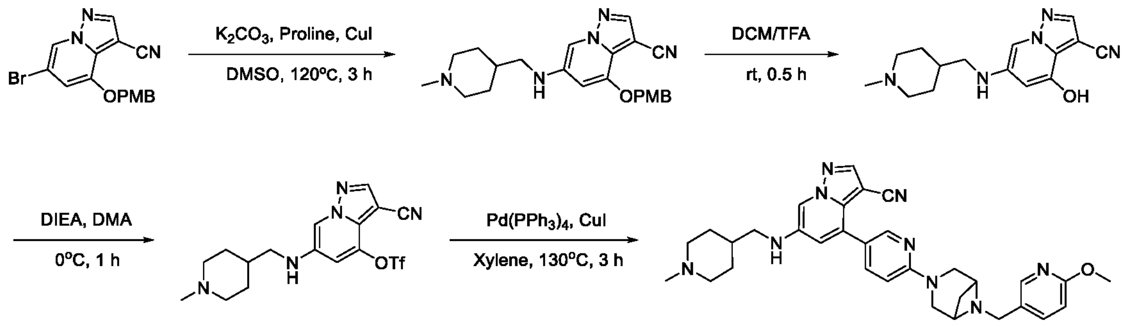


**Step 1: synthesis of 4-((4-methoxybenzyl)oxy)-6-((1-methylpiperidin-4-yl)methyl)amino)pyrazolo [1,5-a]pyridine-3-carbonitrile**

6-bromo-4-((4-methoxybenzyl)oxy)pyrazolo[1,5-a]pyridine-3-carbonitrile (400 mg, 1.12 mmol), CuI (20 mg, 0.1 mmol), L-proline (25 mg, 0.21 mmol), and potassium phosphate (480 mg, 2.2 mmol) were dissolved in 5 mL of anhydrous DMSO at room temperature, and then (1-methylpiperidin-4-yl)carboxamide (422 mg, 3.3 mmol) was added under the protection of $N_2$, and the mixed solution reacted at 120 °C for 3 h. After the reaction was completed, the reaction solution was cooled to room temperature, and then diluted with ethyl acetate (50 mL) and water (50 mL), stirred and filtered. The organic phase was then extracted, dried, filtered, concentrated, and subjected to column chromatography (PE:EA=3:1) to obtain 4-((4-methoxybenzyl)oxy)-6-((1-methylpiperidin-4-yl)methyl)amino)pyrazolo[1,5-a]pyridine-3-carbonitrile (200 mg) with a yield of 44%. MS m/z (ESI): 406.1 $[M+H]^+$.

**Step 2: synthesis of 4-hydroxy-6-((1-methylpiperidin-4-yl)methyl)amino)pyrazolo[1,5-a]pyridine-3-carbonitrile**

4-((4-methoxybenzyl)oxy)-6-((1-methylpiperidin-4-yl)methyl)amino)pyrazolo[1,5-a]pyridine-3-carbonitrile (200 mg, 0.49 mmol) was dissolved in 2 mL of DCM at room temperature, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 271.1 $[M+H]^+$.

**Step 3: synthesis of 3-cyano-6-((1-methylpiperidin-4-yl)methyl)amino)pyrazolo[1,5-a]pyridine-4-trifluoromethanesulfonate**

4-hydroxy-6-((1-methylpiperidin-4-yl)methyl)amino)pyrazolo[1,5-a]pyridine-3-carbonitrile (140 mg, 0.49 mmol) and DIEA (190 mg, 1.47 mmol) were dissolved in 5 mL of DMF at room temperature, N-phenylbis(trifluoromethanesulfonyl)imide (262 mg, 73 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =1:1) to obtain 3-cyano-6-((1-methylpiperidin-4-yl)methyl)amino)pyrazolo[1,5-a]pyridine-4-trifluoromethanesulfonate (90 mg) with a yield of 44%. MS m/z (ESI): 418.1 $[M+H]^+$.

**Step 4: synthesis of 4-(6-(6-((6-methoxypyridin-3-yl)methyl)-3,6-diazabicyclo[3.1.1]heptan-3-yl)pyridin-3-yl)-6-((1-methylpiperidin-4-yl)methyl)amino)pyrazolo[1,5-a]pyridine-3-carbonitrile**

3-cyano-6-((1-methylpiperidin-4-yl)methyl)amino)pyrazolo[1,5-a]pyridine-4-trifluoromethanesulfonate (90 mg, 0.21 mmol), 6-((6-methoxypyridin-3-yl)methyl)-3-(5-(tributyltinyl)pyridin-2-yl)-3,6-diazabicyclo[3.1.1]heptane (126 mg, 0.21 mmol), $Pd(PPh_3)_4$ (24 mg, 0.021 mmol), and CuI (9 mg, 0.048 mmol) were dissolved in 10 mL of xylene, nitrogen

replacement was carried out three time, and the reaction was carried out at 130 °C for 4 h. After the reaction was completed, the reaction solution was cooled and concentrated to remove xylene, and then diluted and extracted; the organic phase was dried, filtered, concentrated, and subjected to column chromatography (DCM:MeOH=10:1) to obtain 6 mg of 4-(6-(6-((6-methoxypyridin-3-yl)methyl)-3,6-diazabicyclo[3.1.1]heptan-3-yl)pyridin-3-yl)-6-((1-methylpiperidin-4-yl)methyl)amino)pyrazolo[1,5-a]pyridine-3-carbonitrile **(compound 64)** with a yield of 5 %. MS m/z (ESI): 563.1[M+H]+.

$^1H$ NMR (400 MHz, DMSO) δ 8.38 (s, 1H), 8.32 (d, 1H), 8.06 (s, 1H), 7.90 (s, 1H), 7.77 (d, 1H), 7.66 (d, 1H), 7.07 (s,1H), 6.75-6.78 (t, 2H), , 3.81 (s,3H),3.63-3.72(m, 4H),3.49-3.52(m, 3H), 2.67 (s, 3H),1.58-1.84(m,7H),0.96-1.24(m, 6H).

## Example 65: Synthesis of compound 65

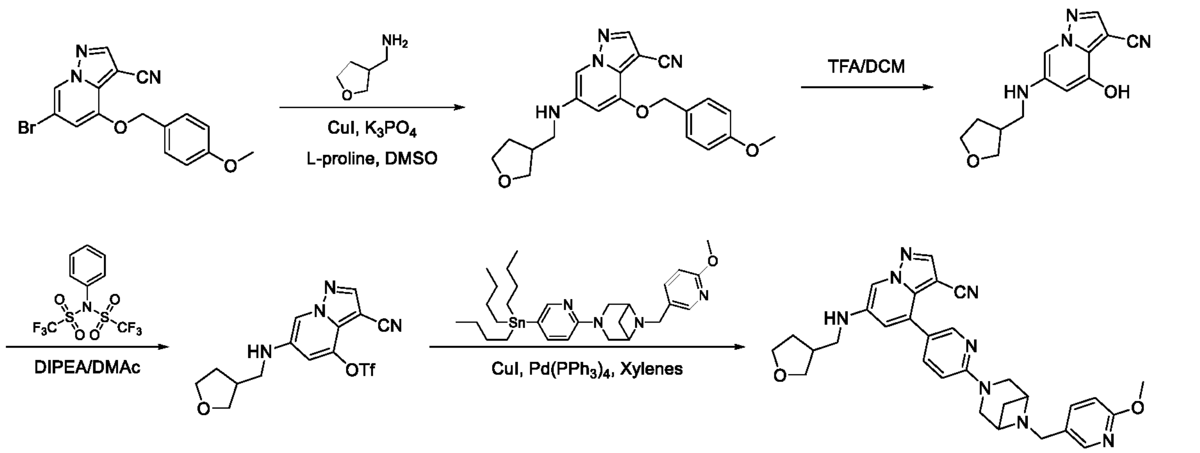


### Step 1: synthesis of 4-((4-methoxybenzyl)oxy)-6-(((tetrahydrofuran-3-yl)methyl)amino)pyrazolo[1,5-a]pyridine-3-carbonitrile

6-bromo-4-((4-methoxybenzyl)oxy)pyrazolo[1,5-a]pyridine-3-carbonitrile (400 mg, 1.12 mmol), potassium phosphate (475 mg, 2.24 mmol), and L-proline (25.8 mg, 0.22 mmol) were dissolved in DMSO (5 mL), and then (tetrahydrofuran-3-yl)methanamine (0.35 ml, 3.36 mmol) and CuI (21.3 mg, 0.11 mmol) were added to the resulting solution; the mixed solution was set under the protection of $N_2$ and then heated to 120 °C and refluxed for 5 h. After the reaction was found to be completed from the monitoring, the reaction solution was cooled, an appropriate amount of water was then added, extraction with ethyl acetate was then carried out, the organic phase was dried and concentrated, and then column chromatography and separation were carried out to obtain 230 mg of 4-((4-methoxybenzyl)oxy)-6-(((tetrahydrofuran-3-yl)methyl)amino)pyrazolo[1,5-a]pyridine-3-carbonitrile with a yield of 54.3%. MS m/z (ESI): 379.0 [M+H]+.

### Step 2: synthesis of 4-hydroxy-6-(((tetrahydrofuran-3-yl)methyl)amino)pyrazolo[1,5-a]pyridine-3-carbonitrile

4-((4-Methoxybenzyl)oxy)-6-(((tetrahydrofuran-3-yl)methyl)amino)pyrazolo[1,5-a]pyridine-3-carbonitrile (230 mg, 0.61 mmol) was dissolved in dichloromethane (4 mL), trifluoroacetic acid (1 mL) was then added to the system in an ice bath, and the reaction solution was then stirred at room temperature for 30 min until the reaction was found to be completed from the monitoring. The reaction solution was then concentrated to obtain a crude product which was directly used in the next step.

### Step 3: synthesis of 3-cyano-6-(((tetrahydrofuran-3-yl)methyl)amino)pyrazolo[1,5-a]pyridin-4-yl trifluoromethanesulfonate

N-phenylbis(trifluoromethanesulfonyl)imide (259 mg, 0.72 mmol) and the crude product obtained from the previous step wew dissolved in DMAc (5 mL), and then DIPEA (0.4 mL, 2.42 mmol) was added to the resulting solution, and the reaction solution was stirred at room temperature for 2 h until the reaction was found to be completed from the monitoring. An appropriate amount of water was then added, extraction with ethyl acetate was carried out, the organic phase was dried and concentrated, and then column chromatography and separation were carried out to obtain 110 mg of 3-cyano-6-(((tetrahydrofuran-3-yl)methyl)amino)pyrazolo[1,5-a]pyridin-4-yl trifluoromethanesulfonate with a yield of

46.6%. MS m/z (ESI): 390.9 [M+H]+.

**Step 4: synthesis of 4-(6-(6-((6-methoxypyridin-3-yl)methyl)-3,6-diazabicyclo[3.1.1]heptyl-3-yl)pyridin-3-yl)-6-(((tetrahydrofuran-3-yl)methyl)amino)pyrazolo[1,5-a]pyridine-3-carbonitrile**

3-cyano-6-(((tetrahydrofuran-3-yl)methyl)amino)pyrazolo[1,5-a]pyridin-4-yl trifluoromethanesulfonate (110 mg, 0.28 mmol), 6-((6-methoxypyridin-3-yl)methyl)-3-(5-(tributyltinyl)pyridin-2-yl)-3,6-diazabicyclo[3.1.1]heptane (166 mg, 0.28 mmol), CuI (5.39 mg, 0.03 mmol) and tetrakistriphenylphosphine palladium (32.66 mg, 0.03 mmol) were dissolved in 10 mL of xylene, and the reaction solution was set under the protection of $N_2$ and then heated to 135 °C and refluxed for 4 h. After the reaction was found to be completed from the monitoring, the reaction solution was cooled, an appropriate amount of water was then added, extraction with dichloromethane was carried out, the organic phase was then dried and concentrated, and then column chromatography and separation were carried out to obtain 20 mg of 4-(6-(6-((6-methoxypyridin-3-yl)methyl)-3,6-diazabicyclo[3.1.1]heptyl-3-yl)pyridin-3-yl)-6-(((tetrahydrofuran-3-yl)methyl)amino)pyrazolo[1,5-a]pyridine-3-carbonitrile **(compound 65)** with a yield of 13.2%. MS m/z (ESI): 537.1 $[M+H]^+$.

$^1$H NMR (300 MHz, DMSO) δ 8.54 (s, 1H), 8.39 (d, *J* = 2.4 Hz, 1H), 8.33 (d, *J* = 1.9 Hz, 1H), 8.05 (d, *J* = 1.7 Hz, 1H), 7.83 (dd, *J* = 8.8, 2.5 Hz, 1H), 7.66 (dd, *J* = 8.5, 2.3 Hz, 1H), 7.50 (d, *J* = 2.0 Hz, 1H), 6.77 (dd, *J* = 8.6, 4.9 Hz, 2H), 6.08 (s, 1H), 3.89 - 3.36 (m, 16H), 3.22 - 3.14 (m, 3H), 1.80 (m, 1H), 1.67(m, 2H), 1.22 (s, 2H).

**Example 66: Synthesis of compound 66**

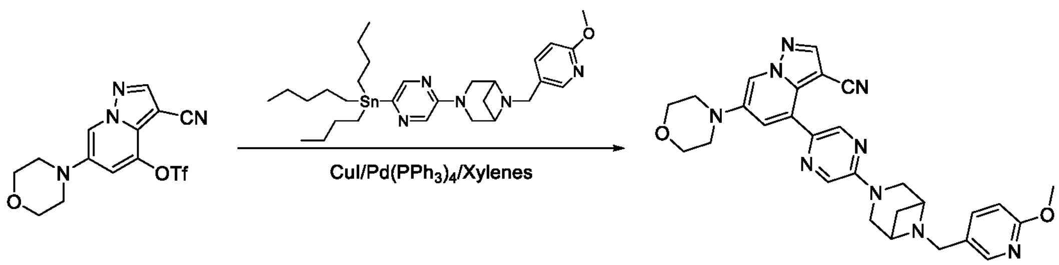


**Step 1: synthesis of 4-(5-(6-((6-methoxypyridin-3-yl)methyl)-3,6-diazabicyclo[3.1.1]heptyl-3-yl)pyrazin-2-yl)-6-morpholinopyrazolo[1,5-a]pyridine-3-carbonitrile**

3-cyano-6-morpholinopyrazolo[1,5-a]pyridin-4-yl trifluoromethanesulfonate (220 mg, 0.6 mmol), 6-((6-methoxypyridin-3-yl)methyl)-3-(5-(tributyltinyl)pyrazin-2-yl)-3,6-diazabicyclo[3.1.1]heptane (370 mg, 0.64 mmol), CuI (11 mg, 0.06 mmol) and tetrakistriphenylphosphine palladium (70 mg, 0.06 mmol) were dissolved in 10 mL of xylene, and the reaction solution was set under the protection of $N_2$ and then heated to 135 °C and refluxed for 3 h. After the reaction was found to be completed from the monitoring, the reaction solution was cooled, an appropriate amount of water was then added, extraction with ethyl acetate was carried out, the organic phase was then dried and concentrated, and then column chromatography and separation were carried out to obtain 0.12 g of 4-(5-(6-((6-methoxypyridin-3-yl)methyl)-3,6-diazabicyclo[3.1.1]heptyl-3-yl)pyrazin-2-yl)-6-morpholinopyrazolo[1,5-a]pyridine-3-carbonitrile **(compound 66).** MS m/z (ESI): 524.5 $[M+H]^+$.

$^1$H NMR (400 MHz, DMSO) δ 8.65 (d, *J* = 1.4 Hz, 1H), 8.54 (s, 1H), 8.35 (d, *J* = 2.0 Hz, 1H), 8.28 (d, *J* = 1.4 Hz, 1H), 8.08 (d, *J* = 1.6 Hz, 1H), 7.77 (d, *J* = 2.0 Hz, 1H), 7.68 (dd, *J* = 8.5, 2.3 Hz, 1H), 6.76 (d, *J* = 8.5 Hz, 1H), 3.86 - 3.74 (m, 9H), 3.69 (d, *J* = 5.1 Hz, 2H), 3.61 (d, *J* = 12.6 Hz, 2H), 3.53 (s, 2H), 3.24 - 3.16 (m, 4H), 2.54 (d, *J* = 5.9 Hz, 2H).

**Example 67: Synthesis of compound 67**

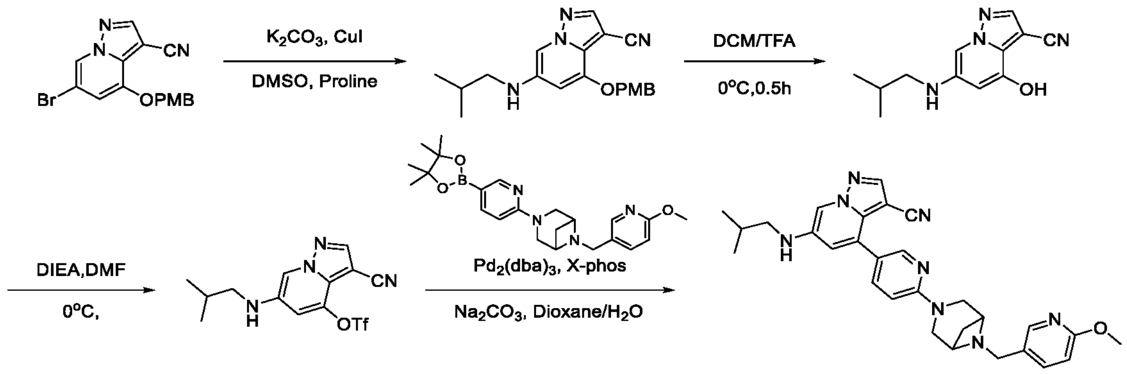


**Step 1: synthesis of 6-(isobutylamino)-4-((4-methoxybenzyl)oxy)pyrazolo[1,5-a]pyridine-3-carbonitrile**

At room temperature, 6-bromo-4-((4-methoxybenzyl)oxy)pyrazolo[1,5-a]pyridine-3-carbonitrile (500 mg, 1.4 mmol), CuI (40 mg, 0.21 mmol), L-proline (32 mg, 0.28 mmol), and potassium carbonate (580 mg, 4.2 mmol) were dissolved in anhydrous DMSO (10 mL), and isobutylamine (310 mg, 4.2 mmol) was then added to the resulting solution under the protection of $N_2$. The reaction was carried out at 120 °C for 6 h. After the reaction was completed, the reaction solution was cooled to room temperature, extracted with EA (50 mL*2), diluted with water (50 mL), stirred, filtered, and separated; and the organic phase was extracted, dried, filtered, concentrated, and subjected to column chromatography (PE: EA=3:1) to obtain 6-(isobutylamino)-4-((4-methoxybenzyl)oxy)pyrazolo[1,5-a]pyridine-3-carbonitrile (200 mg). MS m/z (ESI): 351.1 $[M+H]^+$.

**Step 2: synthesis of 4-hydroxy-6-(isobutylamino)pyrazolo[1,5-a]pyridine-3-carbonitrile**

At room temperature, 6-(isobutylamino)-4-((4-methoxybenzyl)oxy)pyrazolo[1,5-a]pyridine-3-carbonitrile (200 mg, 0.56 mmol) was dissolved in 3 mL of DCM, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 231.2 $[M+H]^+$.

**Step 3: synthesis of 3-cyano-6-(isobutylamino)pyrazolo[1,5-a]pyridin-4-yl trifluoromethanesulfonate**

4-hydroxy-6-(isobutylamino)pyrazolo[1,5-a]pyridine-3-carbonitrile (200 mg, 0.87 mmol) and DIEA (336 mg, 2.6 mmol) were dissolved in 5 mL of DMF, N-phenylbis(trifluoromethanesulfonyl)imide (310 mg, 0.87 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 3-cyano-6-(isobutylamino)pyrazolo[1,5-a]pyridin-4-yl trifluoromethanesulfonate (180 mg). MS m/z (ESI): 363.3 $[M+H]^+$.

**Step 4: synthesis of 6-(isobutylamino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile**

At room temperature, 3-cyano-6-(isobutylamino)pyrazolo[1,5-a]pyridin-4-yl trifluoromethanesulfonate (0.3 g, 0.83 mmol), 6-((6-methoxypyridin-3-yl)methyl)-3-(5-(4,4,5,5-tetramethyl-1,3-dioxaboran-2-yl)pyridine-2-yl)-3,6-diazabicyclo[3.1.1]heptane (0.35 g, 0.83 mmol), $Pd_2(dba)_3$ (73 mg, 0.08 mmol), and X-phos (40 mg, 0.08 mmol) were dissolved in Dioxane/$H_2O$=20 mL/4 mL, nitrogen replacement was carried out three times, and the resulting solution reacted at 100 °C for 6 h. After the reaction was completed, the reaction solution was cooled, concentrated, and extracted with DCM; the organic phase was dried, filtered, concentrated, and subjected to column chromatography (DCM:MeOH=10:1) to obtain 80 mg of 6-(isobutylamino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile **(compound 67)**. MS m/z (ESI): 509.4 $[M+H]^+$

$^1H$ NMR (500 MHz, DMSO) $\delta$ 8.38 (s, 1H), 8.34 (d, *J*= 2.4 Hz, 1H), 8.06 (d, *J* = 1.9 Hz, 1H), 7.92 (d, *J* = 1.8 Hz, 1H), 7.77 (dd, *J* = 8.8, 2.5 Hz, 1H), 7.67 (dd, *J* = 8.5, 2.3 Hz, 1H), 7.10 (d, *J*= 1.9 Hz, 1H), 6.76 (t, *J*= 8.5 Hz, 2H), 5.98 (s, 1H), 3.81 (s, 3H), 3.69 (dd, *J*= 27.6, 8.8 Hz, 5H), 3.51 (d, *J* = 12.3 Hz, 4H), 2.92 - 2.81 (m, 2H), 1.93 - 1.83 (m, 1H), 1.58 (d, *J*= 8.4 Hz, 1H), 0.97 (d, *J* = 6.6 Hz, 6H).

**Example 68: Synthesis of compound 68**

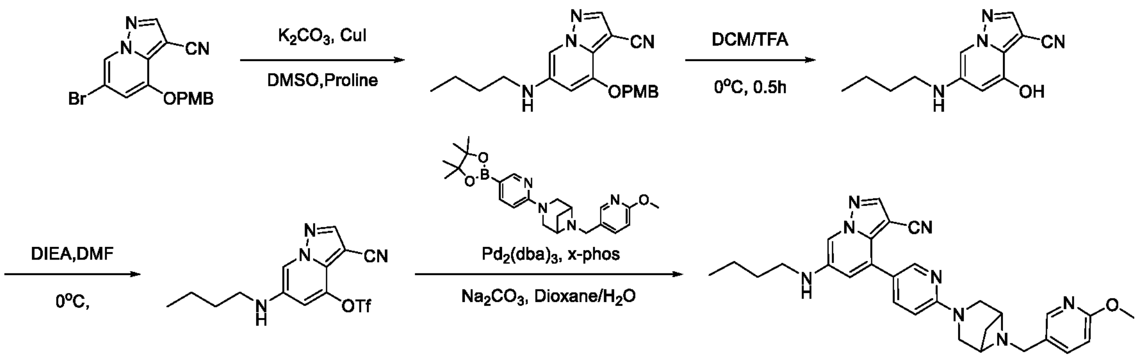


**Step 1: synthesis of 6-(butylamino)-4-((4-methoxybenzyl)oxy)pyrazolo[1,5-a]pyridine-3-carbonitrile**

At room temperature, 6-bromo-4-((4-methoxybenzyl)oxy)pyrazolo[1,5-a]pyridine-3-carbonitrile (500 mg, 1.4 mmol), CuI (40 mg, 0.21 mmol), L-proline (32 mg, 0.28 mmol), and potassium carbonate (580 mg, 4.2 mmol) were dissolved in anhydrous DMSO (10 mL), and n-butylamine (310 mg, 4.2 mmol) was then added to the resulting solution under the protection of $N_2$. The reaction was carried out at 120 °C for 6 h. After the reaction was completed, the reaction solution was cooled to room temperature, extracted with EA (50 mL*2), diluted with water (50 mL), stirred, filtered, and separated; and the organic phase was extracted, dried, filtered, concentrated, and subjected to column chromatography (PE: EA=3:1) to obtain 6-(butylamino)-4-((4-methoxybenzyl)oxy)pyrazolo[1,5-a]pyridine-3-carbonitrile (190 mg). MS m/z (ESI): 351.1 $[M+H]^+$.

**Step 2: synthesis of 6-(butylamino)-4-hydroxypyrazolo[1,5-a]pyridine-3-carbonitrile**

At room temperature, 6-(butylamino)-4-((4-methoxybenzyl)oxy)pyrazolo[1,5-a]pyridine-3-carbonitrile (190 mg, 0.5 mmol) was dissolved in 3 mL of DCM, 1 mL of trifluoroacetic acid was added at 0 °C, and the reaction was carried out for 0.5 h. After the reaction was completed, the reaction solution was concentrated and dried in vacuum at room temperature, and the product was directly used in the next step without further purification. MS m/z (ESI): 231.2 $[M+H]^+$.

**Step 3: synthesis of 6-(butylamino)-3-cyanopyrazolo[1,5-a]pyridin-4-yl trifluoromethanesulfonate**

6-(butylamino)-4-hydroxypyrazolo[1,5-a]pyridine-3-carbonitrile (200 mg, 0.87 mmol) and DIEA (336 mg, 2.6 mmol) were dissolved in 5 mL of DMF, N-phenylbis(trifluoromethanesulfonyl)imide (310 mg, 0.87 mmol) was then added at 0 °C, and the mixed solution was stirred at room temperature for 0.5 h to react until the reaction was completed. Extraction was carried out, and the organic phase was dried, filtered, concentrated, and subjected to column chromatography (PE:EA =3:1) to obtain 6-(butylamino)-3-cyanopyrazolo[1,5-a]pyridin-4-yl trifluoromethanesulfonate (180 mg). MS m/z (ESI): 363.3 $[M+H]^+$.

**Step 4: synthesis of 6-(butylamino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile**

At room temperature, 6-(butylamino)-3-cyanopyrazolo[1,5-a]pyridin-4-yl trifluoromethanesulfonate (0.3 g, 0.83 mmol), 6-((6-methoxypyridin-3-yl)methyl)-3-(5-(4,4,5,5-tetramethyl-1,3-dioxaboran-2-yl)pyridine-2-yl)-3,6-diazabicyclo[3.1.1]heptane (0.35 g, 0.83 mmol), $Pd_2(dba)_3$ (73 mg, 0.08 mmol), and X-phos (40 mg, 0.08 mmol) were dissolved in Dioxane/$H_2O$=20 mL/4 mL, nitrogen replacement was carried out three times, and the resulting solution reacted at 100 °C for 6 h. After the reaction was completed, the reaction solution was cooled, concentrated, and extracted with DCM; the organic phase was dried, filtered, concentrated, and subjected to column chromatography (DCM:MeOH=10:1) to obtain 90 mg of 6-(butylamino)-4-(6-(6-((6-methoxypyridin-3-yl)methyl)-3,6-diazabicyclo[3.1.1]hept-3-yl)pyridin-3-yl)pyrazolo[1,5-a]pyridine-3-carbonitrile (**compound 68**). MS m/z(ESI): 509.4 $[M+H]^+$.

$^1H$ NMR (500 MHz, DMSO) δ 8.38 (s, 1H), 8.33 (d, *J* = 2.4 Hz, 1H), 8.07 (s, 1H), 7.92 (d, *J* = 1.7 Hz, 1H), 7.77 (dd, *J* = 8.8, 2.5 Hz, 1H), 7.67 (d, *J* = 9.8 Hz, 1H), 7.05 (d, *J* = 1.8 Hz, 1H), 6.76 (t, *J* = 8.1 Hz, 2H), 5.92 (t, *J* = 5.4 Hz, 1H), 3.81 (d, *J* = 3.4 Hz, 4H), 3.76 - 3.62 (m, 5H), 3.50 (s, 4H), 3.03 (dd, *J* = 12.5, 6.8 Hz, 2H), 1.58 (d, *J* = 7.3 Hz, 2H),

1.45 - 1.38 (m, 2H), 0.93 (t, *J* = 7.4 Hz, 3H).

**Example 69: Synthesis of compound 69**

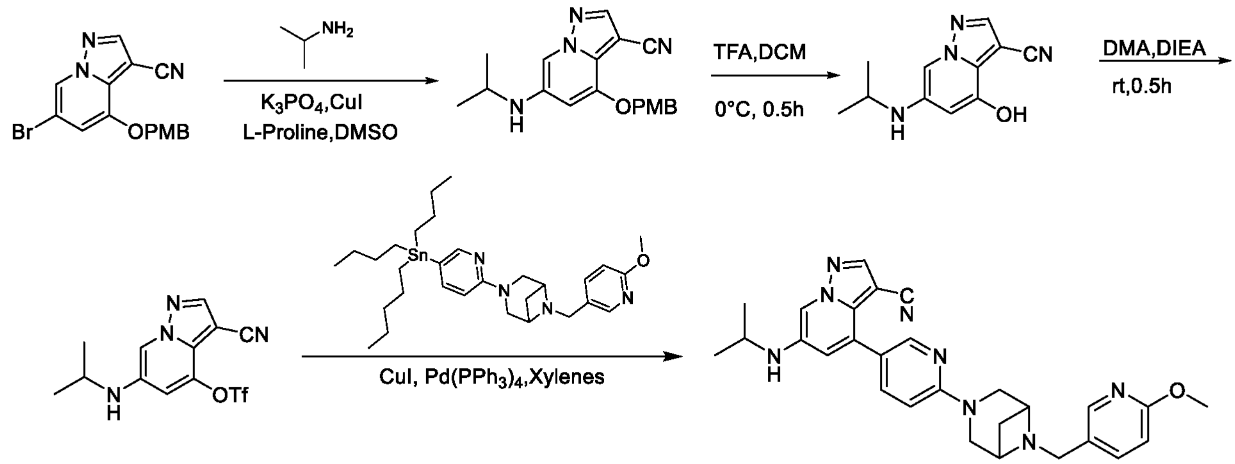


**Step 1: synthesis of 6-(isopropylamino)-4-((4-methoxybenzyl)oxy)pyrazolo[1,5-a]pyridine-3-carbonitrile**

Compound 6-bromo-4-((4-methoxybenzyl)oxy)pyrazolo[1,5-a]pyridine-3-carbonitrile (0.4 g, 1.12 mmol), CuI (53.3 mg, 0.28 mmol), L-proline (51.8 mg, 0.45 mmol), and $K_3PO_4$ (475.5 mg, 2.24 mmol) were dissolved in the DMSO (10 mL) solution, isopropylamine (198.6 mg, 3.36 mmol) was then added to the resulting solution, the system was then switched to nitrogen protection and sealed, and the reaction solution was stirred at 90°C overnight. After the reaction was completed, the reaction solution was washed with 30 mL of saturated NaCl solution, and the organic phase was concentrated and then purified by column chromatography (PE:EA=3:1) to obtain 0.15 g of 6-(isopropylamino)-4-((4-methoxybenzyl)oxy)pyrazolo[1,5-a]pyridine-3-carbonitrile. MS m/z (ESI): 337 [M+H]+.

**Step 2: synthesis of 4-hydroxy-6-(isopropylamino)pyrazolo[1,5-a]pyridine-3-carbonitrile**

6-(isopropylamino)-4-((4-methoxybenzyl)oxy)pyrazolo[1,5-a]pyridine-3-carbonitrile (0.29 g, 0.86 mmol) was dissolved in DMC/$CF_3CO_2H$ (3 mL/1 mL), and the resultion solution was then stirred at room temperature. After the reaction was completed, the reaction solution was diluted with water (10 mL), and 4N aqueous NaOH solution was then added dropwise to adjust the pH to 4 to 5. Extraction with EA (2*10 mL) was then carried out, and the organic phases were then combined, washed with saturated NaCl solution (20 mL), and then concentrated and purified by ISCO (DCM/MeOH = 0 to 10%) to obtain 0.26 g of 6-(isopropyl)-4-hydroxypyrazolo[1,5-a]pyridine-3-carbonitrile. MS m/z (ESI): 217 $[M+H]^+$.

**Step 3: synthesis of 3-cyano-6-(isopropylamino)pyrazolo[1,5-a]pyridin-4-yl trifluoromethanesulfonate**

6-(isopropylamino)-4-hydroxypyrazolo[1,5-a]pyridine-3-carbonitrile (185.76 mg, 0.86 mmol) was dissolved in DMA (10 ml), and then DIPEA (0.6 mL, 3.44 mmol) was added, and $PhNTf_2$ (460.53 mg, 1.29 mmol) was then slowly added to the reaction solution and the resulting solution was stirred at room temperature for 2 h to react. After the reaction was found to be completed from the monitoring of LC-MS, the reaction was quenched with water (10 mL), extraction with EA (10 mL*3) was carried out, and the organic phases were combined and washed with the NaCl solution (20 mL) and then concentrated, and subjected to column chromatography to obtain 260 mg of 3-cyano-6-(isopropylamino)pyrazolo[1,5-a]pyridin-4-yl trifluoromethanesulfonate. MS m/z (ESI): 349 $[M+H]^+$.

Step 4: synthesis of 6-(isopropylamino)-4-(6-(6-((6-methoxypyridin-3-yl)methyl)-3,6-diazabicyclo[3.1.1]heptyl-3-yl)pyridin-3-yl)pyrazolo[1,5-a]pyridine-3-carbonitrile

Compound 3-cyano-6-(isopropylamino)pyrazolo[1,5-a]pyridin-4-yl trifluoromethanesulfonate (0.26 g, 0.75 mmol), (1R, 5S)-6-((6-methoxypyridin-3-yl)methyl)-3-(5-tributylstannyl)pyridin-2-yl)-3,6-diazabicyclo[3.1.1]heptane (480.52 mg, 0.82 mmol), $Pd(PPh_3)_4$ (86.63 mg, 0.075 mmol) and CuI (14.28 mg, 0.075 mmol) were dissolved in 1,4-xylene (10 ml) at 0 °C under the protection of $N_2$, and the reaction system was then stirred thoroughly. The reaction system was then slowly heated up to 140 °C and further stirred overnight to react. After the reaction was found to be completed from the monitoring of LC-MS, the reaction was quenched with 10 mL of saturated aqueous $NaHCO_3$solution (10 mL), extraction with EA (10 mL*3) was then carried out, and the organic phase was then washed with saturated NaCl solution, and then dried, concentrated, and purified by column chromatography (DCM/MeOH=0-10%) to obtain 42 mg of 6-(isopropylamino)-4-(6-(6-((6-methoxypyridin-3-yl)methyl)-3,6-diazabicyclo[3.1.1]heptyl-3-yl)pyridin-3-yl)pyrazolo[1,5-a]pyridine-3-carbonitrile **(compound 69**). MS m/z (ESI): 495 $[M+H]^+$.

$^1H$ NMR (500 MHz, DMSO) δ 8.39 (s, 1H), 8.34 (d, J = 2.3 Hz, 1H), 8.08 (s, 1H), 7.97 (d, J = 1.8 Hz, 1H), 7.77 (dd, J = 8.8, 2.5 Hz, 1H), 7.69 (dd, J = 8.4, 2.1 Hz, 1H), 7.03 (d, J = 1.9 Hz, 1H), 6.77 (dd, J = 8.5, 5.9 Hz, 2H), 5.81 (d, J = 8.0 Hz, 1H), 4.36 (t, J = 5.1 Hz, 1H), 3.77 - 3.63 (m, 4H), 3.62 - 3.47 (m, 5H), 1.18 (d, J = 6.3 Hz, 6H).

**Example 70: Synthesis of compound 70**

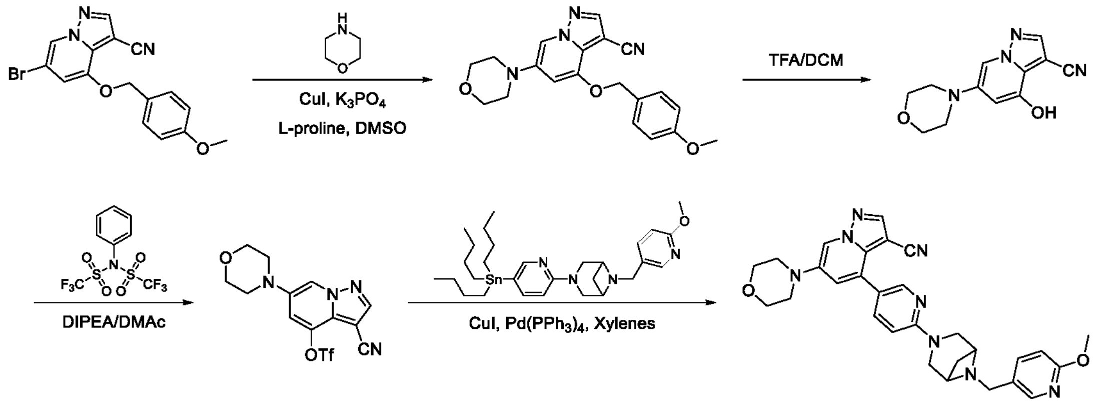


**Step 1: synthesis of 4-((4-methoxybenzyl)oxy)-6-morpholinopyrazolo[1,5-a]pyridine-3-carbonitrile**

6-bromo-4-((4-methoxybenzyl)oxy)pyrazolo[1,5-a]pyridine-3-carbonitrile (1.8 g, 5.04 mmol), potassium phosphate (2.13 g, 10.08 mmol), and L-proline (60 mg, 0.50 mmol) were dissolved in DMSO (18 mL), and then morpholine (1.32 ml, 15.12 mmol) and CuI (96 mg, 0.50 mmol) were added to the resulting solution; the mixed solution was set under the protection of $N_2$ and then heated to 120 °C and refluxed for 5 h. After the reaction was found to be completed from the monitoring, the reaction solution was cooled, an appropriate amount of water was then added, extraction with ethyl acetate was then carried out, the organic phase was dried and concentrated, and then column chromatography and separation were carried out to obtain 270 mg of 4-((4-methoxybenzyl)oxy)-6-morpholinopyrazolo[1,5-a]pyridine-3-carbonitrile with a yield of 14.7%. MS m/z (ESI): 365.0 $[M+H]^+$.

**Step 2: synthesis of 4-hydroxy-6-morpholinopyrazolo[1,5-a]pyridine-3-carbonitrile**

4-((4-methoxybenzyl)oxy)-6-morpholinopyrazolo[1,5-a]pyridine-3-carbonitrile (270 mg, 0.74 mmol) was dissolved in dichloromethane (4 mL), trifluoroacetic acid (1 mL) was then added to the system in an ice bath, and the reaction solution was then stirred at room temperature for 30 min until the reaction was found to be completed from the monitoring. The reaction solution was then concentrated to obtain a crude product which was directly used in the next step.

**Step 3: synthesis of 3-cyano-6-morpholinopyrazolo[1,5-a]pyridin-4-yl trifluoromethanesulfonate**

N-phenylbis(trifluoromethanesulfonyl)imide (316 mg, 0.88mmol) and the crude product obtained from the previous step wew dissolved in DMAc (5 mL), and then DIPEA (0.49 mL, 0.95 mmol) was added to the resulting solution, and the reaction solution was stirred at room temperature for 2 h until the reaction was found to be completed from the monitoring. An appropriate amount of water was then added, extraction with ethyl acetate was carried out, the organic phase was dried and concentrated, and then column chromatography and separation were carried out to obtain 210 mg of 3-cyano-6-morpholinopyrazolo[1,5-a]pyridin-4-yl trifluoromethanesulfonate with a yield of 75.7%. MS m/z (ESI): 376.9 $[M+H]^+$.

**Step 4: synthesis of 4-(6-(6-((6-methoxypyridin-3-yl)methyl)-3,6-diazabicyclo[3.1.1]heptyl-3-yl)pyridin-3-yl)-6-morpholinopyrazolo[1,5-a]pyridine-3-carbonitrile**

3-cyano-6-morpholinopyrazolo[1,5-a]pyridin-4-yl trifluoromethanesulfonate (110 mg, 0.29 mmol), 6-((6-methoxypyridin-3-yl)methyl)-3-(5-(tributyltinyl)pyridin-2-yl)-3,6-diazabicyclo[3.1.1]heptane (188 mg, 0.32 mmol), CuI (5.57 mg, 0.03 mmol) and tetrakistriphenylphosphine palladium (34 mg, 0.03 mmol) were dissolved in 10 mL of xylene, and the reaction solution was set under the protection of $N_2$ and then heated to 135 °C and refluxed for 5 h. After the reaction was found to be completed from the monitoring, the reaction solution was cooled, an appropriate amount of water was then added, extraction with dichloromethane was carried out, the organic phase was then dried and concentrated, and then column chromatography and separation were carried out to obtain 45 mg of 4-(6-(6-((6-methoxypyridin-3-yl)methyl)-3,6-diazabicyclo[3.1.1]heptyl-3-yl)pyridin-3-yl)-6-morpholinopyrazolo[1,5-a]pyridine-3-carbonitrile **(compound 70)** with a yield of 29.5%. MS m/z (ESI): 523.1 $[M+H]^+$.

$^1$H NMR (300 MHz, DMSO) δ 8.54 (s, 1H), 8.39 (d, *J*= 2.4 Hz, 1H), 8.33 (d, *J* = 1.9 Hz, 1H), 8.05 (d, *J*= 1.7 Hz, 1H), 7.83 (dd, *J*= 8.8, 2.5 Hz, 1H), 7.66 (dd, *J*= 8.5, 2.3 Hz, 1H), 7.50 (d, *J*= 2.0 Hz, 1H), 6.77 (dd, *J*= 8.6, 4.9 Hz, 2H), 3.89 - 3.36 (m, 18H), 3.22 - 3.14 (m, 3H), 1.22 (s, 2H)

**Biological activity test example 1:**

**(a) In vitro screening experiment-HTRF method for testing the inhibitory activity of compounds against RET**

Experimental procedure:

1. Preparation of 1x kinase buffer: 5x enzyme buffer was mixed with distilled water at a ratio of 1:4, the final concentration: 5mM $MgCl_2$; 1mM dithiothreitol.
2. The test compound (5 mM stock solution) was diluted 5 folds to 1 mM with 100% dimethyl sulfoxide; the compound was diluted into 10 concentrations at an equal ratio of 1:3 in a 384-well dilution plate.
3.0.2 μL of the serially diluted compound was added to a 384-well plate using an Echo 550 pipetting system, 2 replicate wells for each concentration, and the final concentration of dimethyl sulfoxide was 0.5% (v/v). The gradient concentrations of the test compound were 5000, 1666.7, 555.5, 185.18, 61.72, 20.57, 6.858, 2.286, 0.764, and 0.254 nM.
4. 2x RET (0.1 ng/μl) was prepared using the 1x kinase buffer.
5. 5 μL of 2x RET was added to a 384-well plate, and then centrifuged at 1000g for 30 s, and incubated at room temperature for 10 min.
6. A mixed solution of 2x tyrosine kinase-biotinylated substrate (2 μM) and adenosine triphosphate (20 μM) was prepared using the 1x kinase buffer.
7. 5 μL of the mixed solution of tyrosine kinase-biotinylated substrate and adenosine triphosphate was added to start the reaction. The reaction solution was centrifuged at 1000g for 30 s, and then sealed, and incubated at room temperature for 30 min.
8. A mixed solution of 2x Sa-XL 665 (a reagent) (125 μM) and a tyrosine kinase-antibody-cryptate was prepared using a homogeneous time-resolved fluorescence immunoassay buffer.
9. 10 μL of mixed solution of the Sa-XL 665 and the tyrosine kinase-antibody-cryptate was added to each well, and then centrifuged at 1000g for 30 s, and incubated at room temperature for 1 h.
10. The plate was read at 615 nm and 665 nm on an Envision 2104 microplate reader and the ratio was calculated (665/615 nm).
11. The % inhibition rate was calculated as follows:

$$\text{inhibition rate\%} = [1 - \frac{R1-R2}{R0-R2}] \times 100\%$$

wherein

R0 refers to the average ratio of the microplate reader plate of the vehicle blank group
R1 refers to the ratio of the test compound on the microplate reader plate.
R2 refers to the average ratio on the microplate reader plate in the case of 100% inhibitory activity against RET.

12. $IC_{50}$ was calculated with software GraphPad 6.0 by fitting inhibition values and logarithms of compound concentrations to a nonlinear regression (dose response - variable slope).

**(b) In vitro screening experiment - HTRF method for testing the inhibitory activity of compounds against VEGFR2**

Experimental procedure:

1. Preparation of 1x kinase buffer: 5x enzyme buffer was mixed with distilled water at a ratio of 1:4, the final concentration: 5 mM $MgCl_2$; 1 mM dithiothreitol; 1 mM MnCl.
2. The test compound (5 mM stock solution) was diluted 5 folds to 1 mM with 100% dimethyl sulfoxide; the compound was diluted into 10 concentrations at an equal ratio of 1:3 in a 384-well dilution plate.
3. 0.2 μL of the serially diluted compound was added to a 384-well cell culture plate (Corning, 3570) using an Echo 550 pipetting system, 2 replicate wells for each concentration, and the final concentration of dimethyl sulfoxide was 0.5% (v/v). The gradient concentrations of the test compound were 5000, 1666.7, 555.5, 185.18, 61.72, 20.57, 6.858, 2.286, 0.764, and 0.254 nM.
4. 2x VEGFR2 (0.02 ng/μl) was prepared using the 1x kinase buffer.
5. 5 μl of 2x VEGFR2 was added to a 384-well plate, and then centrifuged at 1000g for 30 s, and incubated at room temperature for 10 min.
6. A mixed solution of 2x tyrosine kinase-biotinylated substrate (2 μM) and ATP (8 μM) was prepared using the 1x kinase buffer.
7. 5 μL of the mixed solution of tyrosine kinase-biotinylated substrate and adenosine triphosphate was added to start the reaction. The reaction solution was centrifuged at 1000g for 30 s, and then sealed, and incubated at room temperature for 40 min.
8. A mixed solution of 2x Sa-XL 665 (125 μM) and a tyrosine kinase-antibody-cryptate was prepared using a homogeneous time-resolved fluorescence immunoassay buffer.
9. 10 μL of mixed solution of Sa-XL 665 and the tyrosine kinase-antibody-cryptate was added to each well, and then centrifuged at 1000g for 30 s, and incubated at room temperature for 1 h.
10. The plate was read at 615 nm and 665 nm on an Envision 2104 microplate reader and the ratio was calculated (665/615 nm).
11. The % inhibition rate was calculated as follows:

$$\text{inhibition rate\%} = [1 - \frac{\text{R1}-\text{R2}}{\text{R0}-\text{R2}}] \times 100\%$$

wherein

R0 refers to the average ratio of the microplate reader plate of the vehicle blank group
R1 refers to the ratio of the test compound on the microplate reader plate.
R2 refers to the average ratio on the microplate reader plate in the case of 100% inhibitory activity against RET.

12. $IC_{50}$ was calculated with software GraphPad 6.0 by fitting inhibition values and logarithms of compound concentrations to a nonlinear regression (dose response - variable slope).

**(c) In vitro screening experiment - CellTiter-Glo luminescence assay for testing the inhibitory activity of compounds against Ba/F3-KIF5B-RET cell**

Experimental procedure:

1. The mammalian cell expression vector containing human KIF5B-RET cDNA was introduced into Ba/F3 cells using the Neon® transfection system method, and the surviving clones screened by puromycin were subjected to cell growth inhibitory function experiments and Western blotting to verify cell lines with stable and high expression of RET.
2. Cells were cultured in RPMI 1640 medium containing 10% fetal bovine serum, 1% penicillin-streptomycin, and 2 μg/mL puromycin, and then placed in a 37°C, 5% carbon dioxide cell incubator.
3. The test compound (5 mM stock) was diluted 2.5 folds to 2 mM with 100% dimethyl sulfoxide; and the compound was diluted into 10 concentrations at an equal ratio of 1:3 in a 384-well dilution plate.
4. 0.2 μL of the serially diluted compound was added to a 384-well cell culture plate (Corning, 3570) using an Echo 550 pipetting system, 2 replicate wells for each concentration, and the final concentration of dimethyl sulfoxide was 0.5% (v/v). The gradient concentrations of the test compound were 5000, 1666.7, 555.5, 185.18, 61.72, 20.57, 6.858, 2.286, 0.764, 0.254 nM.
5.40 μL of Ba/F3-KIF5B-RET cell suspension containing 800 cells was added to each well, and incubated for 72 h

in a 5% $CO_2$ cell incubator.

6.20 μL of Cell Titer-Glo reagent was added to each well of the cell culture plate, and the culture system was then shaken and mixed thoroughly for 2 min to lyse the cells, the cells were then incubated at room temperature for 30 min, and the fluorescence signal values were then read with an Envision 2104 microplate reader.

7. By fitting the data with XLFit 5.0 based on the parameters formula, the $IC_{50}$ value was calculated as:

$$Y=Bottom +(Top-Bottom)/(1+10^\wedge((LogIC50-X)*HillSlope)).$$

**(d) In vitro screening experiment-HTRF method for testing the inhibitory activity of compounds against RET G810R/RET G810S**

Experimental procedure:

1. Preparation of 1x kinase buffer: 5x enzyme buffer was mixed with distilled water at a ratio of 1:4, the final concentration: 5 mM $MgCl_2$; 1 mM dithiothreitol.
2. The test compound (5 mM stock solution) was diluted 5 folds to 1 mM with100% dimethyl sulfoxide; the compound was diluted into 10 concentrations at an equal ratio of 1:3 in a 384-well dilution plate.
3.0.2 μL of the serially diluted compound was added to a 384-well plate using an Echo 550 pipetting system, 2 replicate wells for each concentration, and the final concentration of dimethyl sulfoxide was 0.5% (v/v). The gradient concentrations of the test compound were 5000, 1666.7, 555.5, 185.18, 61.72, 20.57, 6.858, 2.286, 0.764, and 0.254 nM.
4. 2x RET G810R/RET G810S (0.1 ng/μl) was prepared using the 1x kinase buffer.
5. 5 μL of 2x RET G810R/RET G810S was added to a 384-well plate, and then centrifuged at 1000g for 30 s, and incubated at room temperature for 10 min.
6. A mixed solution of 2x tyrosine kinase-biotinylated substrate (2 μM) and adenosine triphosphate (20 μM) was prepared using the 1x kinase buffer.
7. 5 μL of the mixed solution of tyrosine kinase-biotinylated substrate and adenosine triphosphate was added to start the reaction. The reaction solution was centrifuged at 1000g for 30 s, and then sealed, and incubated at room temperature for 40 min.
8. 4x Sa-XL 665 (a reagent) (125 μM) was prepared using a homogeneous time-resolved fluorescence immunoassay buffer.
9. A mixed solution of 5 μL of Sa-XL 665 and 5 μL of tyrosine kinase-antibody-cryptate was added to each well, and then centrifuged at 1000g for 30 s, and incubated at room temperature for 1 h.
10. The plate was read at 615 nm and 665 nm on an Envision 2104 microplate reader and the ratio was calculated (665/615 nm).
11. The % inhibition rate was calculated as follows:

$$\text{inhibition rate\%} = [1 - \frac{R1-R2}{R0-R2}] \times 100\%$$

wherein

R0 refers to the average ratio of the microplate reader plate of the vehicle blank group.
R1 refers to the ratio of the test compound on the microplate reader plate.
R2 refers to the average ratio on the microplate reader plate in the case of 100% inhibitory activity against RET.
$IC_{50}$ was calculated with software GraphPad 6.0 by fitting inhibition values and logarithms of compound concentrations to a nonlinear regression (dose response - variable slope).

The activity of the compounds is summarized in Table 1 below.

Table 1

| Compound | Wild-type RET kinase (nM) | **Ba/F3-KIF5B-RET** cell activity (nM) | VEGFR2 kinase (nM) | RET G810S kinase (nM) | RET G810R kinase (nM) |
|---|---|---|---|---|---|
| LOXO-292 | 0.22 | 7.08 | 86.5 | 11.25 | 64.76 |

(continued)

| Compound | Wild-type RET kinase (nM) | **Ba/F3-KIF5B-RET** cell activity (nM) | VEGFR2 kinase (nM) | RET G810S kinase (nM) | RET G810R kinase (nM) |
|---|---|---|---|---|---|
| 1 | 3.1 | 271.2 | / | / | / |
| 2 | >10000 | / | / | / | / |
| 3 | >10000 | / | / | / | / |
| 4 | 6594 | / | / | / | / |
| 5 | >10000 | / | / | / | / |
| 6 | 678 | / | / | / | / |
| 7 | 12.74 | / | 201.5 | / | / |
| 8 | 249.6 | / | 5000 | / | / |
| 9 | >10000 | / | / | / | / |
| 10 | >10000 | / | / | / | / |
| 11 | >10000 | / | / | / | / |
| 12 | >10000 | / | / | / | / |
| 13 | 0.80 | 223.9 | / | / | / |
| 14 | 1.6 | 259.8 | / | / | / |
| 15 | 1.4 | 225.1 | / | / | / |
| 16 | 1.1 | 203.5 | / | / | / |
| 17 | 0.32 | 61.1 | / | / | / |
| 18 | 0.50 | 123.2 | / | / | / |
| 19 | 0.77 | 182.6 | / | / | / |
| 20 | 2.63 | 19.1 | / | / | / |
| 21 | 0.72 | 191.7 | / | / | / |
| 22 | 1.24 | 151.3 | / | / | / |
| 23 | 1.35 | 1125.1 | / | / | / |
| 24 | 2.19 | 21.3 | / | / | / |
| 25 | 2.89 | 27.7 | / | / | / |
| 26 | 12.73 | 64.2 | / | / | / |
| 27 | 15.65 | 51.7 |  | / | / |
| 28 | 1.3 | 12.6 |  | / | / |
| 29 | 0.181 | 4.43 | 56.0 | 1.93 | 23.8 |
| 30 | 3.98 | 63.4 | / | / | / |
| 31 | 1.40 | 5.1 | / | / | / |
| 32 | 4.14 | 83.3 | / | / | / |
| 33 | 5.73 | 26.0 | / | / | / |
| 34 | 2.04 | 13.8 | / | / | / |
| 35 | 3.08 | 81.5 | / | / | / |
| 36 | 2.44 | 32.1 | 453.7 | / | / |
| 37 | 5.70 |  | 800.9 | / | / |

(continued)

| Compound | Wild-type RET kinase (nM) | **Ba/F3-KIF5B-RET** cell activity (nM) | VEGFR2 kinase (nM) | RET G810S kinase (nM) | RET G810R kinase (nM) |
|---|---|---|---|---|---|
| 38 | 3.22 | 37.3 | 665.9 | / | / |
| 39 | 1.25 | 15.6 | 258.7 | / | / |
| 40 | 0.86 | 207.7 | 112.1 | / | / |
| 41 | 6.2 | 265.6 | / | / | / |
| 42 | 9.2 | / | / | / | / |
| 47 | 0.04 | 1.42 | 33.68 | 1.23 | 8.93 |
| 48 | 2.89 | 104.8 | 875.4 | 13.04 | 63.17 |
| 49 | 0.19 | 1.52 | 28.41 | 0.53 | 1.85 |
| 50 | 0.13 | 5.75 | 59.31 | 1.35 | 5.65 |
| 51 | 0.22 | / | 19.28 | / | / |
| 52 | 2.02 | 141.8 | 39.02 | / | / |
| 53 | 5.54 | / | / | / | / |
| 54 | 2.76 | / | / | / | / |
| 55 | 0.49 | 6.77 | 8.48 | / | / |
| 56 | 0.20 | 8.53 | 102.8 | 4.73 | 19.30 |
| 57 | 0.32 | 1.24 | 31.30 | 2.33 | 10.61 |
| 58 | 10.39 | / | / | / | / |
| 59 | 1.69 | 11.92 | 93.76 | / | / |
| 60 | 29.60 | / | / | / | / |
| 61 | 1.1 | 60.4 | 482.1 | / | / |
| 62 | 0.21 | 1.82 | 19.99 | 0.80 | 6.22 |
| 63 | 0.47 | 3.39 | 65.16 | 14.97 | 76.85 |
| 64 | 4.83 | / | / | / | / |
| 65 | 6.05 | / | / | / | / |
| 66 | 0.82 | 6.21 | 317.9 | 26.65 | 173.3 |
| 67 | 0.15 | 2.19 | 45.38 | 11.02 | 90.90 |
| 68 | 0.37 | 4.19 | 59.80 | 6.18 | 49.07 |
| 69 | 0.47 | 4.14 | 42.24 | 27.02 | 42.39 |
| 70 | 0.24 | 1.97 | 57.55 | 10.41 | 31.40 |
| Note: "/" means no test. | | | | | |

From the results, it can be seen that: the compounds designed in this patent application all have good RET kinase activity, and some of them (such as compounds 29, 47, 48, 49, 50, 56, 57, 62, 63, 66, 67, 68, 69, and 70) also show good inhibitory activity against G810S and G810R, two mutant strains of RET kinase; moreover, the compounds also have good selectivity for VEGFR2 kinase. In addition, the median inhibitory activities ($IC_{50}$) of compounds 29 and 52 against RET gene fusion CCDC6-RET and KIF5B-RET, RET kinase mutants RET V804M, RET M804L and RET M918T are all less than 5 nM. In addition, the compounds all have good activities against RET kinase and RET-sensitive cells.

## Conclusion

1. It can be seen from the above biological activity data: compared with the compound (LOXO-292) with known activity, the compounds of this disclosure where the 6-position of pyrazolo[1,5-a]pyridine (

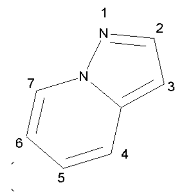

) is an amino group, especially the compounds with an alkylamino or a fluoroalkylamino at the 6-position, have excellent inhibitory activity against G810-mutated RET.
2. When the 6-position is an alkylamino or a fluoroalkylamino, by comparing compounds 47/57/62/67/68/69 having different numbers of carbon atoms, it can be seen that, when the alkyl is straight and has 1 to 3 carbon atoms, the compounds have relatively good RET inhibitory activity against the G810-mutated RET, especially when the 6-position is an ethyl, the strongest inhibitory activity is achieved.
3. When the 6-position is an ethylamino, comparing the inhibitory activity of the compounds 49/50/56/62 unsubstituted and substituted with one or more fluorine atoms against mutated RET, it can be seen that compound 49 having the ethyl substituted with one fluorine atom has better inhibitory activity against G810-mutated RET.

All documents mentioned herein are incorporated by reference in this application as if each document were individually incorporated by reference. In addition, it should be understood that after reading the above teaching content of this disclosure, those skilled in the art can make various changes or modifications to this disclosure, and these equivalent forms also fall within the scope defined by the appended claims of this application.

## Claims

1. A compound of formula I or a pharmaceutically acceptable salt, hydrate, solvate, isotopic compound or prodrug thereof;

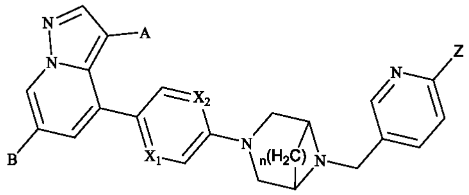

I

wherein

A is selected from: -CN, $-COOR_1$, $-CONR_2R_3$, $-NHCOR_4$ and $-NHCONR_2R_3$;
B is selected from: $R_7$, $-O-(L_1)_{m1}-R_8$, $-OCOR_9$, $-NR_{10}R_{11}$, $-COOR_{12}$, $-CONR_{10}R_{11}$, - $(L_2)_{m2}-R_{12}$, $-(L_2)_{m2}-NR_{10}R_{11}$; wherein each $L_1$ is independently selected from: $-CR_fR_g-$, - CO-, and -CO-NH-; each $L_2$ is independently selected from: $-CR_fR_g-$, -CO-, and -O-CO-;
$X_1$ and $X_2$ are each independently CR or N, wherein R is selected from substituted or unsubstituted groups, said group is selected from C1-C6 alkyl, H, halogen or cyano, wherein the term "substituted" means "substituted with one or more groups selected from C1-C6 alkyl, halogen, cyano, hydroxyl, and amino";
Z is selected from substituted or unsubstituted groups, said group is selected from C1-C6 alkyl, C1-C6 alkoxy, C1-C6 haloalkyl, C3-C8 cycloalkyl, C3-C8 cycloalkoxy, C1-C6 haloalkoxy, C1-C6 alkylamino, and C1-C6 alkylthio, wherein the term "substituted" means "substituted with one or more groups selected from: C1-C6 alkyl,

halogen, cyano, hydroxyl, and amino";

$R_1$, $R_2$, $R_3$ and $R_4$ are each independently selected from substituted or unsubstituted groups, said group is selected from H, C1-C6 alkyl, C3-C8 cycloalkyl, 3- to 8-membered cycloheteroalkyl, C2-C6 alkenyl, C5-C10 aryl, and 5- to 10-membered heteroaryl; or $R_2$ and $R_3$ together with N atom attached thereto constitute a substituted or unsubstituted 3 - to 12-membered heterocyclic group, wherein the term "substituted" means "substituted with one or more groups selected from C1-C6 alkyl, halogen, cyano, hydroxyl, and amino";

$R_7$ is a substituted or unsubstituted 5- to 10-membered heteroaryl, wherein the term "substituted" means "substituted with one or more groups selected from C1-C6 alkyl, halogen, cyano, hydroxyl, and amino";

$R_8$ is selected from substituted or unsubstituted groups, said group is selected from - C1-C6 alkyl-E, C5-C12 fused bicyclic ring, 5- to 12-membered fused heterobicyclic ring, C5-C12 spirobicyclic ring or 5- to 12-membered spiro heterobicyclic ring, wherein E is selected from: -CN, $-COOR_1$, $-OCOR_1$, $-CONR_2R_3$, $-NHCOR_4$, and $-NHCONR_2R_3$, wherein the term "substituted" means "substituted with 1, 2, 3, or 4 groups selected from: H, oxo (=O), halogen, cyano, hydroxy, C1-C6 alkyl, C3-C8 cycloalkyl, C1-C6 haloalkyl, C1-C6 alkoxy, C3-C8 cycloalkoxy, C1-C6 haloalkoxy, C1-C6 alkylamino, and C1-C6 alkylthio";

$R_9$ is selected from substituted or unsubstituted groups, said group is selected from C1-C6 alkoxy, C1-C6 haloalkoxy, $-NR_{10}R_{11}$, $-(L_3)_{m3}$-(3- to 8-membered cycloheteroalkyl), - $(L_3)_{m3}$-(C5-C10 aryl), and $-(L_3)_{m3}$-(5- to 10-membered heteroaryl), wherein each $L_3$ is independently selected from: $-CR_fR_g-$ and $-NR_h-$, wherein the term "substituted" means "substituted with one or more groups selected from: C1-C6 alkyl, halogen, cyano, hydroxyl, and amino";

$R_{10}$ and $R_{11}$ are each independently selected from substituted or unsubstituted groups, said group is selected from H, C1-C6 alkyl, $-(L_2)_{m2}-NQ_1Q_2$, C3-C8 cycloalkyl, 3- to 8-membered cycloheteroalkyl, C6-C10 aryl, and 5- to 10-membered heteroaryl; alternatively, $R_{10}$ and $R_{11}$ together with N atom attached thereto constitute a 3- to 12-membered substituted or unsubstituted heterocyclyl containing 1 to 3 N atoms and 0, 1 or 2 O or S atoms, wherein $Q_1$ and $Q_2$ are each independently selected from: H and substituted or unsubstituted C1-C6 alkyl, or $Q_1$ and $Q_2$ together with N atom attached thereto constitute a 3- to 10-membered substituted or unsubstituted heterocyclyl containing 1 to 3 N atoms and 0, 1 or 2 O or S atoms, wherein the term "substituted" means "substituted with one or more groups selected from: C1-C6 alkyl, cyano, halogen, hydroxyl, C3-C8 cycloalkyl, 3- to 8-membered cycloheteroalkyl, C6-C10 aryl, and 5- to 10-membered heteroaryl";

each $R_{12}$ is independently selected from substituted or unsubstituted groups, said group is selected from C1-C6 alkyl, C3-C8 cycloalkyl, 3- to 8-membered cycloheteroalkyl, C6-C10 aryl, and 5- to 10-membered heteroaryl, the term "substituted" means "substituted with one or more groups selected from: C1-C6 alkyl, cyano, halogen, hydroxy, C3-C8 cycloalkyl, 3- to 8-membered cycloheteroalkyl, C6-C10 aryl, and 5- to 10-membered heteroaryl";

$R_f$ and $R_g$ are each independently selected from: H, halogen, C1-C4 alkyl, C1-C4 haloalkyl, OH, $NH_2$, and C3-C6 cycloalkyl;

$R_h$ is independently selected from: H, C1-C4 alkyl, C1-C4 haloalkyl, and C3-C6 cycloalkyl;

n is 0, 1, or 2;

$m_1$ is 1, 2, 3, 4, 5 or 6;

$m_2$ is 1, 2, 3, 4, 5 or 6;

$m_3$ is 0, 1, or 2;

with the limitation that when B is $R_7$, A is selected from: $-COOR_1$, $-CONR_2R_3$, - $NHCOR_4$, and $-NHCONR_2R_3$.

2. The compound or the pharmaceutically acceptable salt, hydrate, solvate, isotopic compound or prodrug thereof according to claim 1, wherein A is selected from:-CN, - $COOR_1$, $-CONR_2R_3$, $-NHCOR_4$ and $-NHCONR_2R_3$, wherein $R_1$, $R_2$ and $R_3$ are each independently selected from: H, C1-C6 alkyl, C3-C8 cycloalkyl, 3- to 8-membered cycloheteroalkyl, C2-C6 alkenyl, C5-C10 aryl, and 5- to 10-membered heteroaryl; $R_4$ is selected from substituted or unsubstituted groups, said group is selected from C1-C3 alkyl, C3-C6 cycloalkyl, 3- to 6-membered cycloheteroalkyl, C2-C4 alkenyl, phenyl, pyrazolyl, pyridinyl, furyl, thiophenyl, oxazolyl, isoxazolyl, and triazolyl, wherein the term "substituted" means "substituted with C1-C3 alkyl".

3. The compound or the pharmaceutically acceptable salt, hydrate, solvate, isotopic compound or prodrug thereof according to claim 1, wherein B is selected from: $R_7$, $-O-(L_1)_{m1}-R_8$, $-NR_{10}R_{11}$, $-CONR_{10}R_{11}$, $-(L_2)_{m2}-R_{12}$, and $-(L_2)_{m2}-NR_{10}R_{11}$;

with the limitation that when B is $R_7$, A is selected from: $-COOR_1$, $-CONR_2R_3$, - $NHCOR_4$, and $-NHCONR_2R_3$; wherein $R_1$, $R_2$, $R_3$, $R_4$, $R_7$, $L_1$, $L_2$, $m_1$, $m_2$, $R_8$, $R_{10}$, $R_{11}$ and $R_{12}$ are defined as in claim 1.

4. The compound or the pharmaceutically acceptable salt, hydrate, solvate, isotopic compound or prodrug thereof according to claim 1, wherein $-(L_1)_{m1}-$ is selected from: - $(CH_2)_2$-, -CO-, and -CO-NH-.

**5.** The compound or the pharmaceutically acceptable salt, hydrate, solvate, isotopic compound or prodrug thereof according to claim 1, wherein $R_8$ is selected from substituted or unsubstituted groups, said group is selected from 5- to 12-membered fused heterobicyclic ring and 5- to 12-membered spiro heterobicyclic ring, wherein the heterobicyclic ring contains 1 to 3 N atoms and 0, 1 or 2 O or S atoms as ring atoms, and a N atom in the heterobicyclic ring is attached to the $L_1$ moiety, wherein, the term "substituted" means "substituted with 1, 2, 3 or 4 groups selected from: H, oxo (=O), halogen, cyano, hydroxy, C1-C6 alkyl, C3-C8 cycloalkyl, C1-C6 haloalkyl, C1-C6 alkoxy, C3-C8 cycloalkoxy, C1-C6 haloalkoxy, C1-C6 alkylamino, and C1-C6 alkylthio".

**6.** The compound or the pharmaceutically acceptable salt, hydrate, solvate, isotopic compound or prodrug thereof according to claim 1, wherein $R_{10}$ and $R_{11}$ are each independently selected from substituted or unsubstituted groups, said group is selected from H, C1-C3 alkyl, $-(CH_2)_2-NQ_1Q_2$, C3-C6 cycloalkyl, 3- to 6-membered cycloheteroalkyl, phenyl, pyrazolyl, pyridinyl, furyl, thiophenyl, oxazolyl, isoxazolyl, and triazolyl; alternatively, $R_{10}$ and $R_{11}$ together with N atom attached thereto constitute a 3- to 8-membered substituted or unsubstituted heterocyclyl containing 1 to 3 N atoms and 0, 1 or 2 O or S atoms, wherein $Q_1$ and $Q_2$ are each independently selected from: H and C1-C3 alkyl, or $Q_1$ and $Q_2$ together with N atom attached thereto constitute a 3- to 10-membered substituted or unsubstituted heterocyclyl containing 1 to 3 N atoms and 0, 1 or 2 O or S atoms, wherein the term "substituted" means "substituted with one or more groups selected from C1-C3 alkyl, cyano, halogen, and hydroxyl".

**7.** The compound or the pharmaceutically acceptable salt, hydrate, solvate, isotopic compound or prodrug thereof according to claim 1, wherein the compound has a structure of formula II:

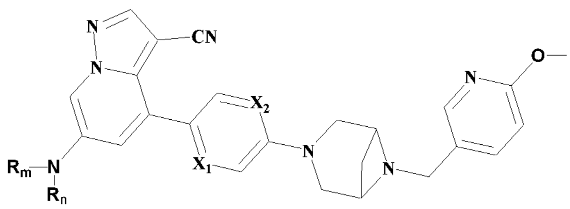

II

wherein

$R_m$ and $R_n$ are each independently selected from substituted or unsubstituted groups, said group is selected from H and C1-C3 alkyl, wherein the term "substituted" means "substituted with 1 to 2 halogen atoms";
$X_1$ and $X_2$ are each independently CH or N.

**8.** The compound or the pharmaceutically acceptable salt, hydrate, solvate, isotopic compound or prodrug thereof according to claim 7, wherein $R_m$ and $R_n$ are each independently selected from: H, methyl, ethyl, n-propyl, $-CH_2F$, $-CHF_2$, $-CH_2CH_2F$, $-CH_2CHF_2$, $-CHFCH_3$, $-CHFCH_2F$, $-CH_2CH_2CH_2F$, and -CH2CHFCH2F.

**9.** The compound or the pharmaceutically acceptable salt, hydrate, solvate, isotopic compound or prodrug thereof according to claim 1, wherein the compound has a structure of formula III:

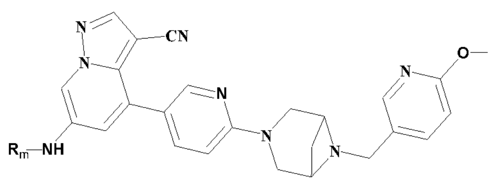

IIi

$R_m$ is selected from: H, methyl, ethyl, n-propyl, $-CH_2F$, $-CHF_2$, $-CH_2CH_2F$, $-CH_2CHF_2$, $-CHFCH_3$, $-CHFCH_2F$, $-CH_2CH_2CH_2F$, and -CH2CHFCH2F.

**10.** The compound or the pharmaceutically acceptable salt, hydrate, solvate, isotopic compound or prodrug thereof according to claim 1, wherein the compound has a structure of formula IV:

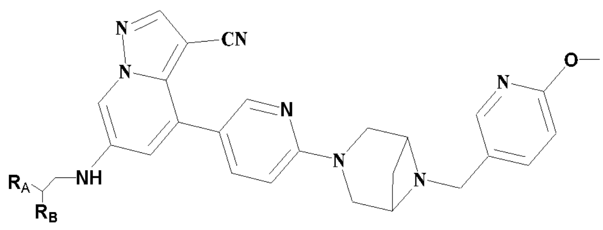

IV

wherein
$R_A$ and $R_B$ are each independently selected from: H, F, and methyl.

**11.** The compound or the pharmaceutically acceptable salt, hydrate, solvate, isotopic compound or prodrug thereof according to claim 1, wherein the compound is selected from:

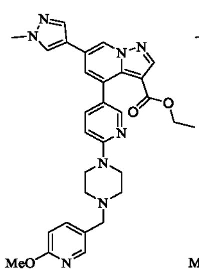
1
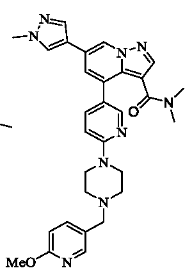
2
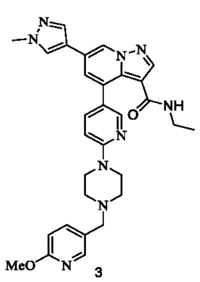
3
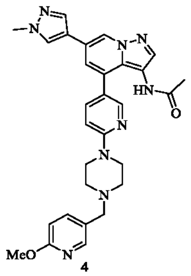
4
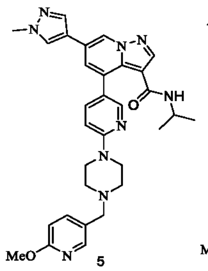
5
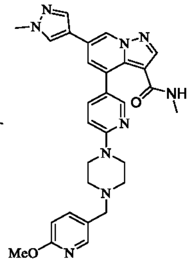
6

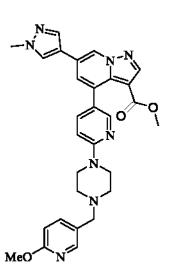
7
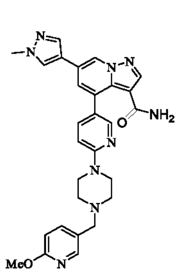
8
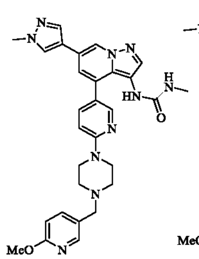
9
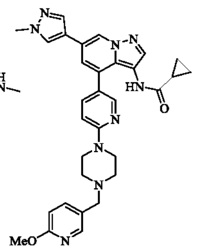
10
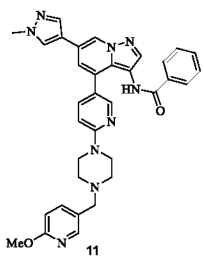
11
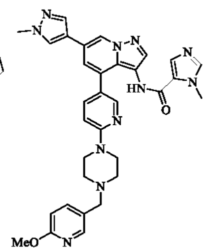
12

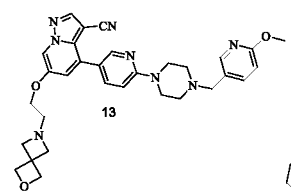
13
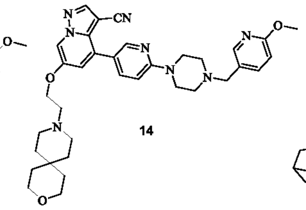
14
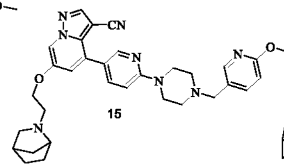
15
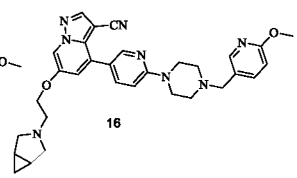
16

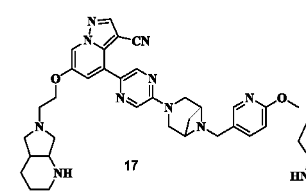
17
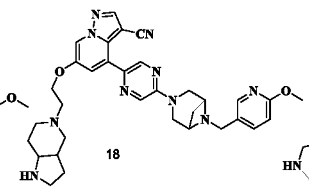
18
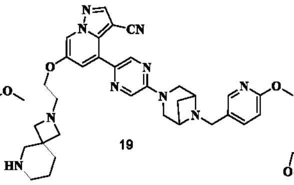
19
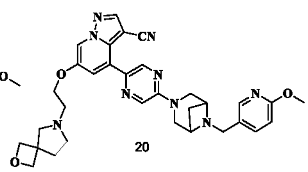
20

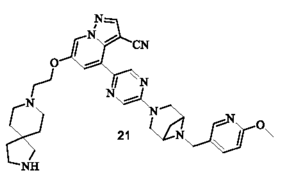
21

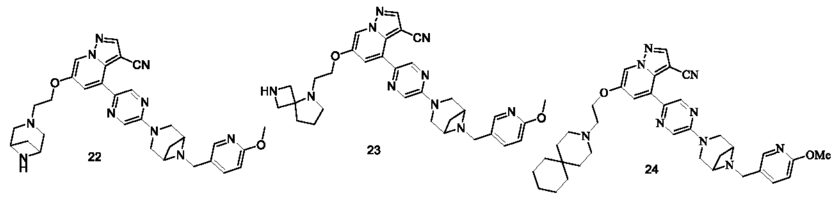
22 23 24

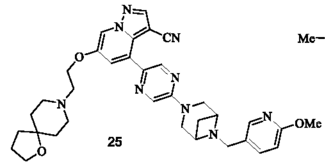
25

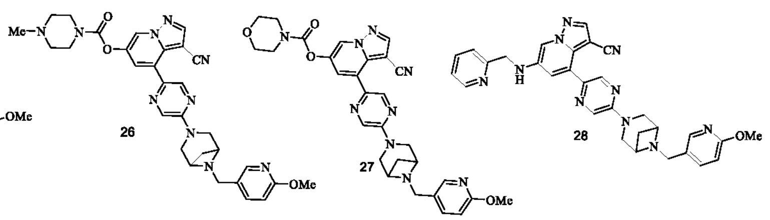
26 27 28

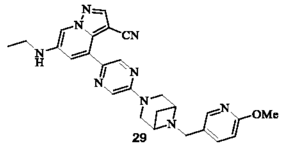
29

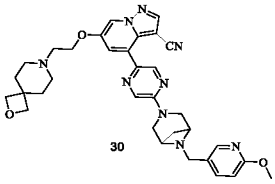
30

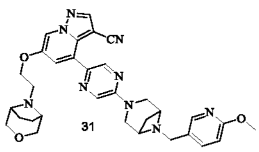
31

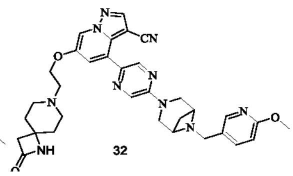
32

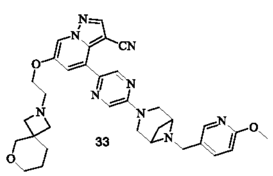
33

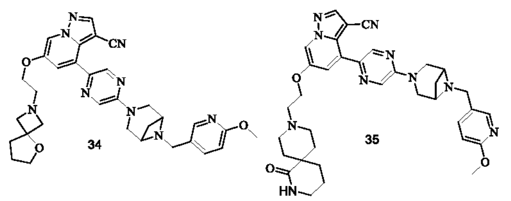
34 35

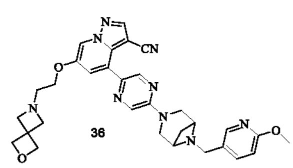
36

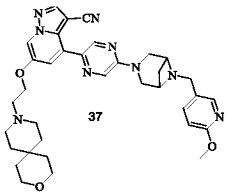
37

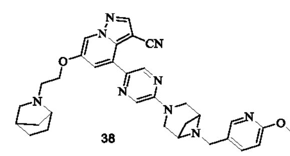
38

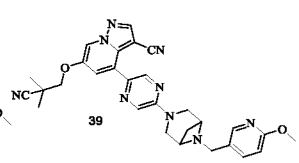
39

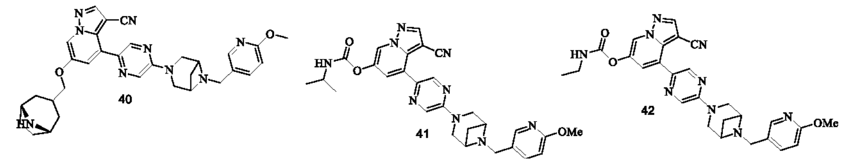
40 41 42

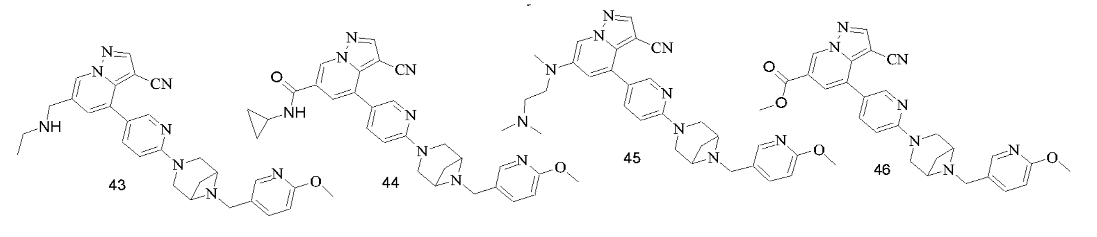
43 44 45 46

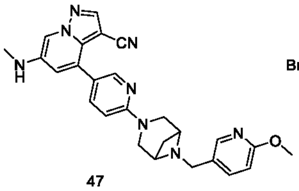 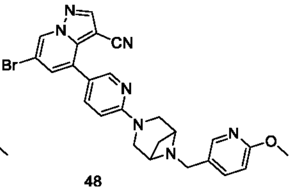 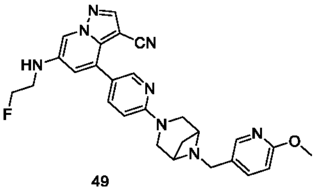 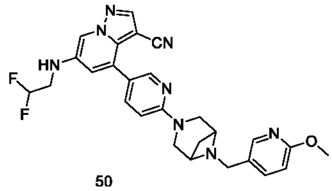

47 48 49 50

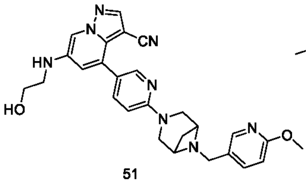 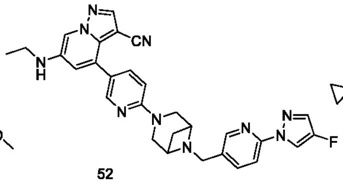 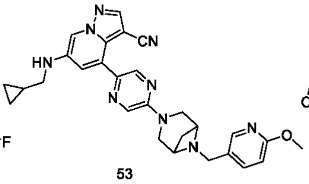 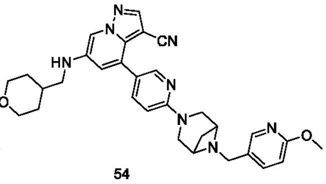

51 52 53 54

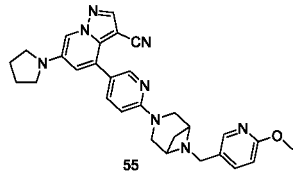 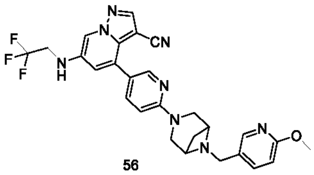 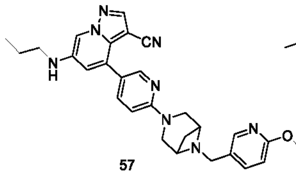 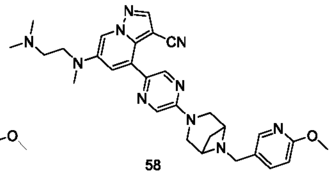

55 56 57 58

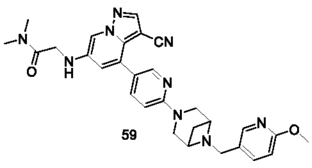 59 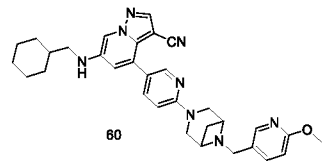 60 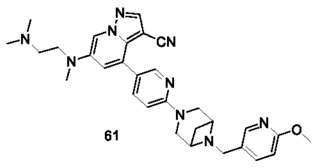 61 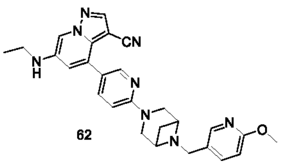 62

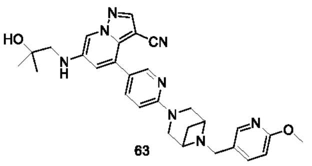 63 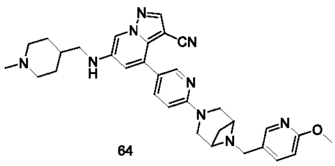 64 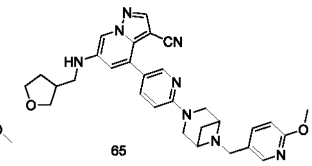 65 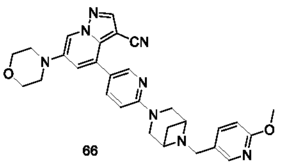 66

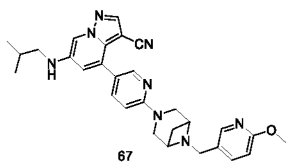 67 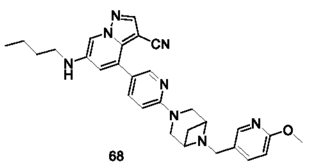 68 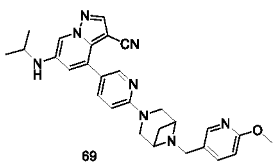 69 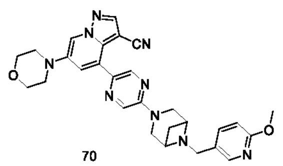 70

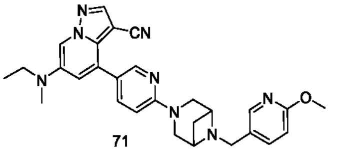 71 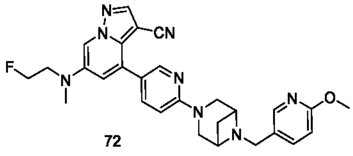 72 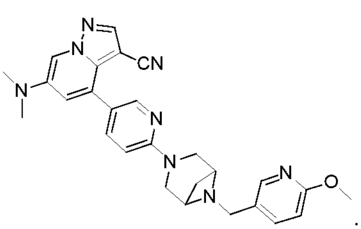.

**12.** The compound or the pharmaceutically acceptable salt, hydrate, solvate, isotopic compound or prodrug thereof according to any one of claims 1 to 11, wherein the pharmaceutically acceptable salt is selected from acetates, adipates, alginates, ascorbates, aspartates, benzoates, besylates, bisulfates, borates, butyrates, citrates, camphorates, camphor sulfonates, cyclopentane propionates, diglycolates, dodecyl sulfates, ethanesulfonates, fumarates, glucoheptonates, glycerophosphates, hemisulfates, heptanoates, hexanoates, hydrochlorides, hydrobromides, hydroiodides, isethionates, lactates, maleates, mesylates, naphthalenesulfonates, nicotinates, nitrates, oxalates, pectates, persulfates, phenylpropionates, phosphates, picrates, pivalates, propionates, salicylates, succinates, sulfates,

sulfonates, tartrates, thiocyanates, tosylates, and dodecanoates.

**13.** A pharmaceutical composition, comprising the compound or the pharmaceutically acceptable salt, hydrate, solvate, isotopic compound or prodrug thereof according to claim 1; and a pharmaceutically acceptable carrier or diluent.

**14.** Use of the compound according to any one of claims 1 to 12 or the pharmaceutical composition according to claim 13 in preparation of a drug for inhibiting activity of a RET kinase in a cell or a subject.

**15.** The use according to claim 14, wherein the drug is used to treat a RET-related disease or a disorder in the expression or activity or level of RET genes, RET kinases, or any one of the RET genes and the RET kinases.

## INTERNATIONAL SEARCH REPORT

International application No.
**PCT/CN2020/135934**

**A. CLASSIFICATION OF SUBJECT MATTER**

C07D 471/04(2006.01)i; A61K 31/437(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNTXT, WPI, CNABS, CNKI, CAPLUS, REGISTRY: 吡唑并吡啶, 哌啶, RET激酶, ret, pyridine, RET INHIBITOR, PYRAZOLO, pyrazolopyridine, structure search

**C. DOCUMENTS CONSIDERED TO BE RELEVANT**

| Category* | Citation of document, with indication, where appropriate, of the relevant passages | Relevant to claim No. |
|---|---|---|
| X | CN 110382494 A (ARRAY BIOPHARMA, INC.) 25 October 2019 (2019-10-25)<br>description, paragraphs 0042-0721, embodiments 1-507, 543-560 | 1-15 |
| PX | WO 2020200316 A1 (MEDSHINE DISCOVERY INC.) 08 October 2020 (2020-10-08)<br>description page 2 paragraph 3 to page 27 paragraph 3, page 76 embodiment 9, page 96 embodiment 30, page 125 embodiment 63 | 1-15 |
| PA | CN 111285873 A (GUANGDONG HEC PHARMACEUTICAL) 16 June 2020 (2020-06-16)<br>description paragraph 0006 to paragraph 0085, paragraph 0277 to paragraph 0292 | 1-15 |
| A | US 2019106438 A1 (LOXO ONCOLOGY INC. et al.) 11 April 2019 (2019-04-11)<br>entire document | 1-15 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 February 2021** | **10 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/135934**

| Patent document cited in search report | Publication date (day/month/year) | Patent family member(s) | Publication date (day/month/year) |
|---|---|---|---|
| CN 110382494 A | 25 October 2019 | EC SP19032676 A | 31 July 2019 |
| | | CO 2019004650 A2 | 21 May 2019 |
| | | IL 265916 D0 | 30 June 2019 |
| | | RS 60536 B1 | 31 August 2020 |
| | | CA 3039760 A1 | 19 April 2018 |
| | | BR 112019007144 B1 | 14 July 2020 |
| | | MX 2019004205 A | 21 August 2019 |
| | | PT 3523301 T | 26 August 2020 |
| | | US 10555944 B2 | 11 February 2020 |
| | | CA 3039760 C | 08 September 2020 |
| | | EP 3523301 A1 | 14 August 2019 |
| | | US 2018134702 A1 | 17 May 2018 |
| | | DK 3523301 T3 | 03 August 2020 |
| | | KR 20190076976 A | 02 July 2019 |
| | | US 10172851 B2 | 08 January 2019 |
| | | US 2018133200 A1 | 17 May 2018 |
| | | JP 6776446 B2 | 28 October 2020 |
| | | SI 3523301 T1 | 31 August 2020 |
| | | KR 102143899 B1 | 13 August 2020 |
| | | DO P2019000090 A | 30 June 2019 |
| | | US 2018133213 A1 | 17 May 2018 |
| | | US 2019183886 A1 | 20 June 2019 |
| | | PH 12019500775 A1 | 01 July 2019 |
| | | CR 20190218 A | 14 October 2019 |
| | | US 10137124 B2 | 27 November 2018 |
| | | LT 3523301 T | 27 July 2020 |
| | | BR 112019007144 A2 | 02 July 2019 |
| | | AU 2017342022 B2 | 05 December 2019 |
| | | TW 201825488 A | 16 July 2018 |
| | | EP 3523301 B1 | 27 May 2020 |
| | | HR P20201008 T1 | 16 October 2020 |
| | | JP 2020503247 A | 30 January 2020 |
| | | US 10112942 B2 | 30 October 2018 |
| | | WO 2018071447 A1 | 19 April 2018 |
| | | PE 20190918 A1 | 26 June 2019 |
| | | AU 2017342022 A1 | 23 May 2019 |
| | | SG 11201903144 P A | 30 May 2019 |
| | | CL 2019000941 A1 | 13 December 2019 |
| WO 2020200316 A1 | 08 October 2020 | None | |
| CN 111285873 A | 16 June 2020 | WO 2020114487 A1 | 11 June 2020 |
| US 2019106438 A1 | 11 April 2019 | US 2020308194 A1 | 01 October 2020 |
| | | UY 37927 A | 30 April 2019 |
| | | AR 113758 A1 | 10 June 2020 |
| | | CN 111263760 A | 09 June 2020 |
| | | EP 3694853 A1 | 19 August 2020 |
| | | CA 3079019 A1 | 18 April 2019 |
| | | TW 201922757 A | 16 June 2019 |
| | | US 10745419 B2 | 18 August 2020 |
| | | WO 2019075092 A1 | 18 April 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ALEXANDER DRILON.** *Nature Reviews Clinical Oncology,* 2018, vol. 15, 151-167
- **ROSELL R ; KARACHALIOU N.** *Lancet Oncol.,* 2016, vol. 17, 1623-1625
- **MULLIGAN LM.** *Nat Rev Cancer.,* 2014, vol. 14, 173-86
- **SOLOMON BJ ; TAN L ; LIN JJ et al.** *J Thorac Oncol.,* April 2020, vol. 15 (4), 541-549
- **SAMBROOK et al.** Molecular Cloning: Laboratory Manual. Cold Spring Harbor Laboratory Press, 1989